# EUROPEAN PATENT SPECIFICATION

(11) **EP 0 726 955 B1**
(45) Date of publication and mention of the grant of the patent: **07.04.2004**
(21) Application number: 95900453.2
(22) Date of filing: 27.10.1994
(51) Int. Cl.: C12N 15/31, C12N 15/62, C07K 14/20, A61K 39/02, G01N 33/50, C07K 16/12

(54) **CHIMERIC PROTEINS COMPRISING BORRELIA POLYPEPTIDES: USES THEREFOR**
CHIMERE PROTEINE, WELCHE BORRELIA POLYPEPTIDE BEINHALTEN, VERWENDUNGEN DAFÜR
PROTEINES CHIMERES COMPRENANT DES POLYPEPTIDES DE BORRELIA ET LEURS UTILISATIONS

(30) Priority: 01.11.1993 US 148191; 29.04.1994 US 235836
(43) Date of publication of application: 21.08.1996
(73) Proprietor: Brookhaven Science Associates, Upton, New York 11973 (US)
(72) Inventor: DUNN, John, J., Bellport, NY 11713 (US); LUFT, Benjamin, J., Port Jefferson, NY 11777 (US)
(74) Representative: Gambell, Derek
(86) International application number: PCT/US1994/012352
(87) International publication number: WO 1995/012676

(56) References cited:
- WO-A-91/13630
- WO-A-93/08306
- WO-A-94/20536
- MOLECULAR MICROBIOLOGY, vol.6, no.20, 1992 pages 3031 - 3040 ROSA P. A. ET AL. 'Recombination between genes encoding major outer surface proteins A and B of Borrelia burgdorferi'
- GINSBERG H. S. ET AL. (EDS.) 'Vaccines 93. Modern approaches to new vaccines including prevention of AIDS. Tenth annual meeting, Cold Spring Harbor, New York, USA, September 1992.' 1993 , COLD SPRING HARBOR LABORATORY PRESS , NEW YORK cited in the application McGrath B. C. et al.: 'Biochemical and biophysical characterization of the major outer surface protein from north american and european isolates of Borrelia burgdorferi' see page 365 - page 370 see page 369, last paragraph
- JOURNAL OF BACTERIOLOGY, vol.175, no.9, May 1993 pages 2516 - 2522 KITTEN T. ET AL. 'Intragenic recombination and a chimeric outer membrane protein in the relapsing fever agent Borrelia hermsii'

## Description

### Background of the Invention

Lyme borreliosis is the most common tick-borne infectious disease in North America, Europe, and northern Asia. The causative bacterial agent of this disease, *Borrelia burgdorferi*, was first isolated and cultivated in 1982 (Burgdorferi, W.A. *et al.,* Science 216: 1317-1319 (1982); Steere, A.R. *et al*., N. Engl. J. Med. 308: 733-740 (1983)). With that discovery, a wide array of clinical syndromes, described in both the European and American literature since the early 20th century, could be attributed to infection by B. burgdorferi (Afzelius, A., Acta Derm. Venereol. 2: 120-125 (1921); Bannwarth, A., Arch. Psychiatr. Nervenkrankh. 117: 161-185 (1944); Garin, C. and A. Bujadouz, J. Med. Lyon 71: 765-767 (1922); Herxheimer, K. and K. Hartmann, Arch. Dermatol. Syphilol. 61: 57-76, 255-300 (1902)).

The immune response to *B. burgdorferi* is characterized by an early, prominent, and persistent humoral response to the end of flagellar protein, p41 (fla), and to a protein constituent of the protoplasmic cylinder, p93 (Szczepanski, A., and J.L. Benach, Microbiol. Rev. 55:21 (1991)). The p41 flagellin antigen is an immunodominant protein; however, it shares significant homology with flagellins of other microorganisms and therefore is highly cross reactive. The p93 antigen is the largest immunodominant antigen of *B*. *burgdorferi*. Both the p41 and p93 proteins are physically cryptic antigens, sheathed from the immune system by an outer membrane whose major protein constituents are the outer surface proteins A and B (OspA and OspB). OspA is a basic lipoprotein of approximately 31 kd, which is encoded on a large linear plasmid along with OspB, a basic lipoprotein of approximately 34 kd (Szczepanski, A., and J.L. Benach, Microbiol. Rev. 55:21 (1991)). Analysis of isolates of *B. burgdorferi* obtained from North America and Europe has demonstrated that OspA has antigenic variability, and that several distinct groups can be serologically and genotypically defined (Wilske, B., *et al*., World J. Microbiol. 7: 130 (1991)). Other *Borrelia* proteins demonstrate similar antigenic variability. Surprisingly, the immune response to these outer surface proteins tends to occur late in the disease, if at all (Craft, J. E. *et al.,* J. Clin Invest. 78: 934-939 (1986); Dattwyler, R.J. and B.J. Luff, Rheum. Clin. North Am. 15: 727-734 (1989)). Furthermore, patients acutely and chronically infected with *B*. *burgdorferi* respond variably to the different antigens, including ospA, OspB, OspC, OspD, p39, p41 and p93.

Vaccines against Lyme borreliosis have been attempted. Mice immunized with a recombinant form of OspA are protected from challenge with the same strain of *B. burgdorferi* from which the protein was obtained (Fikrig, E., *et al.,* Science 250: 553-556 (1990)). Furthermore, passively transferred anti-OspA monoclonal antibodies (Mabs) have been shown to be protective in mice, and vaccination with a recombinant protein induced protective immunity against subsequent infection with the homologous strain of *B.burgdorferi* (Simon, M.M., *et al.,* J. Infect. Dis. 164: 123 (1991)). Unfortunately, immunization with a protein from one strain does not necessarily confer resistance to a heterologous strain (Fikrig, E. *et al.,* J. Immunol. 7: 2256-1160 (1992)), but rather, is limited to the homologous 'species' from which the protein was prepared. Furthermore, immunization with a single protein from a particular strain of *Borrelia* will not confer resistance to that strain in all individuals. There is considerable variation displayed in OspA and OspB, as well as p93, including the regions conferring antigenicity. Therefore, the degree and frequency of protection from vaccination with a protein from a single strain depend upon the response of the immune system to the particular variation, as well as the frequency of genetic variation in *B. burgdorferi.* Currently, a need exists for a vaccine which provides immunogenicity across species and to more epitopes within a species, as well as immunogenicity against more than one protein.

Rosa *et al*. (*Molec. Biol*. 6:3031-3040 (1992)), describe the isolation of clonal *B. burgdorferi* strains where recombination has occurred within the ospA/ospB operon.

### Summary of the Invention

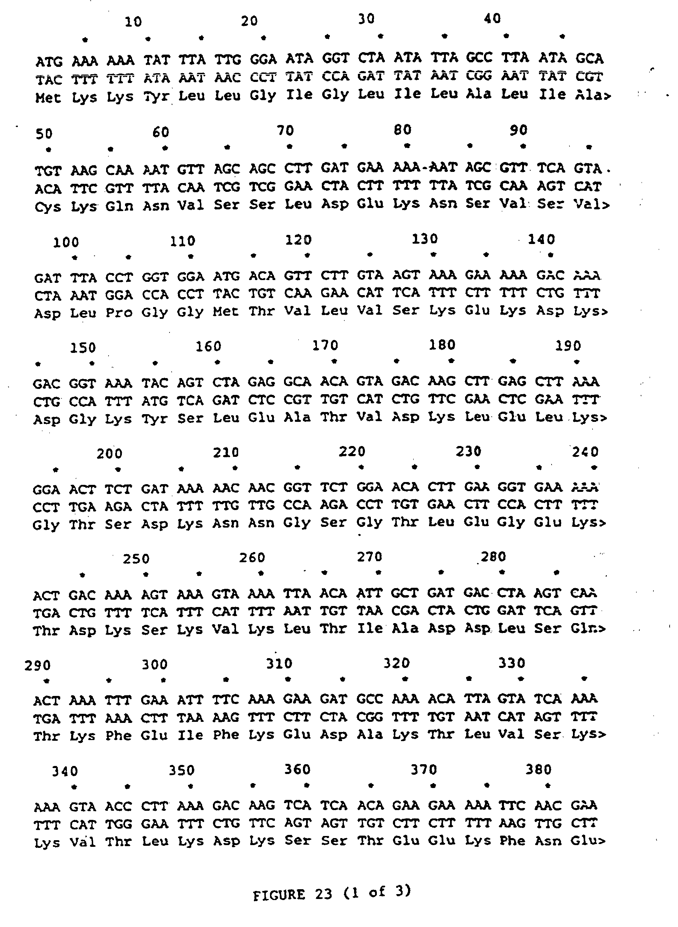

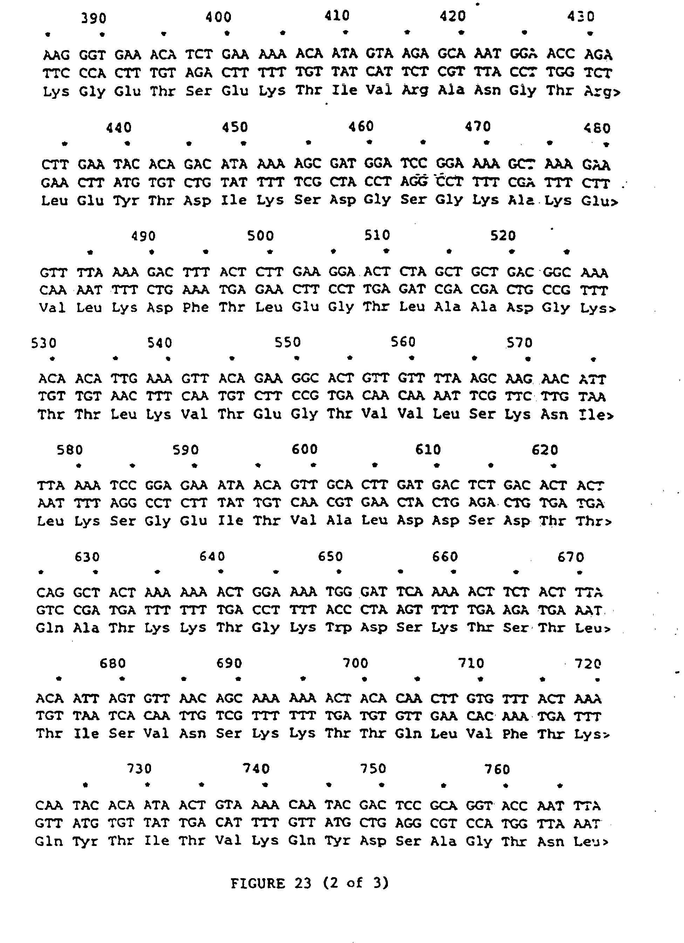

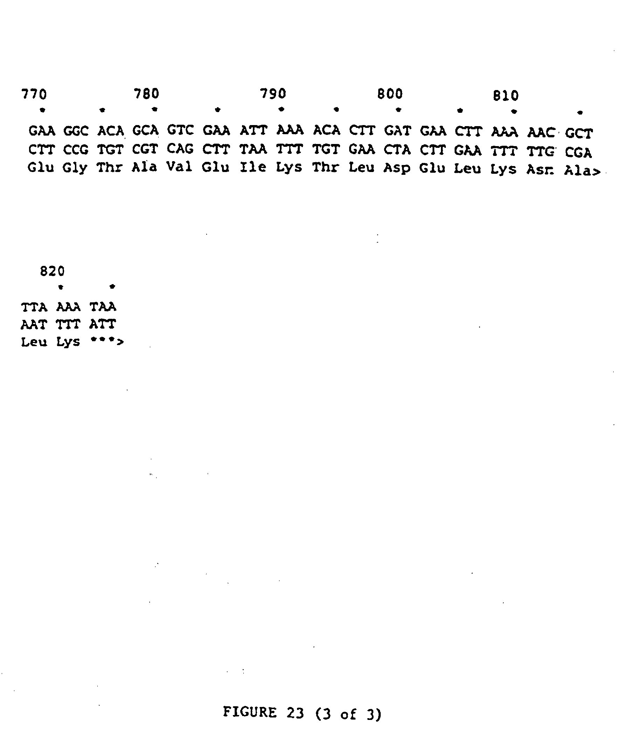

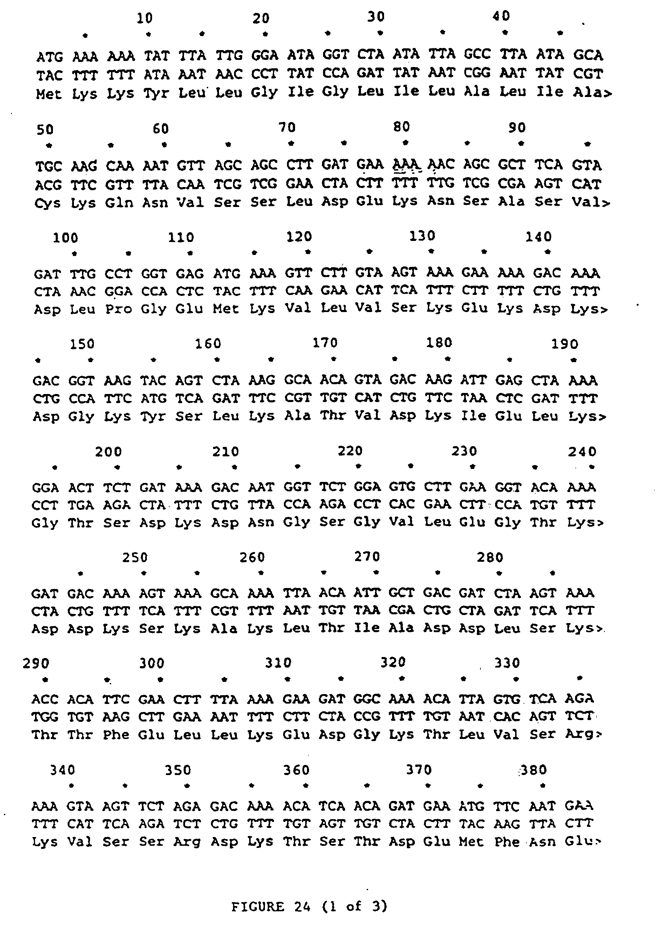

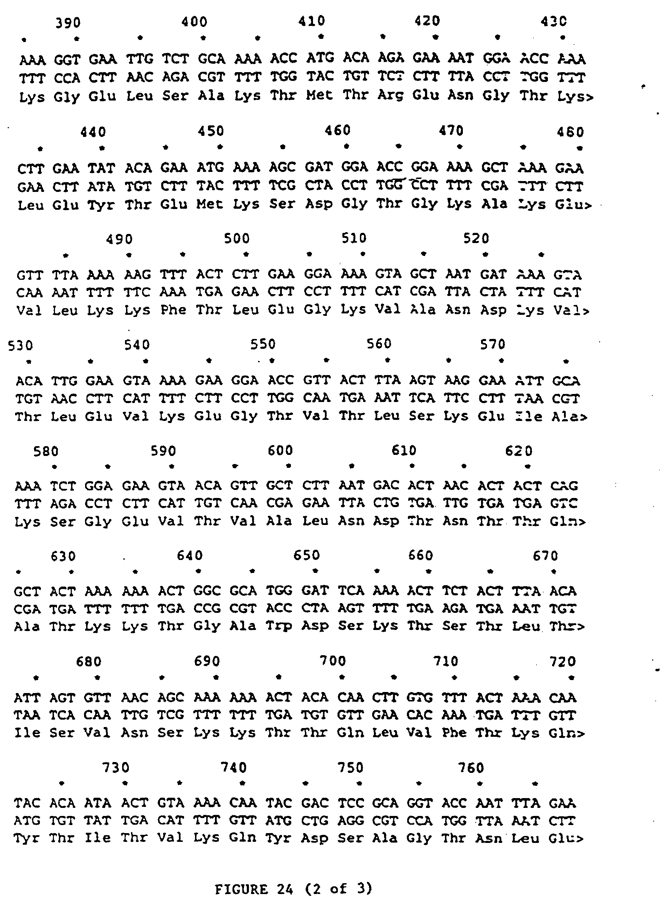

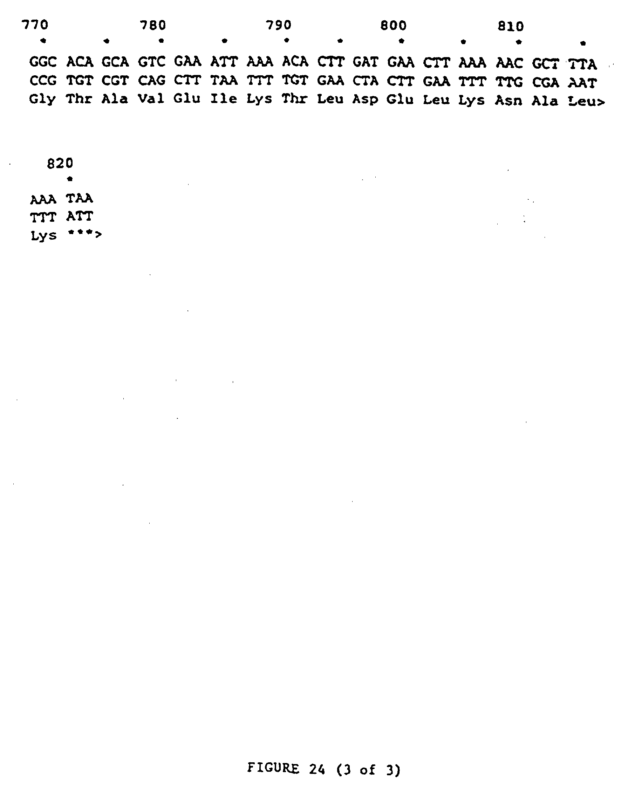

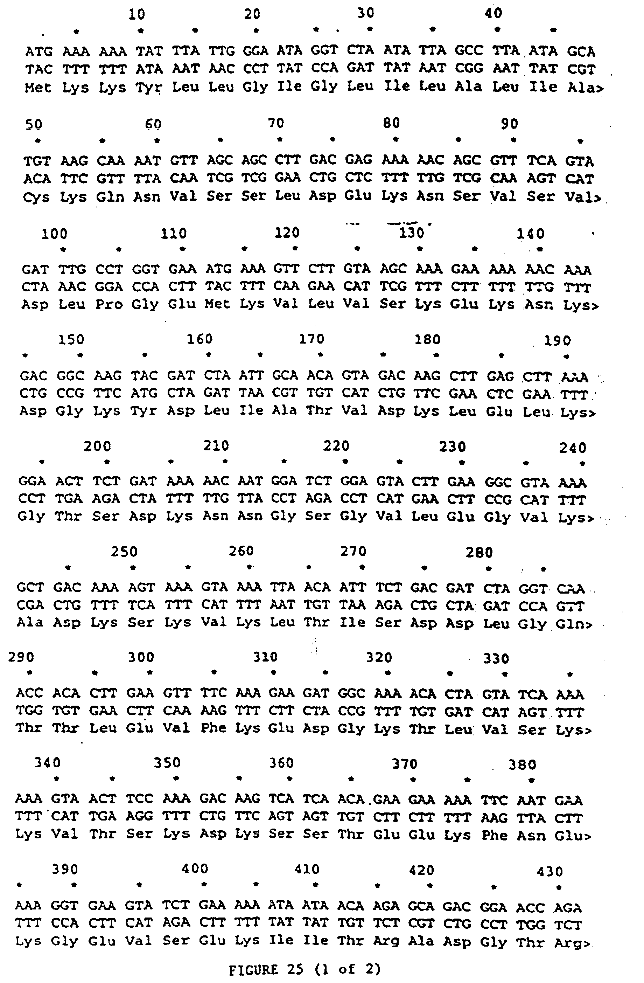

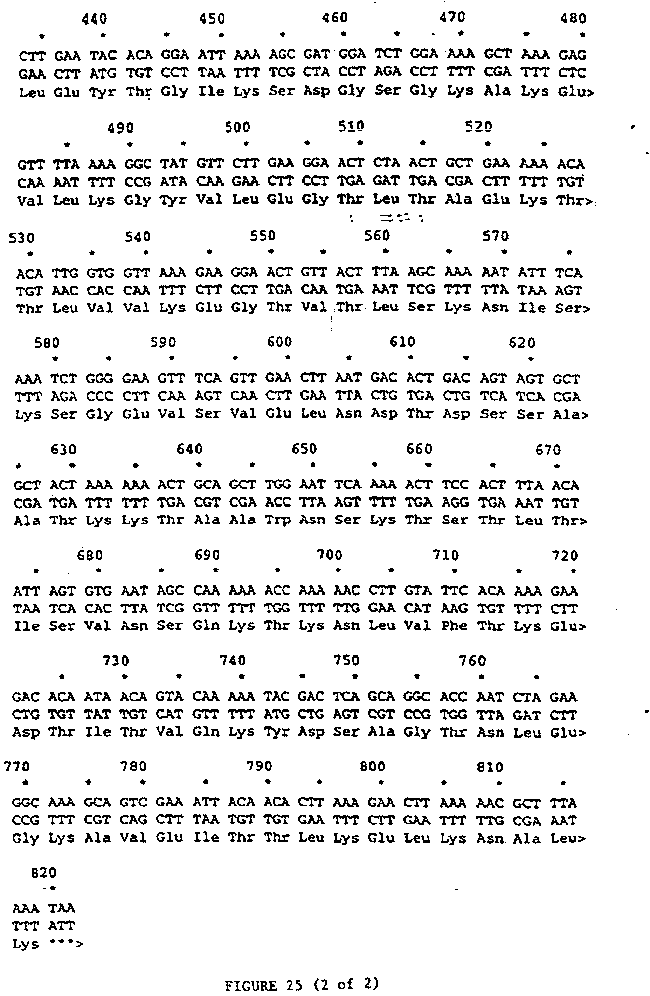

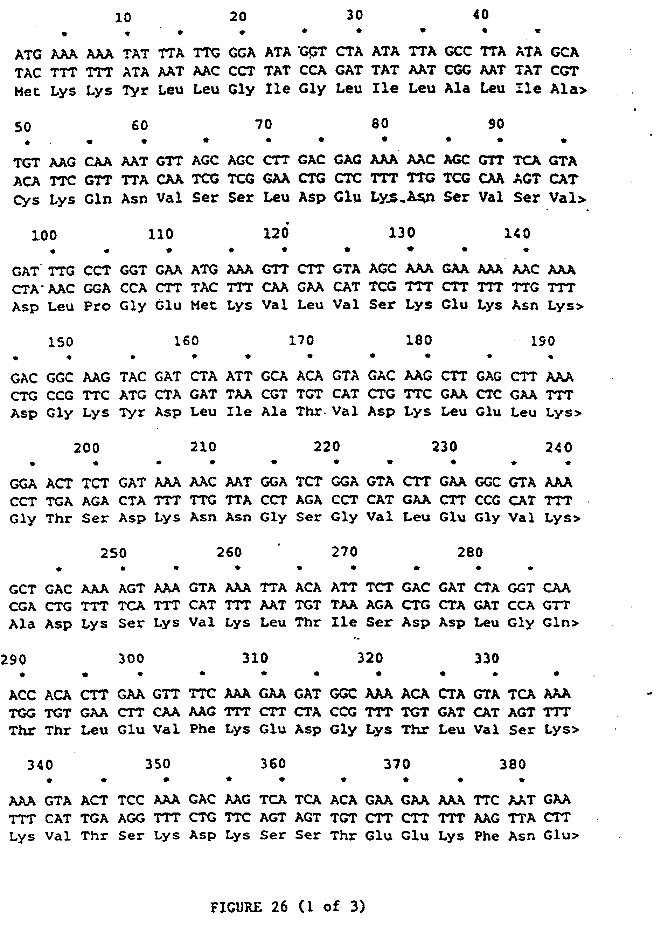

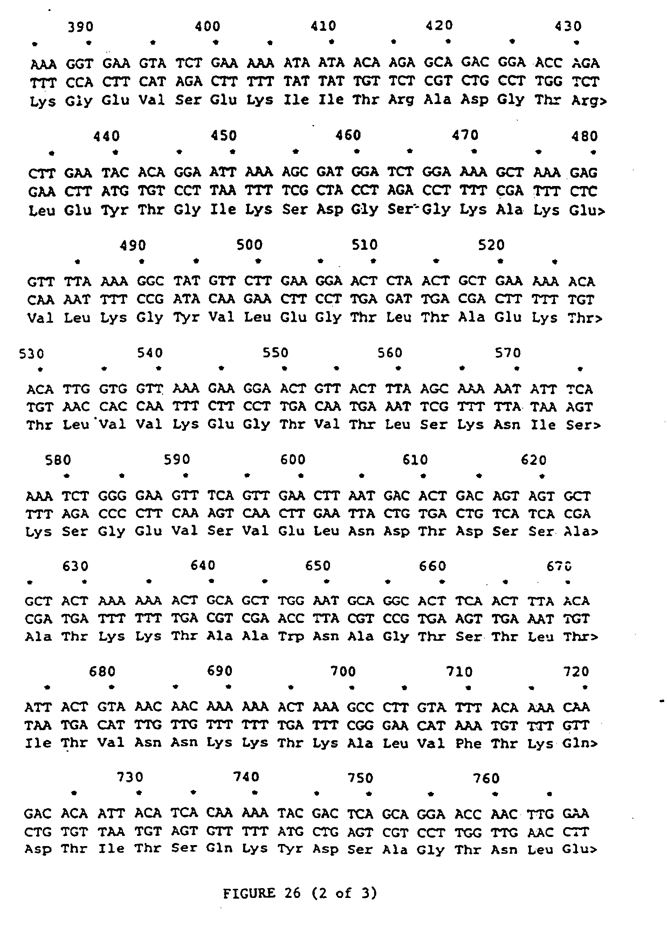

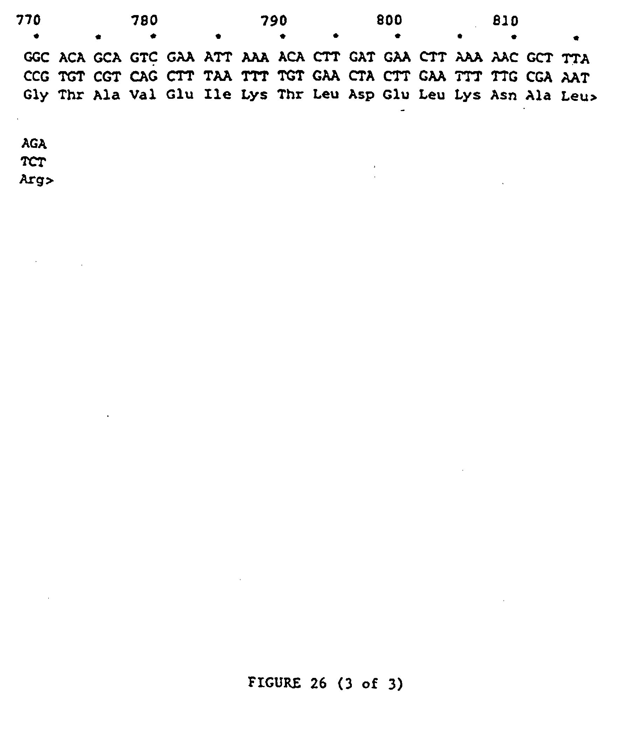

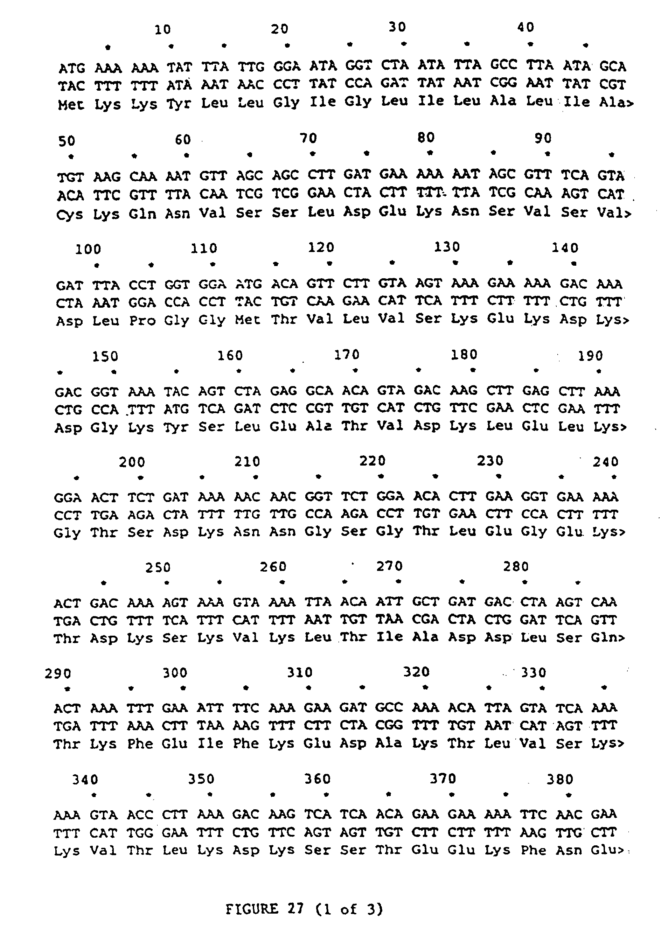

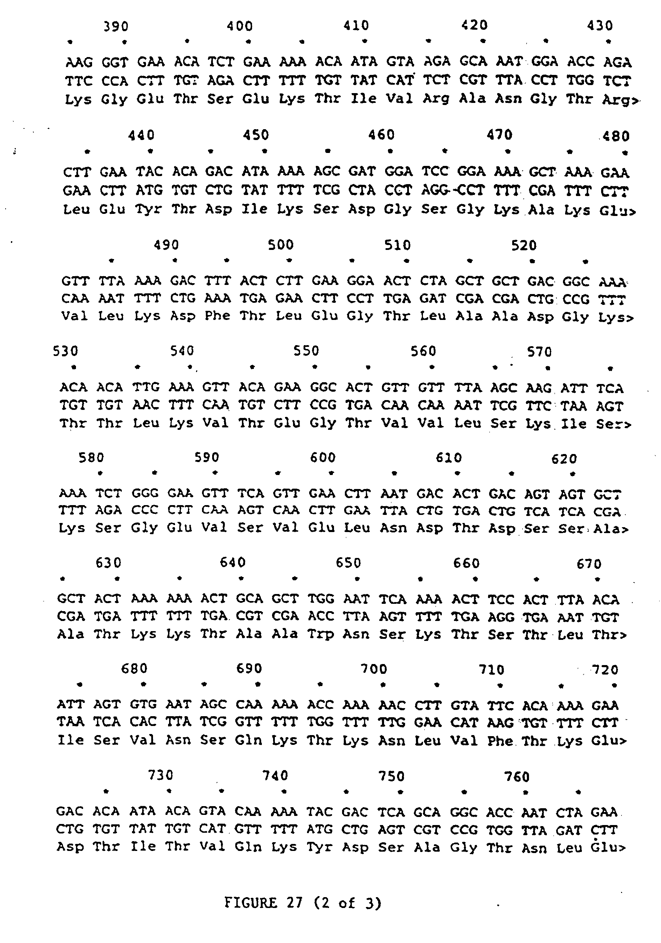

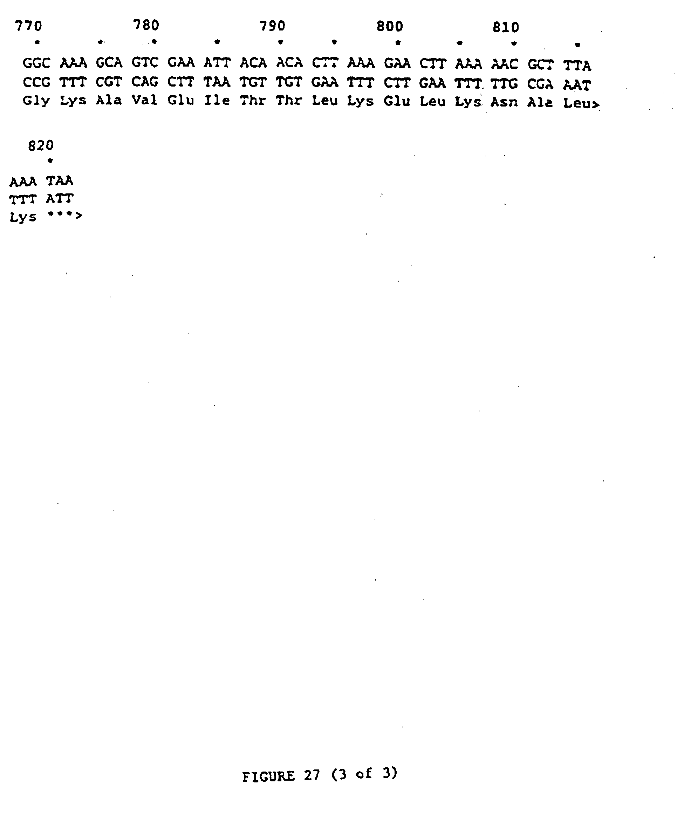

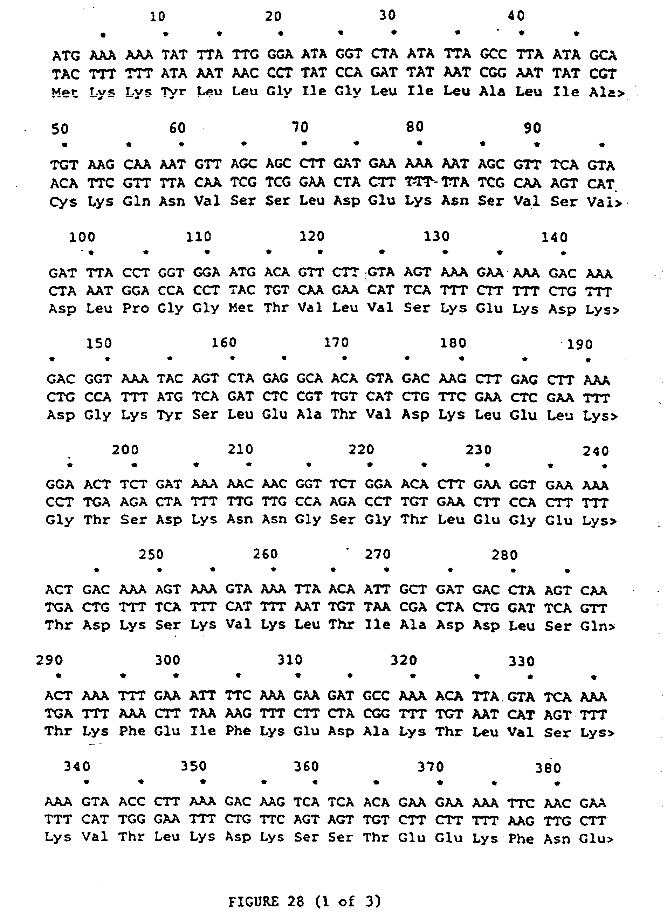

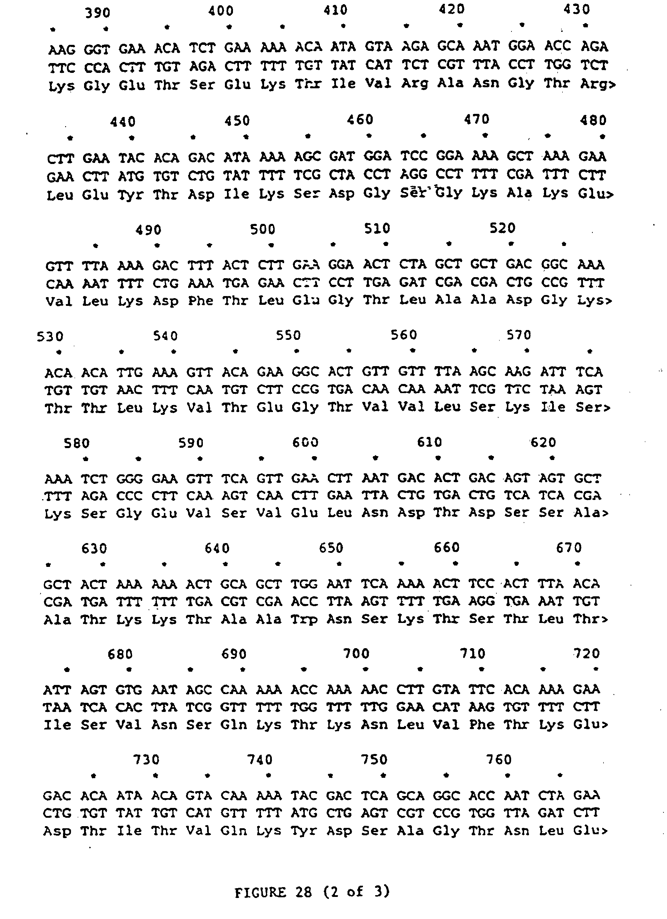

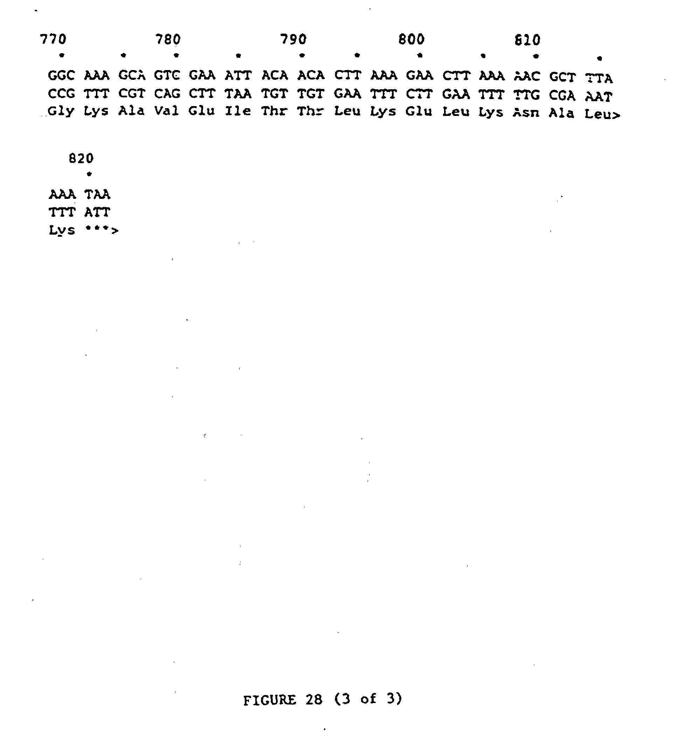

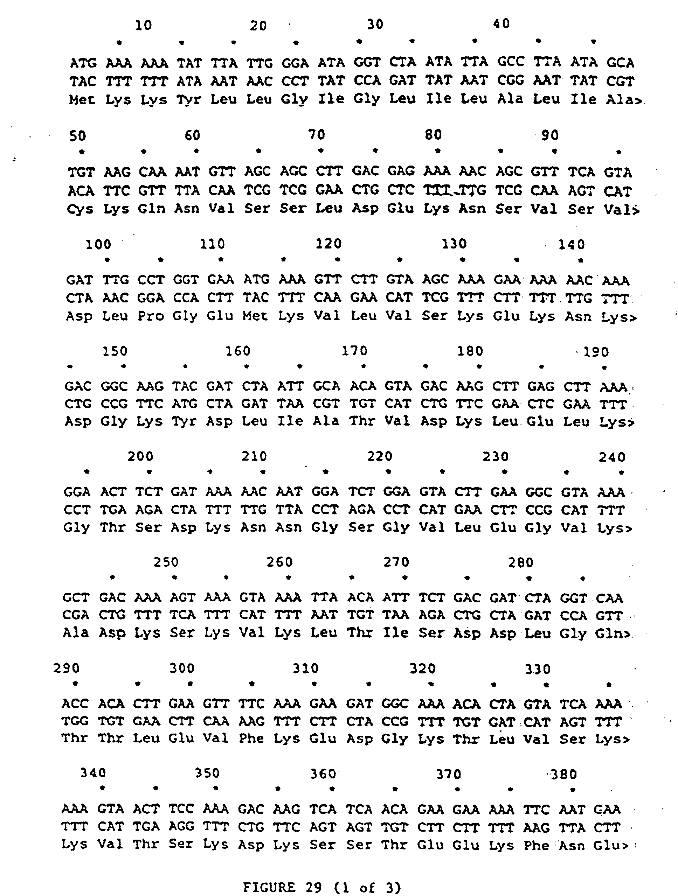

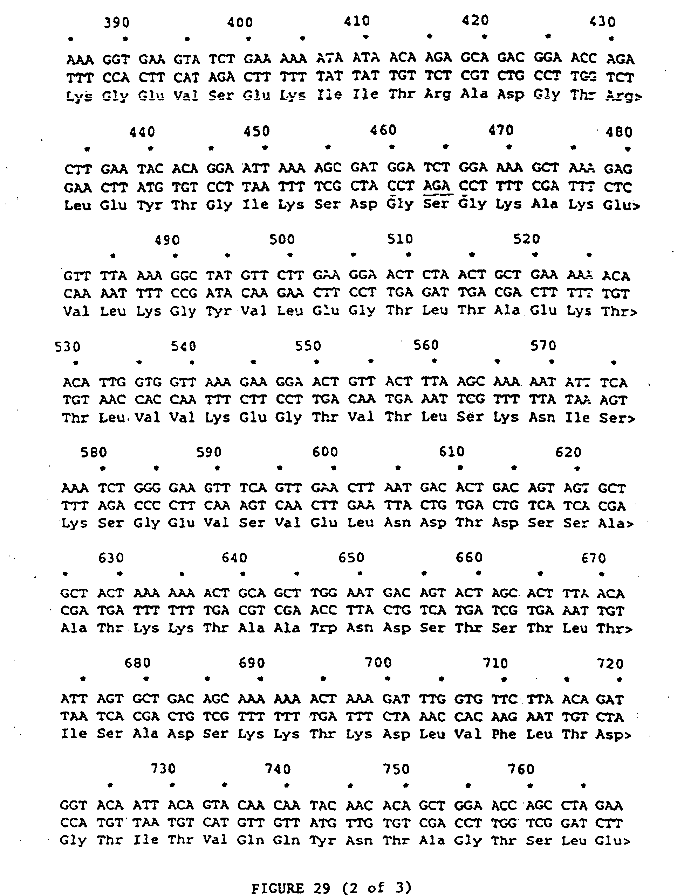

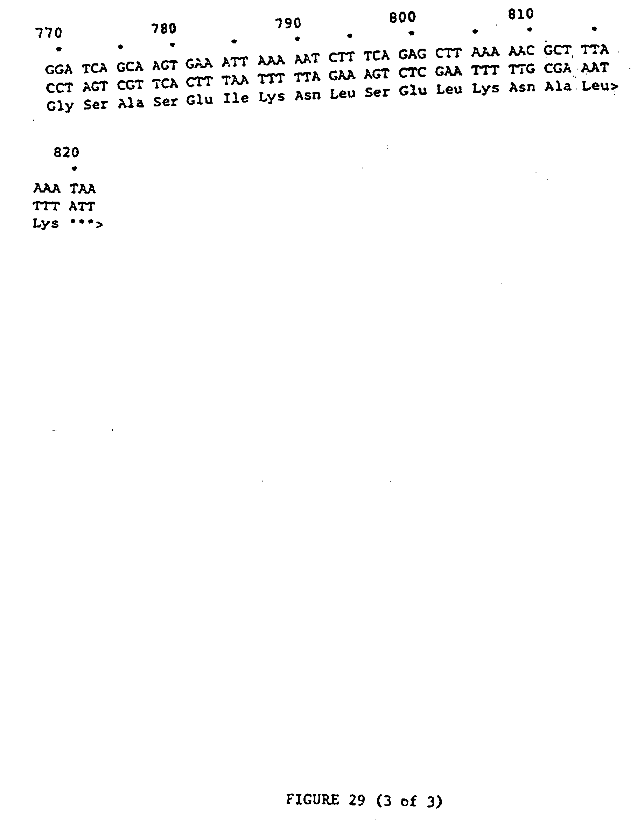

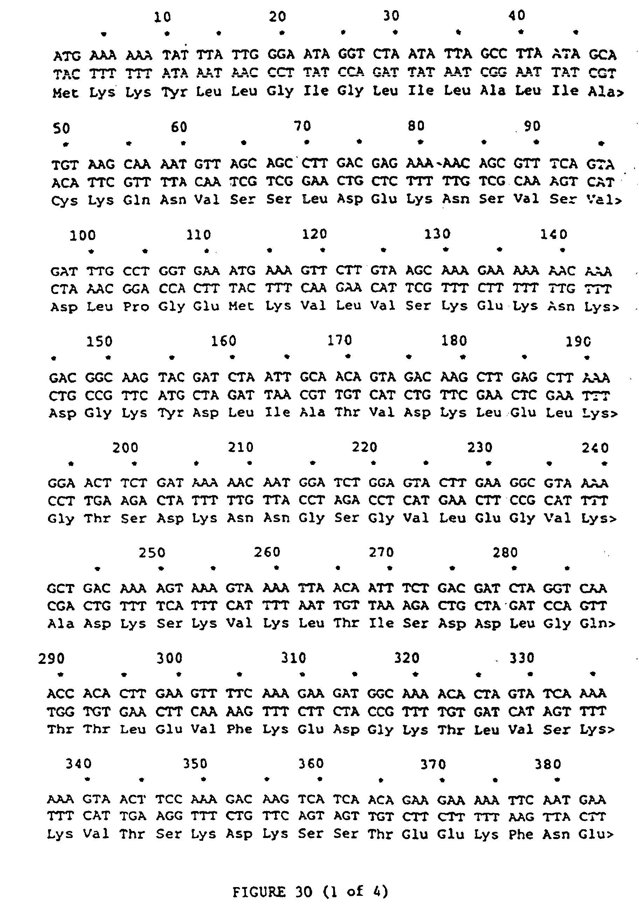

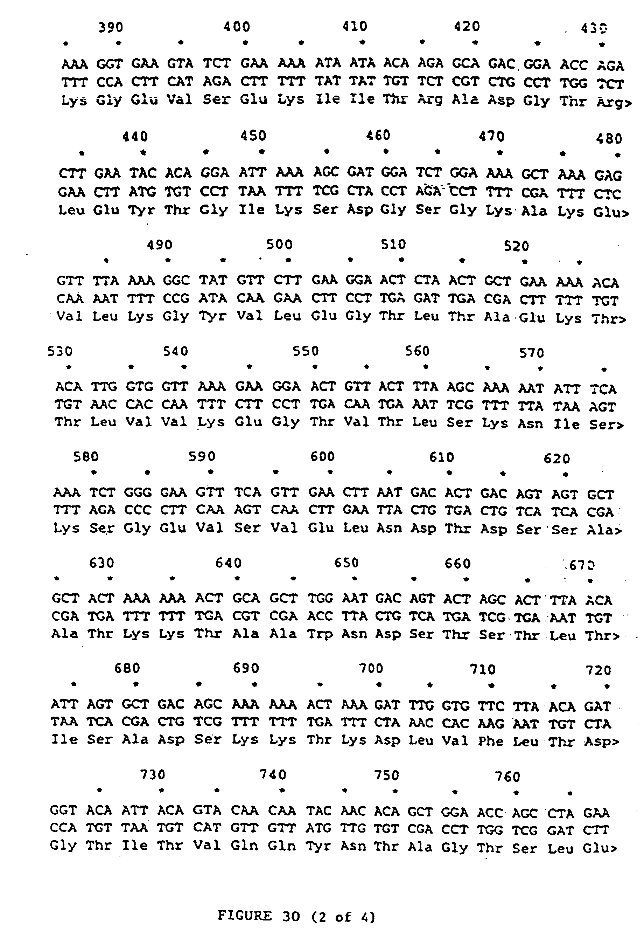

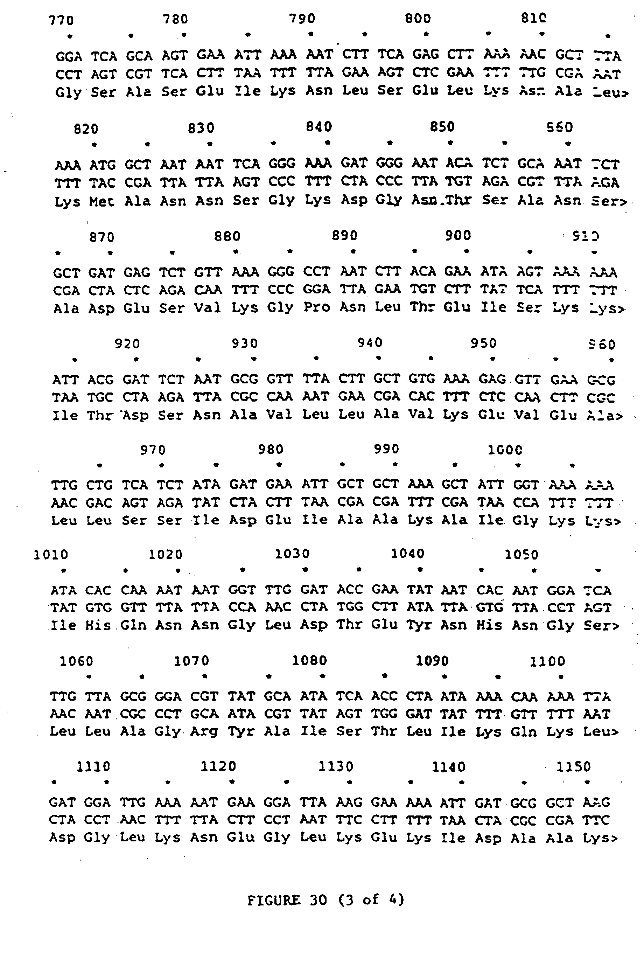

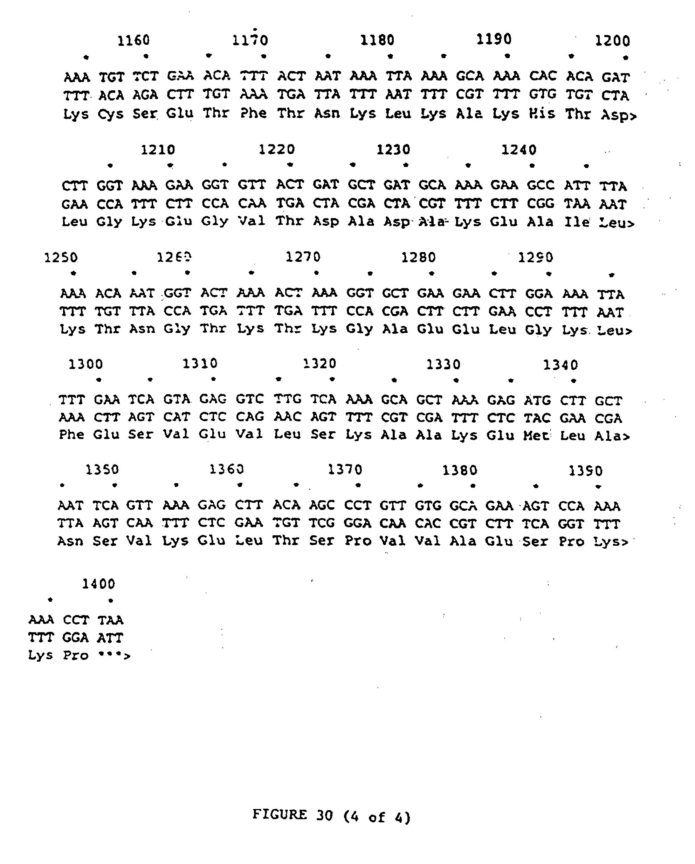

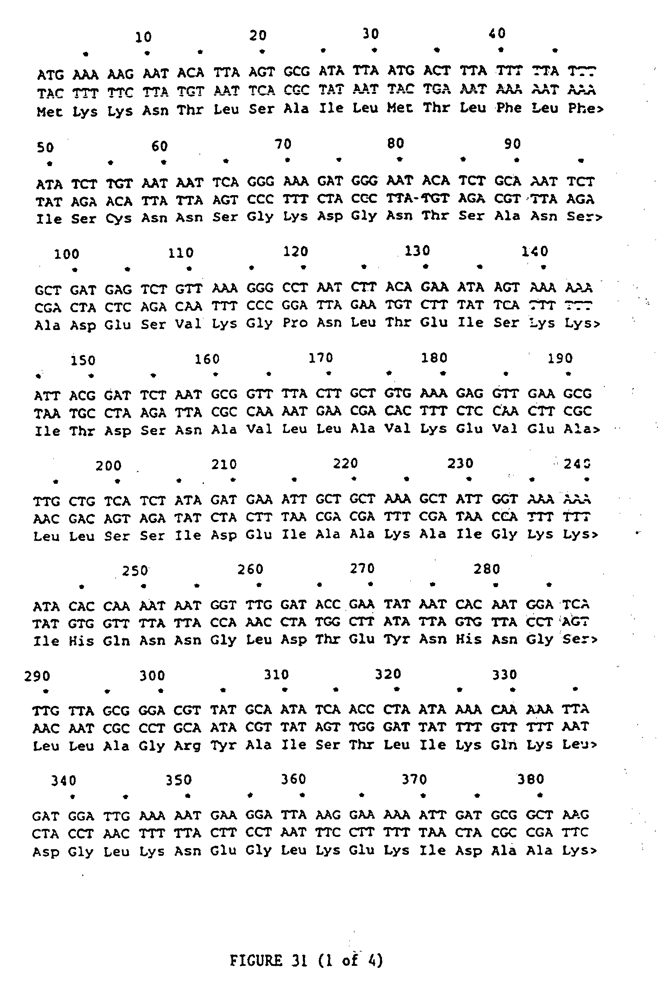

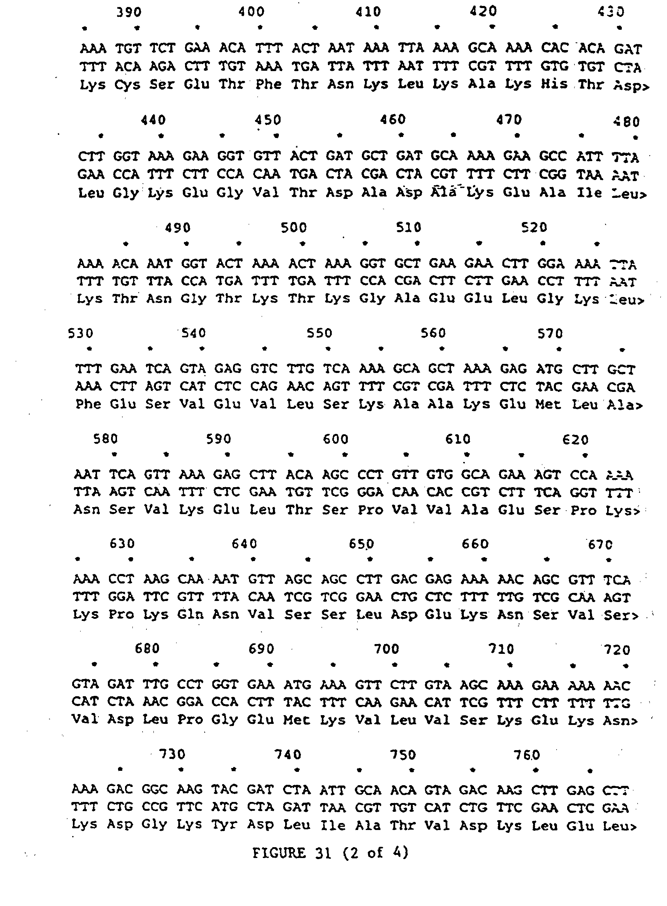

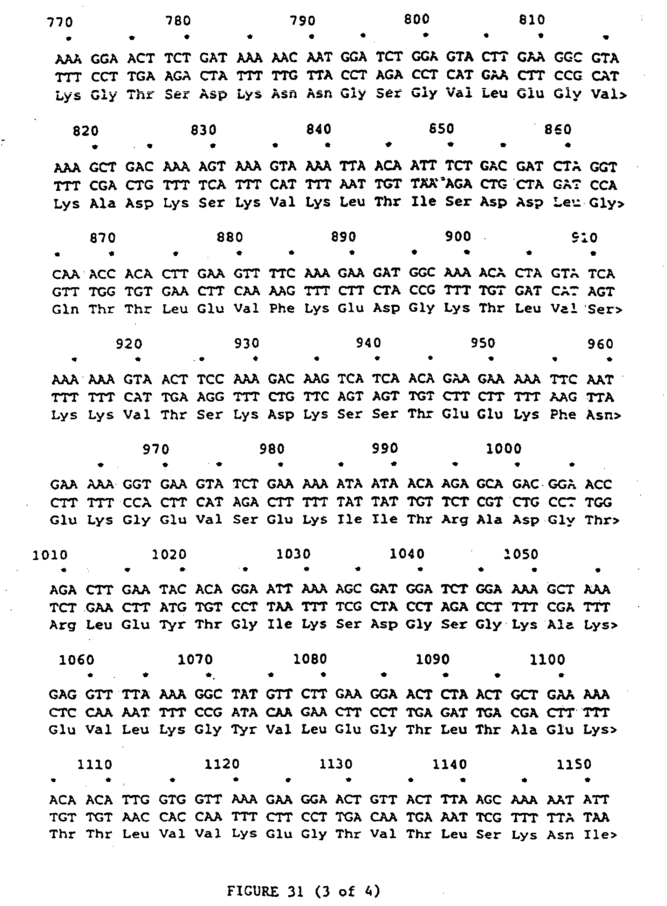

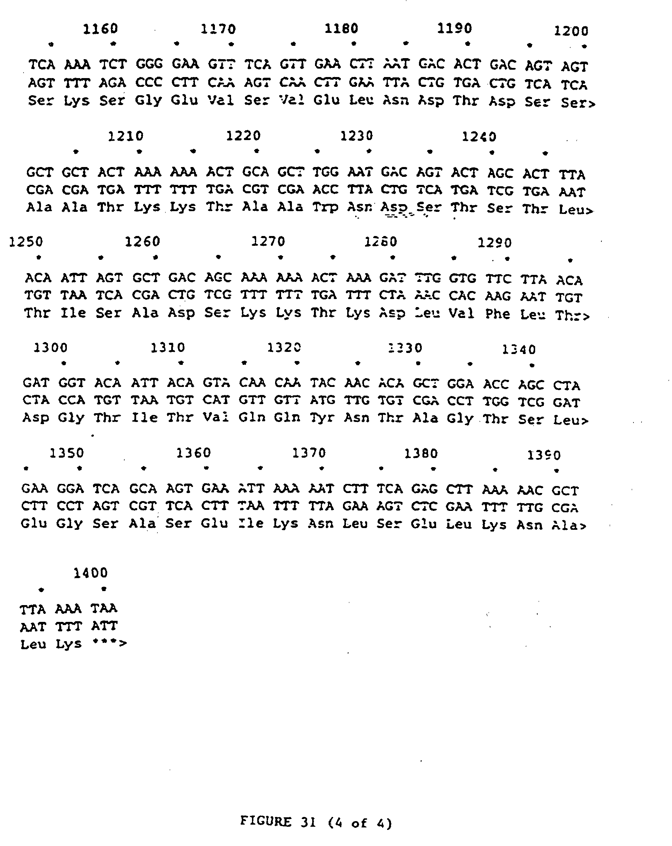

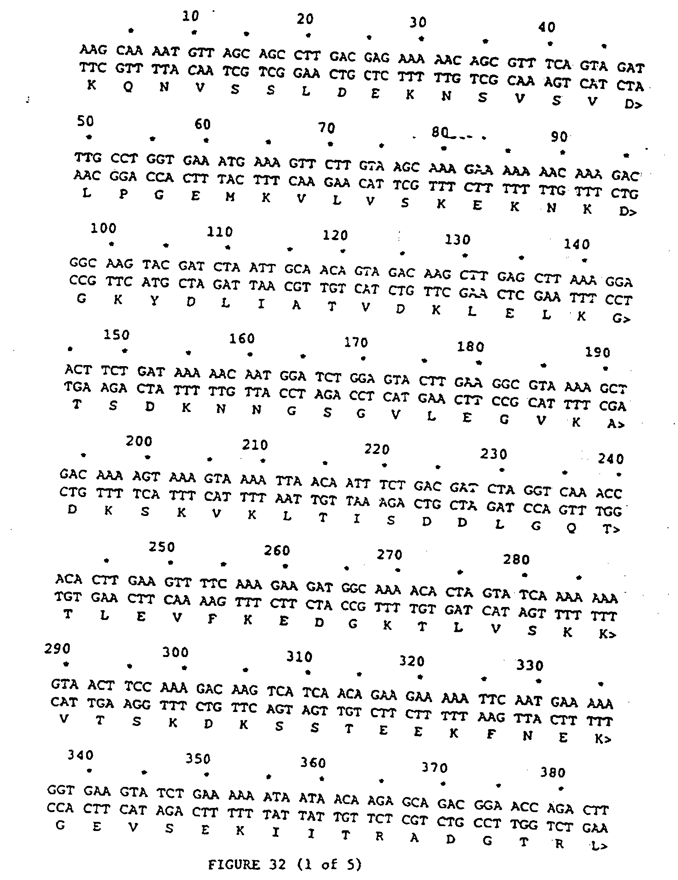

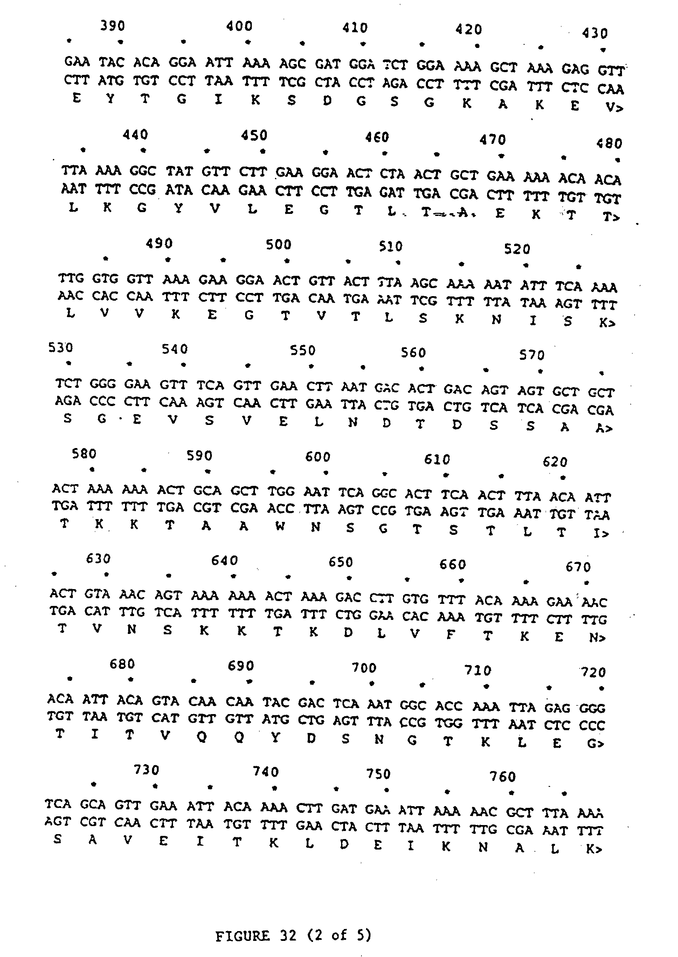

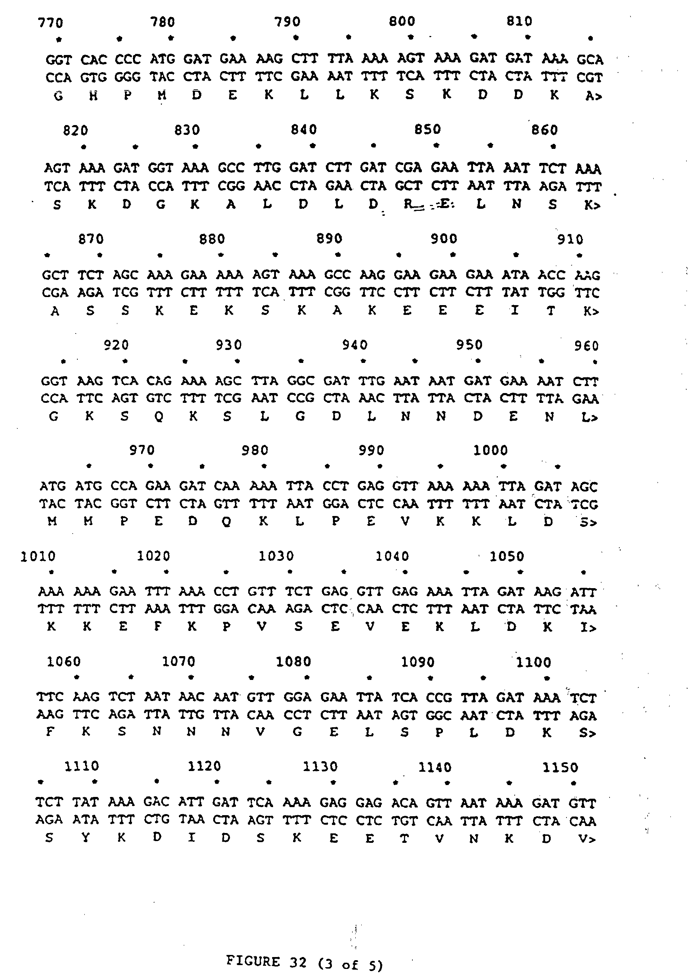

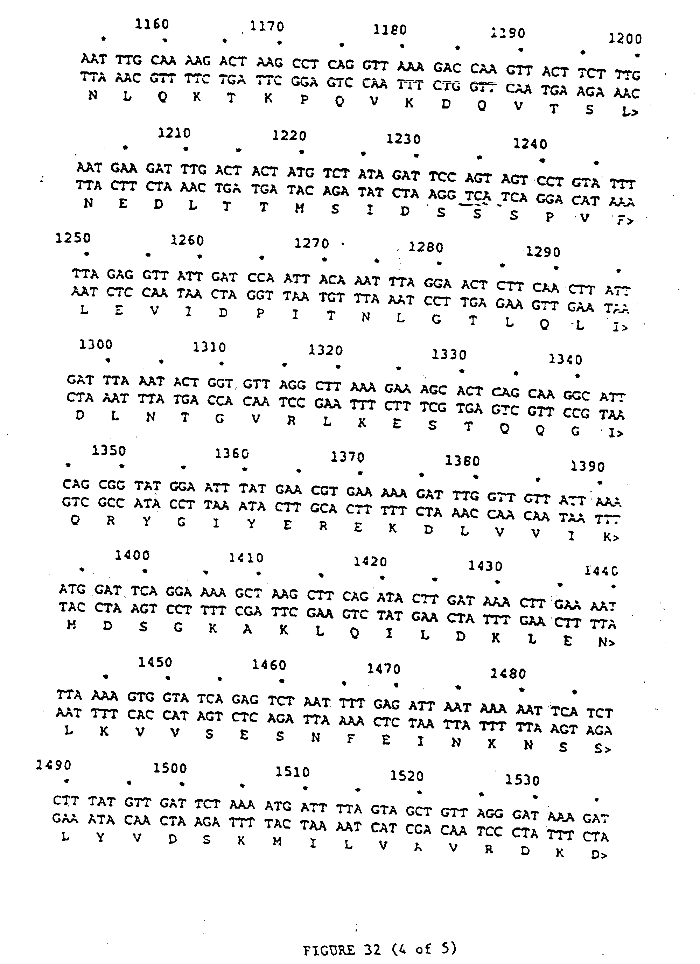

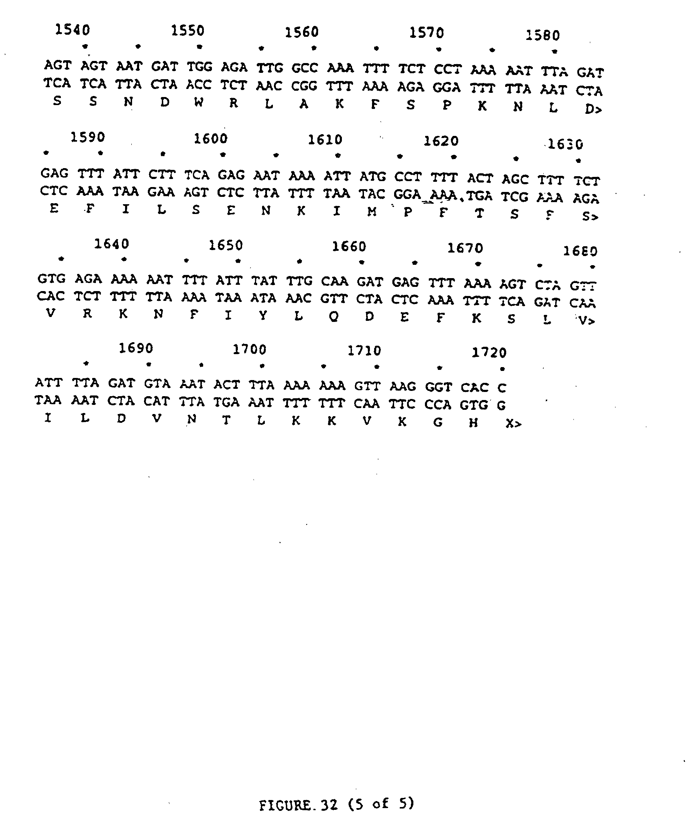

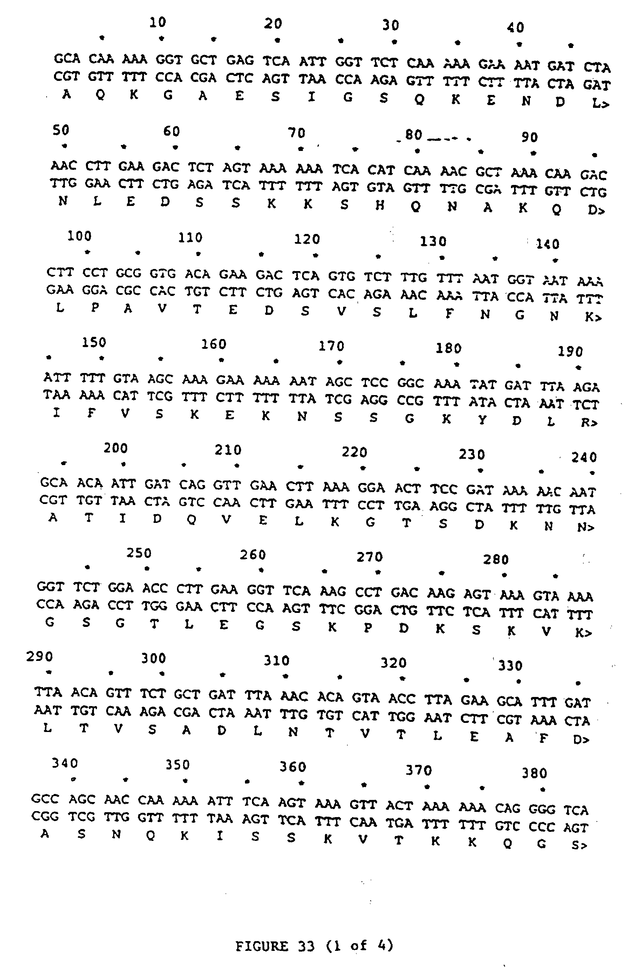

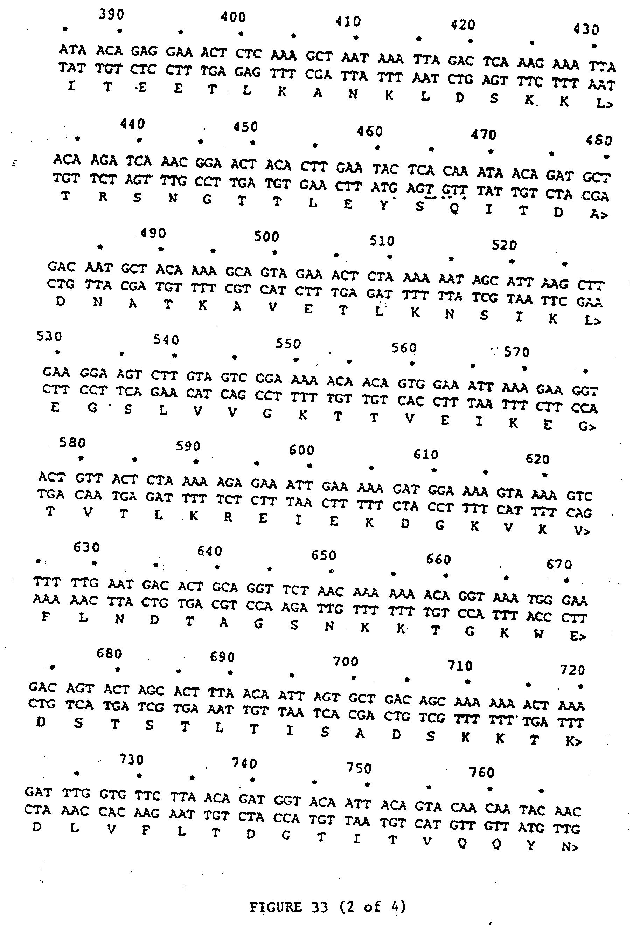

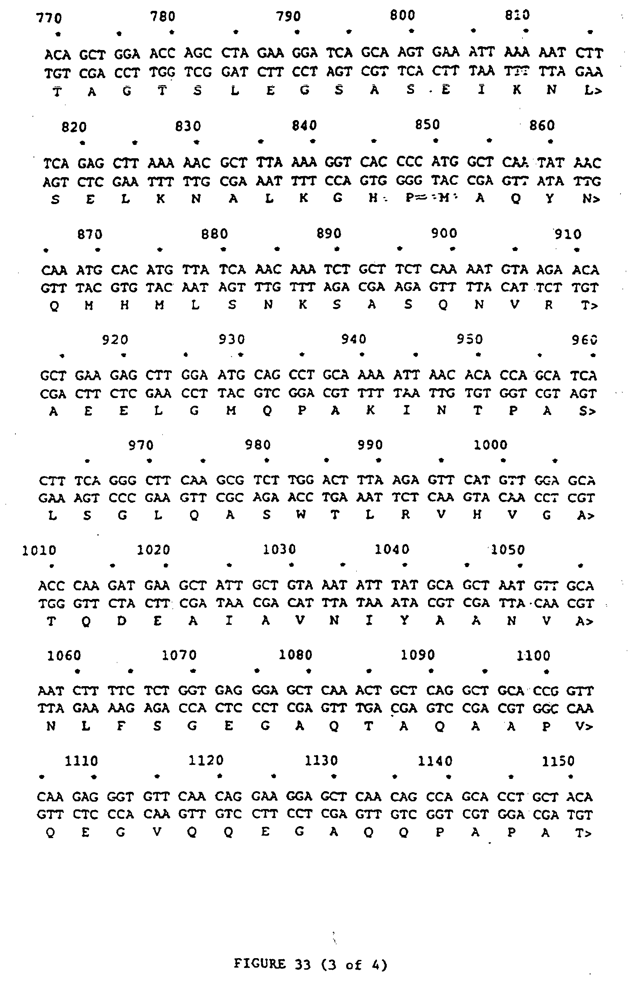

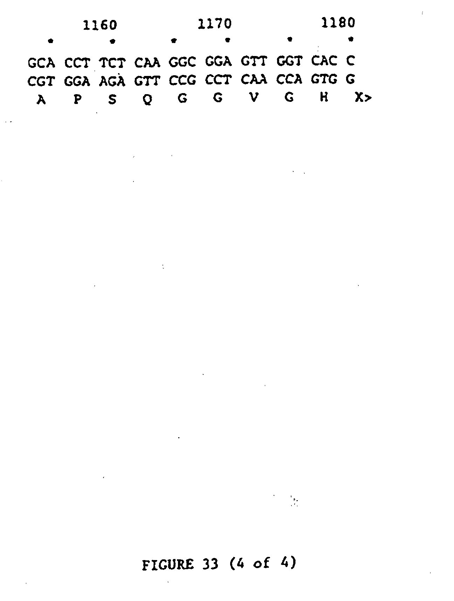

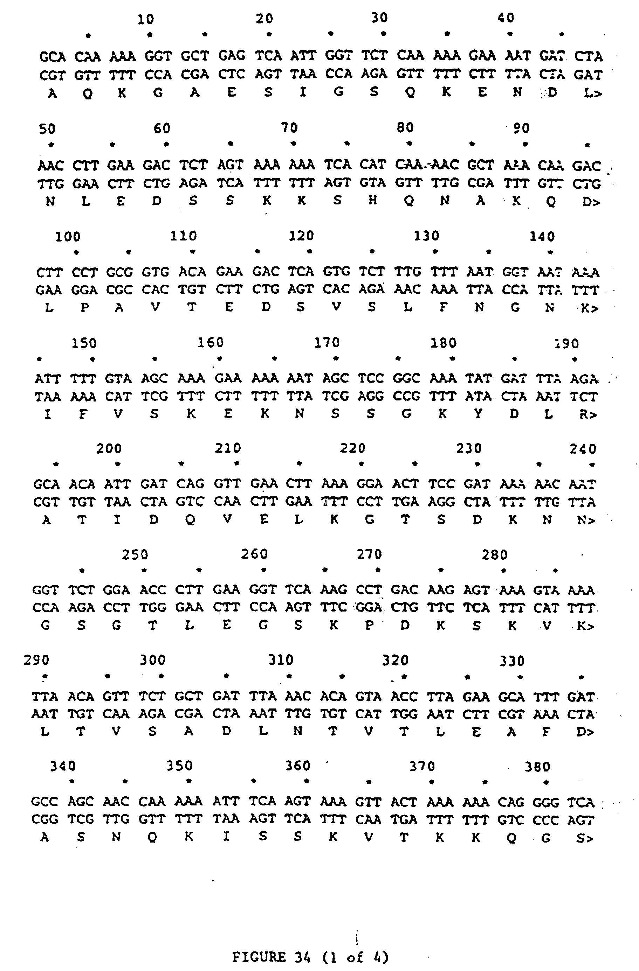

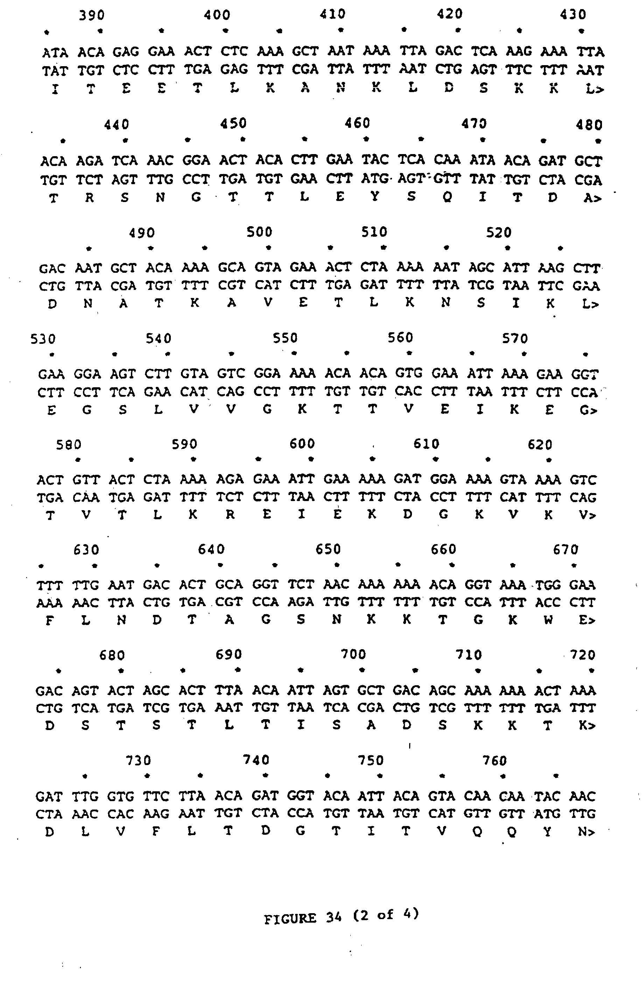

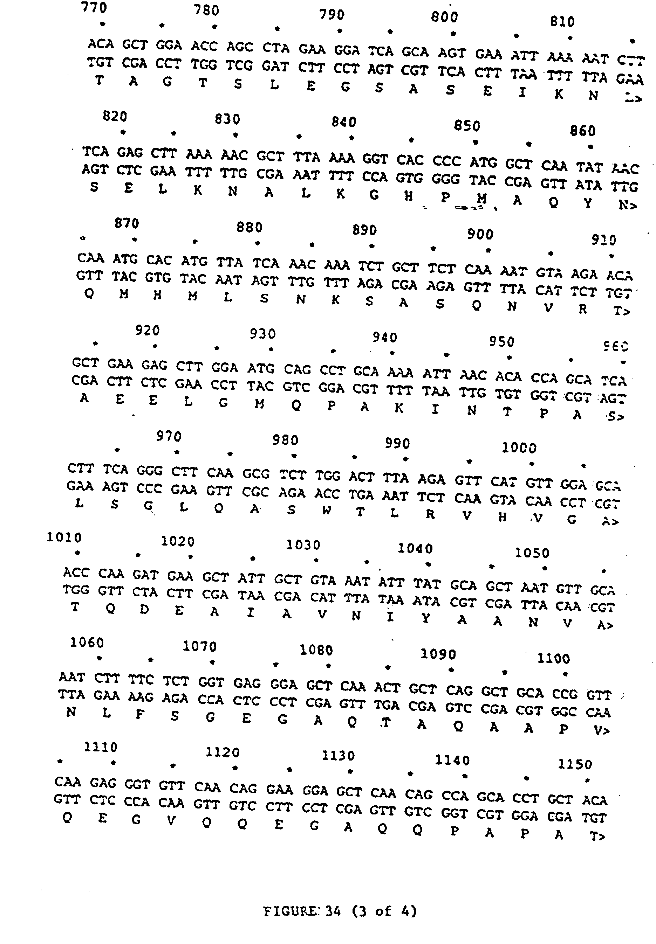

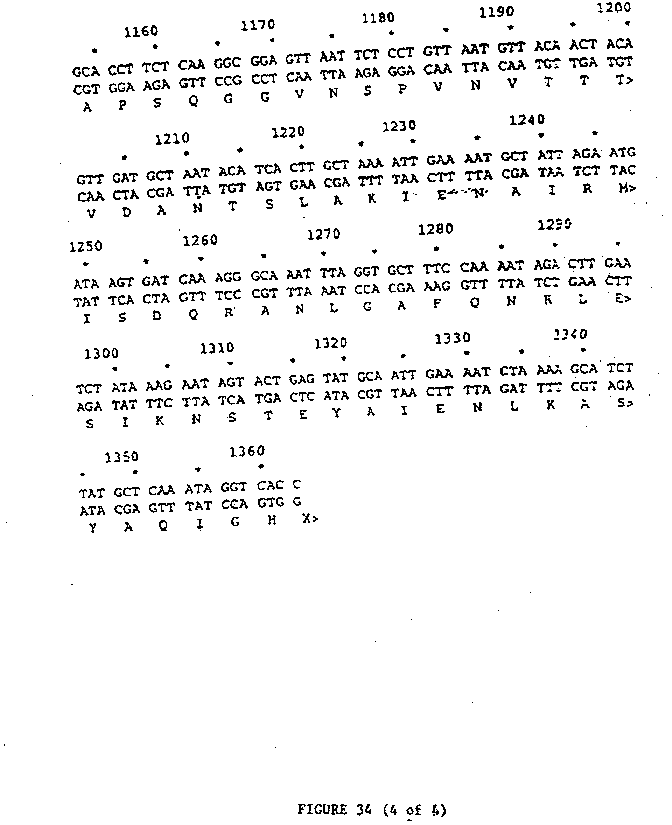

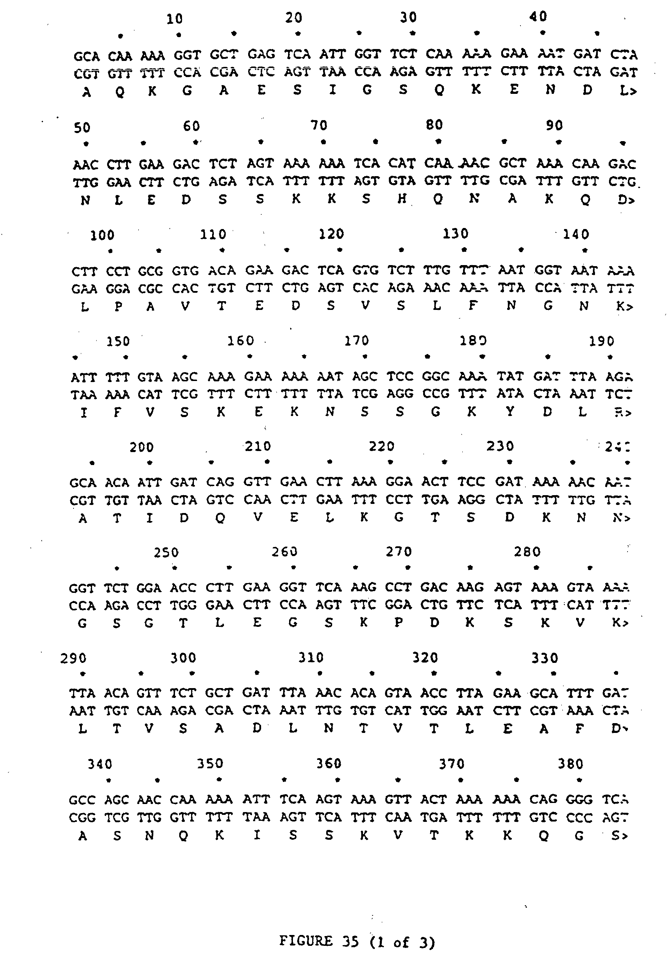

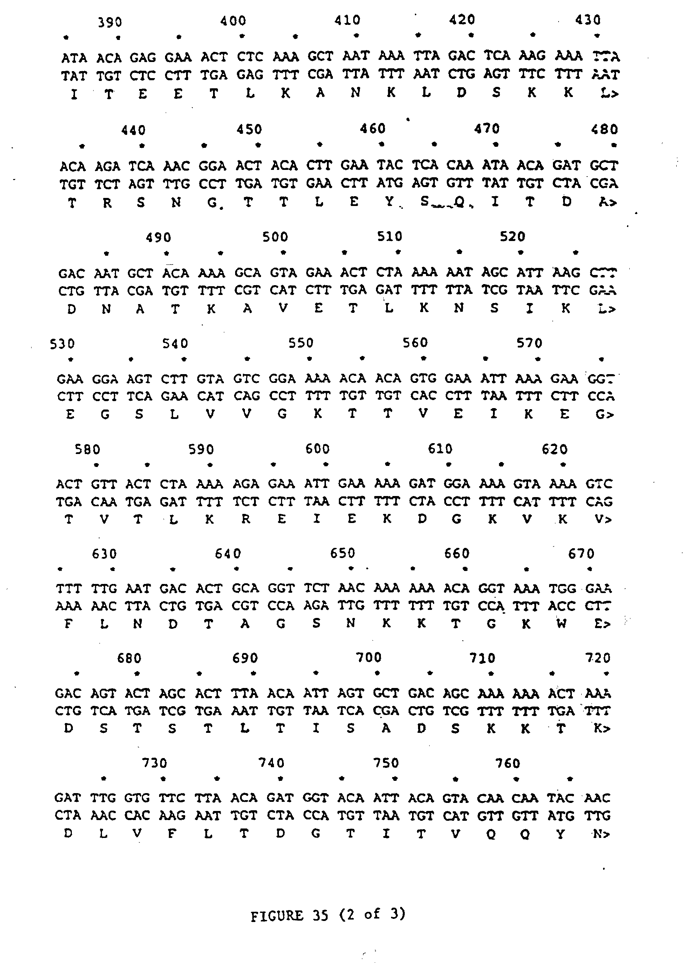

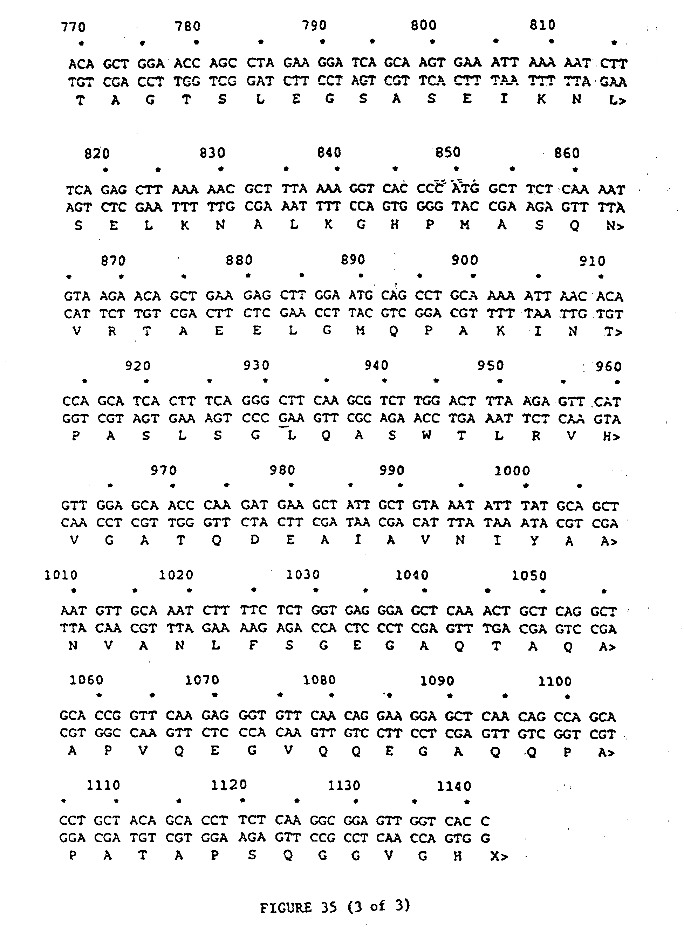

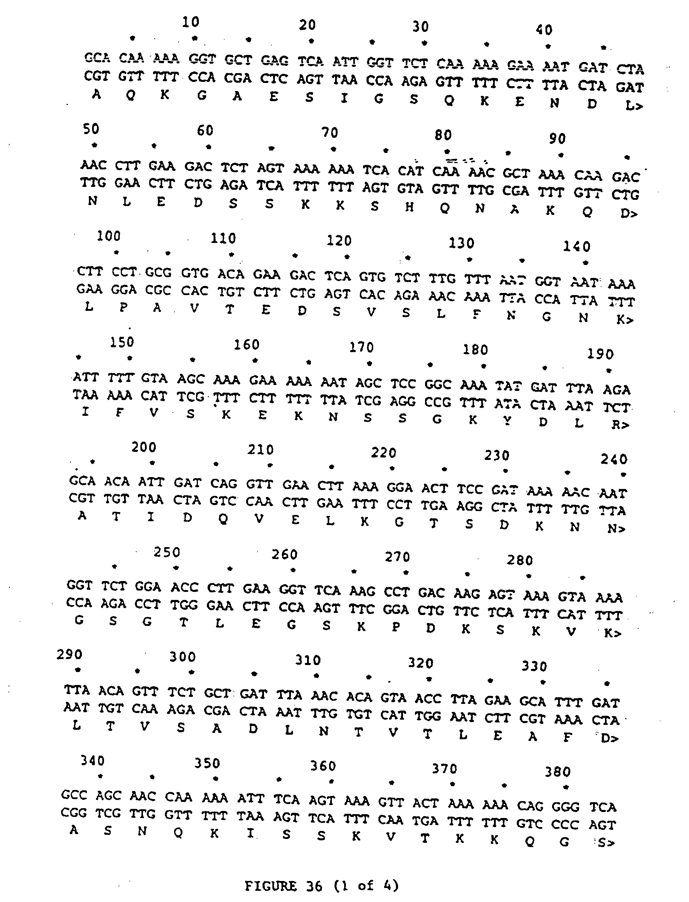

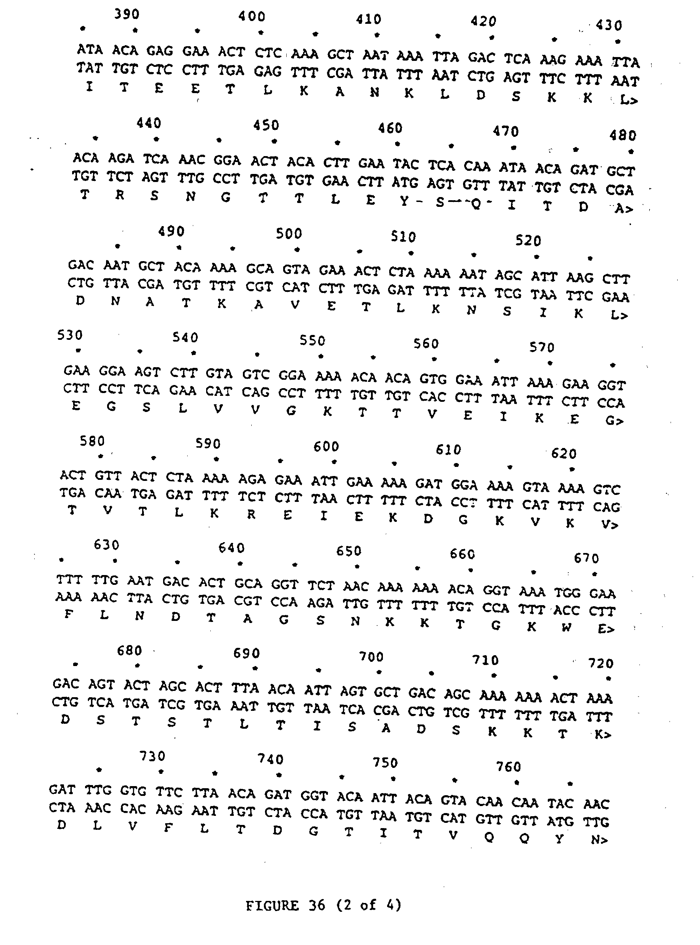

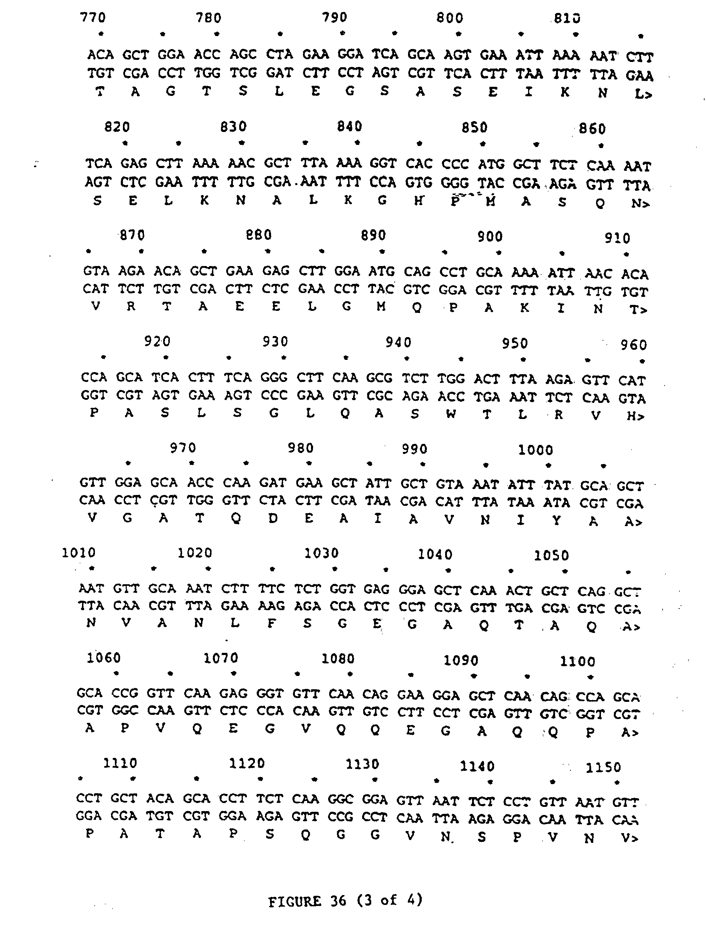

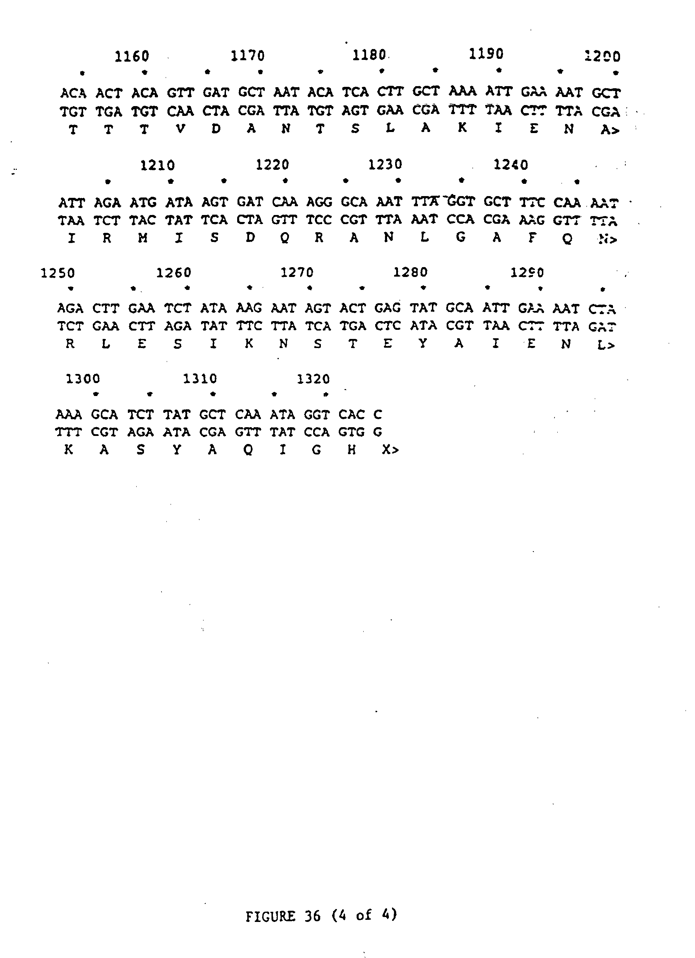

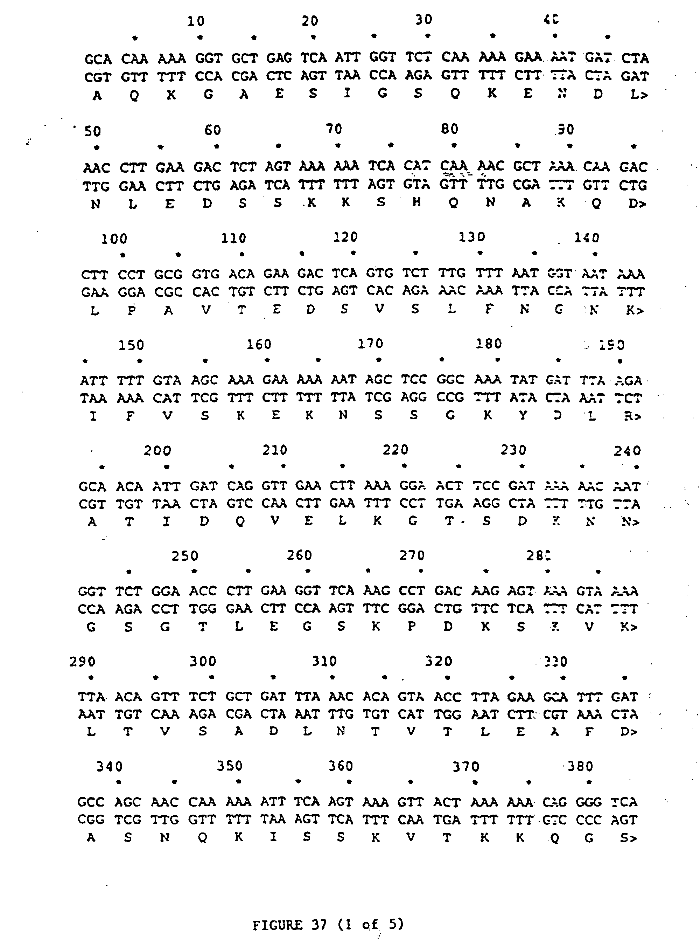

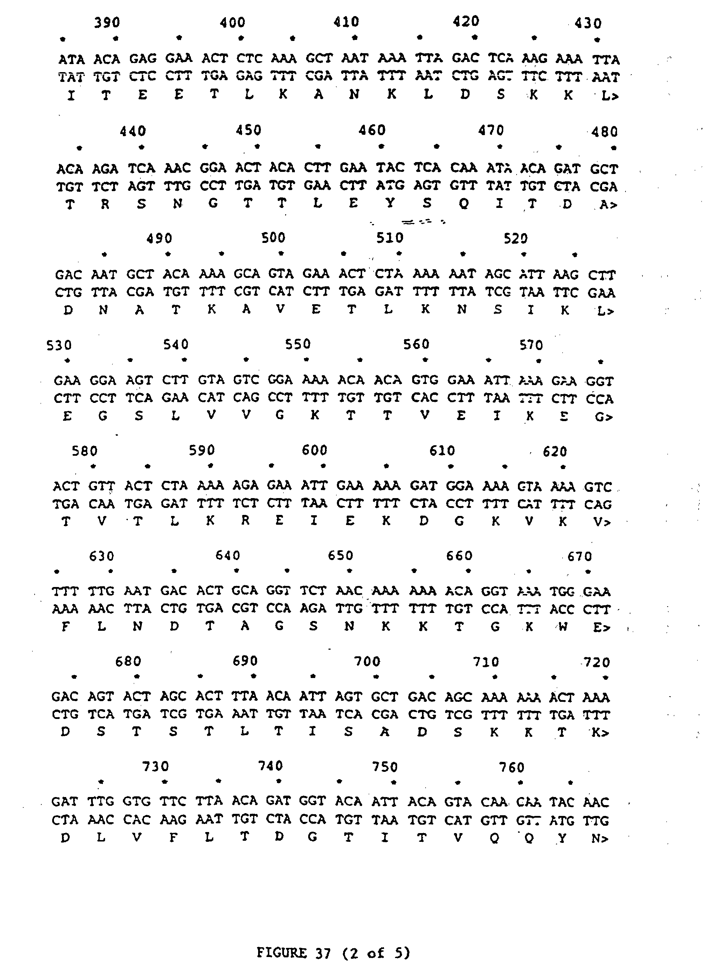

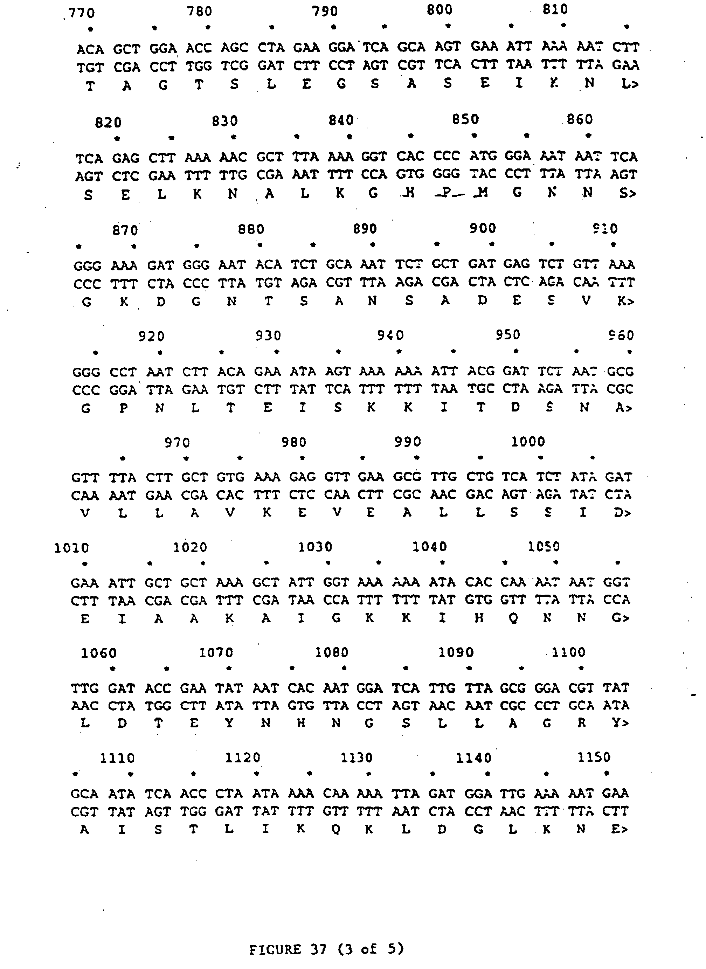

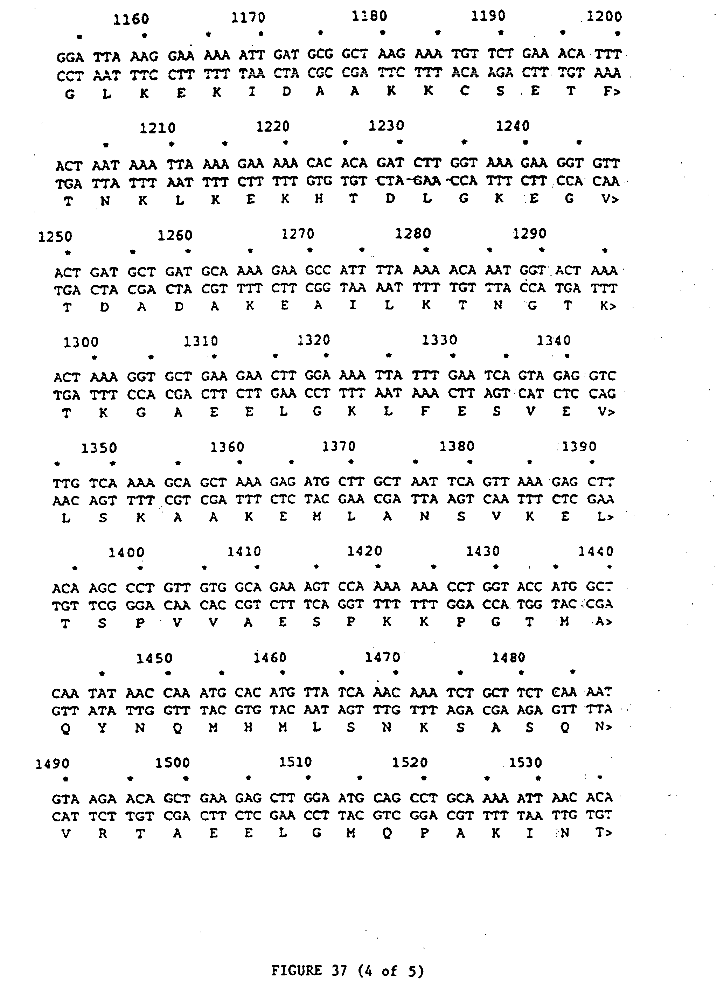

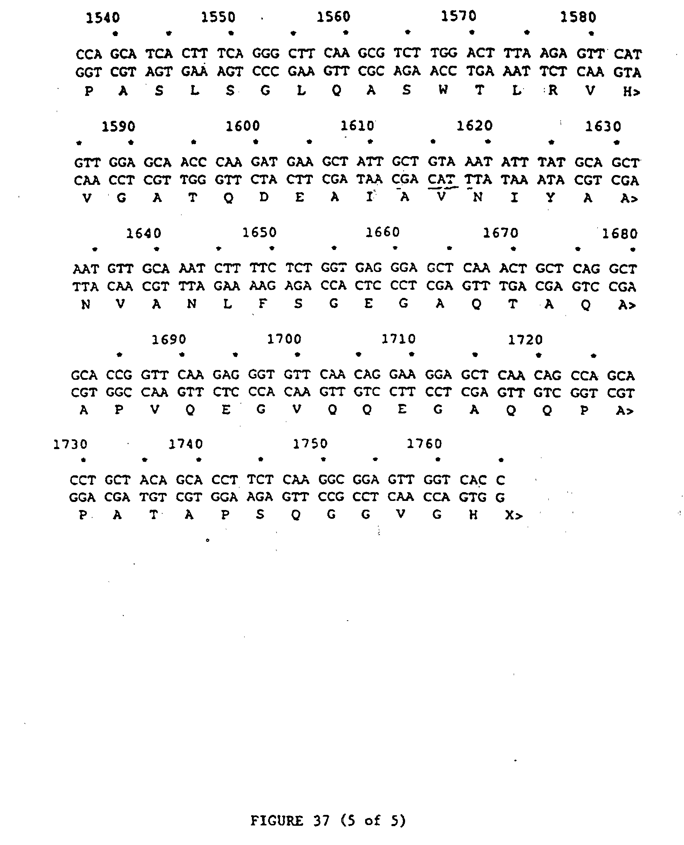

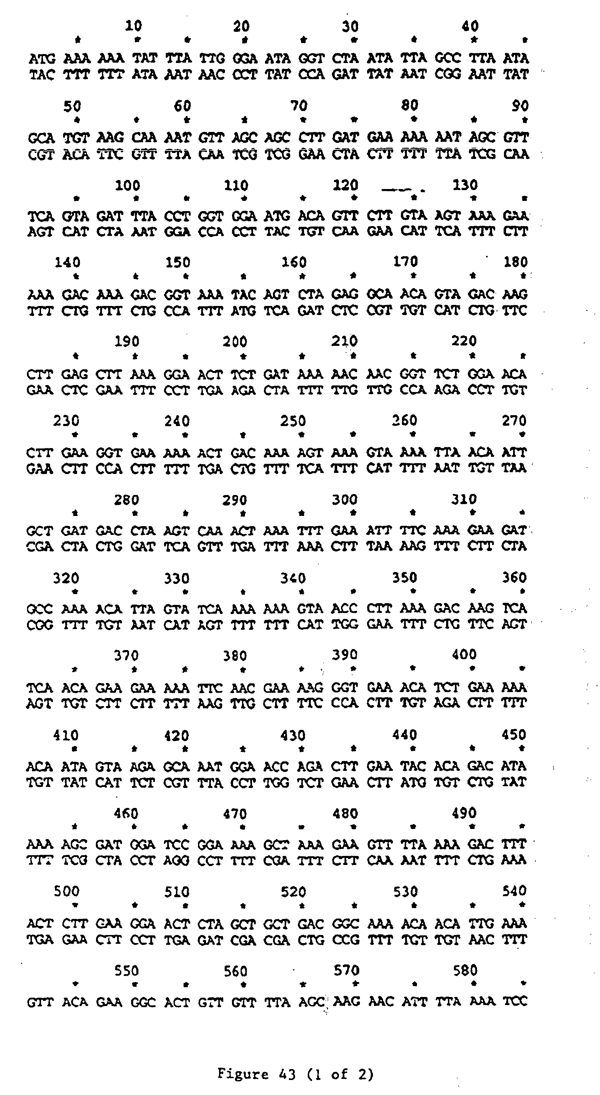

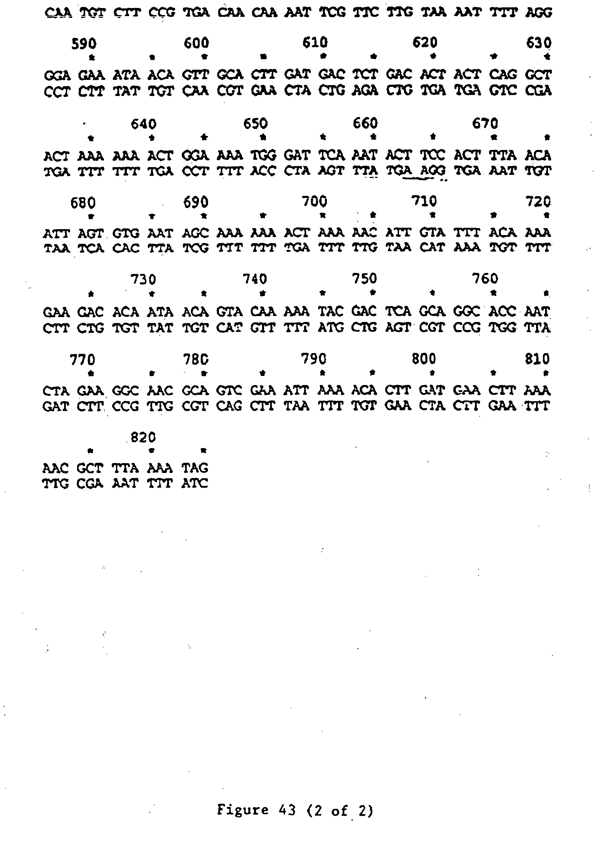

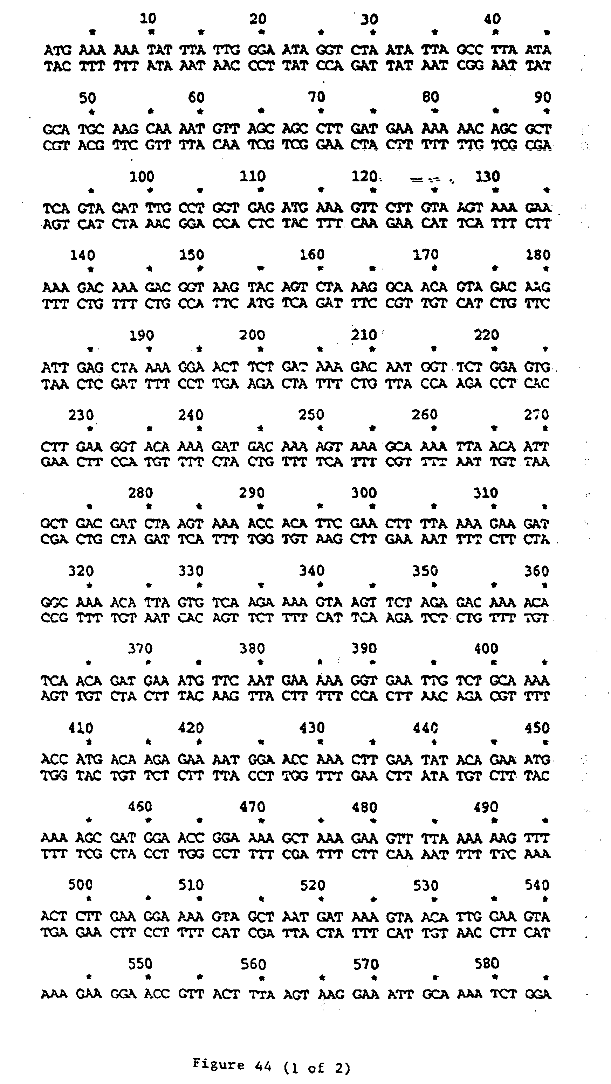

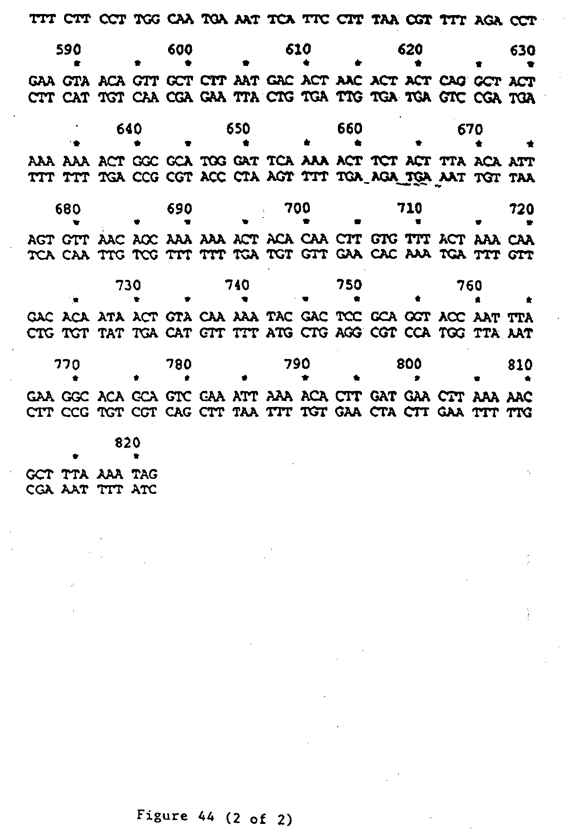

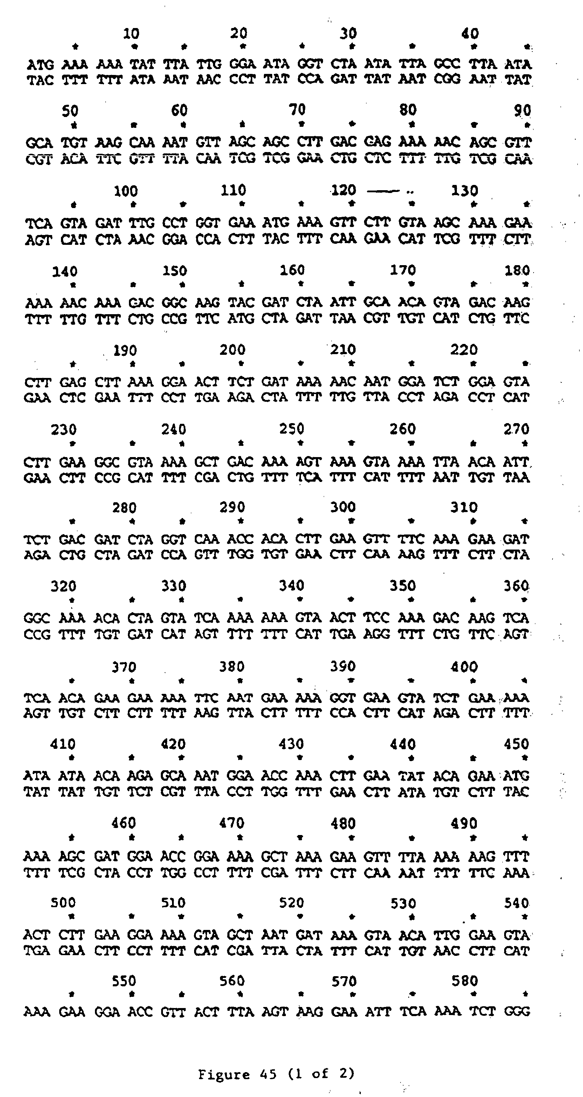

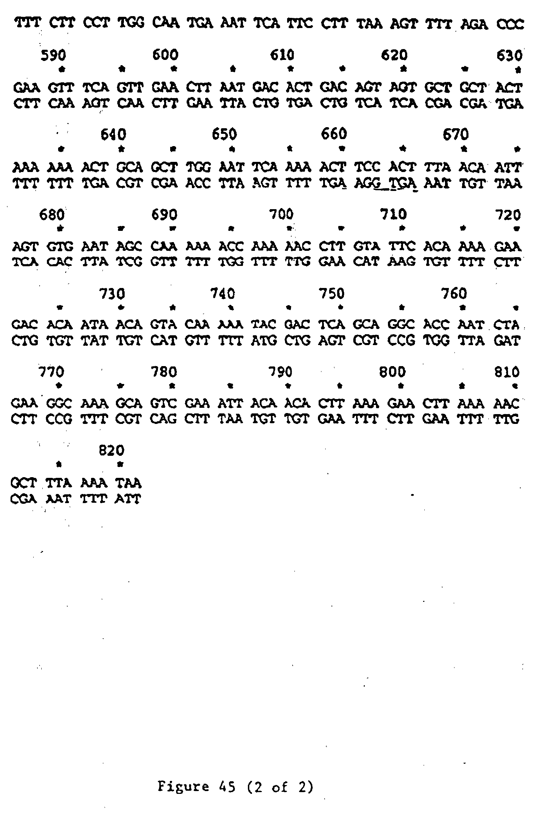

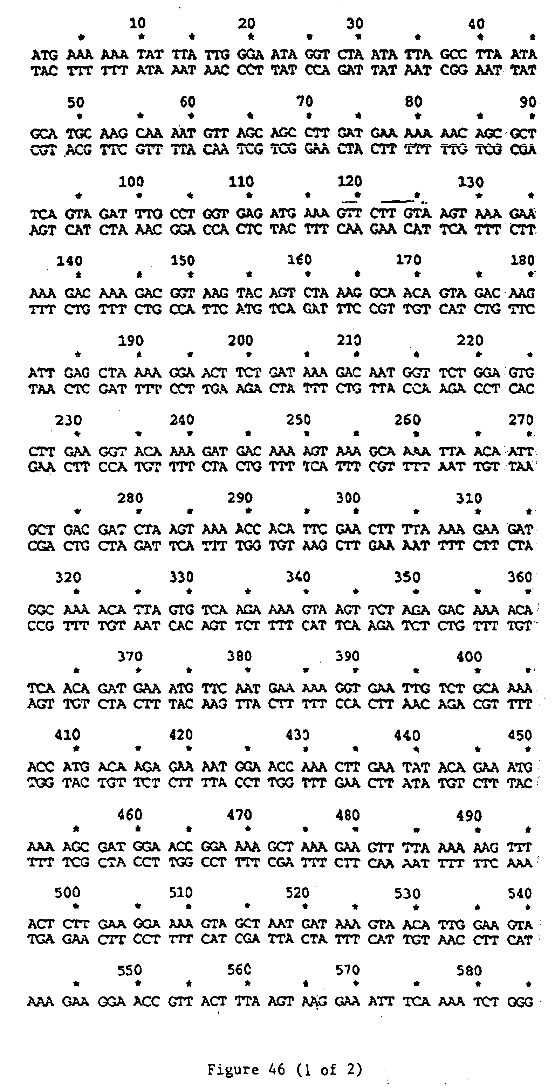

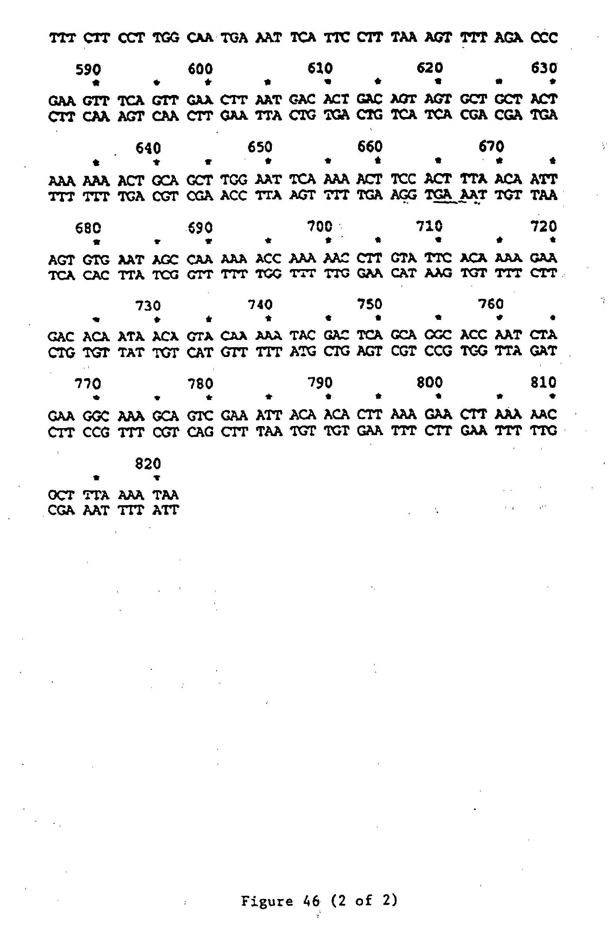

The current invention pertains to chimeric *Borrelia* proteins which include two or more antigenic *Borrelia* polypeptides which do not occur naturally (in nature) in the same protein in *Borrelia,* as well as the nucleic acids encoding such chimeric proteins. The antigenic polypeptides incorporated in the chimeric proteins are derived from any *Borrelia* protein from any strain of *Borrelia*, and include outer surface protein (Osp) A, OspB, OspC, OspD, p12, p39, p41, p66, and p93. The proteins from which the antigenic polypeptides are derived can be from the same strain of *Borrelia,* from different strains, or from combinations of proteins from the same and from different strains. If the proteins from which the antigenic polypeptides are derived are OspA or OspB, the antigenic polypeptide can be derived from either the portion of the OspA or OspB protein present between the amino terminus and the conserved tryptophan of the protein (referred to as a proximal portion), or the portion of the OspA or OspB protein present between the conserved tryptophan of the protein and the carboxy terminus (referred to as a distal portion). Particular chimeric proteins, and the nucleotide sequences encoding them, are set forth in Figures 23-37 and 43-46.

The chimeric proteins of the current invention provide antigenic polypeptides of a variety of *Borrelia* strains and/or proteins within a single protein. Such proteins are particularly useful in immunodiagostic assays to detect the presence of antibodies to native Borrelia in potentially infected individuals as well as to measure T-cell reactivity, and can therefore be used as immunodiagnostic reagents. The chimeric proteins of the current invention are additionally useful as vaccine immunogens against Borrelia infection.

For a better understanding of the present invention together with other and further objects, reference is made to the following description, taken together with the accompanying drawings.

### Brief Description of the Drawings

Figure 1 summarizes peptides and antigenic domains localized by proteolytic and chemical fragmentation of OspA.
Figure 2 is a comparison of the antigenic domains depicted in Figure 1, for OspA in nine strains of *B. burgdorferi*.
Figure 3 is a graph depicting a plot of weighted polymorphism versus amino acid position among 14 OspA variants. The marked peaks are: a) amino acids 132-145; b) amino acids 163-177; c) amino acids 208-221. The lower dotted line at polymorphism value 1.395 demarcates statistically significant excesses of polymorphism at p = 0.05. The upper dotted line at 1.520 is the same, except that the first 29 amino acids at the monomorphic N-terminus have been removed from the original analysis.
Figure 4 depicts the amino acid alignment of residues 200 through 220 for OspAs from strains B31 and K48 as well as for the site-directed mutants 613, 625, 640, 613/625, and 613/640. Arrow indicates Trp216. Amino acid changes are underlined.
Figure 5 is a helical wheel projection of residues 204-217 of B31 OspA. Capital letters indicate hydrophobic residues; lower case letters indicate hydrophilic residues; +/- indicate positively/negatively charged residues. Dashed line indicates division of the alpha-helix into hydrophobic arc (above the line) and polar arc (below the line). Adapted from France et al. (Biochem. Biophys. Acta 1120: 59 (1992)).

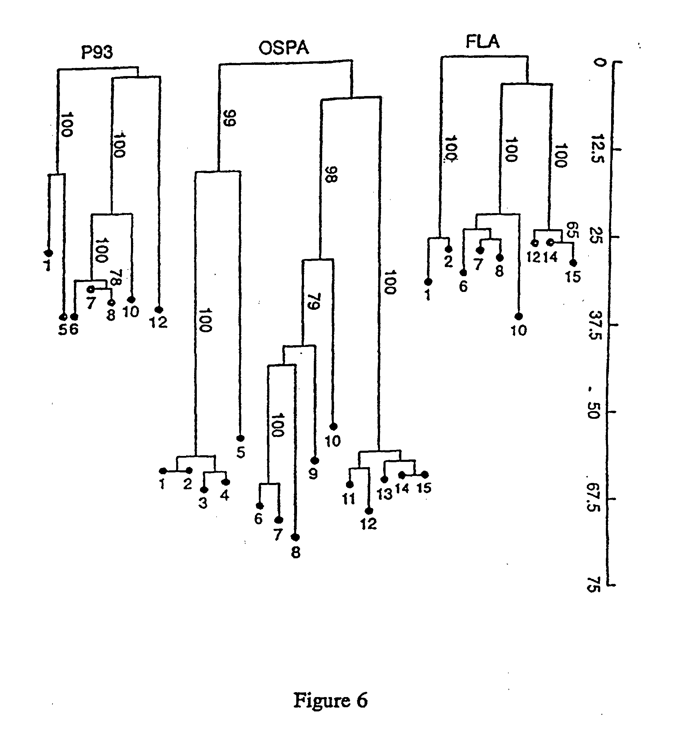

Figure 6 depicts a phylogenic tree for strains of *Borrelia* described in Table I. The strains are as follows: 1 = B31; 2 = Pkal; 3 = ZS7; 4 = N40; 5 = 25015; 6 = K48; 7 = DK29; 8 = PHei; 9 = Ip90; 10 = PTrob; 11 = ACAI; 12 = PGau; 13 = Ip3; 14 = PBo; 15 = PKo.

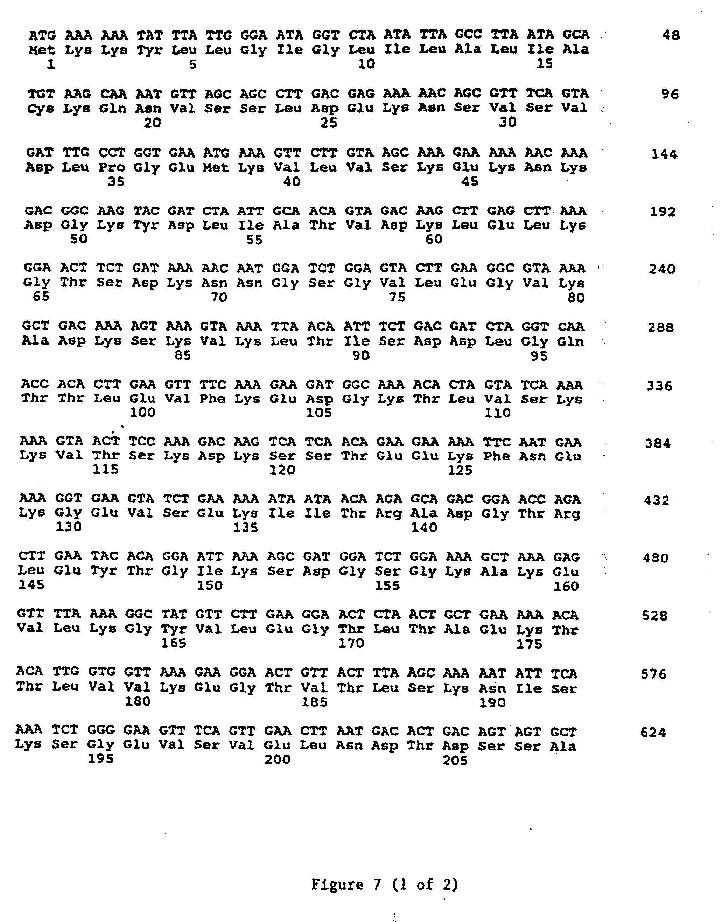

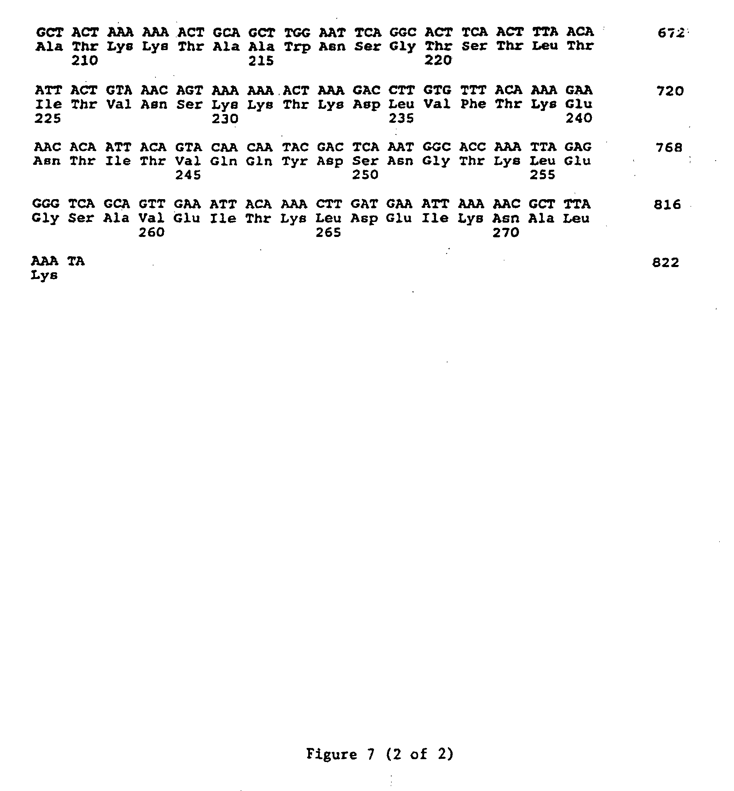

Figure 7 depicts the nucleic acid sequence of OspA-B31 (SEW ID NO. 6), and the encoded protein sequence (SEQ ID NO. 7).

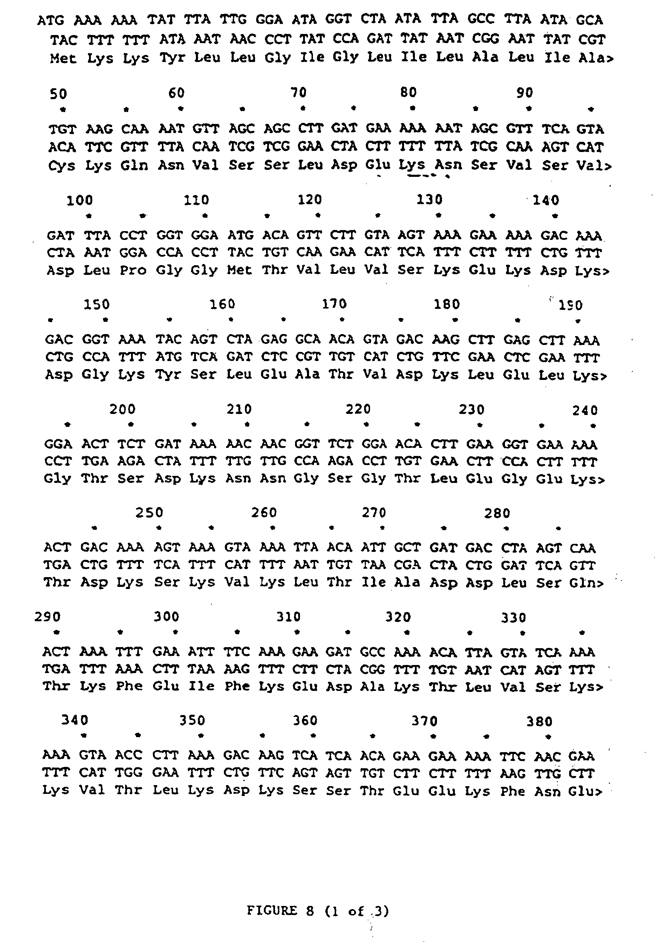

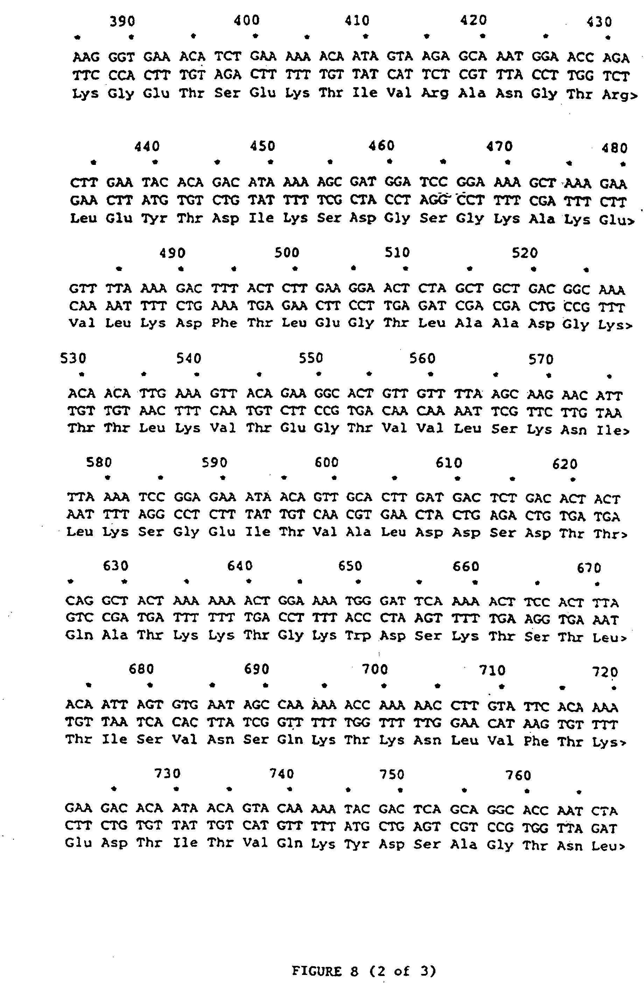

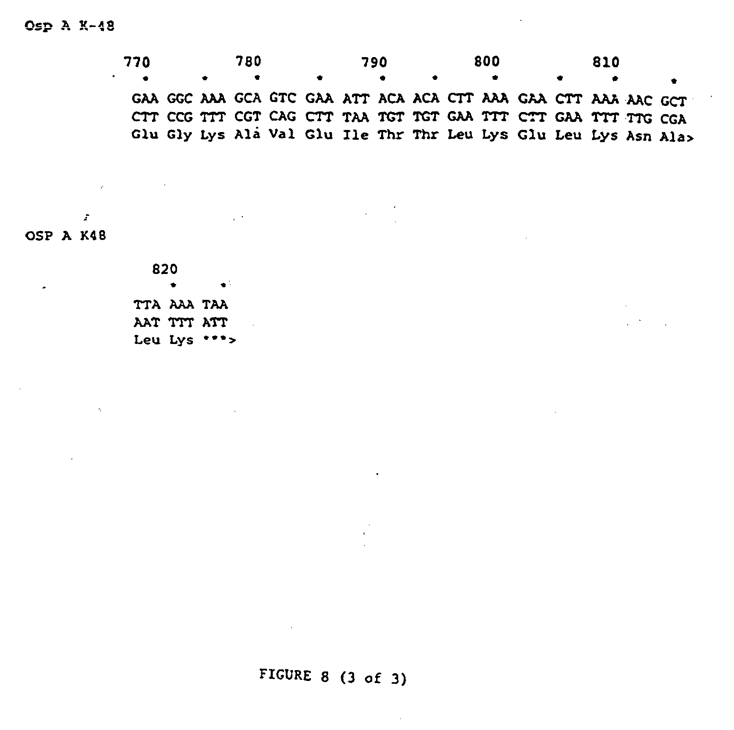

Figure 8 depicts the nucleic acid sequence of OspA-K48 (SEQ ID NO. 8), and the encoded protein sequence (SEQ ID NO. 9).

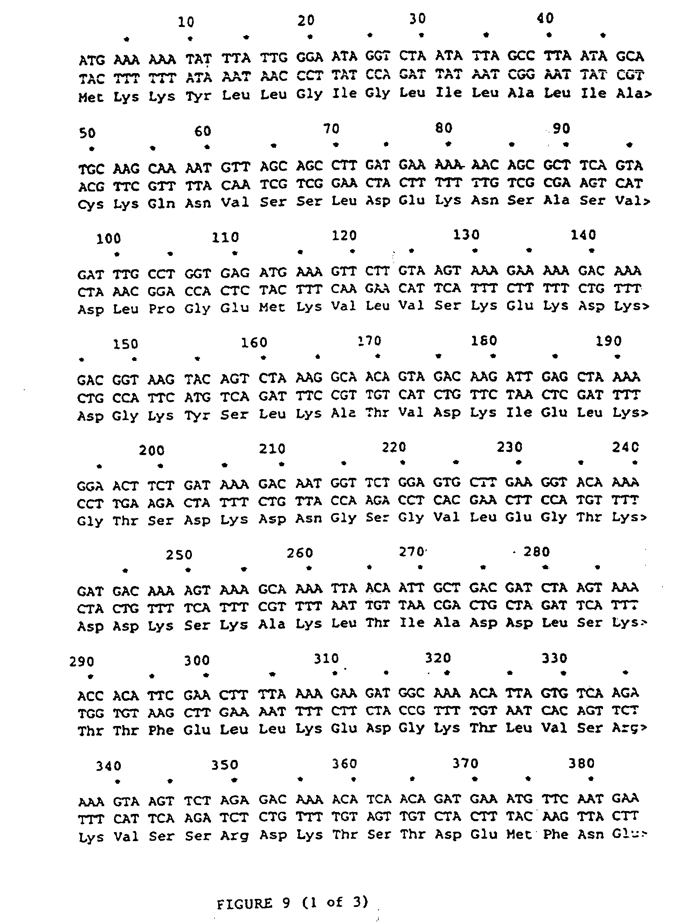

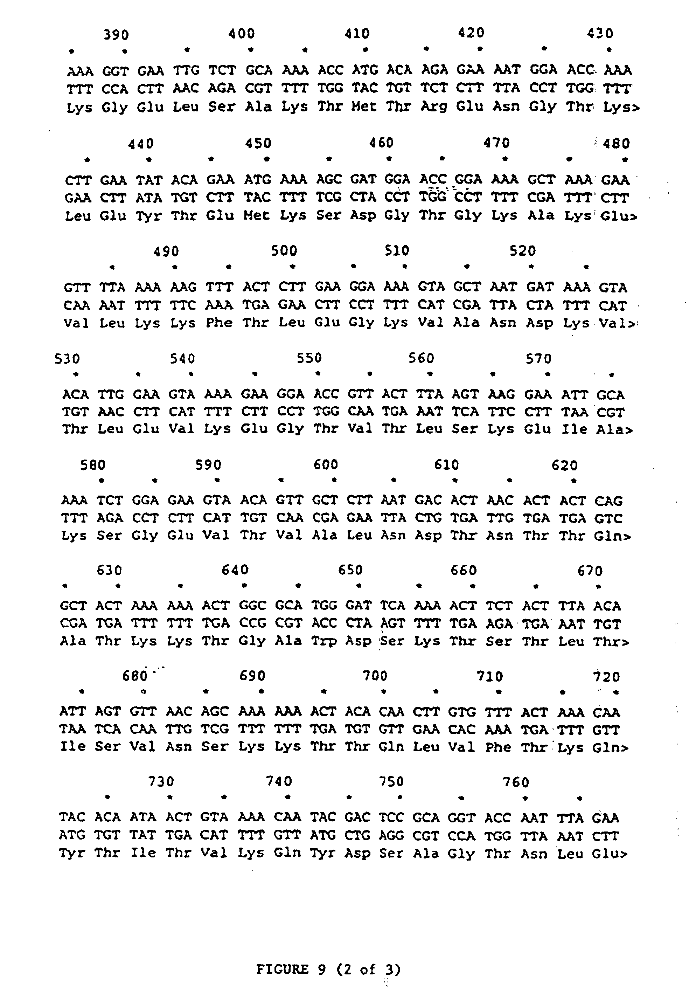

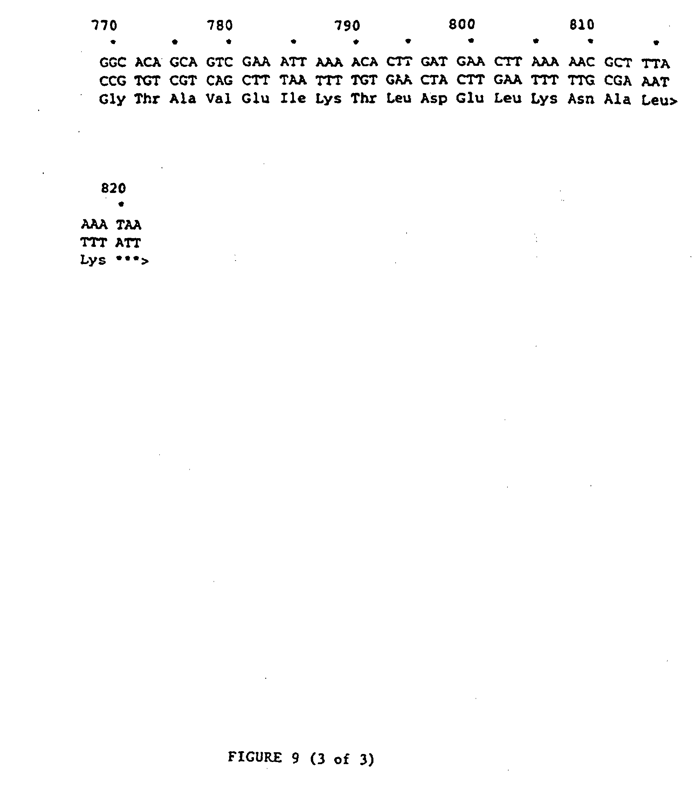

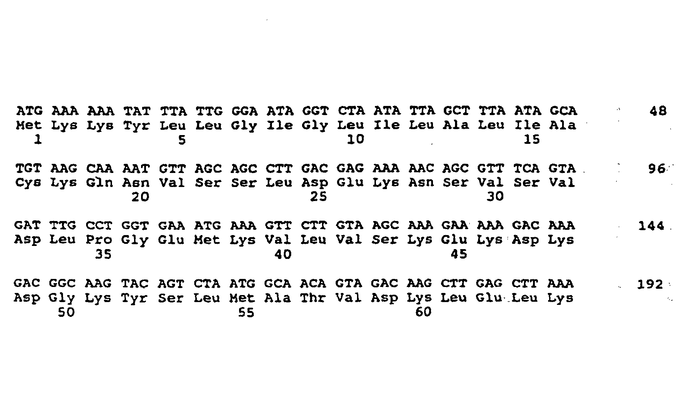

Figure 9 depicts the nucleic acid sequence of OspA-PGau (SEQ ID NO. 10), and the encoded protein sequence (SEQ ID NO. 11).

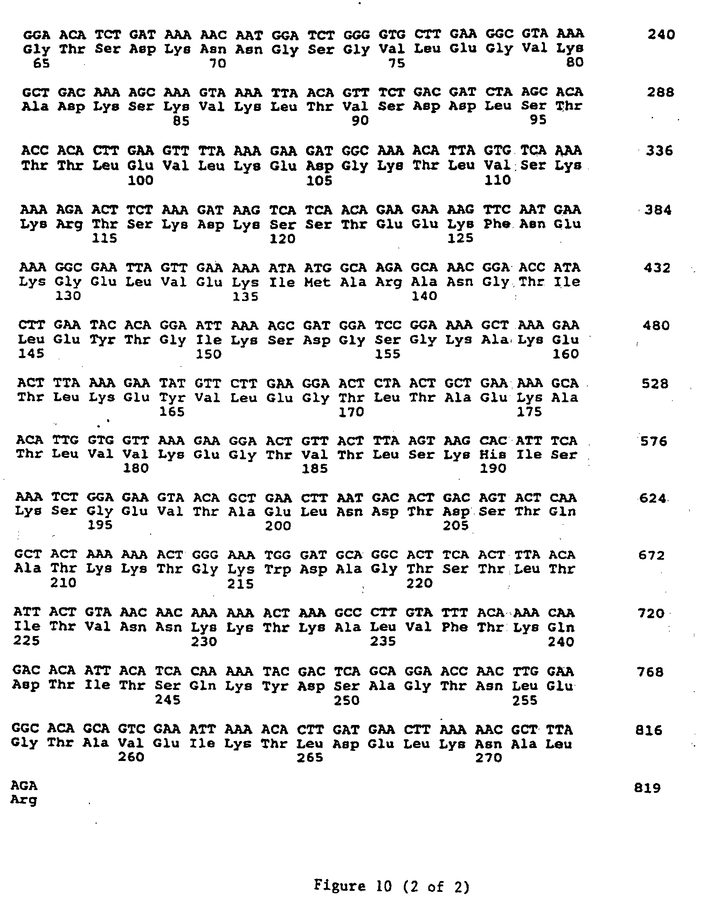

Figure 10 depicts the nucleic acid sequence of OspA-25015 (SEQ ID NO. 12), and the encoded protein sequence (SEQ ID NO. 13).

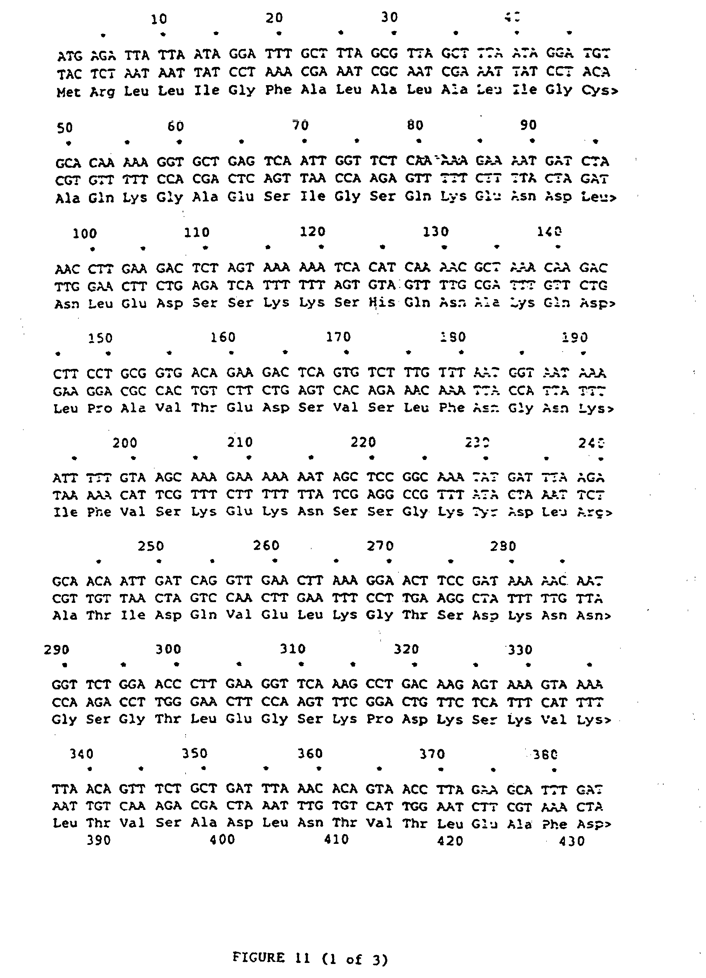

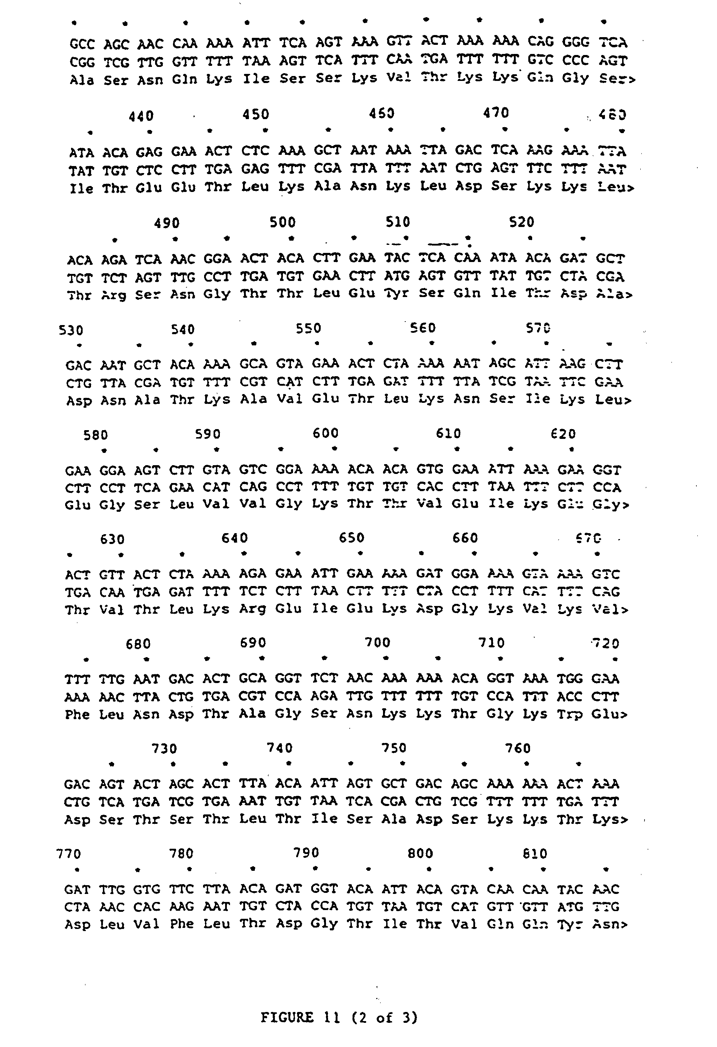

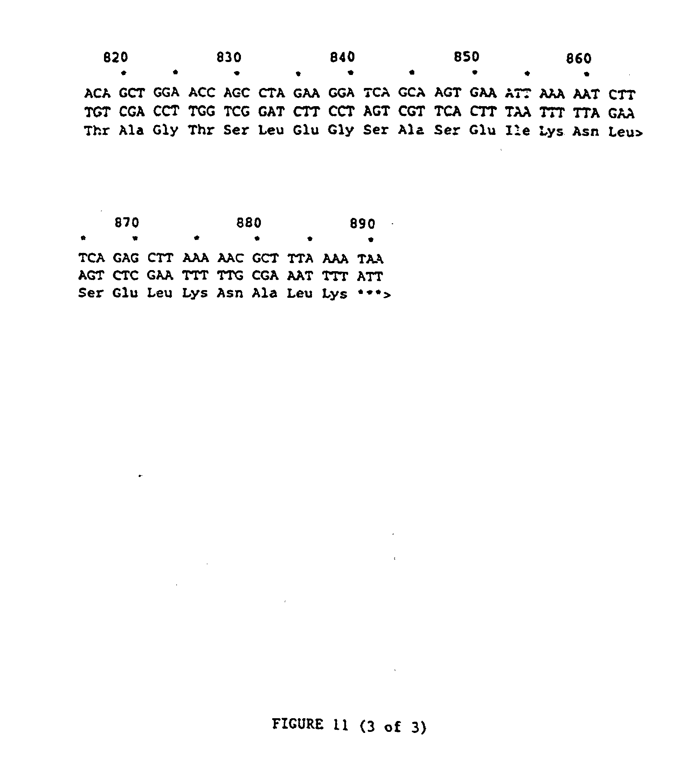

Figure 11 depicts the nucleic acid sequence of OspB-B31 (SEQ ID NO. 21), and the encoded protein sequence (SEQ ID NO. 22).

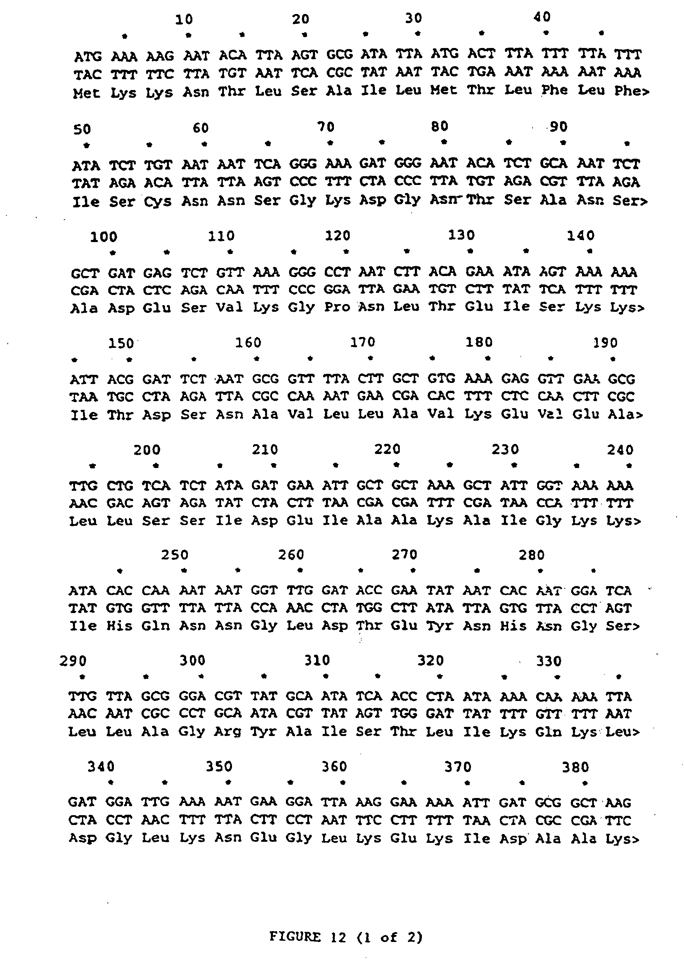

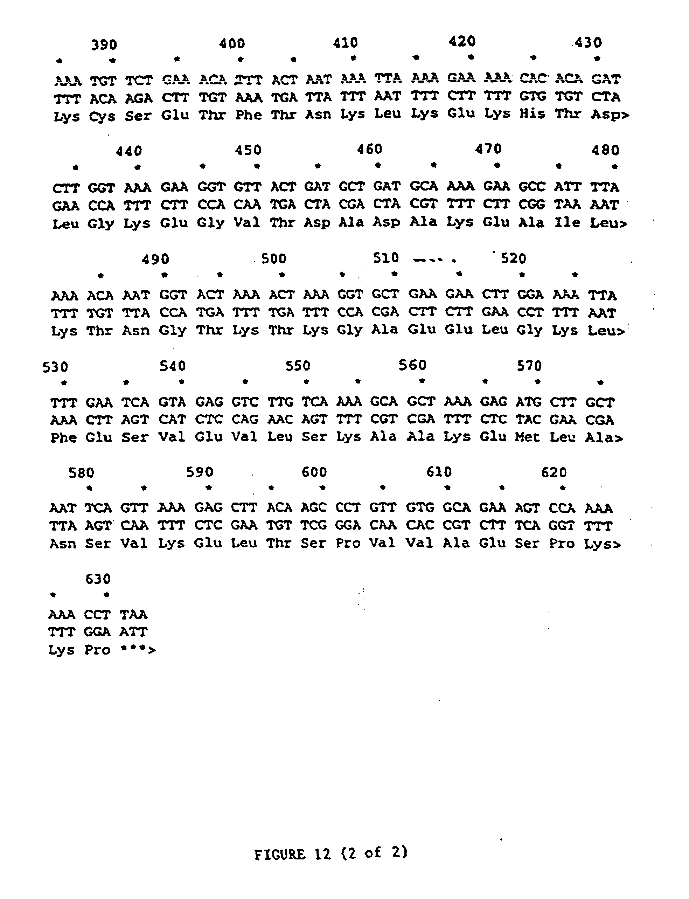

Figure 12 depicts the nucleic acid sequence of OspC-B31 (SEQ ID NO. 29), and the encoded protein sequence (SEQ ID NO. 30).

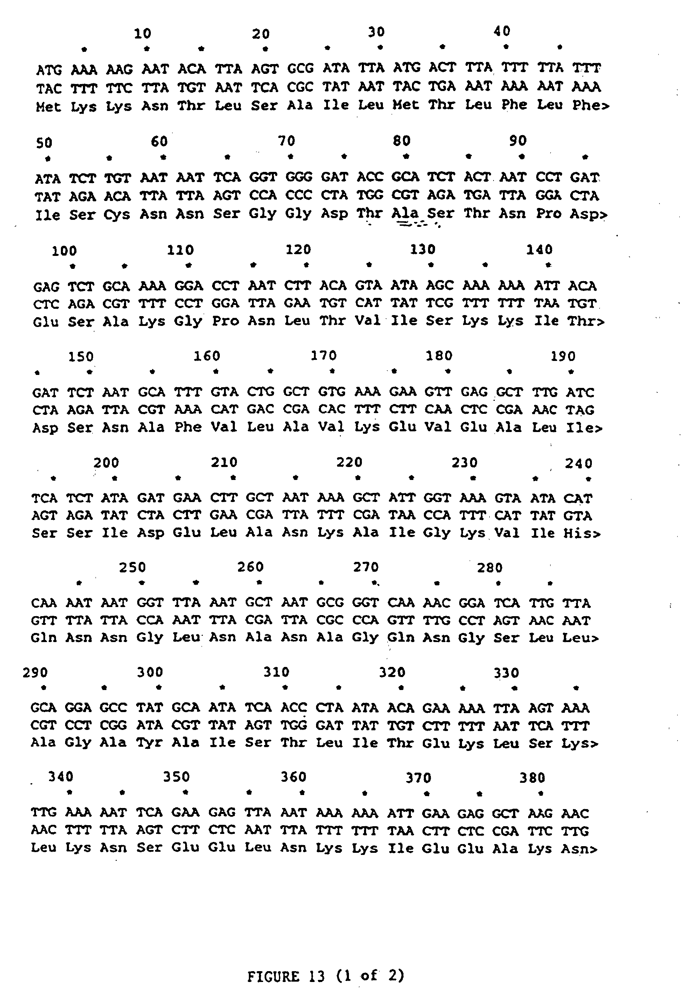

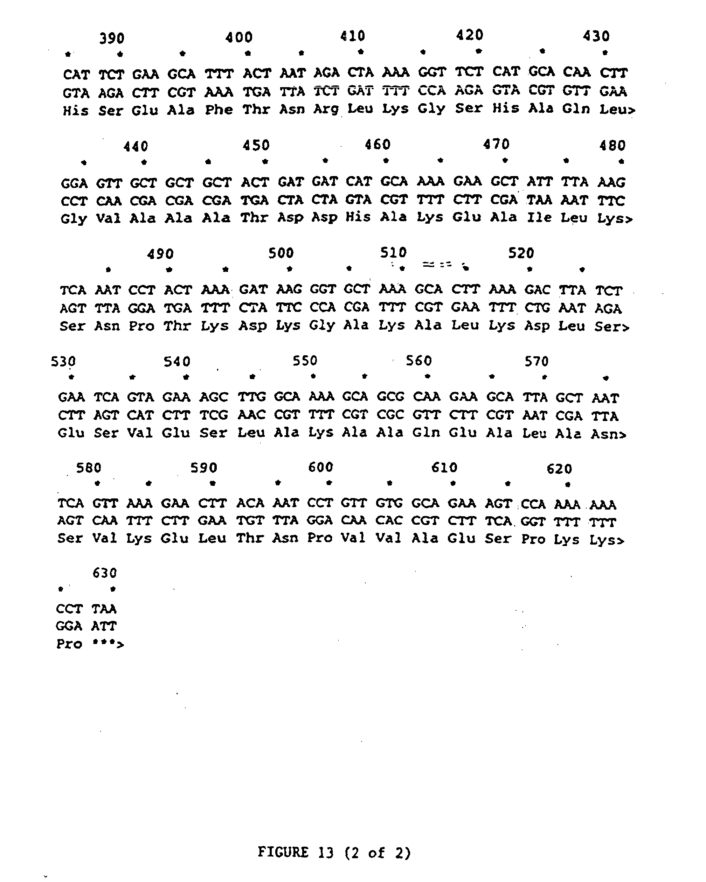

Figure 13 depicts the nucleic acid sequence of OspC-K48 (SEQ ID NO. 31), and the encoded protein sequence (SEQ ID NO. 32).

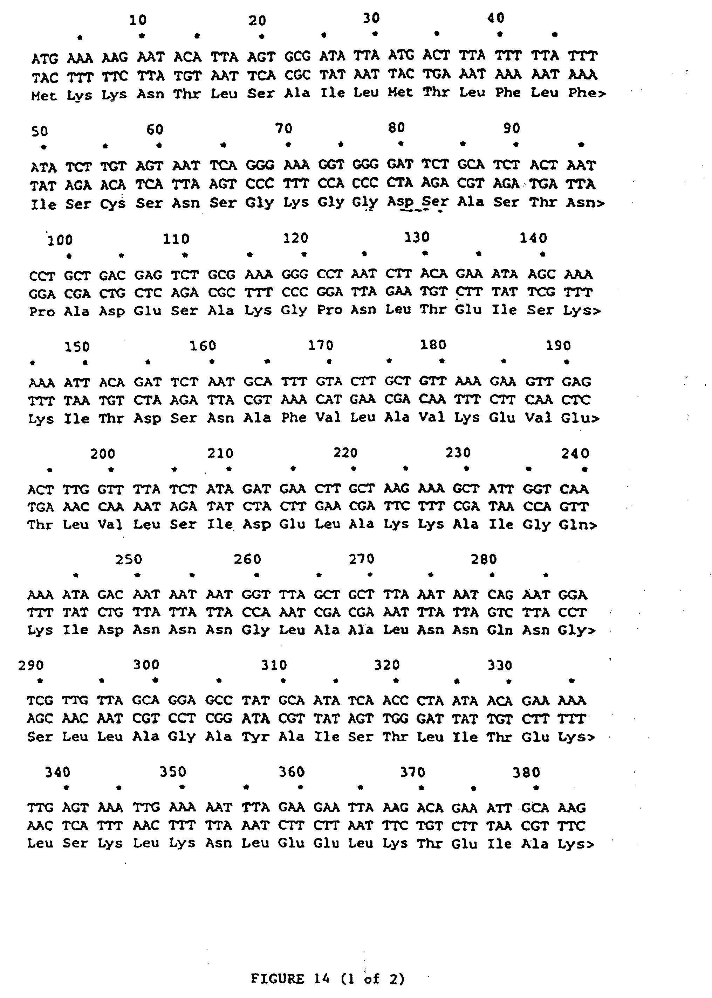

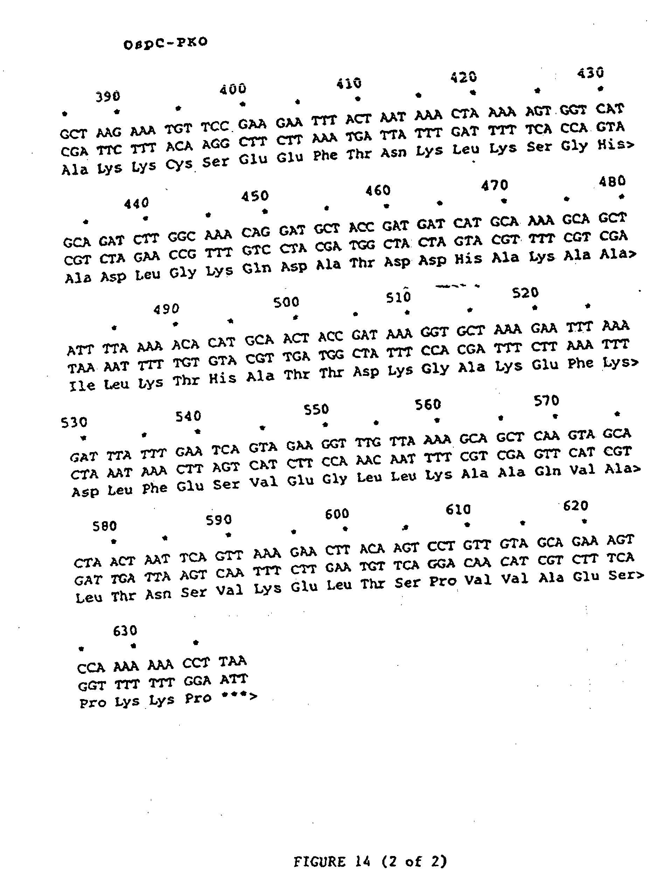

Figure 14 depicts the nucleic acid sequence of OspC-PKo (SEQ ID NO. 33), and the encoded protein sequence (SEQ ID NO. 34).

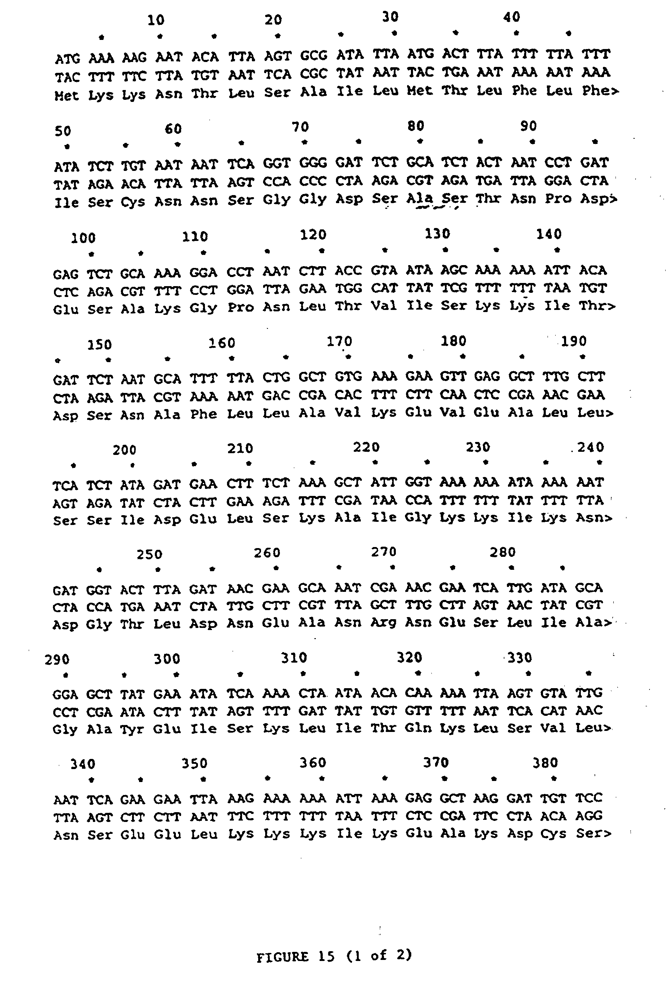

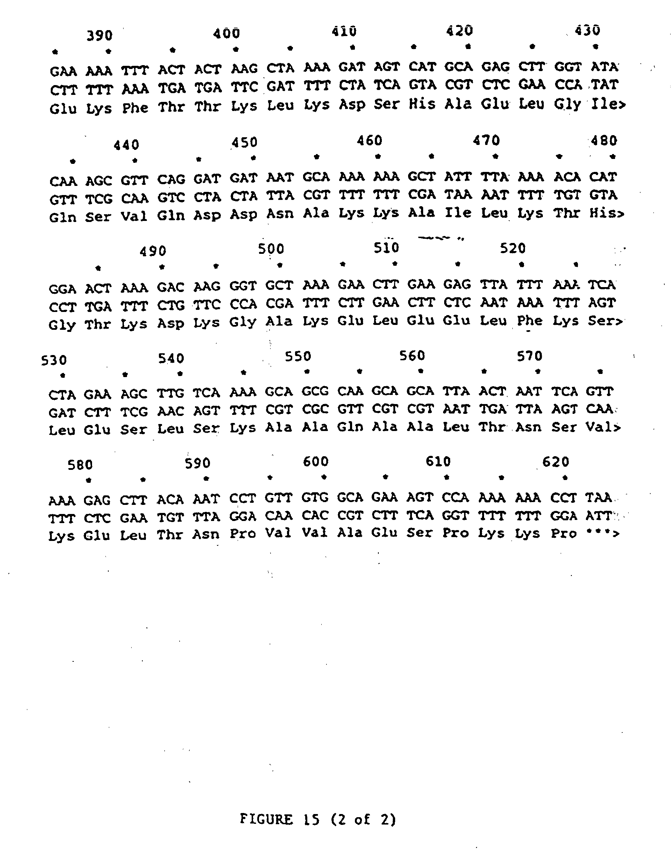

Figure 15 depicts the nucleic acid sequence of OspC-pTrob (SEQ ID NO. 35) and the encoded protein sequence (SEQ ID NO. 36).

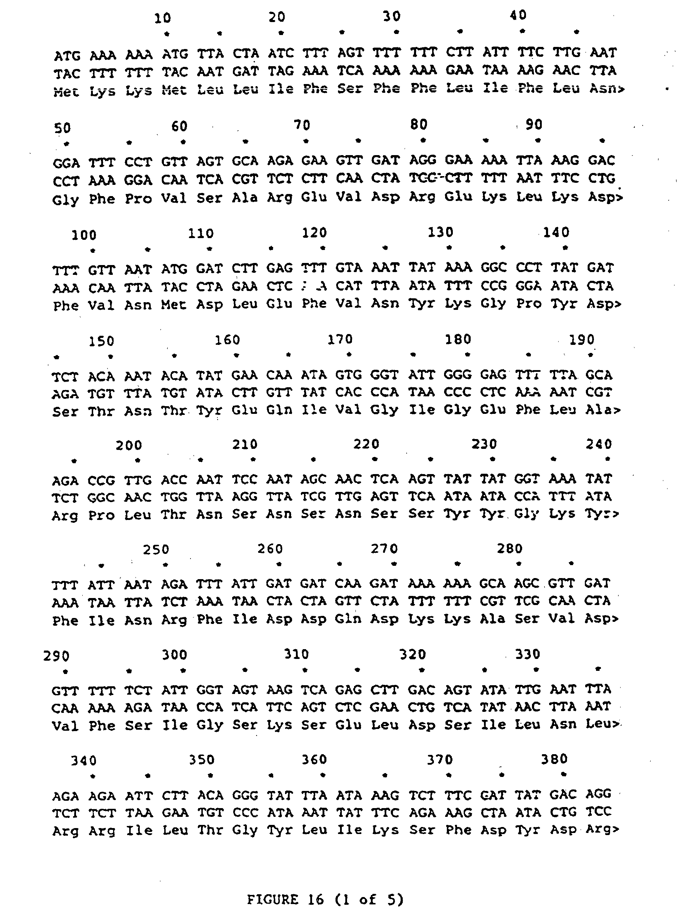

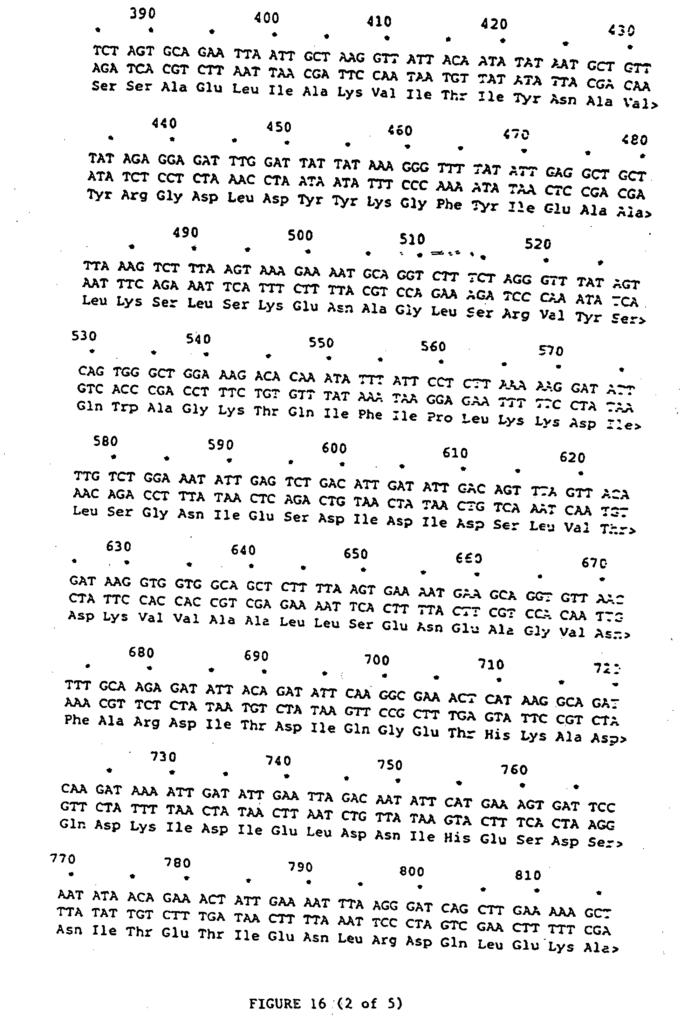

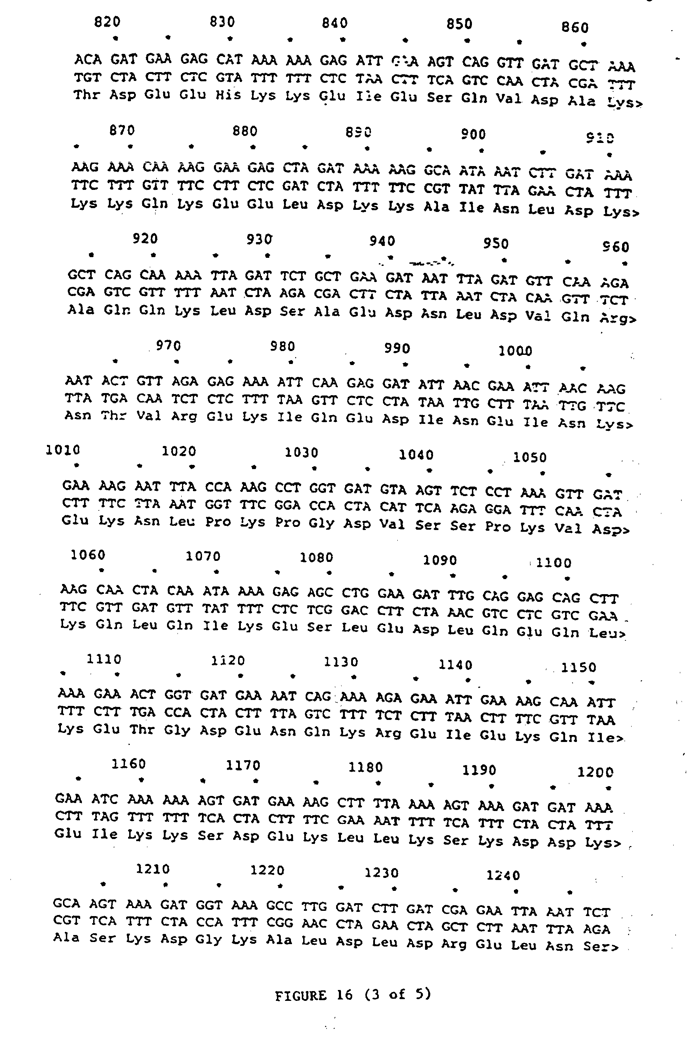

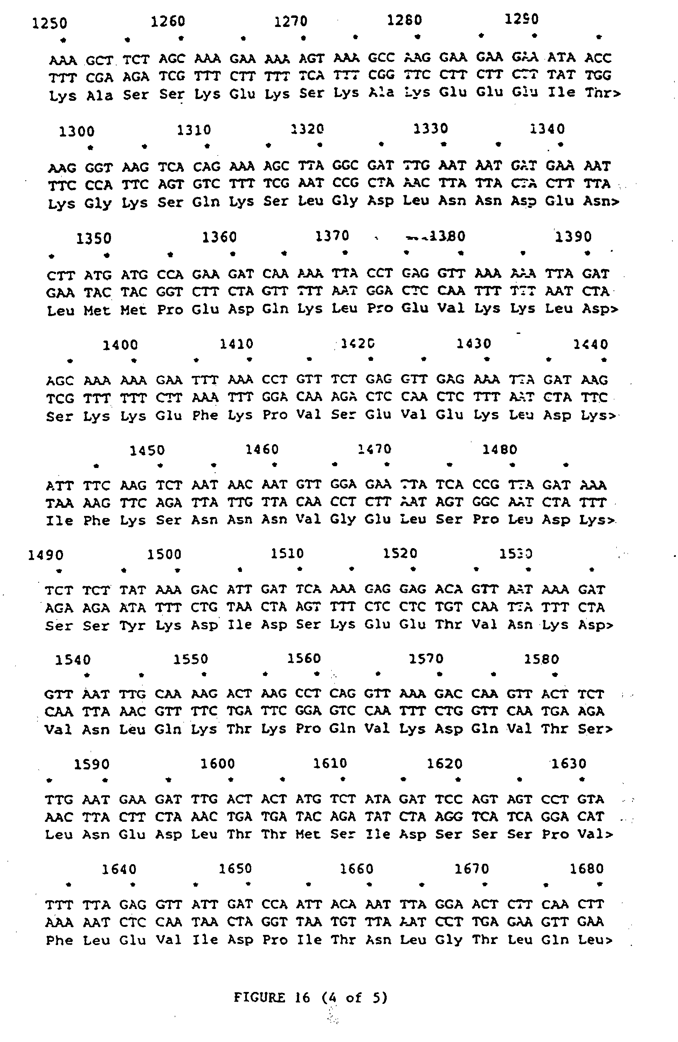

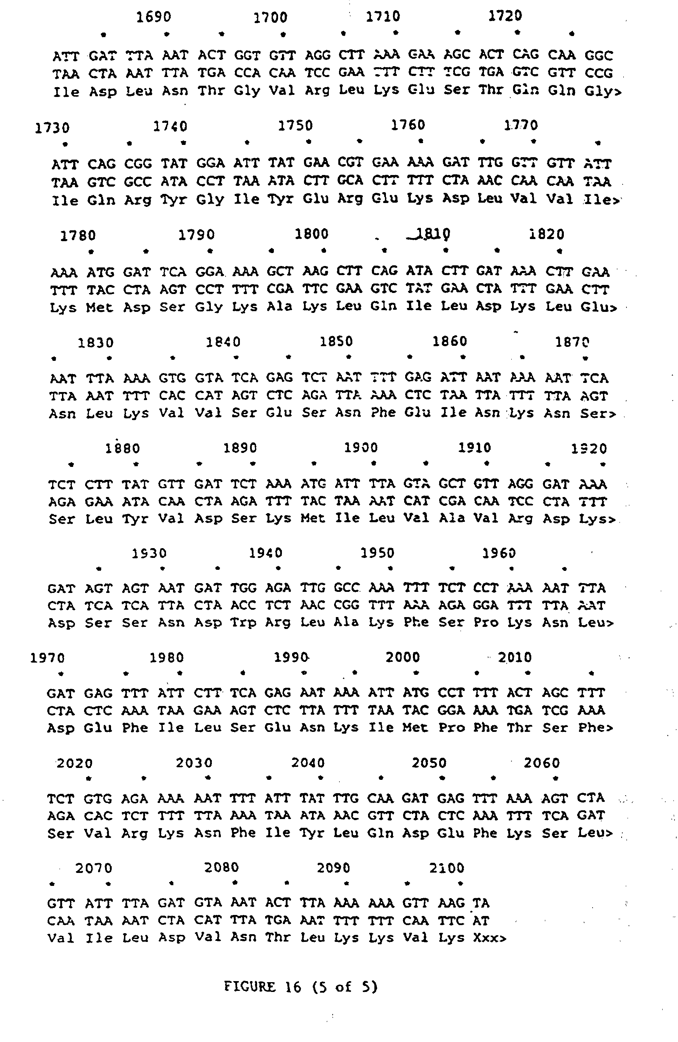

Figure 16 depicts the nucleic acid sequence of p93-B31 (SEQ ID NO. 65) and the encoded protein sequence (SEQ ID NO. 66).

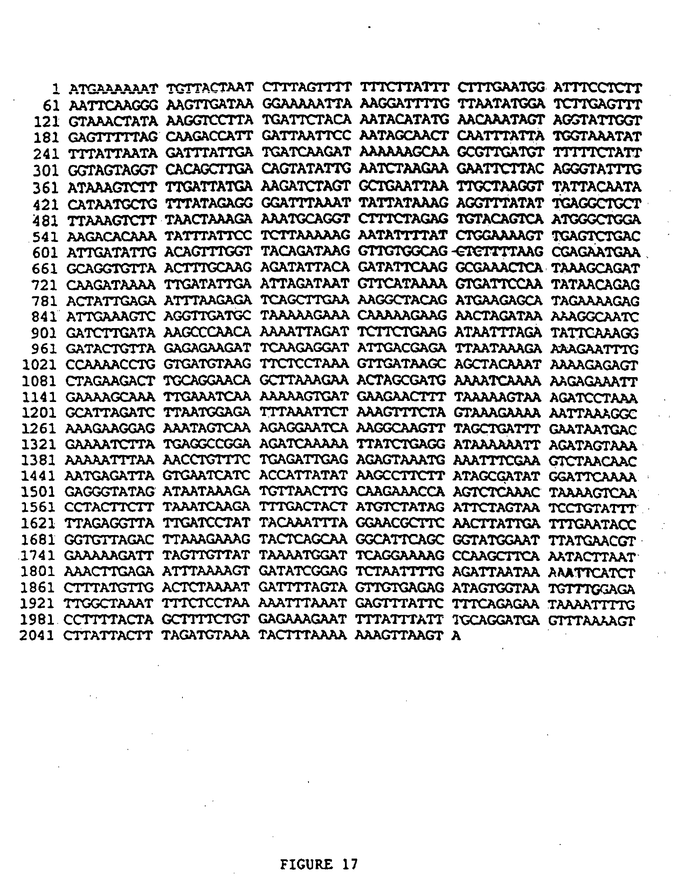

Figure 17 depicts the nucleic acid sequence of p93-K48 (SEQ ID NO. 67).

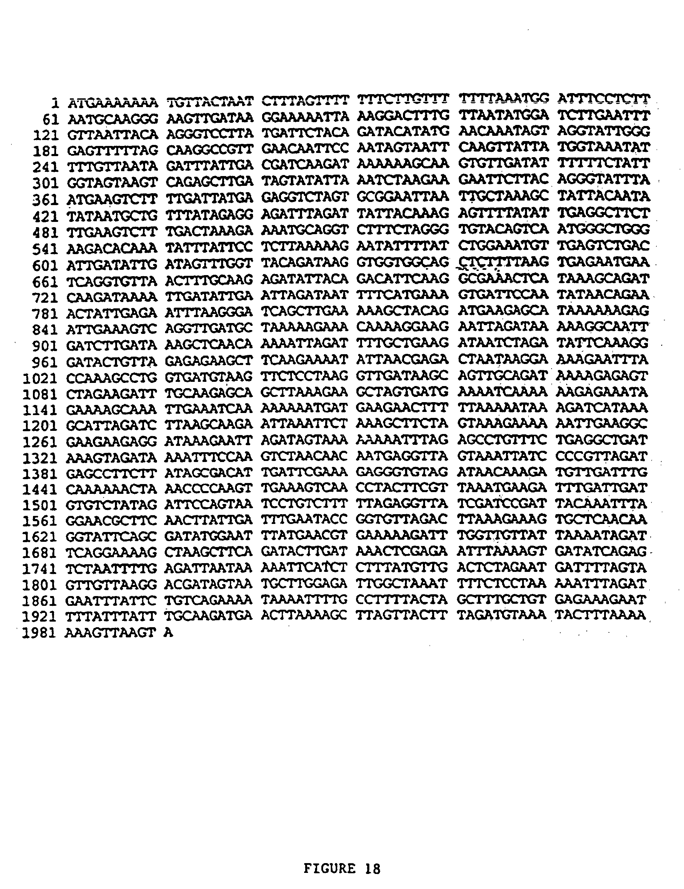

Figure 18 depicts the nucleic acid sequence of p93-PBo (SEQ ID NO. 69).

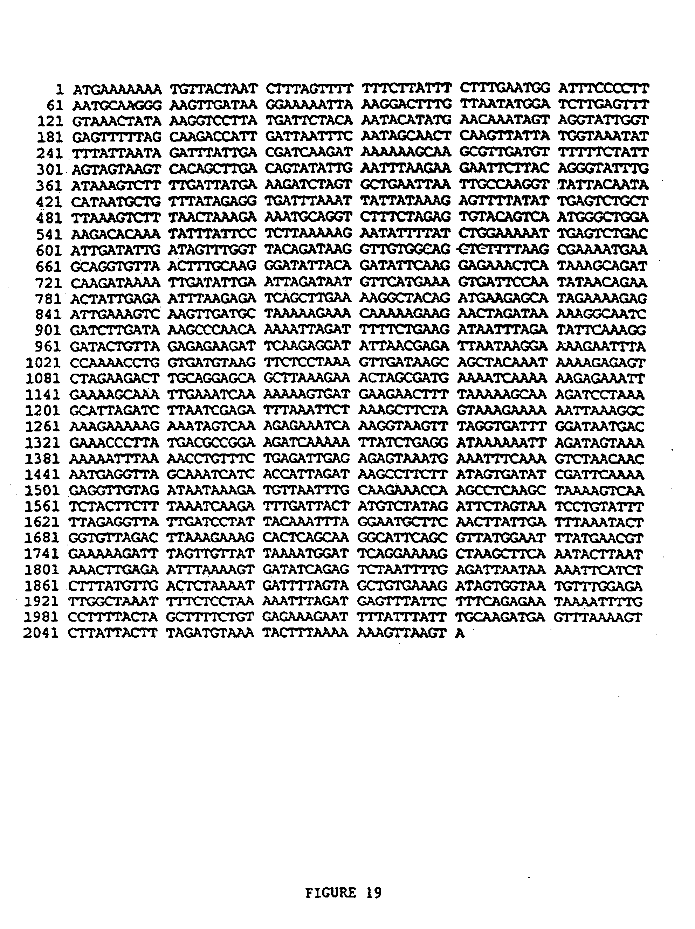

Figure 19 depicts the nucleic acid sequence of p93-pTrob (SEQ ID NO. 71).

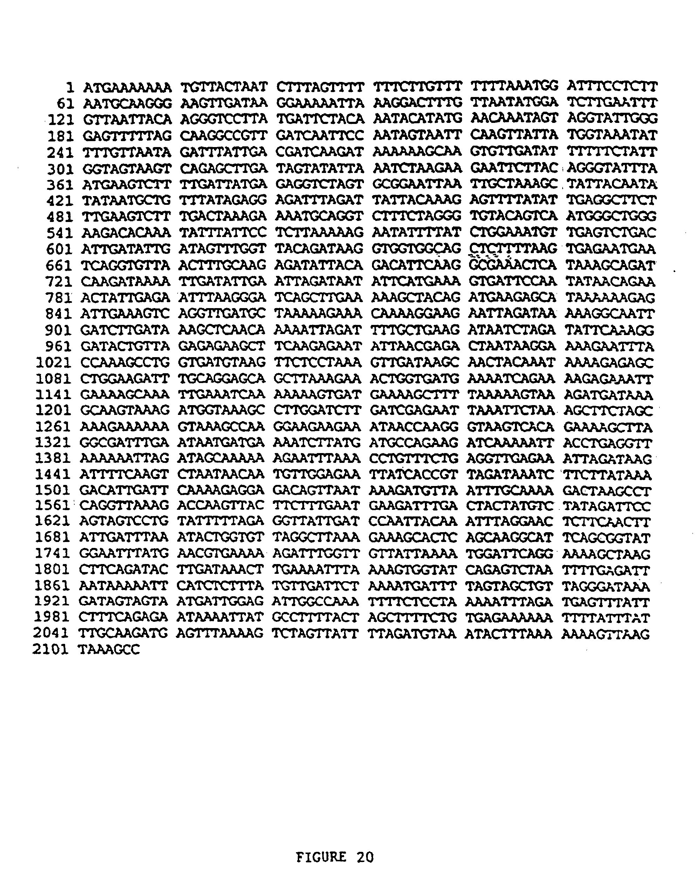

Figure 20 depicts the nucleic acid sequence of p93-pGau (SEQ ID NO. 73).

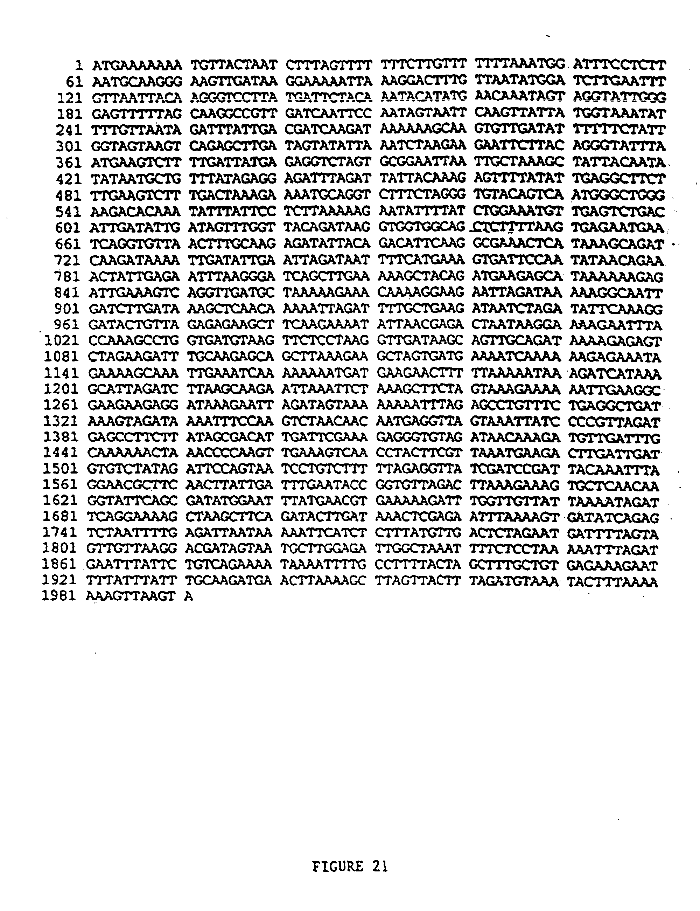

Figure 21 depicts the nucleic acid sequence of p93-25015 (SEQ ID NO. 75).

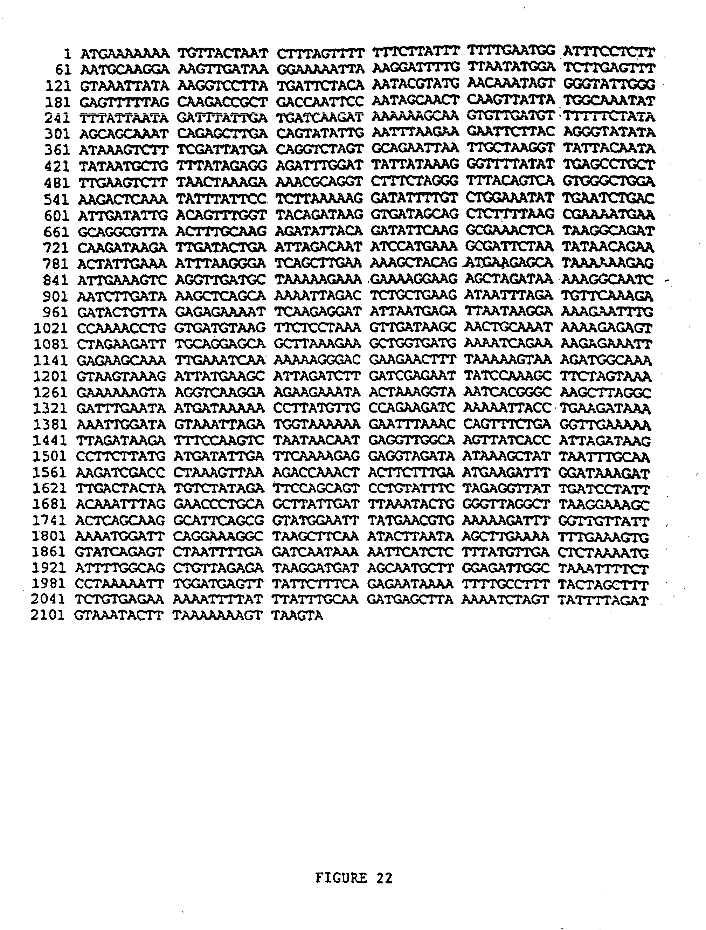

Figure 22 depicts the nucleic acid sequence of p93-pKo (SEQ ID NO. 77).
Figure 23 depicts the nucleic acid sequence of the OspA-K48/OspA-PGau chimer (SEQ ID NO. 85) and the encoded chimeric protein sequence (SEQ ID NO. 86).
Figure 24 depicts the nucleic acid sequence of the OspA-B31/OspA-PGau chimer (SEQ ID NO. 88) and the encoded chimeric protein sequence (SEQ ID NO. 89).
Figure 25 depicts the nucleic acid sequence of the OspA-B31/OspA-K48 chimer (SEQ ID NO. 91) and the encoded chimeric protein sequence (SEQ ID NO. 92).
Figure 26 depicts the nucleic acid sequence of the OspA-B31/OspA-25015 chimer (SEQ ID NO. 94) and the encoded chimeric protein sequence (SEQ ID NO. 95).
Figure 27 depicts the nucleic acid sequence of the OspA-K48/OspA-B31/OspA-K48 chimer (SEQ ID NO. 97) and the encoded chimeric protein sequence (SEQ ID NO. 98).
Figure 28 depicts the nucleic acid sequence of the OspA-B31/OspA-K48/OspA-B31/OspA-K48 chimer (SEQ ID NO. 100) and the encoded chimeric protein sequence (SEQ ID NO. 101).
Figure 29 depicts the nucleic acid sequence of the OspA-B31/OspB-B31 chimer (SEQ ID NO. 103) and the encoded chimeric protein sequence (SEQ ID NO. 104).
Figure 30 depicts the nucleic acid sequence of the OspA-B31/OspB-B31/OspC-B31 chimer (SEQ ID NO. 106) and the encoded chimeric protein sequence (SEQ ID NO. 107).
Figure 31 depicts the nucleic acid sequence of the OspC-B31/OspA-B31/OspB-B31 chimer (SEQ ID NO. 109) and the encoded chimeric protein sequence (SEQ ID NO. 110).
Figure 32 depicts the nucleic acid sequence of the OspA-B31/p93-B31 chimer (SEQ ID NO. 111) and the encoded chimeric protein sequence (SEQ ID NO. 112).
Figure 33 depicts the nucleic acid sequence of the OspB-B31/p41-B31 (122-234) chimer (SEQ ID NO. 113) and the encoded chimeric protein sequence (SEQ ID NO. 114).
Figure 34 depicts the nucleic acid sequence of the OspB-B31/p41-B31 (122-295) chimer (SEQ ID NO. 115) and the encoded chimeric protein sequence (SEQ ID NO. 116).
Figure 35 depicts the nucleic acid sequence of the OspB-B31/p41-B31 (140-234) chimer (SEQ ID NO. 117) and the encoded chimeric protein sequence (SEQ ID NO. 118).
Figure 36 depicts the nucleic acid sequence of the OspB-B31/p41-B31 (140-295) chimer (SEQ ID NO. 119) and the encoded chimeric protein sequence (SEQ ID NO. 120).
Figure 37 depicts the nucleic acid sequence of the OspB-B31/p41-B31 (122-234)/OspC-B31 chimer (SEQ ID NO. 121) and the encoded chimeric protein sequence (SEQ ID NO. 122).
Figure 38 depicts an alignment of the nucleic acid sequences for OspC-B31 (SEQ ID NO. 29), OspC-PKo (SEQ ID NO. 33), OspC-pTrob (SEQ ID NO. 35), and OspC-K48 (SEQ ID NO. 31). Nucleic acids which are identical to those in the lead nucleic acid sequence (here, OspC-B31) are represented by a period (.); differing nucleic acids are shown in lower case letters.
Figure 39 depicts an alignment of the nucleic acid sequences for OspD-pBO (SEQ ID NO. 123), OspD-PGau (SEq ID NO. 124), OspD-DK29 (SEQ ID NO. 125), and OspD-K48 (SEQ ID NO. 126). Nucleic acids which are identical to those in the lead nucleic acid sequence (here, OspD-pBo) are represented by a period (.); differing nucleic acids are shown in lower case letters.

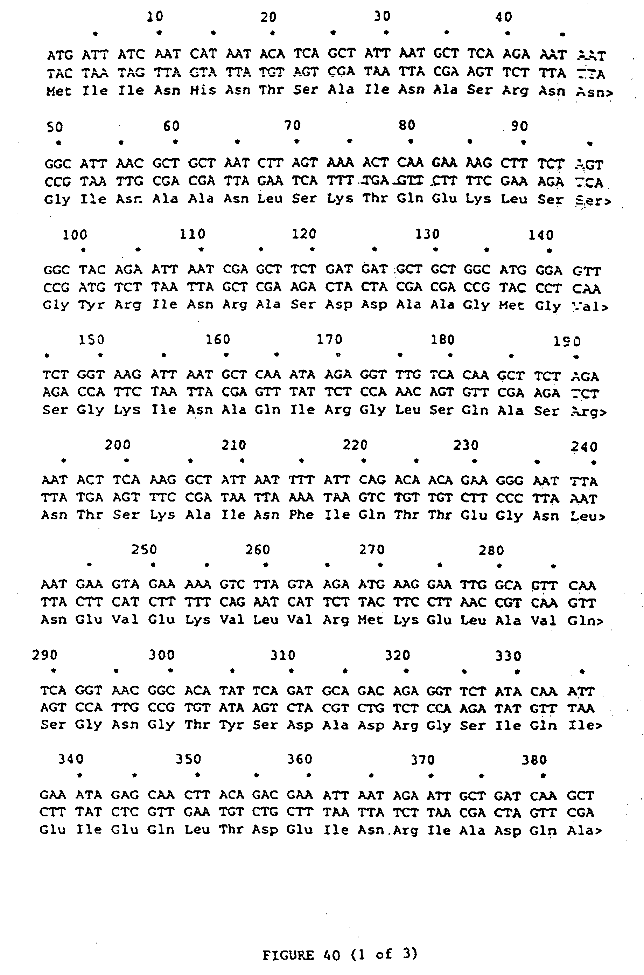

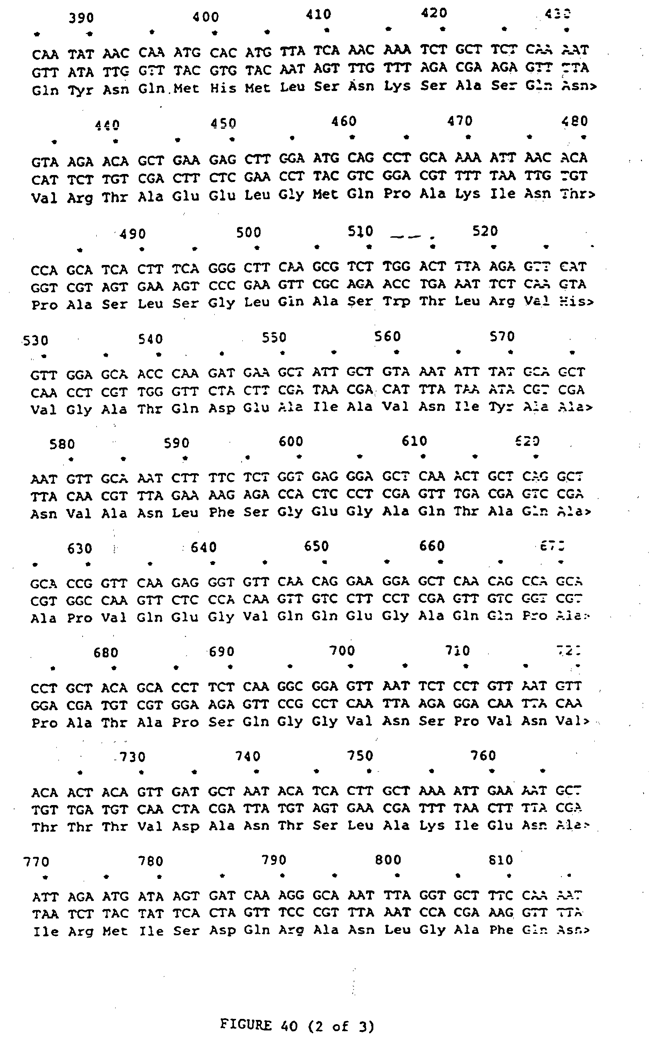

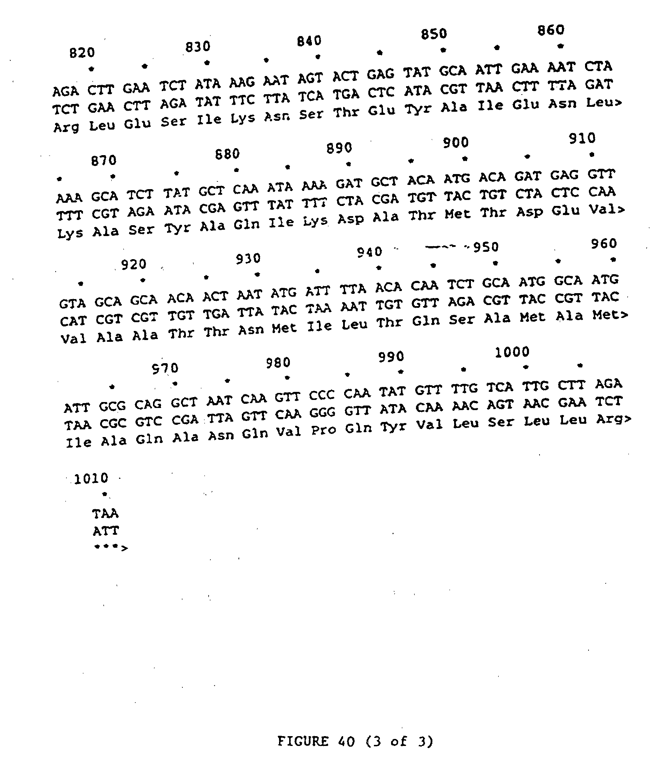

Figure 40 depicts the nucleic acid sequence of p41-B31 (SEq ID NO. 127) and then encoded protein sequence (SEQ ID NO. 128).
Figure 41 depicts an alignment of the nucleic acid sequences for p41-B31 (SEQ ID NO. 127), p41-pKa1 (SEQ ID NO. 129), p41-PGau (SEQ ID NO. 51), p41-PBo (SEQ ID NO. 130), p41-DK29 (SEQ ID NO. 53), and p41-PKo (SEQ ID NO. 131). Nucleic acids which are identical to those in the lead nucleic acid sequence (here, p41-B31) are represented by a period (.); differing nucleic acids are shown in lower case letters.

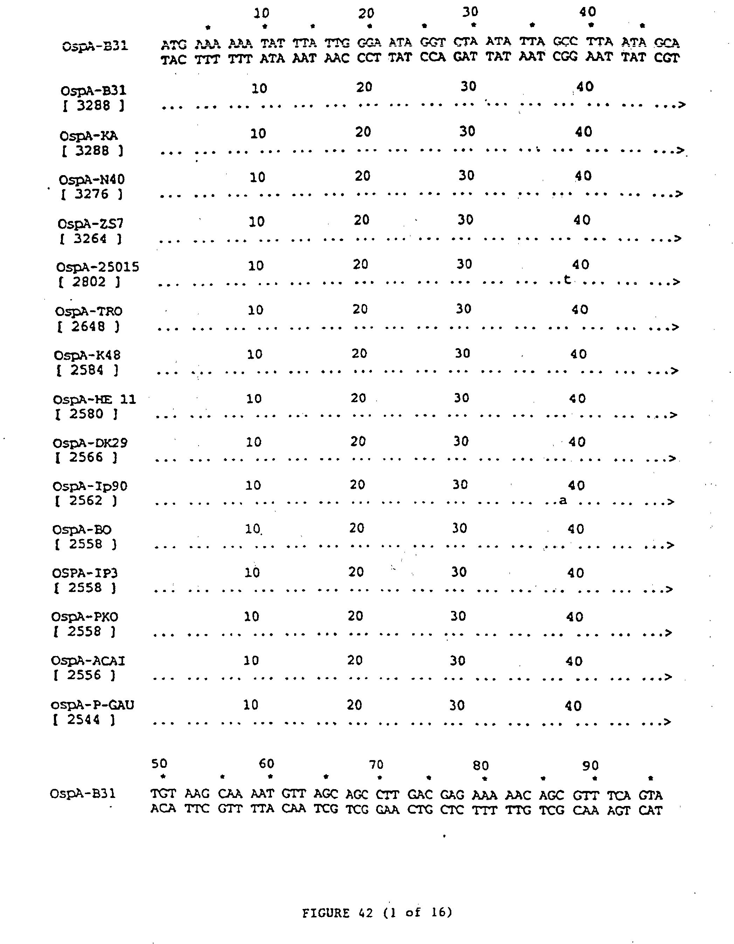

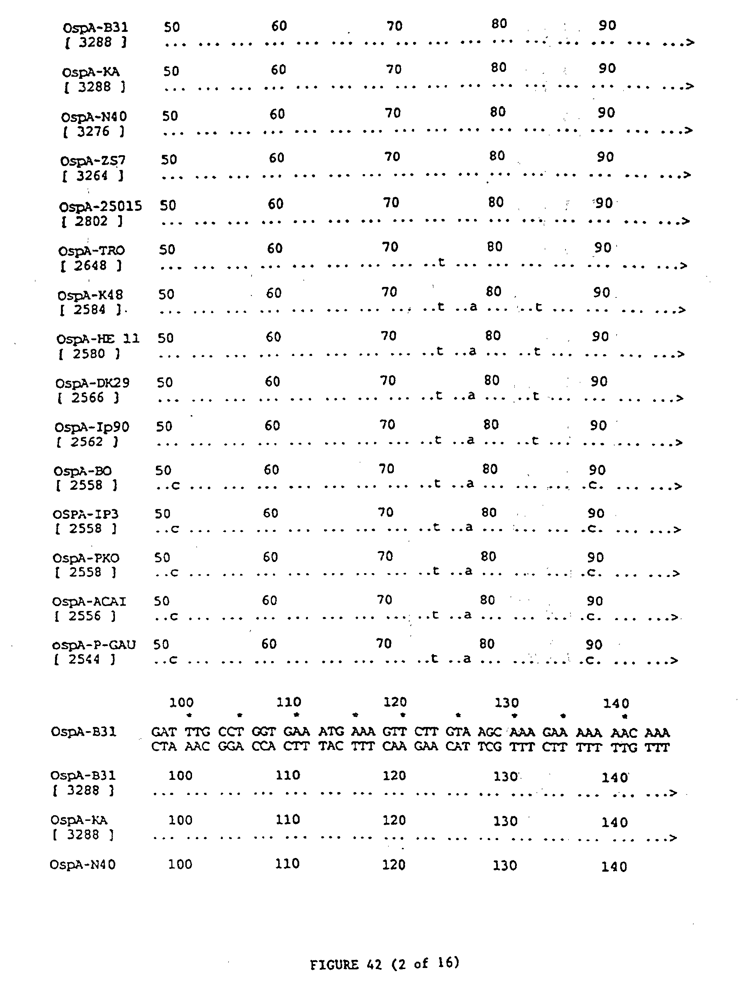

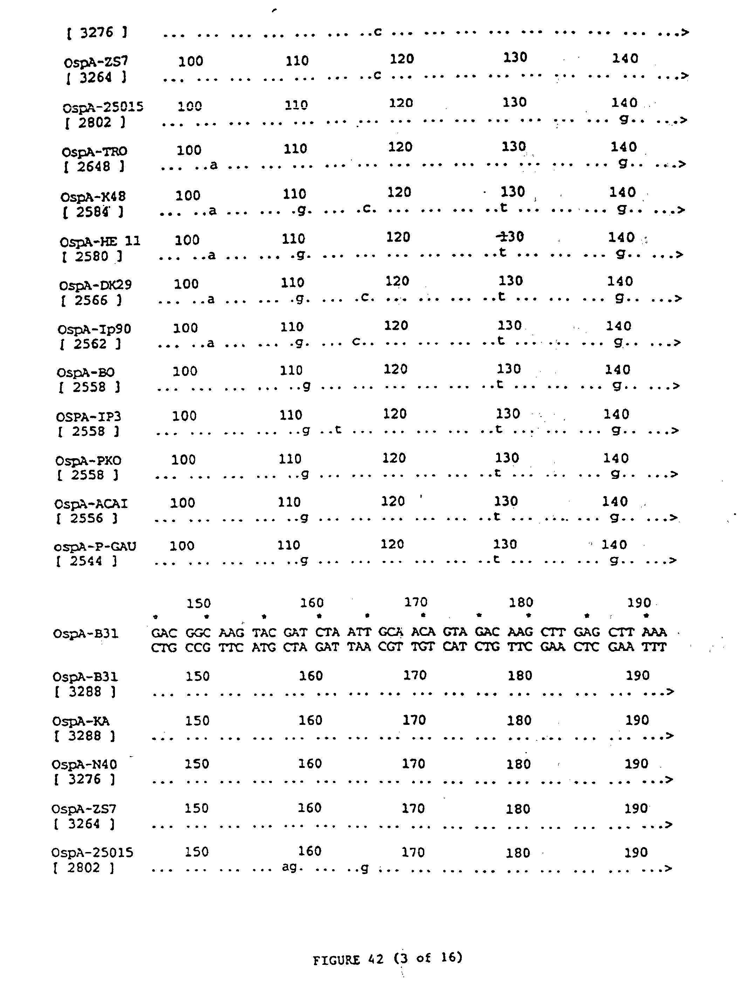

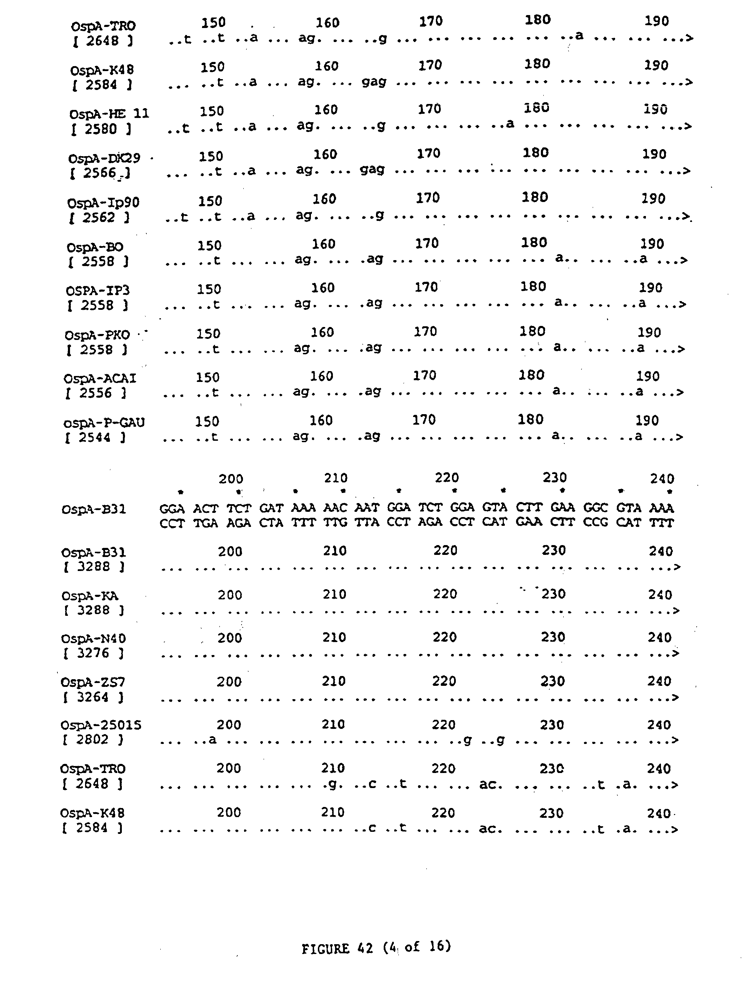

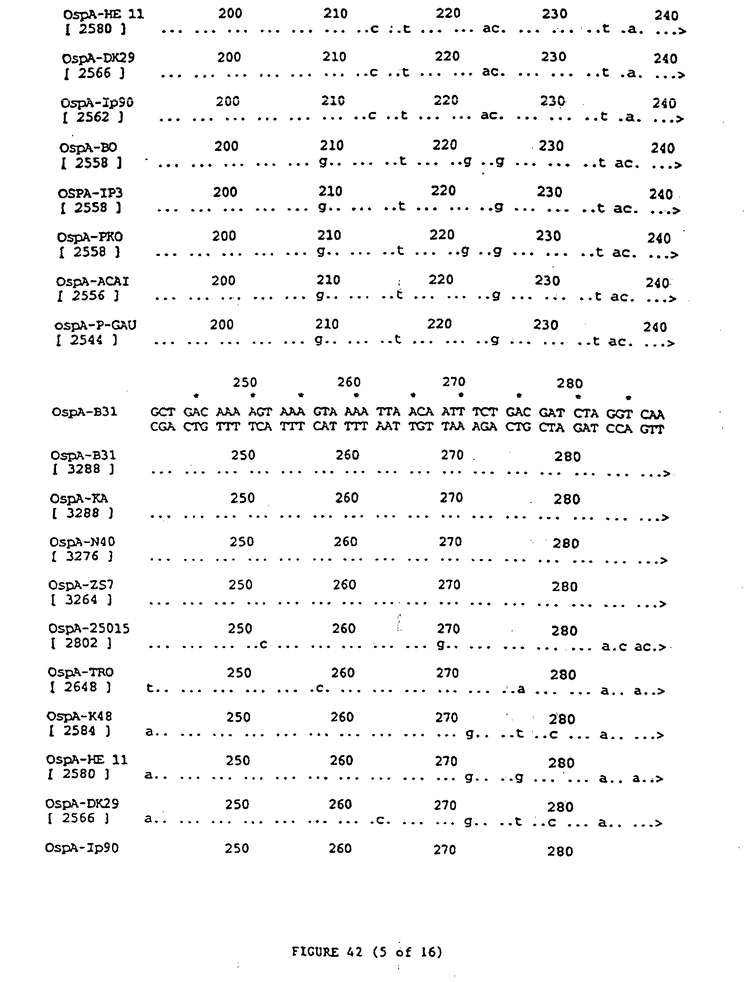

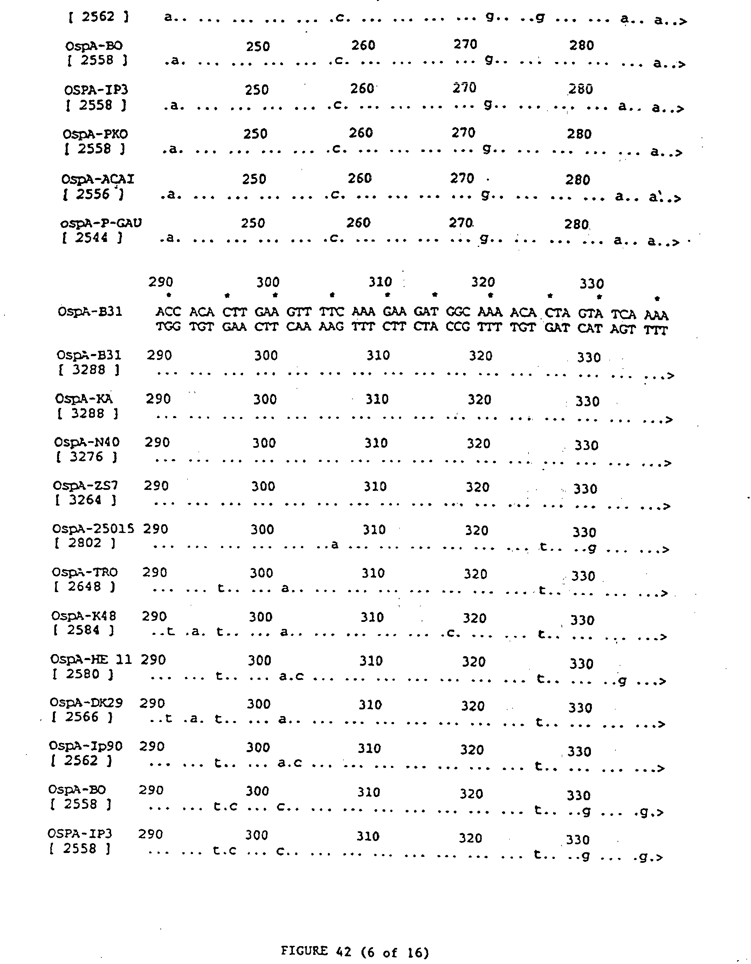

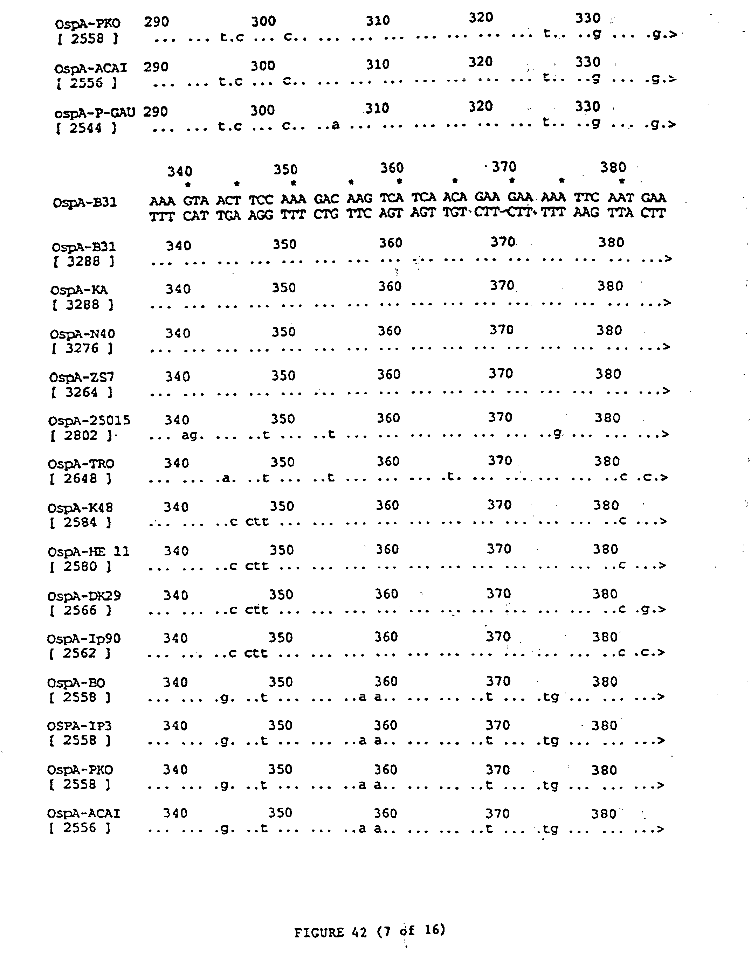

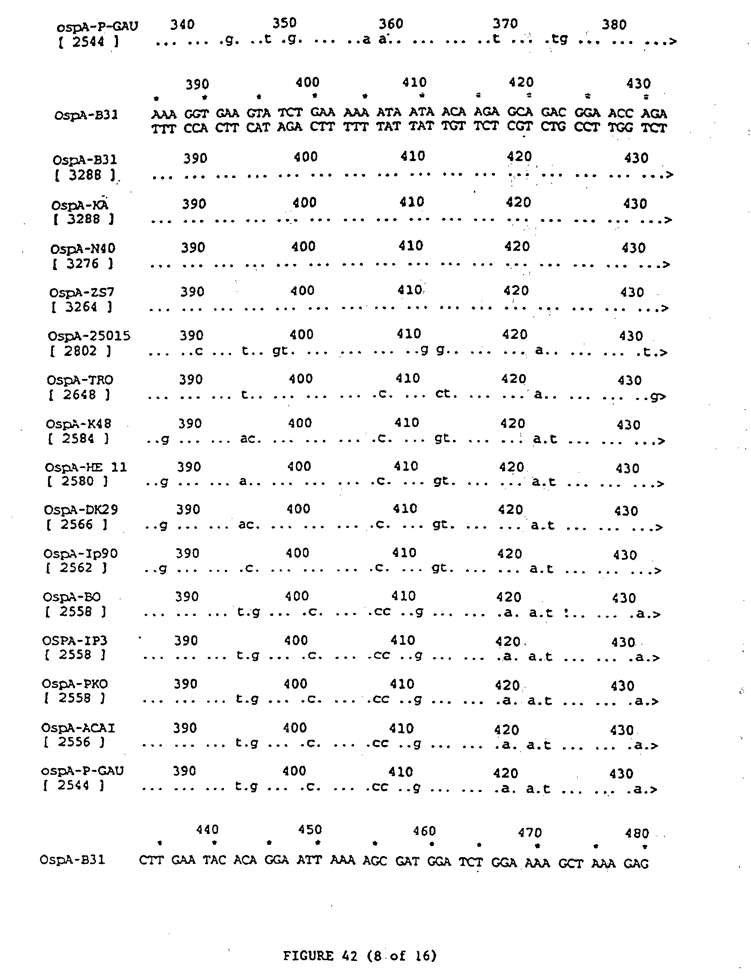

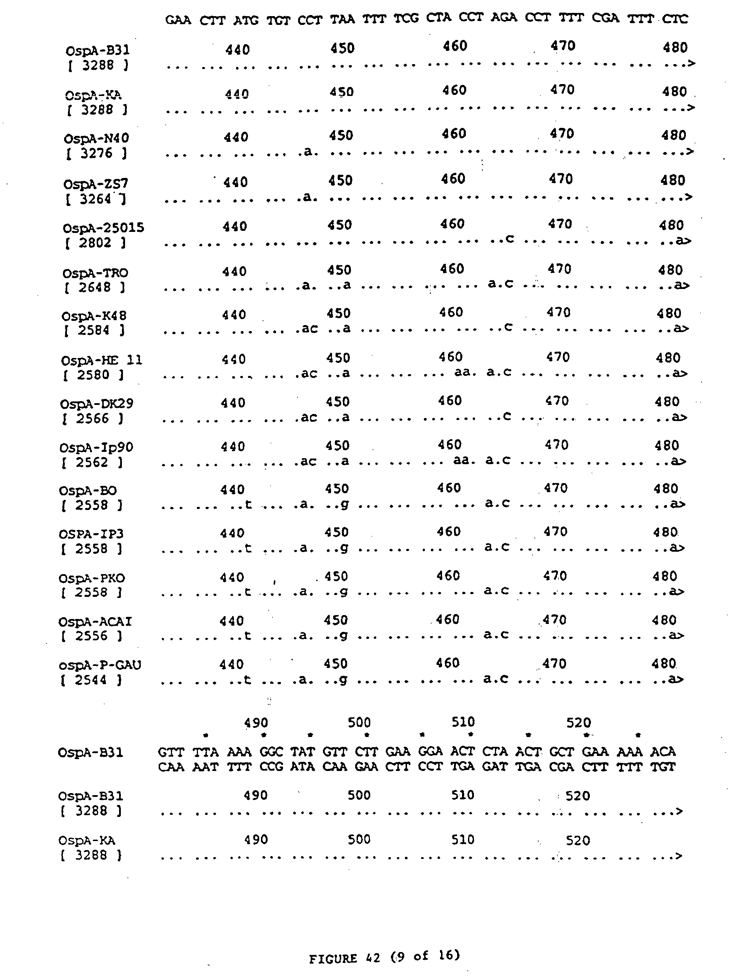

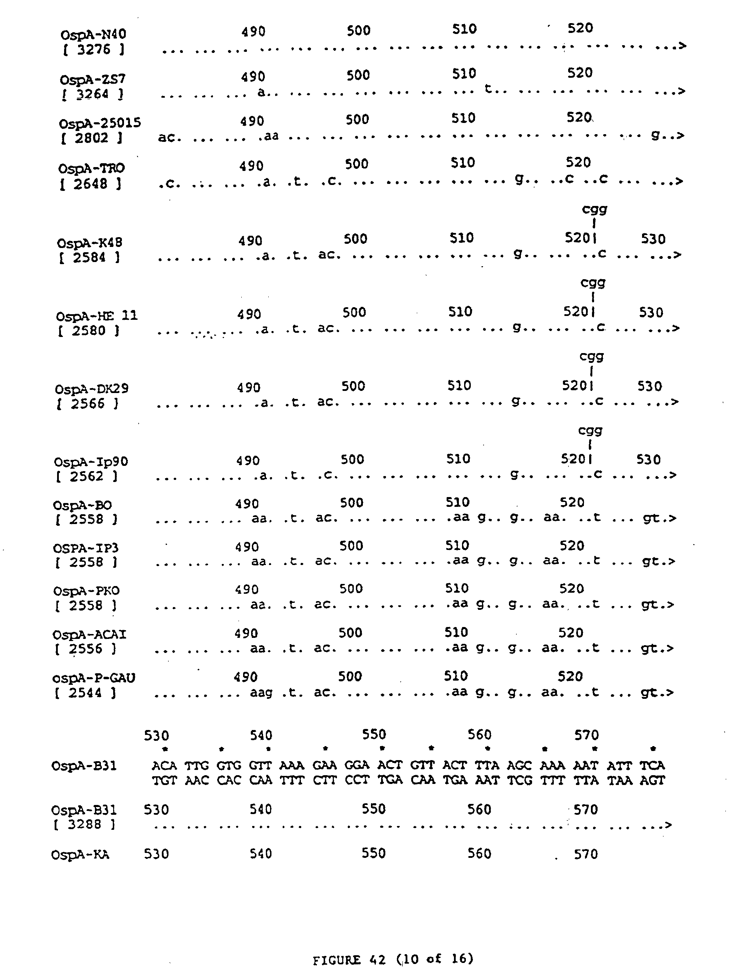

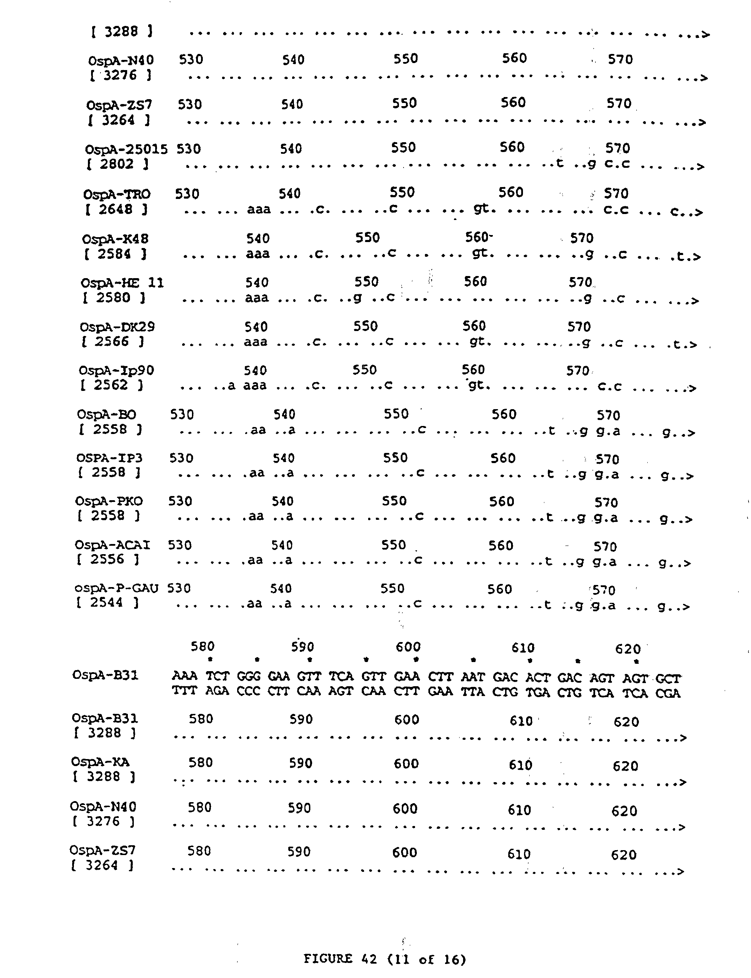

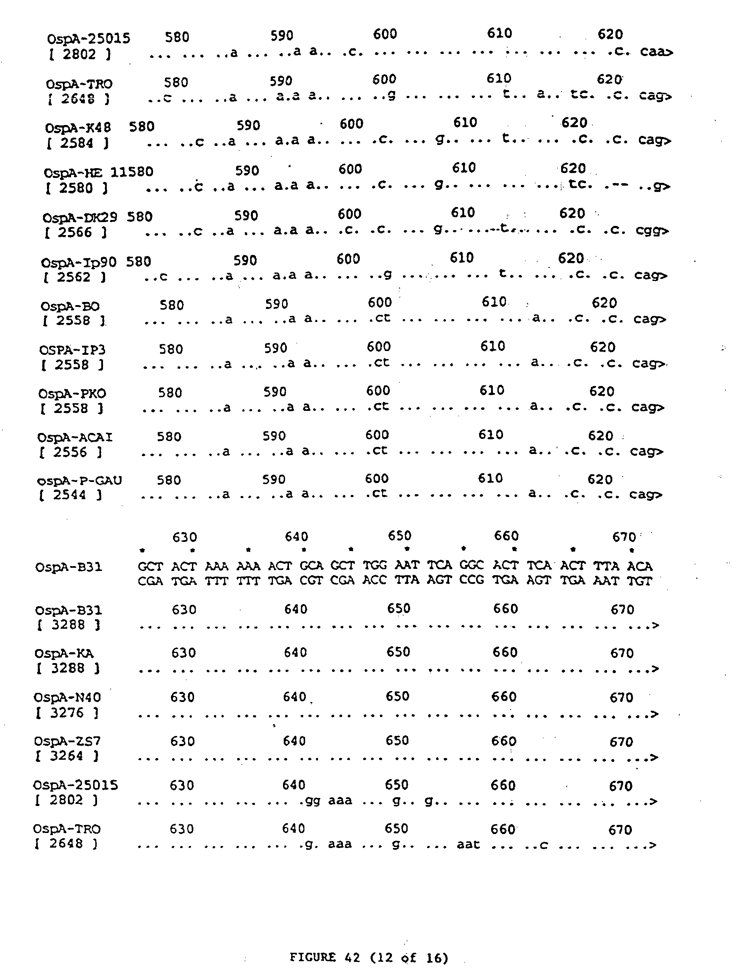

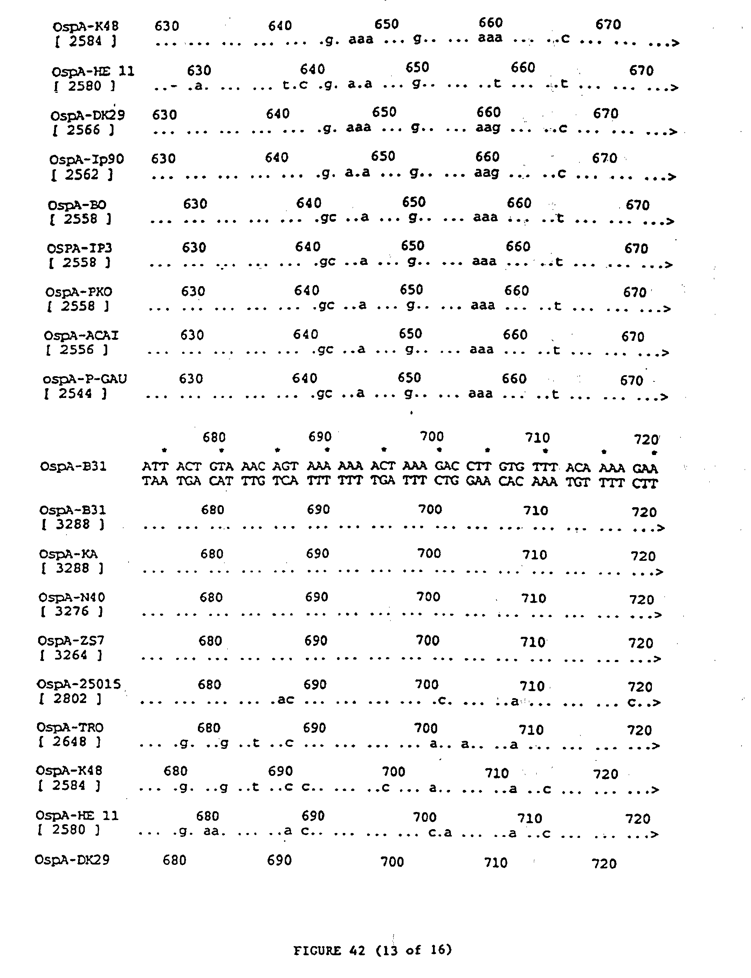

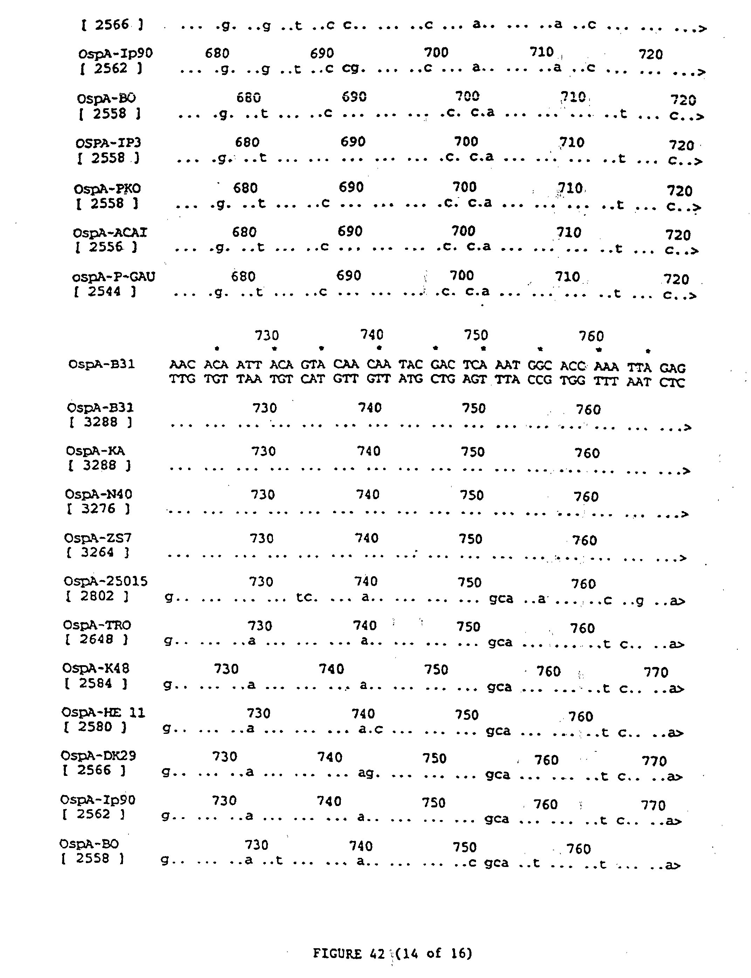

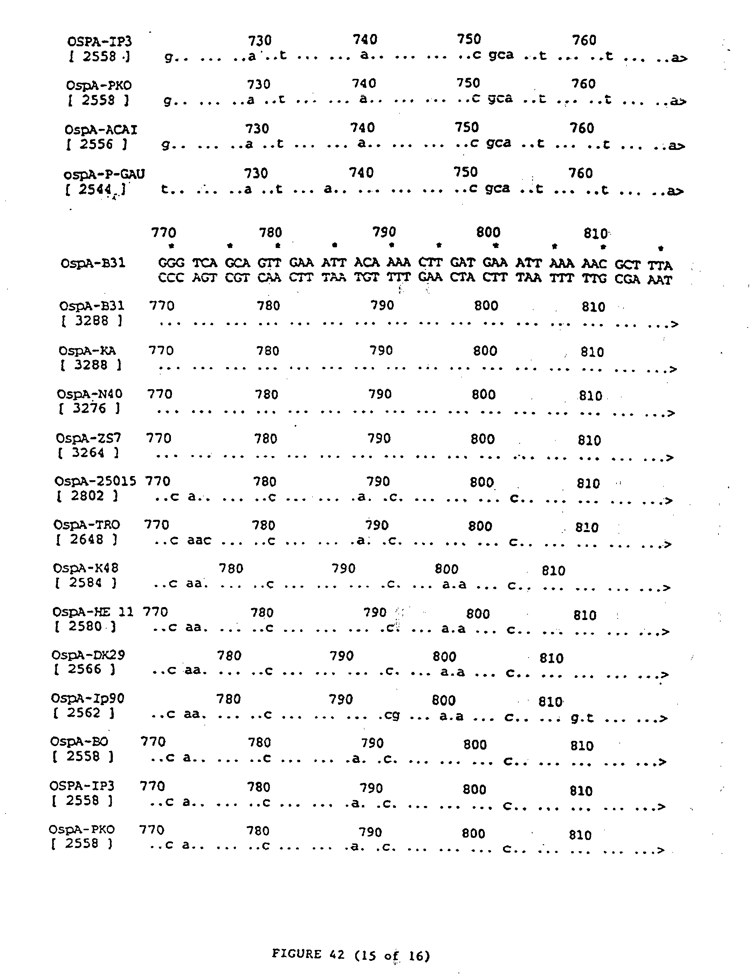

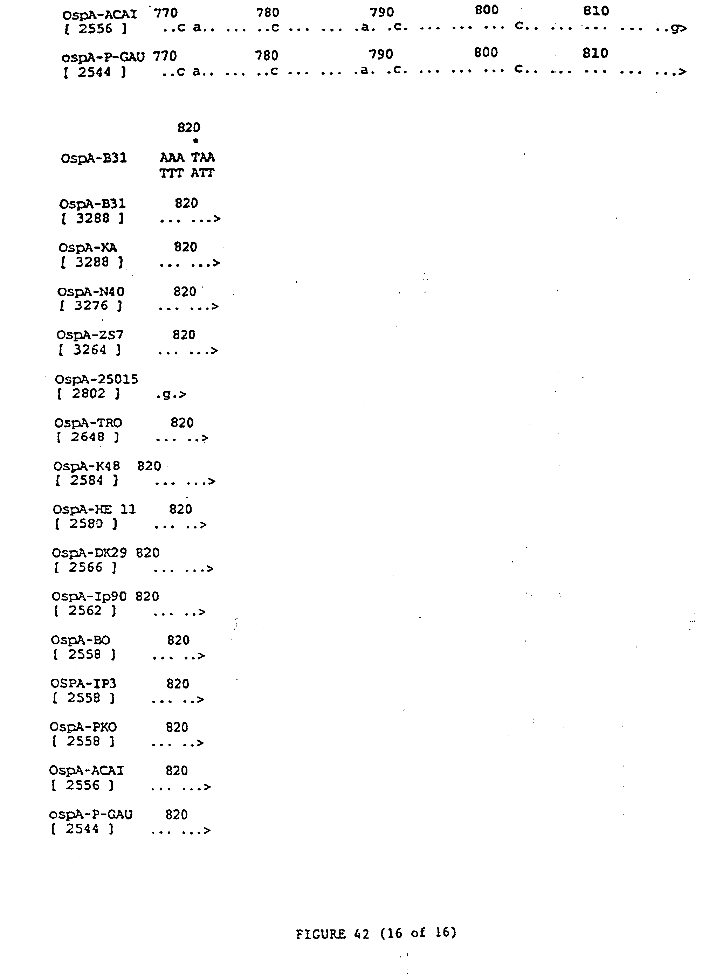

Figure 42 depicts an alignment of the nucleic acid sequences for OspA-B31 (SEQ ID NO. 6), OspA-pKa1 (SEQ ID NO. 132), OspA-N40 (SEQ ID NO. 133), OspA-ZS7 (SEQ ID NO. 134), OspA-25015 (SEQ ID NO. 12), OspA-pTrob (SEQ ID NO. 135), OspA-K48 (SEQ ID NO. 8), OspA-Hei (SEQ ID NO. 136), OspA-DK29 (SEQ ID NO. 49), OSpA-Ip90 (SEQ ID NO. 50), OspA-pBo (Seq ID NO. 55), OspA-Ip3 (SEQ ID NO. 56), OspA-PKo (SEQ ID NO. 57), OspA-ACAI (SEQ ID NO. 58), and OspA-PGau (SEQ ID NO. 10). Nucleic acids which are identical to those in the lead nucleic acid sequence (here, OspA-B31) are represented by a period (.); differing nucleic acids are shown in lower case letters.
Figure 43 depicts the nucleic acid sequence of the OspA-Tro/OspA-Bo chimer (SEQ ID NO. 137) and the encoded chimeric protein sequence (SEQ ID NO. 138).
Figure 44 depicts the nucleic acid sequence of the OspA-PGau/OspA-Bo chimer (SEQ ID NO. 139) and the encoded chimeric protein sequence (SEQ ID NO. 140).
Figure 45 depicts the nucleic acid sequence of the OspA-B31/OspA-PGau/OspA-B31/OspA-K48 chimer (SEQ ID NO. 141) and the encoded chimeric protein sequence (SEQ ID NO. 142).
Figure 46 depicts the nucleic acid sequence of the OspA-PGau/OspA-B31/OspA-K48 chimer (SEQ ID NO. 143) and the encoded chimeric protein sequence (SEQ ID NO. 144).

### Detailed Description of the Invention

The current invention pertains to chimeric proteins comprising antigenic *Borrelia* polypeptides which do not occur in nature in the same Borrelia protein. The chimeric proteins are a combination of two or more antigenic polypeptides derived from *Borrelia* proteins. The antigenic polypeptides can be derived from different proteins from the same species of *Borrelia*, or different proteins from different Borrelia species, as well as from corresponding proteins from different species. As used herein, the term "chimeric protein" describes a protein comprising two or more polypeptides which are derived from corresponding and/or non-corresponding native *Borrelia* protein. A polypeptide "derived from" a native *Borrelia* protein is a polypeptide which has an amino acid sequence the same as an amino acid sequence present in a *Borrelia* protein, an amino acid sequence equivalent to the amino acid sequence of a naturally occurring *Borrelia* protein, or an amino acid sequence substantially similar to the amino acid sequence of a naturally occurring Borrelia protein (e.g., differing by few amino acids) such as when a nucleic acid encoding a protein is subjected to site-directed mutagenesis. "Corresponding" proteins are equivalent proteins from different species or strains of *Borrelia*, such as outer surface protein A (OspA) from strain B31 and OspA from strain K48. The invention additionally pertains to nucleic acids encoding these chimeric proteins.

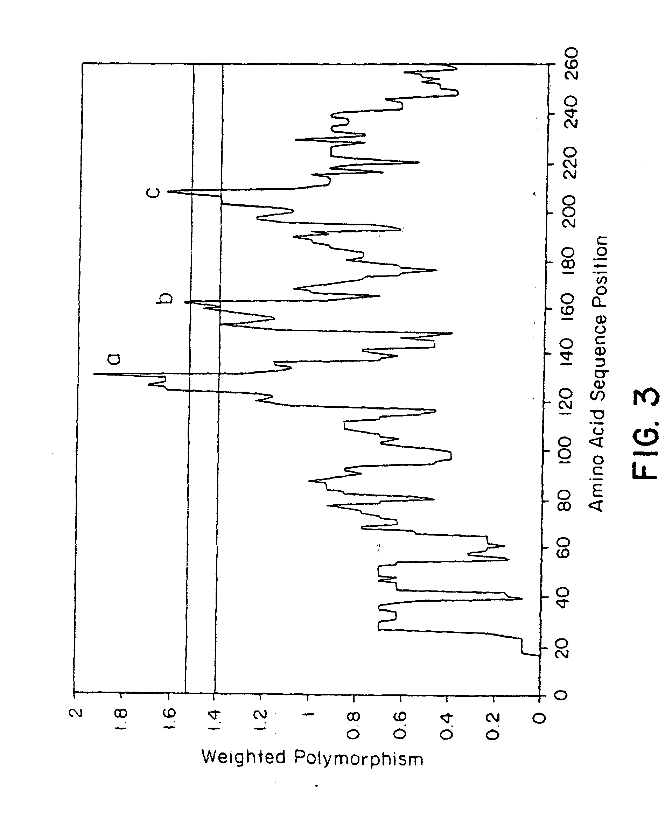

As described below, Applicants have identified two separate antigenic domains of OspA and OspB which flank the sole conserved tryptophan present in OspA and in OspB. These domains share cross-reactivity with different genospecies of Borrelia. The precise amino acids responsible for antigenic variability were determined through site-directed mutagenesis, so that proteins with specific amino acid substitutions are available for the development of chimeric proteins. Furthermore, Applicants have identified immunologically important hypervariable domains in OspA proteins, as described below in Example 2. The first hypervariable domain of interest for chimeric proteins, Domain A, includes amino acid residues 120-140 of OspA, the second hypervariable domain, Domain B, includes residues 150-180 and the third hypervariable domain, Domain C, includes residues 200-216 or 217 (depending on the position of the sole conserved tryptophan residue in the OspA of that particular species of Borrelia) (see Figure 3). In addition, Applicants have sequenced the genes for several *Borrelia* proteins.

These discoveries have aided in the development of novel recombinant *Borrelia* proteins which include two or more amino acid regions or sequences which do not occur in the same *Borrelia* protein in nature. The recombinant proteins comprise polypeptides from a variety of *Borrelia* proteins, including, but not limited to, OspA, OspB, OspC, OspD, p12, p39, p41, p66, and p93. Antigenically relevant polypeptides from each of a number of proteins are combined into a single chimeric protein.

In one embodiment of the current invention, chimers are now available which include antigenic polypeptides flanking a tryptophan residue. The antigenic polypeptides are derived from either the proximal portion from the tryptophan (the portion of the OspA or OspB protein present between the amino terminus and the conserved tryptophan of the protein), or the distal portion from the tryptophan (the portion of the OspA or OspB protein present between the conserved tryptophan of the protein and the carboxy terminus) in OspA and/or OspB. The resultant chimers can be OspA-OspA chimers (i.e., chimers incorporating polypeptides derived from OspA from different strains of *Borrelia*), OspA-OspB chimers, or OspB-OspB chimers, and are constructed such that amino acid residues amino-proximal to an invariant tryptophan are from one protein and residues carboxy-proximal to the invariant tryptophan are from the other protein. For example, one available chimer consists of a polypeptide derived from the amino-proximal region of OspA from strain B31, followed by the tryptophan residue, followed by a polypeptide derived from the carboxy-proximal region of OspA from strain K48 (SEQ ID NO. 92). Another available chimer includes a polypeptide derived from the amino-proximal region of OspA from strain B31, and a polypeptide derived from the carboxy-proximal region of OspB from strain B31 (SEQ ID NO. 104). If the polypeptide proximal to the tryptophan of these chimeric proteins is derived from OspA, the proximal polypeptide can be further subdivided into the three hypervariable domains (Domains A, B, and C), each of which can be derived from OspA from a different strain of *Borrelia.* These chimeric proteins can further comprise antigenic polypeptides from another protein, in addition to the antigenic polypeptides flanking the tryptophan residue.

In another embodiment of the current invention, chimeric proteins are available which incorporate antigenic domains of two or more *Borrelia* proteins, such as Osp proteins (Osp A, B, C and/or D) as well as p12, p39, p41, p66, and/or p93.

The chimers described herein can be produced so that they are highly soluble, hyper-produced in *E*. *coli*, and non-lipidated. In addition, the chimeric proteins can be designed to end in an affinity tag (His-tag) to facilitate purification. The recombinant proteins described herein have been constructed to maintain high levels of antigenicity. In addition, recombinant proteins specific for the various genospecies of *Borrelia* that cause Lyme disease are now available, because the genes from each of the major genospecies have been sequenced; the sequences are set forth below. These recombinant proteins with their novel biophysical and antigenic properties will be important diagnostic reagent and vaccine candidates.

The chimeric proteins of the current invention are advantageous in that they retain specific reactivity to monoclonal and polyclonal antibodies against wild-type *Borrelia* proteins, are immunogenic, and inhibit the growth or induce lysis of *Borrelia in vitro.* Furthermore, in some embodiments, the proteins provide antigenic domains of two or more *Borrelia* strains and/or proteins within a single protein. Such proteins are particularly useful in immuno-diagostic assays. For example, proteins of the present invention can be used as reagents in assays to detect the presence of antibodies to native *Borrelia* in potentially infected individuals. These proteins can also be used as immunodiagnostic reagents, such as in dot blots, Western blots, enzyme linked immunosorbed assays, or agglutination assays. The chimeric proteins of the present invention can be produced by known techniques, such as by recombinant methodology, polymerase chain reaction, or mutagenesis.

Furthermore, the proteins of the current invention are useful as vaccine immunogens against Borrelia infection. Because Borrelia has been shown to be clonal, a protein comprising antigenic polypeptides from a variety of Borrelia proteins and/or species, will provide immunoprotection for a considerable time when used in a vaccine. The lack of significant intragenic recombination, a process which might rapidly generate novel epitopes with changed antigenic properties, ensures that Borrelia can only change antigenic type by accumulating mutational change, which is slow when compared with recombination in generating different antigenic types. The chimeric protein can be combined with a physiologically acceptable carrier and administered to a vertebrate animal through standard methods (e.g., intravenously or intramuscularly, for example).

The current invention is illustrated by the following Examples, which are not to be construed to be limiting in any way.

### Example 1. Purification of Borrelia burgorferi Outer Surface Protein A and Analysis of Antibody Binding Domains

This example details a method for the purification of large amounts of native outer surface protein A (OspA) to homogeneity, and describes mapping of the antigenic specificities of several anti-OspA MAbs. OspA was purified to homogeneity by exploiting its resistance to trypsin digestion. Intrinsic labeling with ¹⁴C-palmitic acid confirmed that OspA was lipidated, and partial digestion established lipidation at the amino-terminal cysteine of the molecule.

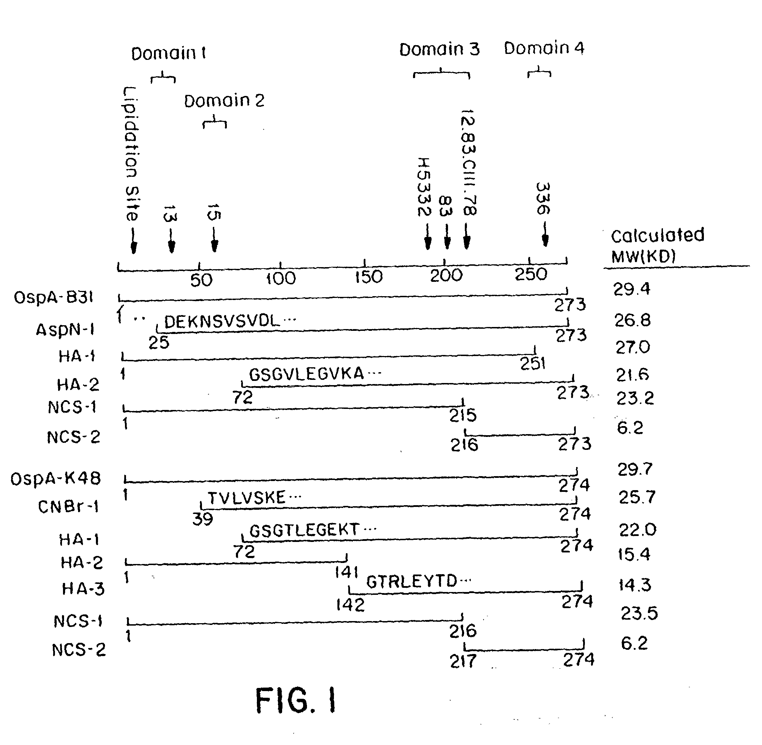

The reactivity of seven anti-OspA murine monoclonal antibodies to nine different *Borrelia* isolates was ascertained by Western blot analysis. Purified OspA was fragmented by enzymatic or chemical cleavage, and the monoclonal antibodies were able to define four distinct immunogenic domains (see Figure 1). Domain 3, which included residues 190-220 of OspA, was reactive with protective antibodies known to agglutinate the organism *in vitro*, and included distinct specificities, some of which were not restricted to a genotype of *B*. *burgdorferi*.

### A. Purification of Native OspA

Detergent solubilization of *B*. *burgdorferi* strips the outer surface proteins and yields partially-purified preparations containing both OspA and outer surface protein B (Osp B) (Barbour, A.G. *et al.,* Infect. Immun. 52 (5): 549-554 (1986); Coleman, J.L. and J.L. Benach, J Infect. Dis. 155 (4): 756-765 (1987); Cunningham, T.M. et al., Ann. NY Acad. Sci. 539: 376-378 (1988); Brandt, M.E. *et al.,* Infect. Immun. 58: 983-991 (1990); Sambri, V. and R. Cevenini, Microbiol. 14:307-314 (1991)). Although both OspA and OspB are sensitive to proteinase K digestion, in contrast to OspB, OspA is resistant to cleavage by trypsin (Dunn, J. *et al.,* Prot. Exp. Purif. 1: 159-168 (1990); Barbour, A.G. *et al.,* Infect. Immun. 45:94-100 (1984)). The relative insensitivity to trypsin is surprising in view of the fact that Osp A has a high (16% for B31) lysine content, and may relate to the relative configuration of Osp A and B in the outer membrane.

### Intrinsic Radiolabeling of Borrelia

Labeling for lipoproteins was performed as described by Brandt *et al*. (Infect. Immun. 58:983-991 (1990)). ¹⁴C-palmitic acid (ICN, Irvine, California) was added to the BSK II media to a final concentration of 0.5 µCi per milliliter (ml). Organisms were cultured at 34°C in this medium until a density of 10⁸ cells per ml was achieved.

### Purification of OspA Protein from Borrelia Strain B31

*Borrelia burgdorferi,* either ¹⁴C-palmitic acid-labeled or unlabeled, were harvested and washed as described (Brandt, M.E. *et al.,* Infect. Immun. 58:983-991 (1990)). Whole organisms were trypsinized according to the protocol of Barbour et al. (Infect. Immun. 45:94-100 (1984)) with some modifications. The pellet was suspended in phosphate buffered saline (PBS, 10mM, pH 7.2), containing 0.8% tosyl-L-phenylalanine chloromethyl ketone (TPCK)-treated trypsin (Sigma, St. Louis, Missouri), the latter at a ratio of 1 µg per 10⁸ cells. Reaction was carried out at 25°C for 1 hour, following which the cells were centrifuged. The pellet was washed in PBS with 100 µg/ml phenylmethylsulfonyl fluoride (PMSF). Triton X-114 partitioning of the pellet was carried out as described by Brandt *et al*. (Infect. Immun. 58:983-991 (1990)). Following trypsin treatment, cells were resuspended in ice-cold 2% (v/v) Triton X-114 in PBS at 10⁹ cells per ml. The suspension was rotated overnight at 4°C, and the insoluble fraction removed as a pellet after centrifugation at 10,000 X g for 15 minutes at 4°C. The supernatant (soluble fraction) was incubated at 37°C for 15 minutes and centrifuged at room temperature at 1000 X g for 15 minutes to separate the aqueous and detergent phases. The aqueous phase was decanted, and ice cold PBS added to the lower Triton phase, mixed, warmed to 37°C, and again centrifuged at 1000 X g for 15 minutes. Washing was repeated twice more. Finally, detergent was removed from the preparation using a spin column of Bio-beads SM2 (BioRad, Melville, New York) as described (Holloway, P.W., Anal. Biochem. 53:304-308 (1973)).

Ion exchange chromatography was carried out as described by Dunn *et al*. (Prot. Exp. Purif. 1: 159-168 (1990)) with minor modifications. Crude OspA was dissolved in buffer A (1% Triton X-100, 10mM phosphate buffer (pH 5.0)) and loaded onto a SP Sepharose resin (Pharmacia, Piscataway, New Jersey), pre-equilibrated with buffer A at 25°C. After washing the column with 10 bed-volumes of buffer A, the bound OspA was eluted with buffer B (1% Triton X-100, 10mM phosphate buffer (pH 8.0)). OspA fractions were detected by protein assay using the BCA method (Pierce, Rockford, Illinois), or as radioactivity when intrinsically labeled material was fractionated. Triton X-100 was removed using a spin column of Bio-beads SM2.

This method purifies OspA from an outer surface membrane preparation. In the absence of trypsin-treatment, OspA and B were the major components of the soluble fraction obtained after Triton partitioning of strain B31. In contrast, when Triton extraction was carried out after trypsin-treatment, the OspB band is not seen. Further purification of OspA-B31 on a SP Sepharose column resulted in a single band by SDS-PAGE. The yield following removal of detergent was approximately 2 mg per liter of culture. This method of purification of OspA, as described herein for strain B31, can be used for other isolates of *Borrelia* as well. For strains such as strain K48, which lack OspB, trypsin treatment can be omitted.

### Lipidation site of OspA-B31

¹⁴C-palmitic acid labeled OspA from strain B31 was purified as described above and partially digested with endoproteinase Asp-N (data not shown). Following digestion, a new band of lower molecular weight was apparent by SDS-PAGE, found by direct amino-terminal sequencing to begin at Asp₂₅. This band had no trace of radioactivity by autoradiography (data not shown). OspA and B contain a signal sequence (L-X-Y-C) similar to the consensus described for lipoproteins of *E. coli*, and it has been predicted that the lipidation site of OspA and B should be the amino-terminal cysteine (Brandt, M.E. et *al*., Infect. Immun 58: 983-991 (1990)). The results presented herein support this prediction.

### B. Comparison of OspA Antibody Binding Regions in Nine Strains of Borrelia burgdorferi

The availability of the amino acid sequenced for OspA from a number of different isolates, combined with peptide mapping and Western blot analysis, permitted the identification of the antigenic domains recognized by monoclonal antibodies (MAbs) and allowed inference of the key amino acid residues responsible for specific antibody reactivity.

### Strains of Borrelia burgdorferi

Nine strains of *Borrelia,* including seven European strains and two North American strains, were used in this study of antibody binding domains of several proteins. Information concerning the strains is summarized in Table I, below.

**Table I.**

| Representative *Borrelia* Strains | | |
|---|---|---|
| Strain | Location and Source | Reference for Strain |
| K48 | Czechoslovakia, *Ixodes ricinus* | none |
| PGau | Germany, human ACA | Wilske, B. *et al.,* J. Clin. Microbiol. 32:340-350 (1993) |
| DK29 | Denmark, human EM | Wilske, B. *et al*. |
| PKo | Germany, human EM | Wilske, B. *et al*. |
| PTrob | Germany, human skin | Wilske, B. *et al*. |
| Ip3 | Khabarovsk, Russia, *I. persulcatus* | Asbrink, E. *et al*., Acta Derm. Venereol. 64: 506-512 (1984) |
| Ip90 | Khabarovsk, Russia, *I*. *persulcatus* | Asbrink, E. *et al*. |
| 25015 B31 | Millbrook, NY, I. *persulcatus* | Barbour, A.G. *et al*., Curr. Microbiol. 8:123-126 (1983) |
| | Shelter Island, NY, *I. scapularis* | Luff, B.J. *et al*., Infect. Immun. 60: 4309-4321 (1992); ATCC 35210 |
| PKal | Germany, human CSF | Wilske, B. *et al*. |
| ZS7 N40 | Freiburg, Germany, *I. ricinus* | Wallich, R. *et al.,* Nucl. Acids Res. 17: 8864 (1989) |
| | Westchester Co., NY | Fikrig, E. *et al*., Science 250:553-556 (1990) |
| PHei | Germany, human CSF | Wilske, B. *et al*. |
| ACAI | Sweden, human ACA | Luft, B. J. *et al*., FEMS Microbiol. Lett. 93:73-68 (1992) |
| PBo | Germany, human CSF | Wilske, B. *et al.* |
| ACA = patient with acrodermatitis chronica atrophicans; EM = patient with erythema migrans; CSF = cerebrospinal fluid of patient with Lyme disease | | |

Strains K48, PGau and DK29 were supplied by R. Johnson, University of Minnesota; PKo and pTrob were provided by B. Wilske and V. Preac-Mursic of the Pettenkhofer Institute, Munich, Germany; and Ip3 and Ip90 were supplied by L. Mayer of the Center for Disease Control, Atlanta, Georgia. The North American strains included strain 25015, provided by J. Anderson of the Connecticut Department of Agriculture; and strain B31 (ATCC 35210).

### Monoclonal Antibodies

Seven monoclonal antibodies (MAbs) were utilized in this study. Five of the MAbs (12, 13, 15, 83 and 336) were produced from hybridomas cloned and subcloned as previously described (Schubach, W.H., *et al.,* Infect. Immun. 59(6):1911-1915 (1991)). MAb H5332 (Barbour, A.G. *et al*., Infect. Immun. 41:795-804 (1983)) was a gift from Drs. Alan Barbour, University of Texas, and MAb CIII.78 (Sears, J.E. *et al*., J. Immunol. 147(6):1995-2000 (1991)) was a gift from Richard A. Flavell, Yale University. MAbs 12 and 15 were raised against whole sonicated B3; MAb 336 was produced against whole PGau; and MAbs 13 and 83 were raised to a truncated form of OspA cloned from the K48 strain and expressed in *E. coli* using the T7 RNA polymerase system (McGrath, B.C. *et al*., Vaccines, Cold Spring Harbor Laboratory Press, Plainview, New York, pp. 365-370 (1993)). All MAbs were typed as being Immunoglobulin G (IgG).

### Methods of Protein Cleavage, Western Blotting, and Amino-Terminal Sequencing

Prediction of the various cleavage sites was achieved by knowledge of the primary amino acid sequence derived from the full nucleotide sequences of OspA, many of which are currently available (see Table II, below). Cleavage sites can also be predicted based on the peptide sequence of OspA, which can be determined by standard techniques after isolation and purification of OspA by the method described above. Cleavage of several OspA isolates was conducted to determine the localization of monoclonal antibody binding of the proteins.

Hydroxylamine-HCl (HA), N-chlorosuccinimide (NCS), and cyanogen bromide cleavage of OspA followed the methods described by Bornstein (Biochem. 9 (12):2408-2421 (1970)), Shechter *et al*., (Biochem. 15 (23):5071-5075 (1976)), and Gross (in Hirs, C.H.W. (ed): Methods in Enzymology, (N.Y. Acad. Press), 11:238-255 (1967)) respectively. Protease cleavage by endoproteinase, Asp-N (Boehringer Mannheim, Indianapolis, Indiana), was performed as described by Cleveland D.W. *et al*., (J. Biol. Chem. 252:1102-1106 (1977)). Ten micrograms of OspA were used for each reaction. The ratio of enzyme to OspA was approximately 1 to 10 (w/w).

Proteins and peptides generated by cleavage were separated by SDS-polyacrylamide gel electrophoresis (SDS-PAGE) (Laemmli, U.K., Nature (London) 227:680-685 (1970)), and electroblotted onto immobilon Polyvinylidine Difluoride (PVDF) membranes (Ploskal, M.G. *et al*., Biotechniques 4:272-283 (1986)). They were detected by amido black staining or by immunostaining with murine MAbs, followed by alkaline phosphatase-conjugated goat antimouse IgG. Specific binding was detected using a 5-bromo-4-chloro-3-indolylphosphate (BCIP)/nitroblue tetrazolium (NBT) developer system (KPL Inc., Gathersburg, Maryland).

In addition, amino-terminal amino acid sequence analysis was carried out on several cleavage products, as described by Luff *et al*. (Infect. Immun. 57:3637-3645 (1989)). Amido black stained bands were excised from PVDF blots and sequenced by Edman degradation using a Biosystems model 475A sequenator with model 120A PTH analyzer and model 900A control/data analyzer.

### Cleavage Products of Outer Surface Protein A Isolates

Purified OspA-B31, labeled with ¹⁴C-palmitic acid, was fragmented with hydroxylamine-HCl (HA) into two peptides, designated HA1 and HA2 (data not shown). The HA1 band migrated at 27 KD and retained its radioactivity, indicating that the peptide included the lipidation site at the N-terminus of the molecule (data not shown). From the predicted cleavage point, HA1 should correspond to residues 1 to 251 of OspA-B31. HA2 had a MW of 21.6 KD by SDS-PAGE, with amino-terminal sequence analysis showing it to begin at Gly72, i.e. residues 72 to 273 of OspA-B31. By contrast, HA cleaved OspA-K48 into three peptides, designated HA1, HA2, and HA3 with apparent MWs of 22KD, 16 KD and 12 KD, respectively. Amino-terminal sequencing showed HAl to start at Gly72, and HA3 at Gly142. HA2 was found to have a blocked amino-terminus, as was observed for the full-length OspA protein. HA1, 2 and 3 of OspA-K48 were predicted to be residues 72-274, 1 to 141 and 142 to 274, respectively.

N-Chlorosuccinimide (NCS) cleaves tryptophan (W), which is at residue 216 of OspA-B31 or residue 217 of OspA-K48 (data not shown). NCS cleaved OspA-B31 into 2 fragments, NCS1, with MW of 23 KD, residues 1-216 of the protein, and NCS2 with a MW of 6.2 KD, residues 217 to 273 (data not shown). Similarly, K48 OspA was divided into 2 pieces, NCS1 residues 1-217, and NCS2 residues 218 to 274 (data not shown).

Cleavage of OspA by cyanogen bromide (CNBr) occurs at the carboxy side of methionine, residue 39. The major fragment, CNBr1, has a MW of 25.7 KD, residues 39-274 by amino-terminal amino acid sequence analysis (data not shown). CNBr2 (about 4 KD) could not be visualized by amido black staining; instead, lightly stained bands of about 20 KD MW were seen. These bands reacted with anti-OspA MAbs, and most likely were degradation products due to cleavage by formic acid.

### Determination of Antibody Binding Domains for Anti-OspA Monoclonal Antibodies

The cleavage products of OspA-B31 and OspA-K48 were analyzed by Western blot to assess their ability to bind to the six different MAbs. Preliminary Western blot analysis of the cleavage products demonstrated that strains K48 and DK29 have similar patterns of reactivity, as do IP3, PGau and PKo. The OspA of strain PTrob was immunologically distinct from the others, being recognized only by MAb 336. MAb 12 recognized only the two North American strains, B31 and 25015. When the isolates were separated into genogroups, it was remarkable that all the MAbs, except MAb 12, crossed over to react with multiple genogroups.

MAb12, specific for OspA-B31, bound to both HA1 and HA2 of OspA-B31. However, cleavage of OspA-B31 by NCS at residue Trp216 created fragments which did not react with MAb12, suggesting that the relevant domain is near or is structurally dependent upon the integrity of this residue (data not shown). MAb 13 bound only to OspA-K48, and to peptides containing the amino-terminus of that molecule (e.g. HA2; NCS1). It did not bind to CNBr1 residues 39 to 274. Thus the domain recognized by MAb13 is in the amino-terminal end of OspA-K48, near Met38.

MAb15 reacts with the OspA of both the B31 and K48 strains, and to peptides containing the N-terminus of OspA, such as HA1 of OspA-B31 and NCS1, but not to peptides HA2 of OspA-B31 and HA1 of OspA-K48 (data not shown). Both peptides include residue 72 to the C-terminus of the molecules. MAb15 bound to CNBr1 of OspA-K48, indicating the domain for this antibody to be residues 39 to 72, specifically near Gly72 (data not shown).

MAb83 binds to OspA-K48, and to peptides containing the C-terminal portion of the molecule, such as HA1. They do not bind to HA2 of OspA-K48, most likely because the C-terminus of HA2 of OspA-K48 ends at 141. Similar to MAb12 and OspA-B31, binding of MAbs 83 and CIII.78 is eliminated by cleavage of OspA at the tryptophan residue. Thus binding of MAbs 12, 83 and CIII.78 to OspA depends on the structural integrity of the Trp₂₁₆ residue, which appears to be critical for antigenicity. Also apparent is that, although these MAbs bind to a common antigenic domain, the precise epitopes which they recognize are distinct from one another given the varying degrees of cross-reactivity to these MAbs among strains.

Although there is similar loss of binding activity of MAb336 with cleavage at Trp₂₁₆, this MAb does not bind to HA1 of OspA-B31, suggesting the domain for this antibody includes the carboxy-terminal end of the molecule, inclusive of residues 251 to 273. Low MW peptides, such as HA3 (10 KD) and NCS2 (6KD), of OspA-K48 do not bind this MAb on Western blots. In order to confirm this observation, we tested binding of the 6 MAbs with a recombinant fusion construct p3A/EC that contains a trpE leader protein fused with residues 217 to 273 of OspA-B31 (Schubach, W.H. *et al.,* Infect. Immun. 59(6): 1911-1915 (1991)). Only MAb336 reacted with this construct (data not shown). Peptides and antigenic domains localized by fragmentation of OspA are summarized in Figure 1.

### Mapping of Domains to Define the Molecular Basis for the Serotype Analysis

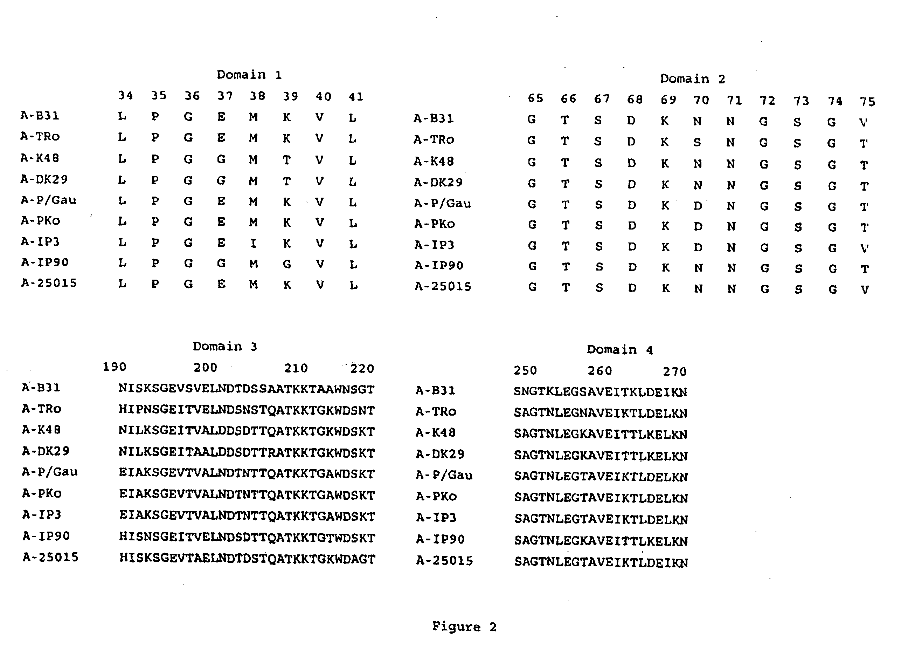

To define the molecular basis for the serotype analysis of OspA, we compared the derived amino acid sequences of OspA for the nine isolates (Figure 2). At the amino terminus of the protein, these predictions can be more precise given the relatively small number of amino acid substitutions in this region compared to the carboxy terminus. Domain 1, which is recognized by MAb13, includes residues Leu34 to Leu41. MAb13 only binds to the OspA of species K48, DK29 and IP90. Within this region, residue 37 is variable, however Gly37 is conserved amongst the three reactive strains. When Gly37 is changed to Glu37, as it is in OspA of strains B31, pTrob, PGau, and PKo, MAb13 does not recognize the protein (data not shown). By similar analysis, it can be seen that Asp70 is a crucial residue for Domain 2, which includes residues 65 to 75 and is recognized by MAb15. Domain 3 is reactive with MAbs H5332, 12 and 83, and includes residues 190-220. It is clear that significant heterogeneity exists between MAbs reactive with this domain, and that more than one conformational epitope must be contained within the sequence. Domain 4 binds MAb336, and includes residues 250 to 270. In this region, residue 266 is variable and therefore may be an important determinant. It is apparent, however, that other determinants of the reactivity of this monoclonal antibody reside in the region comprising amino acids 217-250. Furthermore, the structural integrity of Trp216 is essential for antibody reactivity in the intact protein. Finally, it is important to stress that Figure 2 indicates only the locations of the domains, and does not necessarily encompass the entire domain. Exact epitopes are being analyzed by site-directed mutagenesis of specific residues.

Overall, evidence suggests that the N-terminal portion is not the immunodominant domain of OspA, possibly by virtue of its lipidation, and the putative function of the lipid moiety in anchoring the protein to the outer envelope. The C-terminal end is immunodominant and includes domains that account in part for structural heterogeneity (Wilske, B. *et al.,* Med. Microbiol. Immunol. 181: 191-207 (1992)), and may provide epitopes for antibody neutralization (Sears, J.E. et al., J. Immunol. 147(6): 1995-2000 (1991)), and relate to other activities, such as the induction of T-cell proliferation (Shanafel, M.M., et al., J. Immunol. 148: 218-224 (1992)). There are common epitopes in the carboxy-end of the protein that are shared among genospecies which may have immunoprotective potential (Wilske, B., *et al.,* Med. Microbiol. Immunol. 181: 191-207 (1992)).

Prediction of secondary structure on the basis of hydropathy analysis and circular dichroism and fluorescence spectroscopy measurements (McGrath, B.C., *et al.,* Vaccines, Cold Spring Harbor Laboratory Press, Plainview, New York; pp. 365-370 (1993)) suggest domains 3 and 4 to be in a region of the molecule with a propensity to form alpha-helix, whereas domains 1 and 2 occur in regions predicted to be beta-sheets (see Figure 1). These differences may distinguish domains in accessibility to antibody or to reactive T-cells (Shanafel, M.M. *et al.,* J. Immunol. 148: 218-224 (1992)). Site-directed mutagenesis of specific epitopes, as described below in Example 2, aids in identifying exact epitopes.

### Example 2. Identification of an Immunologically Important Hypervariable Domain of the Major Outer Surface Protein A of Borrelia

This Example describes epitope mapping studies using chemically cleaved OspA and TrpE-OspA fusion proteins. The studies indicate a hypervariable region surrounding the single conserved tryptophan residue of OspA (at residue 216, or in some cases 217), as determined by a moving window population analysis of OspA from fifteen European and North American isolates of *Borrelia.* The hypervariable region is important for immune recognition.

Site-directed mutagenesis was also conducted to examine the hypervariable regions more closely. Fluorescence and circular dichroism spectroscopy have indicated that the conserved tryptophan is part of an alpha-helical region in which the tryptophan is buried in a hydrophobic environment (McGrath, B.C., *et al.,* Vaccines, Cold Spring Harbor Laboratory Press, Plainview, New York; pp. 365-370 (1993)). More polar amino acid side-chains flanking the tryptophan are likely to be exposed to the hydrophilic solvent. The hypervariability of these solvent-exposed residues among the various strains of Borrelia suggested that these amino acid residues may contribute to the antigenic variation in OspA. Therefore, site-directed mutagenesis was performed to replace some of the potentially exposed amino acid side chains in the protein from one strain with the analogous residues of a second strain. The altered proteins were then analyzed by Western Blot using monoclonal antibodies which bind OspA on the surface of the intact, non-mutated spirochete. The results indicated that certain specific amino acid changes near the tryptophan can abolish reactivity of OspA to these monoclonal antibodies.

### A. Verification of Clustered Polymorphisms in Outer Surface Protein A Sequences

Cloning and sequencing of the OspA protein from fifteen European and North American isolates (described above in Table I) demonstrated that amino acid polymorphism is not randomly distributed throughout the protein; rather, polymorphism tended to be clustered in three regions of OspA. The analysis was carried out by plotting the moving, weighted average polymorphism of a window (a fixed length subsection of the total sequence) as it is slid along the sequence. The window size in this analysis was thirteen amino acids, based upon the determination of the largest number of significantly deviating points as established by the method of Tajima (J. Mol. Evol. 33: 470-473 (1991)). The average weighted polymorphism was calculated by summing the number of variant alleles for each site. Polymorphism calculations were weighted by the severity of amino acid replacement (Dayhoff, M.O. *et al.,* in: Dayhoff, M.O. (ed.) Atlas of Protein Sequence and Structure NBRF, Washington, Vol. 5, Suppl. 3: 345 (1978)). The sum was normalized by the window size and plotted. The amino acid sequence position corresponds to a window that encompasses amino acids 1 through 13. Bootstrap resampling was used to generate 95% confidence intervals on the sliding window analysis. Since *Borrelia* has been shown to be clonal, the bootstrap analysis should give a reliable estimate of the expected variance out of polymorphism calculations. The bootstrap was iterated five hundred times at each position, and the mean was calculated from the sum of all positions. The clonal nature of *Borrelia* ensures that the stochastic variance that results from differing genealogical histories of the sequence positions (as would be expected if recombination were prevalent) will be minimized.

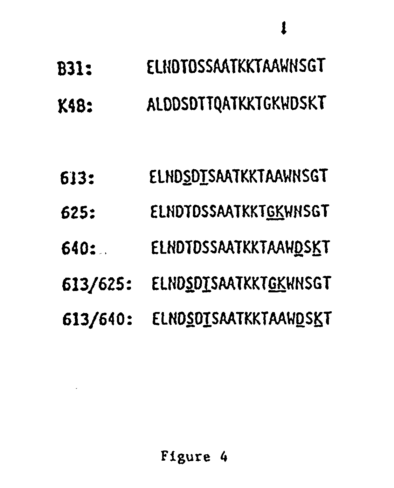

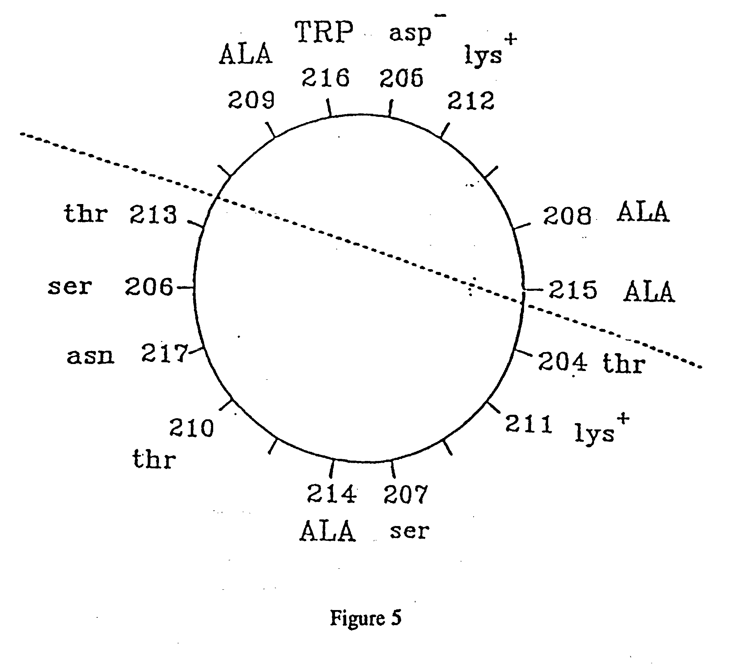

This test verified that the three regions around the observed peaks all have significant excesses of polymorphism. Excesses of polymorphism were observed in the regions including amino acid residues 132-145, residues 163-177, and residues 208-221 (Figure 3). An amino acid alignment between residues 200 and 220 for B31, K48 and the four site-directed mutants is shown in Figure 4. The amino acid 208-221 region includes the region of OspA which has been modeled as an oriented alpha-helix in which the single tryptophan residue at amino acid 216 is buried in a hydrophobic pocket, thereby exposing more polar amino acids to the solvent (Figure 5) (France, L.L., *et al.,* Biochem. Biophys. Acta 1120: 59 (1992)). These potentially solvent-exposed residues showed considerable variability among the OspAs from various strains and may be an important component of OspA antigenic variation. For the purposes of generating chimeric proteins, the hypervariable domains of interest are Domain A, which includes amino acid residues 120-140 of OspA; Domain B, which includes residues 150-180; and Domain C, which includes residues 200-216 or 217.

### B. Site-Directed Mutagenesis of the Hypervariable Region

Site-directed mutagenesis was performed to convert residues within the 204-219 domain of the recombinant B31 OspA to the analogous residues of a European OspA variant, K48. In the region of OspA between residues 204 and 219, which includes the helical domain (amino acids 204-217), there are seven amino acid differences between OspA-B31 and OspA-K48. Three oligonucleotides were generated, each containing nucleotide changes which would incorporate K48 amino acids at their analogous positions in the B31 OspA protein. The oligos used to create the site-directed mutants were:
5'-CTTAATGACTCTGACACTAGTGC-3' (#613, which converts threonine at position 204 to serine, and serine at 206 to threonine (Thr204-Ser, Thr206-Ser)) (SEQ ID NO. 1);
5'-GCTACTAAAAAAACCGGGAAATGGAATTCA-3' (#625, which converts alanine at 214 to glycine, and alanine at 215 to lysine (Ala214-Gly, Ala215-Lys)) (SEQ ID NO. 2); and
5'-GCAGCTTGGGATTCAAAAACATCCACTTTAACA-3' (#640, which converts asparagine at 217 to aspartate, and glycine at 219 to lysine (Asn217-Asp, Gly219-Lys)) (SEQ ID NO. 3).
Site-directed mutagenesis was carried out by performing mutagenesis with pairs of the above oligos. Three site-directed mutants were created, each with two changes: OspA 613 (Thr204-Ser, Thr206-Ser), OspA 625 (Ala214-Gly, Ala215-Lys), and 640 (Asn217-Asp, Gly219-Lys). There were also two proteins with four changes: OspA 613/625 (Thr204-Ser, Thr206-Ser, Ala214-Gly, Ala215-Lys) and OspA 613/640 (Thr204-Ser, Thr206-Ser, Asn217-Asp, Gly219-Lys).

### Specificity of Antibody Binding to Epitopes of the Non-mutated Hypervariable Region

Monoclonal antibodies that agglutinate spirochetes, including several which are neutralizing in vitro, recognize epitopes that map to the hypervariable region around Trp216 (Barbour, A.G. *et al.,* Infect. and Immun. 41: 759 (1983); Schubach, W.H. *et al.,* Infect. and Immun. 59: 1911 (1991)). Western Blot analysis demonstrated that chemical cleavage of OspA from the B31 strain at Trp 216 abolishes reactivity of the protein with the agglutinating Mab 105, a monoclonal raised against B31 spirochetes (data not shown). The reagent, n-chlorosuccinimide (NCS), cleaves OspA at the Trp 216, forming a 23.2kd fragment and a 6.2kd peptide which is not retained on the Imobilon-P membrane after transfer. The uncleaved material binds Mab 105; however, the 23.2kd fragment is unreactive. Similar Western blots with a TrpE-OspA fusion protein containing the carboxy-terminal portion of the OspA protein demonstrated that the small 6.2kd piece also fails to bind Mab 105 (Schubach, W.H. *et al*., Infect. and Immun. 59: 1911 (1991)).

Monoclonal antibodies H5332 and H3TS (Barbour, A.G. *et al.,* Infect. and Immun. 41: 759 (1983)) have been shown by immunofluorescence to decorate the surface of fixed spirochetes (Wilske, B. *et al.,* World J. Microbiol. 7: 130 (1991)). These monoclonals also inhibit the growth of the organism in culture. Epitope mapping with fusion proteins has confirmed that the epitopes which bind these Mabs are conformationally determined and reside in the carboxy half of the protein. Mab H5332 is cross-reactive among all of the known phylogenetic groups, whereas Mab H3TS and Mab 105 seem to be specific to the B31 strain to which they were raised. Like Mab 105, the reactivities of H5332 and H3TS to OspA are abrogated by fragmentation of the protein at Trp216 (data not shown). Mab 336 was raised to whole spirochetes of the strain P/Gau. It cross-reacts to OspA from group 1 (the group to which B31 belongs) but not to group 2 (of which K48 is a member). Previous studies using fusion proteins and chemical cleavage have indicated that this antibody recognizes a domain of OspA in the region between residues 217 and 273 (data not shown). All of these Mabs will agglutinate the B31 spirochete.

### Western Blot Analysis of Antibody Binding to Mutated Hypervariable Regions

Mabs were used for Western Blot analysis of the site-directed OspA mutants induced in E.coli using the T7 expression system (Dunn, J.J. *et al.,* Protein Expression and Purification 1: 159 (1990)). *E. coli* cells carrying Pet9c plasmids having a site-directed OspA mutant insert were induced at mid-log phase growth with IPTG for four hours at 37°C. Cell lysates were made by boiling an aliquot of the induced cultures in SDS gell loading dye, and this material was then loaded onto a 12% SDS gell (BioRad mini-Protean II), and electrophoresed. The proteins were then transferred to Imobilon-P membranes (Millipore) 70V, 2 hour at 4°C using the BioRad mini transfer system. Western analysis was carried out as described by Schubach *et al*. (Infect. Immun. 59: 1911 (1991)).

Western Blot analysis indicated that only the 625 mutant (Ala214-Gly and Ala215-Lys) retained binding to the agglutinating monoclonal H3TS (data not shown). However, the 613/625 mutant which has additional alterations to the amino terminus of Trp216 (Ser204-Thr and Thr206-Ser) did not bind this monoclonal. Both 640 and 613/640 OspAs which have the Asn217-Asp and Gly219-Lys changes on the carboxy-terminal side of Trp216 also failed to bind Mab H3TS. This indicated that the epitope of the B31 OspA which binds H3TS is comprised of amino acid side-chains on both sides of Trp216.

The 613/625 mutant failed to bind Mabs 105 and H5332, while the other mutants retained their ability to bind these Mabs. This is important in light of the data using fusion proteins that indicate that Mab 105 behaves more like Mab H3TS in terms of its serotype specificity and binding to OspA (Wilske, B. *et al.,* Med. Microbiol. Immunol. 181: 191 (1992)). The 613/625 protein has, in addition to the differences at residues Thr204 and Ser206, changes immediately amino-terminal to Trp216 (Ala214-Gly and Ala215-Lys). The abrogation of reactivity of Mabs 105 and H5332 to this protein indicated that the epitopes of OspA which bind these monoclonals are comprised of residues on the amino-terminal side of Trp216.

The two proteins carrying the Asn217-Asp and Gly219-Lys replacements on the carboxy-terminal side of Trp216 (OspAs 640 and 613/640) retained binding to Mabs 105 and H5332; however, they failed to react with Mab 336, a monoclonal which has been mapped with TrpE-OspA fusion proteins and by chemical cleavage to a more carboxy-terminal domain. This result may explain why Mab 336 failed to recognize the K48-type of OspA (Group 2).

It is clear that amino acids Ser204 and Thr206 play an important part in the agglutinating epitopes in the region of the B31 OspA flanking Trp216. Replacement of these two residues altered the epitopes of OspA that bind Mabs 105, H3TS and H5332. The ability of the 640 changes alone to abolish reactivity of Mab 336 indicated that Thr204 and Ser206 are not involved in direct interaction with Mab 336.

The results indicated that the epitopes of OspA which are available to Mabs that agglutinate spirochetes are comprised at least in part by amino acids in the immediate vicinity of Trp216. Since recent circular dichroism analysis indicated that the structures of B31 and K48 OspA differ very little within this domain, it is unlikely that the changes made by mutation have radically altered the overall structure of the OspA protein (France, L.L. *et al*., Biochem. Biophys. Acta 1120: 59 (1992); and France *et al.,* Biochem. Biophys Acta, submitted (1993)). This hypothesis is supported by the finding that the recombinant, mutant OspAs exhibit the same high solubility and purification properties as the parent B31 protein (data not shown).

In summary, amino acid side-chains at Ser204 and Thr206 are important for many of the agglutinating epitopes. However, a limited set of conservative changes at these sites were not sufficient to abolish binding of all of the agglutinating Mabs. These results suggested that the agglutinating epitopes of OspA are distinct, yet may have some overlap. The results also supported the hypothesis that the surface-exposed epitope around Trp216 which is thought to be important for immune recognition and neutralization is a conformationally-determined and complex domain of OspA.

### EXAMPLE 3. Borrelia Strains and Proteins

Proteins and genes from any strain of Borrelia can be utilized in the current invention. Representative strains are summarized in Table I, above.

### A. Genes Encoding Borrelia Proteins

The chimeric peptides of the current invention can comprise peptides derived from any Borrelia proteins. Representative proteins include OspA, OspB, OspC, OspD, p12, p39, p41 (fla), p66, and p93. Nucleic acid sequences encoding several Borrelia proteins are presently available (see Table II, below); alternatively, nucleic acid sequences encoding *Borrelia* proteins can be isolated and characterized using methods such as those described below.

**Table II.**

| References for Nucleic Acid Sequences for Several Proteins of Various *Borrelia* Strains | | | |
|---|---|---|---|
| Strain | p93 | OspA | p41 (fla) |
| K48 | X69602 (SID 67) | X62624 (SID 8) | X69610 (SID 49) |
| PGau | SID 73 | X62387 (SID 10) | X69612 (SID 51) |
| DK29 | - | X63412 (SID 137) | X69608 (SID 53) |
| PKo | X69803 (SID 77) | X65599 (SID 141) | X69613 (SID 131) |
| PTrob | X69604 (SID 71) | X65598 (SID 135) | X69614 (SID 55) |
| Ip3 | - | X70365 (SID 140) | - |
| Ip90 | ND | Kryuchechnikov, V.N. *et al.,* J.Microbiol. Epid. Immunobiol. 12:41-44 (1988) (SID 138) | - |
| 25015 | X70365 (SID 75) | Fikrig, E.S. *et al*., J. Immunol. 7:2256-2260 1992) SID 12) | - |
| B31 | Perng, G.C. *et al*., Infect. Immun. 59:2070-74 (1992); Luft, B.J. *et al*., Infect. Immun. 60:4309-4321 (1992) (SID 65) | Bergstrom, S. *et al*., Mol. Microbiol. 3:479-486 (1989) (SID 6) | Gassmann, G.S. *et al*., Nucl. Acids Res. 17: 3590 (1989) (SID 127) |
| PKal | - | X69606 (SID 132) | X69611 (SID 129) |
| ZS7 | - | Jonsson, M. *et al*., Infect. Immun. 60:1845-1853 (1992) (SID 134) | - |
| N40 | - | Kryuchechnikov, V.N. *et al*. (SID 133) | - |
| PHei | - | X65600 (SID 136) | - |
| ACAI | - | Kryuchechnikov, V.N. *et al*. (SID 142) | - |
| PBo | X69601 (SID 69) | X65605 (SID 139) | X69610 (SID 130) |
| Numbers with an "X" prefix are GenBank data base accession numbers. SID = SEQ ID NO. | | | |

### B. Isolation of Borrelia Genes

Nucleic acid sequences encoding full length, lipidated proteins from known Borrelia strains were isolated using the polymerase chain reaction (PCR) as described below. In addition, nucleic acid sequences were generated which encoded truncated proteins (proteins in which the lipidation signal has been removed, such as by eliminating the nucleic acid sequence encoding the first 18 amino acids, resulting in non-lipidated proteins). Other proteins were generated which encoded polypeptides of a particular gene (i.e., encoding a segment of the protein which has a different number of amino acids than the protein does in nature). Using similar methods as those described below, primers can be generated from known nucleic acid sequences encoding *Borrelia* proteins and used to isolate other genes encoding Borrelia proteins. Primers can be designed to amplify all of a gene, as well as to amplify a nucleic acid sequence encoding truncated protein sequences, such as described below for OspC, or nucleic acid sequences encoding a polypeptide derived from a *Borrelia* protein. Primers can also be designed to incorporate unique restriction enzyme cleavage sites into the amplified nucleic acid sequences. Sequence analysis of the amplified nucleic acid sequences can then be performed using standard techniques.

### Cloning and Sequencing of OspA Genes and Relevant Nucleic Acid Sequences

*Borrelia* OspA sequences were isolated in the following manner: 100 µl reaction mixtures containing 50 mM KCl, 10 mM TRIS-HCl (pH 8,3), 1.5 mM MgCl₂, 200 µM each NTP, 2.5 units of TaqI DNA polymerase (Amplitaq, Perkin-Elmer/Cetus) and 100 pmol each of the 5' and 3' primers (described below) were used. Amplification was performed in a Perkin-Elmer/Cetus thermal cycler as described (Schubach, W.H. et *al*., Infect. Immun. 59:1811-1915 (1991)). The amplicon was visualized on an agarose gel by ethidium bromide staining. Twenty nanograms of the chloroform-extracted PCR product were cloned directly into the PC-TA vector (Invitrogen) by following the manufacturer's instructions. Recombinant colonies containing the amplified fragment were selected, the plasmids were prepared, and the nucleic acid sequence of each OspA was determined by the dideoxy chain-termination technique using the Sequenase kit (United States Biochemical). Directed sequencing was performed with M13 primers followed by OspA-specific primers derived from sequences, previously obtained with M13 primers.

Because the 5' and 3' ends of the OspA gene are highly conserved (Fikrig, E.S. *et al.,* J. Immunol. 7:2256-2260 (1992); Bergstrom, S. *et al*., Mol. Microbiol. 3: 479-486 (1989); Zumstein, G. *et al*., Med. Microbiol. Immunol. 181: 57-70 (1992)), the 5' and 3' primers for cloning can be based upon any known OspA sequences. For example, the following primers based upon the OspA nucleic acid sequence from strain B31 were used:
5'-GGAGAATATATTATGAAA-3' (-12 to +6) (SEQ ID NO. 4); and
5'-CTCCTTATTTTAAAGCG-3' (+826 to +809) (SEQ ID NO. 5). (Schubach, W.H. et al., Infect. Immun 59:1811-1915 (1991)).

OspA genes isolated in this manner include those for strains B31, K48, PGau, and 25015; the nucleic acid sequences are depicted in the sequence listing as SEQ ID NO. 6 (OspA-B31), SEQ ID NO. 8 (OspA-K48), SEQ ID NO. 10 (OspA-PGau), and SEQ ID NO. 12 (OspA-25015). An alignment of these and other OspA nucleic acid sequences is shown in Figure 42. The amino acid sequences of the proteins encoded by these nucleic acid sequences are represented as SEQ ID NO. 7 (OspA-B31), SEQ ID NO. 9 (OspA-K48), SEQ ID NO. 11 (OspA-PGau), and SEQ ID NO. 13 (OspA-25015).

The following primers were used to generate specific nucleic acid sequences of the OspA gene, to be used to generate chimeric nucleic acid sequences (as described in Example 4):
5'-GTCTGCAAAAACCATGACAAG-3' (plus strand primer #369) (SEQ ID NO. 14);
5'-GTCATCAACAGAAGAAAAATTC-3' (plus strand primer #357) (SEQ ID NO 15);
5'-CCGGATCCATATGAAAAAATATTTATTGGG-3' (plus strand primer #607) (SEQ ID NO. 16);
5'-CCGGGATCCATATGGCTAAGCAAAATGTTAGC-3' (plus strand primer #584) (SEQ ID NO. 17);
5'-GCGTTCAAGTACTCCAGA-3' (minus strand primer #200) (SEQ ID NO. 18);
5'-GATATCTAGATCTTATTTTAAAGCGTT-3' (minus strand primer #586) (SEQ ID NO. 19); and
5'-GGATCCGGTGACCTTTTAAAGCGTTTTTAAT-3' (minus strand primer #1169) (SEQ ID NO. 20).

### Cloning and Sequencing of OspB

Similar methods were also used to isolate OspB genes. One OspB genes isolated is represented as SEQ ID NO. 21 (OspB-B31); its encoded amino acid sequence is SEQ ID NO. 22.

The following primers were used to generate specific nucleic acid sequences of the OspB gene, to be used in generation of chimeric nucleic acid sequences (see Example 4) :
5'-GGTACAATTACAGTACAA-3' (plus strand primer #721) (SEQ ID NO. 23) ;
5'-CCGAGAATCTCATATGGCACAAAAAGGTGCTGAGTCAATTGG-3' (plus strand primer #1105) (SEQ ID NO. 24);
5'-CCGATATCGGATCCTATTTTAAAGCGTTTTTAAGC-3' (minus strand primer # 1106) (SEQ ID NO. 25); and
5'-GGATCCGGTGACCTTTTAAAGCGTTTTTAAG-3' (minus strand primer #1170) (SEQ ID NO. 26).

### Cloning and Sequencing of OspC

Similar methods were also used to isolate OspC genes. The following primers were used to isolate entire OspC genes from Borrelia strains B31, K48, PKO, and pTrob:
5'-GTGCGCGACCATATGAAAAAGAATACATTAAGTGCG-3' (plus strand primer having Ndel site combined with start codon) (SEQ ID NO. 27), and
5'-GTCGGCGGATCCTTAAGGTTTTTTTGGACTTTCTGC-3' (minus strand primer having BamH1 site followed by stop codon) (SEQ ID NO. 28).

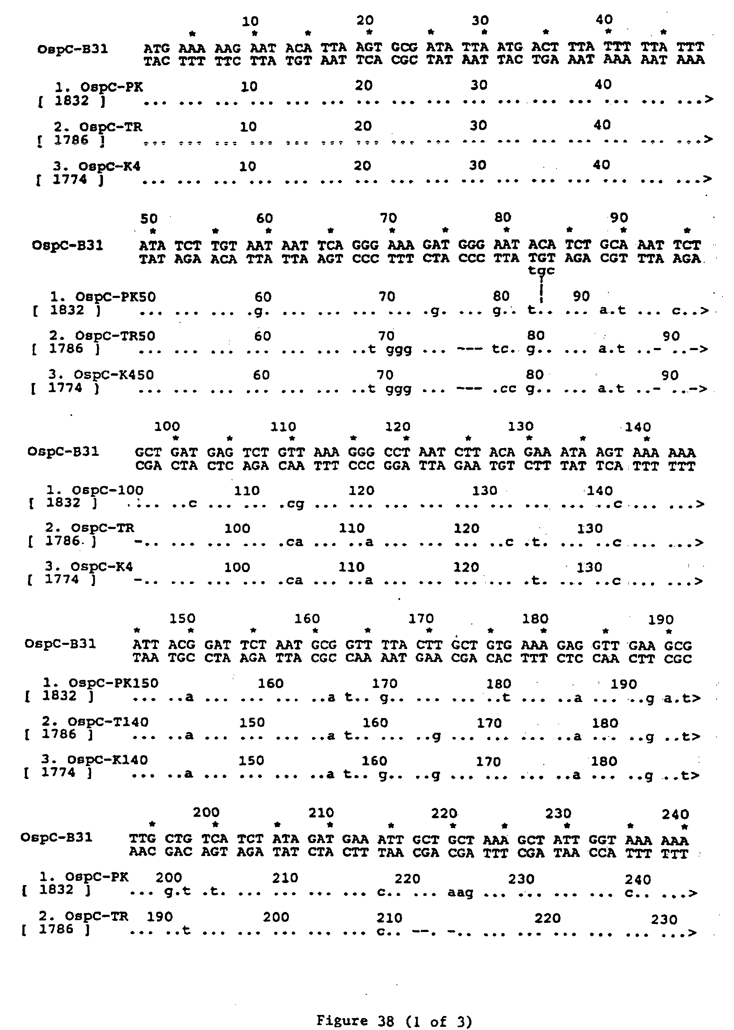

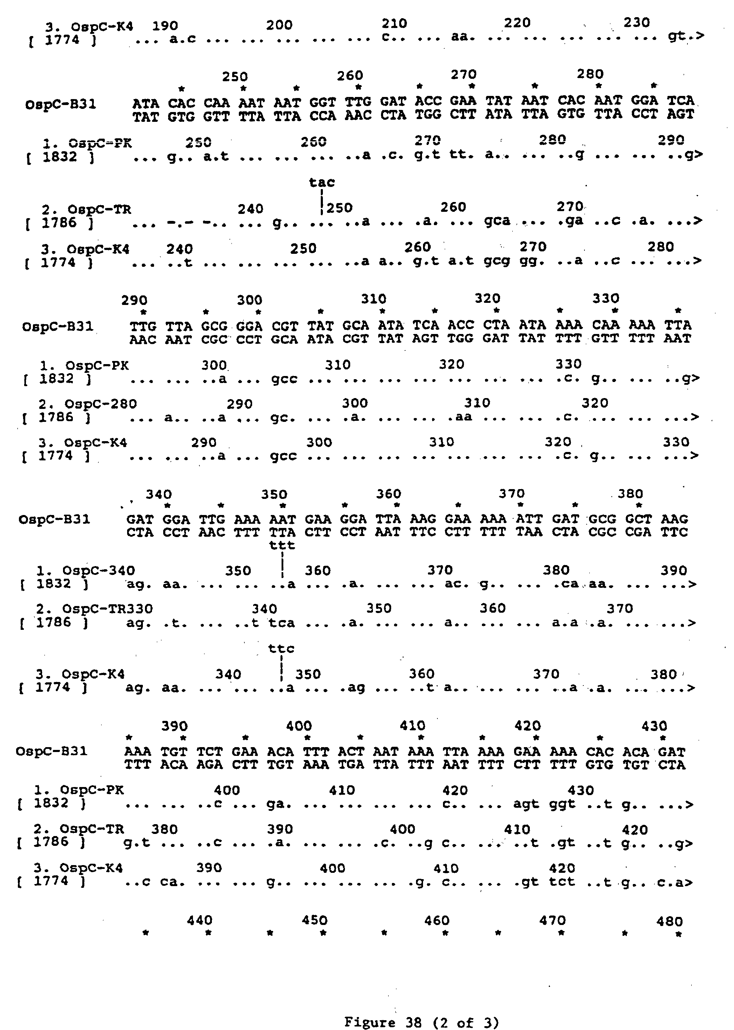

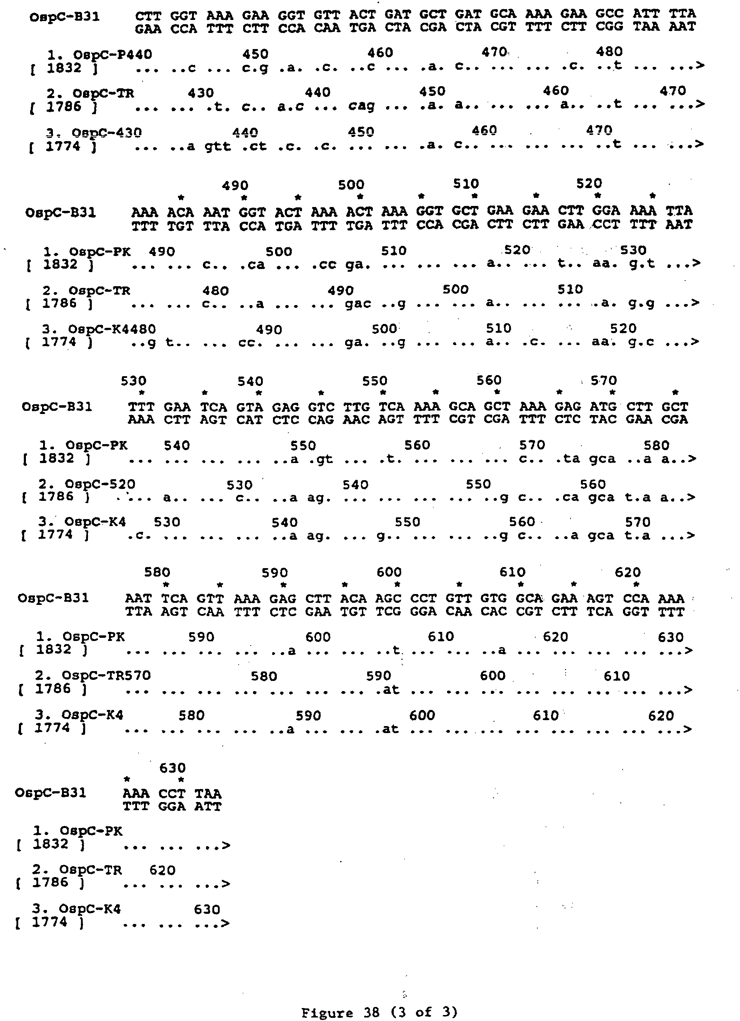

The nucleic acid sequences of the OspC genes were then determined by the dideoxy chain-termination technique using the Sequenase kit (United States Biochemical). OspC genes isolated and sequenced in this manner include those for strains B31, K48, PKo, and Tro; the nucleic acid sequences are depicted in the sequence listing as SEQ ID NO. 29 (OspC-B31), SEQ ID NO. 31 (OspC-K48), SEQ ID NO. 33 (OspC-PKo), and SEQ ID NO. 35 (OspC-Tro). An alignment of these sequences is shown in Figure 38. The amino acid sequences of the proteins encoded by these nucleic acid sequences are represented as SEQ ID NO. 30 (OspC-B31), SEQ ID NO. 32 (OspC-K48), SEQ ID NO. 34 (OspC-PKo), and SEQ ID NO. 36 (OspC-Tro).

Truncated OspC genes were generated using other primers. These primers were designed to amplify nucleic acid sequences, derived from the OspC gene, that lacked the nucleic acids encoding the signal peptidase sequence of the full-length protein. The primers corresponded to bp 58-75 of the natural protein, with a codon for Met-Ala attached ahead. For strain B31, the following primer was used:
5'-GTGCGCGACCATATGGCTAATAATTCAGGGAAAGAT-3' (SEQ ID NO. 37).

For strain PKo,
5'-GTGCGCGACCATATGGCTAGTAATTCAGGGAAAGGT-3' (SEQ ID NO. 38)
was used.

For strains pTrob and K48,
5'-GTGCGCGACCATATGGCTAATAATTCAGGTGGGGAT-3' (SEQ ID NO. 39)
was used.

Additional primers were also designed to amplify nucleic acids encoding particular polypeptides, for use in creation of chimeric nucleic acid sequences (see Example 4). These primers included:
5'-CTTGGAAAATTATTTGAA-3' (plus strand primer #520) (SEQ ID NO. 40) ;
5'-CACGGTCACCCCATGGGAAATAATTCAGGGAAAGG-3' (plus strand primer #58) (SEQ ID NO. 41);
5'-TATAGATGACAGCAACGC-3' (minus strand primer #207) (SEQ ID NO. 42); and
5'-CCGGTGACCCCATGGTACCAGGTTTTTTTGGACTTTCTGC-3' (minus strand primer #636) (SEQ ID NO. 43).

### Cloning and Sequencing of OspD

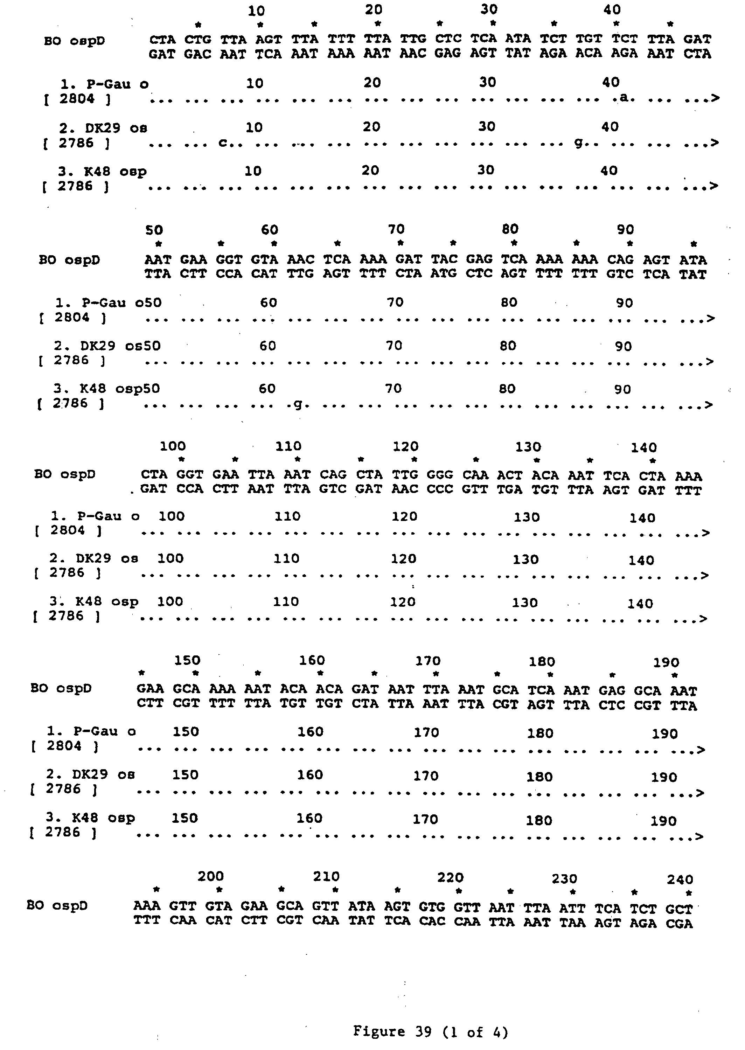

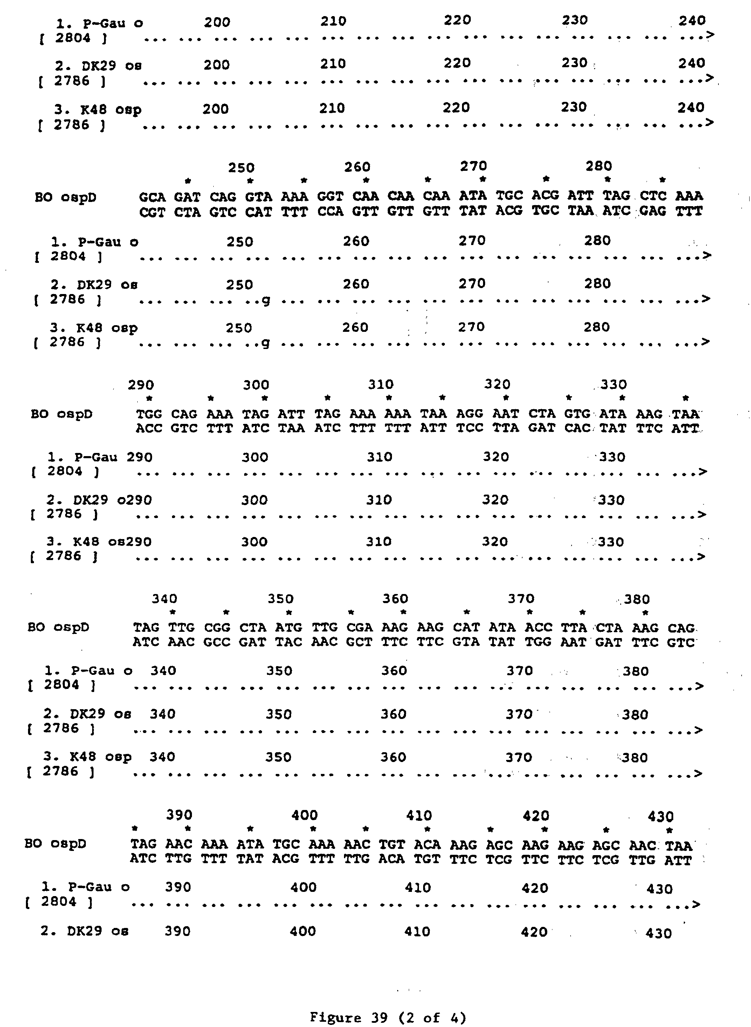

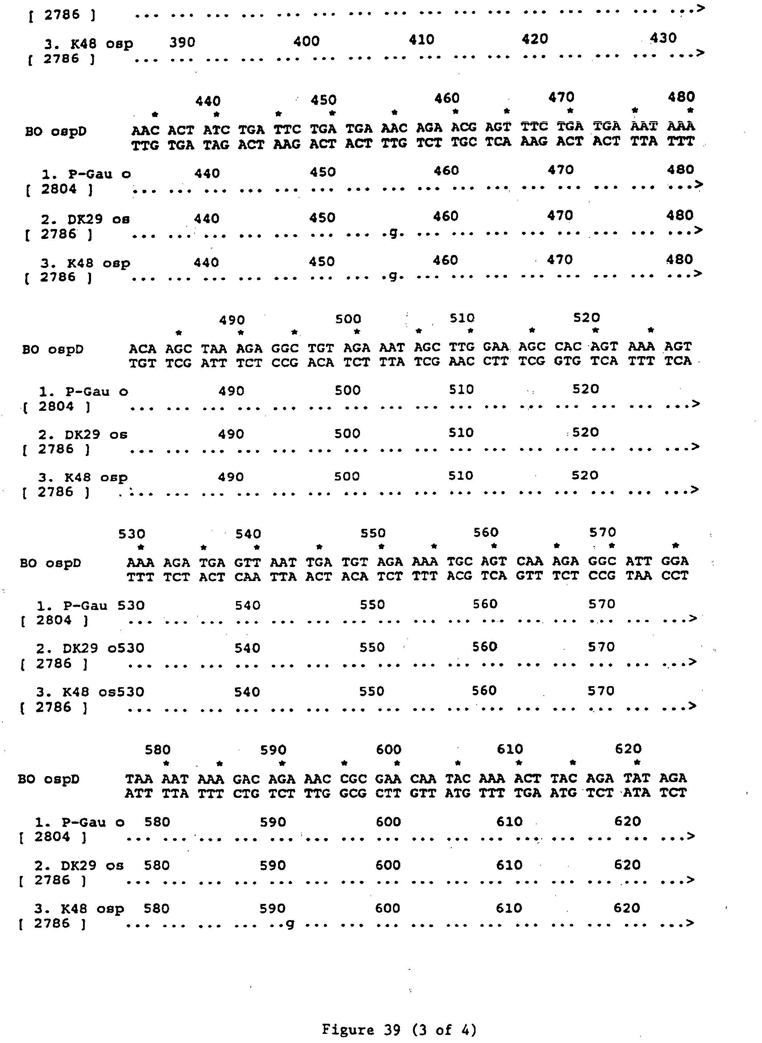

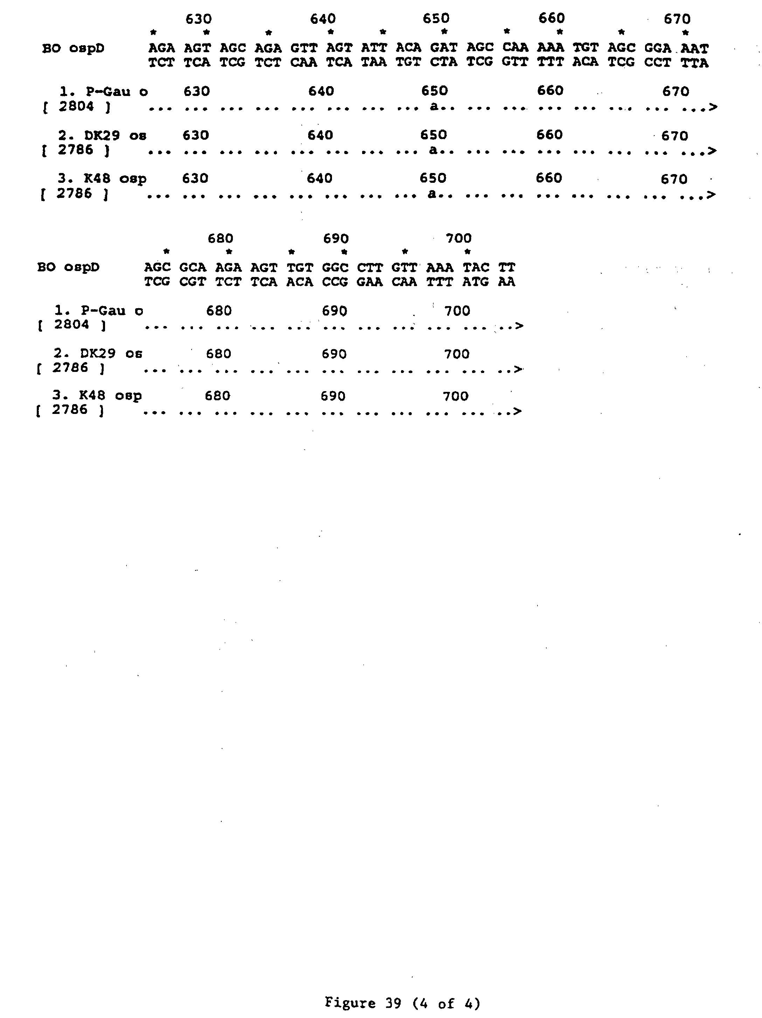

Similar methods can be used to isolate OspD genes. An alignment of four OspD nucleic acid sequences (from strains pBo, PGau, DK29, and K48) is shown in Figure 39.

### Cloning and Sequencing of p12

The p12 gene was similarly identified. Primers used to clone the entire p12 gene included: 5'- CCGGATCCATATGGTTAAAAAAATAATATTTATTTC-3' (forward primer # 757) (SEQ ID NO. 44); and 5'- GATATCTAGATCTTTAATTGCTCTGCTCACTCTCTTC-3' (reverse primer #758) (SEQ ID NO. 45).

To amplify a truncated p12 gene (one in which the transcribed protein is non-lipidated, and begins at amino acid 18 of the native sequence), the following primers were used: 5'-CCGGGATCCATATGGCTAGTGCAATTGGTCGTGG-3' (forward primer # 759) (SEQ ID NO. 46); and primer #758 (SEQ ID NO. 45).

### Cloning and Sequencing of p41 (fla)

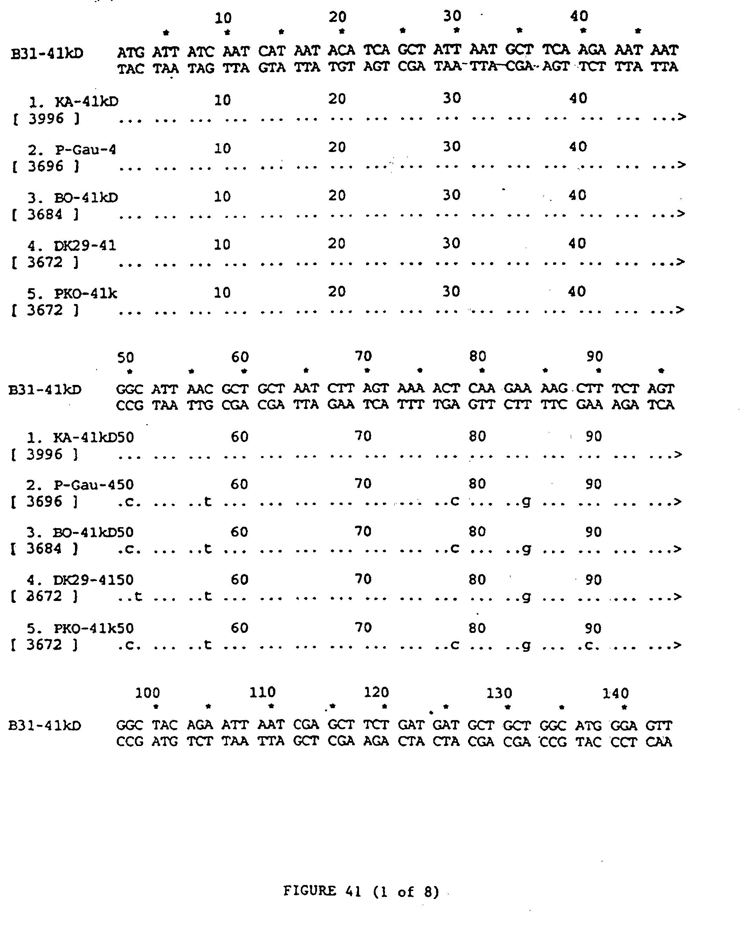

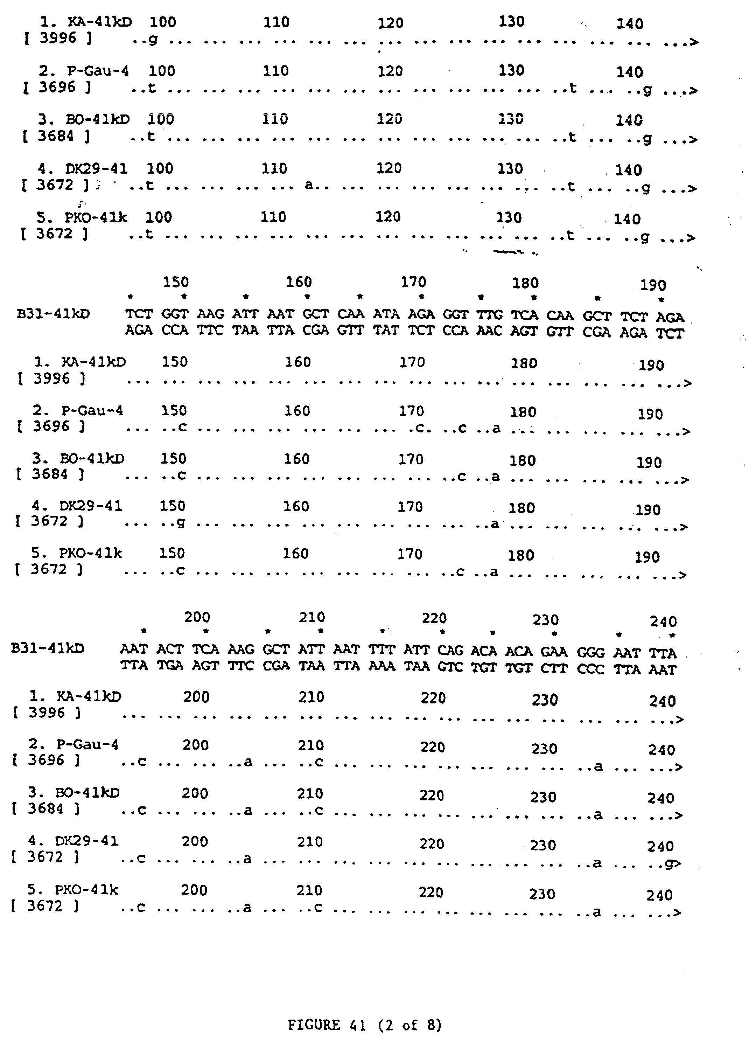

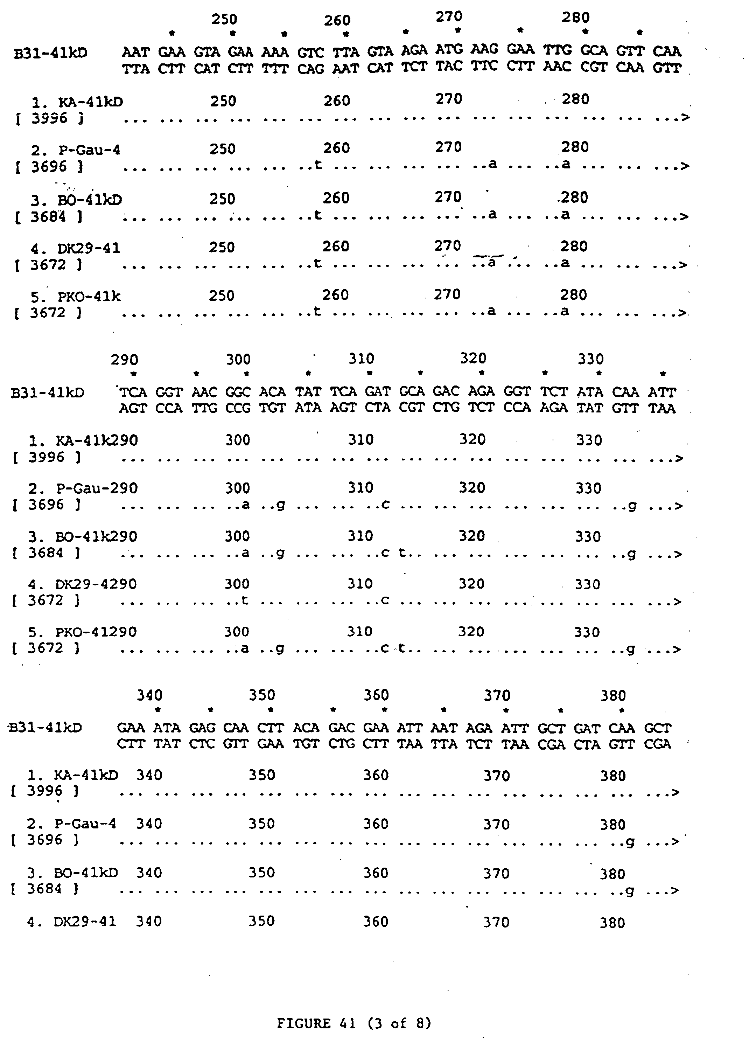

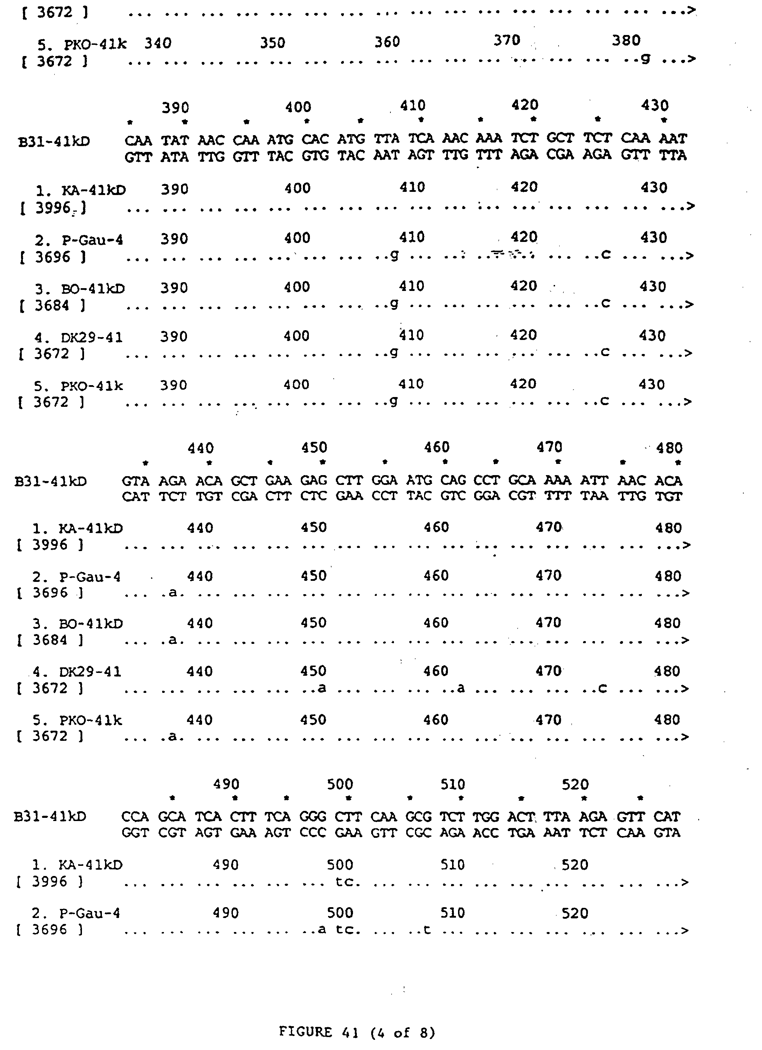

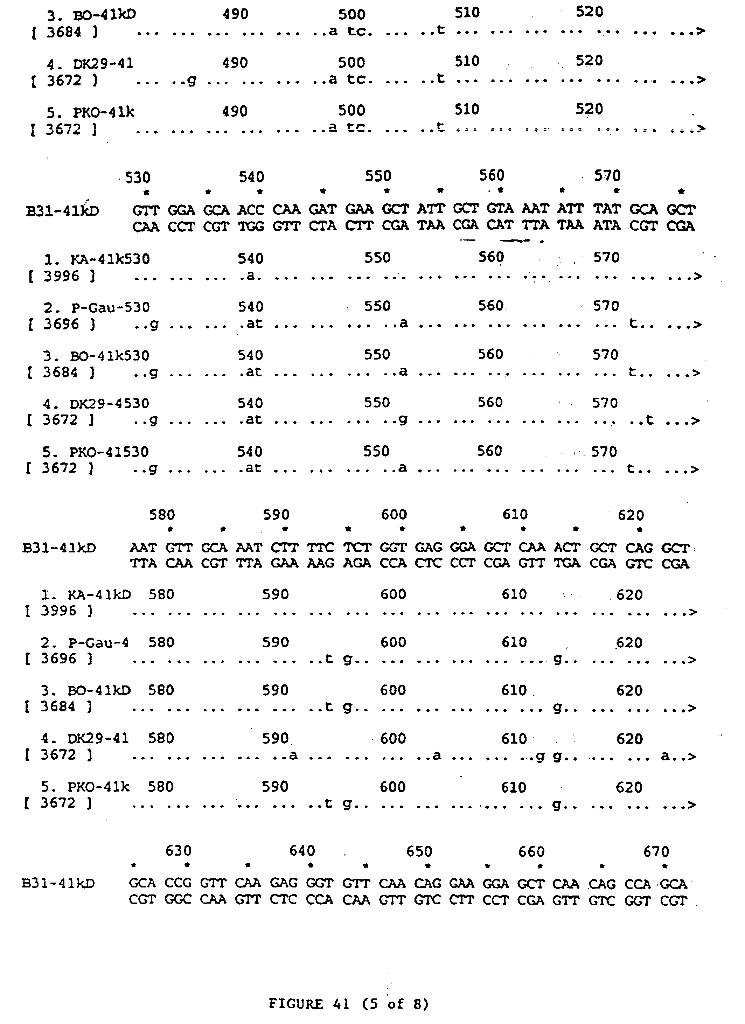

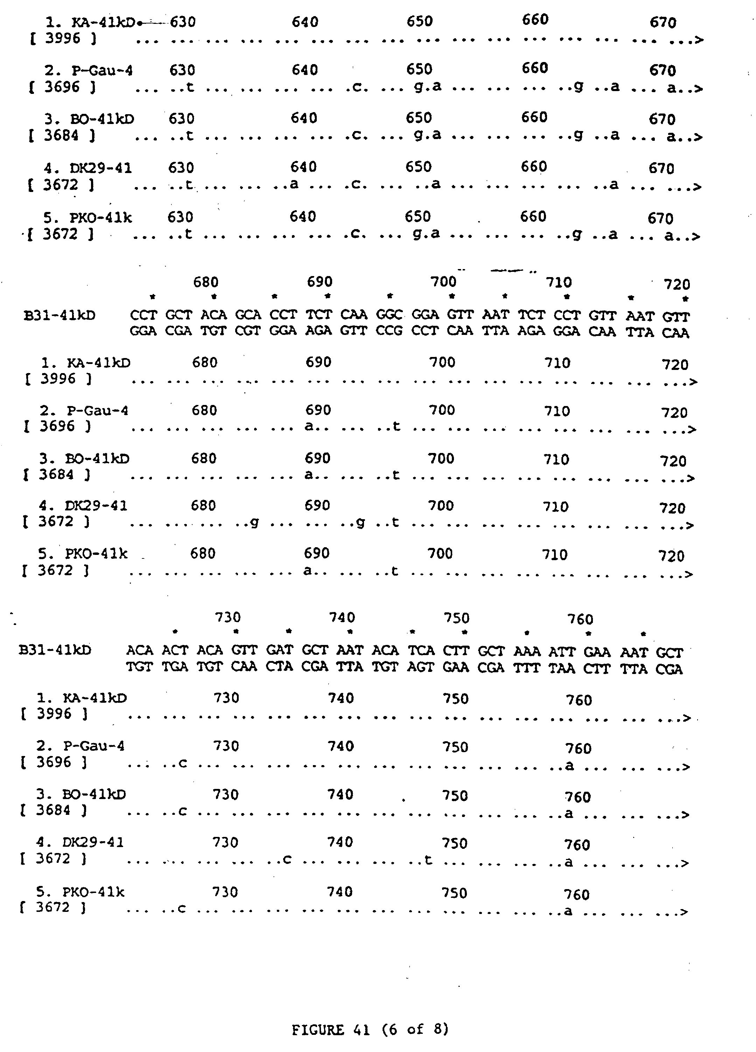

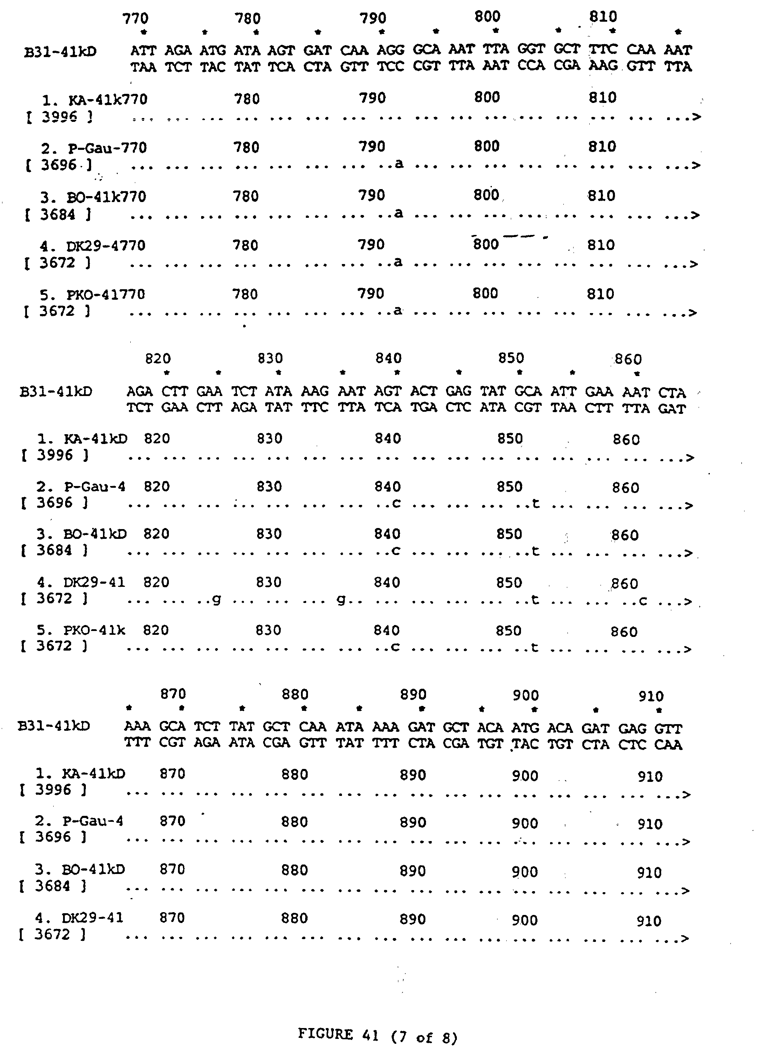

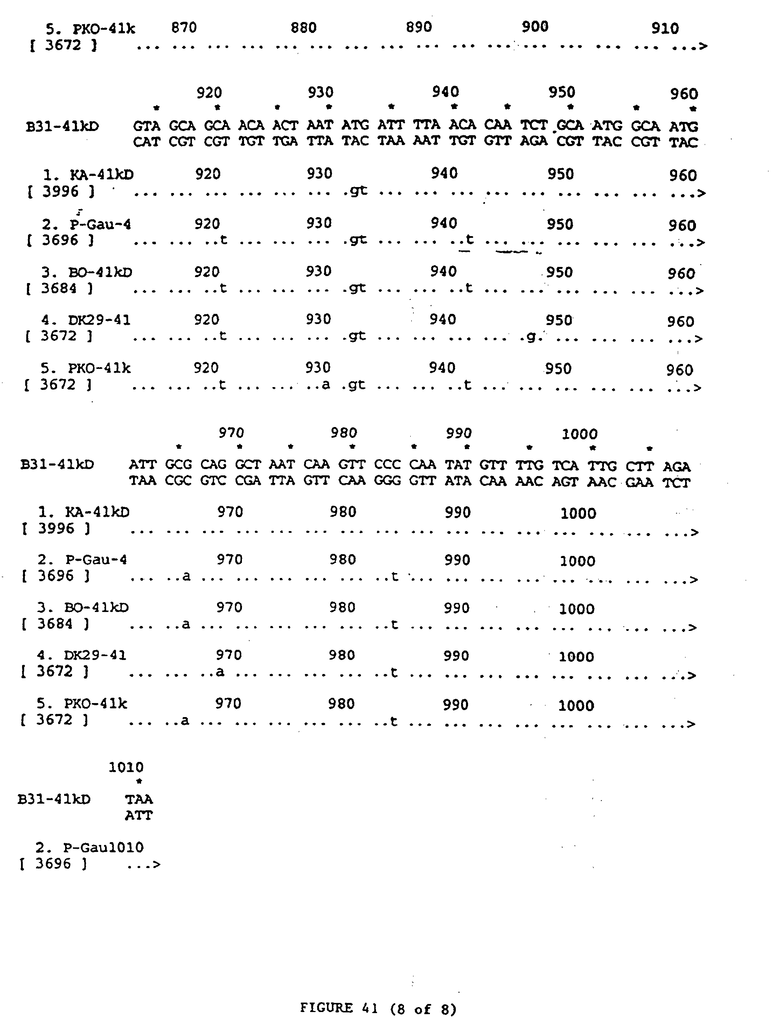

A similar approach was used to clone and sequence genes encoding the p41 (fla) protein. The p41 sequences listed in Table II with GenBank accession numbers were isolated using the following primers from strain B31:
5'-ATGATTATCAATCATAAT-3' (+1 to +18) (SEQ ID NO. 47); and
5'-TCTGAACAATGACAAAAC-3' (+1008 to +991) (SEQ ID NO. 48).
The nucleic acid sequences of p41 isolated in this manner are depicted in the sequence listing as SEQ ID NO. 51 (p41-PGau), and SEQ ID NO. 53 (p41-DK29). An alignment of several p41 nucleic acid sequences, including those for strains B31, pKa1, PGau, pBo, DK29, and pKo, is shown in Figure 41. The amino acid sequences of the proteins encoded by these nucleic acid sequences are represented as SEQ ID NO. 50 (p41-K48), SEQ ID NO. 52 (p41-PGau), SEQ ID NO. 54 (p41-DK29), SEQ ID NO. 56 (p41-PTrob), and SEQ ID NO. 58 (p41-PHei).

Other primers were designed to amplify nucleic acid sequences encoding polypeptides of p41, to be used in chimeric nucleic acid sequences. These primers included:
5'-TTGGATCCGGTCACCCCATGGCTCAATATAACCAATG-3' (minus strand primer #122) (SEQ ID NO. 59);
5'-TTGGATCCGGTCACCCCATGGCTTCTCAAAATGTAAG-3' (plus strand primer # 140) (SEQ ID NO. 60);
5'-TTGGATCCGGTGACCAACTCCGCCTTGAGAAGG-3' (minus strand primer # 234) (SEQ ID NO. 61); and
5'-TTGGATCCGGTGACCTATTTGAGCATAAGATGC-3' (minus strand primer #141) (SEQ ID NO. 62).

### Cloning and Sequencing of p93

The same approach was also used to clone and sequence p93 protein. Genes encoding p93, as listed in Table II with GenBank accession numbers, were isolated by this method with the following primers from strain B31:
5'-GGTGAATTTAGTTGGTAAGG-3' (-54 to -35) (SEQ ID NO. 63); and
5'-CACCAGTTTCTTTAAGCTGCTCCTGC-3' (+1117 to +1092) (SEQ ID NO. 64).

The nucleic acid sequences of p93 isolated in this manner are depicted in the sequence listing as SEQ ID NO. 65 (p93-B31), SEQ ID NO. 67 (p93-K48) SEQ ID NO. 69 (p93-PBo), SEQ ID NO. 71 (p93-PTrob), SEQ ID NO. 73 (p93-PGau), SEQ ID NO. 75 (p93-25015), and SEQ ID NO. 77 (p93-PKo). The amino acid sequences of the proteins encoded by these nucleic acid sequences are represented as SEQ ID NO. 66 (p93-B31), SEQ ID NO. 68 (p93-K48) SEQ ID NO. 70 (p93-PBo), SEQ ID NO. 72 (p93-PTrob), SEQ ID NO. 74 (p93-PGau), SEQ ID NO. 76 (p93-25015), and SEQ ID NO. 78 (p93-PKo).

Other primers were used to amplify nucleic acid sequences encoding polypeptides of p93 to be used in generating chimeric nucleic acid sequences. These primers included:
5'-CCGGTCACCCCATGGCTGCTTTAAAGTCTTTA-3' (plus strand primer #475) (SEQ ID NO. 79);
5'-CCGGTCACCCCATGAATCTTGATAAAGCTCAG-3' (plus strand primer #900) (SEQ ID NO. 80);
5'-CCGGTCACCCCATGGATGAAAAGCTTTTAAAAAGT-3' (plus strand primer #1168) (SEQ ID NO. 81);
5'-CCGGTCACCCCCATGGTTGAGAAATTAGATAAG-3' (plus strand primer #1423) (SEQ ID NO. 82); and
5'-TTGGATCCGGTGACCCTTAACTTTTTTTAAAG-3' (minus strand primer # 2100) (SEQ ID NO. 83).

### C. Expression of Proteins from Borrelia Genes

The nucleic acid sequences described above can be incorporated into expression plasmids, using standard techniques, and transfected into compatible host cells in order to express the proteins encoded by the nucleic acid sequences. As an example, the expression the p12 gene and the isolation of p12 protein is set forth.

Amplification of the p12 nucleic acid sequence was conducted with primers that included a NdeI restriction site into the nucleic acid sequence. The PCR product was extracted with phenol/chloroform and precipitated with ethanol. The precipitated product was digested and ligated into an expression plasmid as follows: 15 µl (approximately 1 µg) of PCR DNA was combined with 2 µl 10X restriction buffer for NdeI (Gibco/BRL), 1 µl NdeI (Gibco/BRL), and 2 µl distilled water, and incubated overnight at 37°C. This mixture was subsequently combined with 3 µl 10X buffer (buffer 3, New England BioLabs), 1 µl BamHI (NEB), and 6 µl distilled water, and incubated at 37° for two hours. The resultant material was purified by preparative gel electrophoresis using low melting point agarose, and the band was visualized under long wave ultraviolet light and excised from the gel. The gel slice was treated with Gelase using conditions recommended by the manufacturer (Epicentre Technologies). The resulting DNA polled was resuspended in 25-50 µl of 10 mM TRIS-CL (pH 8.0) and 1 mM EDTA (TE). An aliquot of this material was ligated into the Pet9c expression vector (Dunn, J. J. *et al.,* Protein Expression and Purification 1: 159 (1990)).

To ligate the material into the Pet9c expression vector, 20-50 ng of p12 nucleic acid sequences cut and purified as described above was combined with 5 µl 10 One-Phor-All (OPA) buffer (Pharmacia), 30-60 ng Pet9c cut with NdeI and BamHI, 2.5 µl 20 mM ATP, 2 µl T4 DNA ligase (Pharmacia) diluted 1:5 in 1X OPA buffer, and sufficient distilled water to bring the final volume to 50 µl. The mixture was incubated at 12°C overnight.

The resultant ligations were transformed into competent DH5-alpha cells and plated on nutrient agar plates containing 50 µg/ml kanamycin and incubated overnight at 37 °C. DH5-alpha is used as a "storage strain" for T7 expression clones, because it is RecA deficient, so that recombination and concatenation are not problematic, and because it lacks the T7 RNA polymerase gene necessary to express the cloned gene. The use of this strain allows for cloning of potentially toxic gene products while minimizing the chance of deletion and/or rearrangement of the desired genes. Other cell lines having similar properties may also be used.

Kanamycin resistant colonies were single-colony purified on nutrient agar plates supplemented with kanamycin at 50 µg/ml. A colony from each isolate was inoculated into 3-5 ml of liquid medium containing 50 µg/ml kanamycin, and incubated at 37°C without agitation. Plasmid DNA was obtained from 1 ml of each isolate using a hot alkaline lysis procedure (Mantiatis, T. *et al*., Molecular Cloning: A Laboratory Manual, cold Spring Harbor Laboratory, Cold Spring Harbor, NY (1982)).

Plasmid DNA was digested with EcoRI and BglII in the following manner: 15 µl plasmid DNA was combined with 2 µl 10X buffer 3 (NEB), 1 µ EcoRI (NEB), 1 µl BglII (NEB) and 1 µl distilled water, and incubated for two hours at 37°C. The entire reaction mixture was electrophoresed on an analytical agarose gel. Plasmids carrying the p12 insert were identified by the presence of a band corresponding to 925 base-pairs (full length p12) or 875 base-pairs (nonlipidated p12).
One or two plasmid DNAs from the full length and nonlipidated p12 clones in Pet9c were used to transform BL21 DE3 pLysS to kanamycin resistance as described by Studier *et al*. (Methods in Enzymology, Goeddel, D. (Ed.), Academic Press, 185: 60-89 (1990)). One or two transformants of the full length and nonlipidated clones were single-colony purified on nutrient plates containing 25 µg/ml chloramphenicol (to maintain pLysS) and 50 µg/ml kanamycin at 37 °C. One colony of each isolate was inoculated into liquid medium supplemented with chloramphenicol and kanamycin and incubated overnight at 37°C. The overnight culture was subcultured the following morning into 500 ml of liquid broth with chloramphenicol (25 µg/ml) and kanamycin (50 µg/ml) and grown with aeration at 37°C in an orbital air-shaker until the absorbance at 600 nm reached 0.4-0.7. Isopropyl-thio-galactoside (IPTG) was added to a final concentration of 0.5 mM, for induction, and the culture was incubated for 3-4 hours at 37° as before. The induced cells were pelleted by centrifugation and resuspended in 25 ml of 20 mM NaPO₄ (pH 7.7). A small aliquot was removed for analysis by gel electrophoresis. Expressing clones produced proteins which migrated at the 12 kDa position.

A crude cell lysate was prepared from the culture as described for recombinant OspA by Dunn, J.J. *et al*., (Protein Expression and Purification 1: 159 (1990)). The crude lysate was first passed over a Q-sepharose column (Pharmacia) which had been pre-equilibrated in Buffer A: 10 mM NaPO₄ (pH 7.7), 10 mM NaCl, 0.5 mM PMSF. The column was washed with 10 mM NaPO₄, 50 mM NaCl and 0.5 mM PMSF and then p12 was eluted in 10 mM NaPO₄, 0.5 mM PMSF with a NaCl gradient from 50-400 mM. p12 eluted approximately halfway through the gradient between 100 and 200 mM NaCl. The peak fractions were pooled and dialyzed against 10 mM NaPo4 (pH 7.7), 10 mM NaCl, 0.5 mM PMSF. The protein was then concentrated and applied to a Sephadex G50 gel filtration column of approximately 50 ml bed volume (Pharmacia), in 10 mM NaPO₄, 200 mM NaCl, 0.5 mM PMSF. p12 would typically elute shortly after the excluded volume marker. Peak fractions were determined by running small aliquots of all fractions on a gel. The p12 peak was pooled and stored in small aliquots at -20°C.

### Example 4. Generation of Chimeric Nucleic Acid Sequences and Chimeric Proteins

### A. General Protocol for Creation of Chimeric Nucleic Acid Sequences

The megaprimer method of site directed mutagenesis and its modification were used to generate chimeric nucleic acid sequences (Sarkar and Sommer, Biotechniques 8(4) : 404-407 (1990); Aiyar, A. and J. Leis, Biotechniques 14(3) : 366-369 (1993)). A 5' primer for the first genomic template and a 3' fusion oligo are used to amplify the desired region. the fusion primer consists of a 3' end of the first template (DNA that encodes the amino-proximal polypeptide of the fusion protein), coupled to a 5' end of the second template (DNA that encodes the carboxy-proximal polypeptide of the fusion protein).

The PCR amplifications are performed using Taq DNA polymerase, 10X PCR buffer, and MgCl₂ (Promega Corp., Madison, WI), and Ultrapure dNTPs (Pharmacia, Piscataway, NJ). One µg of genomic template 1, 5 µ of 10 µM 5' oligo and 5 µl of 10 µM fusion oligo are combined with the following reagents at indicated final concentrations: 10X Buffer-Mg FREE (1X), MgCl₂ (2 mM), dNTP mix (200 µM each dNTP), Taq DNA polymerase (2.5 units), water to bring final volume to 100 µl. A Thermal Cycler (Perkin Elmer Cetus, Norwalk, CT) is used to amplify under the following conditions: 35 cycles at 95°C for one minute, 55°C for two minutes, and 72° for three minutes. This procedure results in a "megaprimer".

The resulting megaprimer is run on a 1X TAE, 4% low-melt agarose gel. The megaprimer band is cut from the gel and purified using the Promega Magic PCR Preps DNA purification system. Purified megaprimer is then used in a second PCR step. One µg of genomic template 2, approximately 0.5 µg of the megaprimer, and 5 µ of 10 µM 3' oligo are added to a cocktail of 10X buffer, MgCl₂, dNTPs and Taq at the same final concentrations as noted above, and brought to 100 µl with water. PCR conditions are the same as above. The fusion product resulting from this amplification is also purified using the Promega Magic PCR Preps DNA purification system.

The fusion product is then ligated into TA vector and transformed into *E. coli* using the Invitrogen (San Diego, CA) TA Cloning Kit. Approximately 50 ng of PCR fusion product is ligated to 50 ng of pCRII vector with 1X Ligation Buffer, 4 units of T4 ligase, and brought to 10 Nl with water. This ligated product mixture is incubated at 12°C overnight (approximately 14 hours). Two µl of the ligation product mixture is added to 50 µl competent INC F' cells and 2 µ beta mercaptoethanol. The cells are then incubated for 30 minutes, followed by heat shock treatment at 42°C for 60 seconds, and an ice quenching for two minutes. 450 µl of warmed SOC media is then added to the cells, resulting in a transformed cell culture which is incubated at 37°C for one hour with slight shaking. 50 µl of the transformed cell culture is plated on LB + 50 µg/µl ampicillin plates and incubated overnight at 37°C. Single white colonies are picked and added to individual overnight cultures containing 3 ml LB with ampicillin (50 µg/µl).

The individual overnight cultures are prepared using Promega's Magic Miniprep DNA purification system. A small amount of the resulting DNA is cut using a restriction digest as a check. DNA sequencing is then performed to check the sequence of the fusion nucleic acid sequence, using the United States Biochemical (Cleveland, OH) Sequenase Version 2.0 DNA sequencing kit. Three to five µg of plasmid DNA is used per reaction. 2 µl 2M NaOH/2mM EDTA are added to the DNA, and the volume is brought to 20 µl with water. The mixture is then incubated at room temperature for five minutes. 7 µl water, 3µl 3M NaAc, 75 µl EtOH are added. The resultant mixture is mixed by vortex and incubated for ten minutes at -70°C, and then subjected to microfugation. After microfuge for ten minutes, the supernatant is aspirated off, and the pellet is dried in the speed vac for 30 second. 6 µl water, 2 µl annealing buffer, and 2 µl of 10 µM of the appropriate oligo is then added. This mixture is incubated for 10 minutes at 37°C and then allowed to stand at room temperature for 10 minutes. Subsequently, 5.5 µl of label cocktail (described above) is added to each sample of the mixture, which are incubated at room temperature for an additional five minutes. 3.5 µl labeled DNA is then added to each sample which is then incubated for five minutes at 37°C. 4 µl stop solution is added to each well. The DNA is denatured at 95° for two minutes, and then placed on ice.

Clones with the desired fusion nucleic acid sequences are then recloned in frame in the pEt expression system in the lipidated (full length) and non-lipidated (truncated, i.e., without first 17 amino acids) forms. The product is amplified using restriction sites contained in the PCR primers. The vector and product are cut with the same enzymes and ligated together with T4 ligase. The resultant plasmid is transformed into competent *E. coli* using standard transformation techniques. Colonies are screened as described earlier and positive clones are transformed into expression cells, such as E. coli BL21, for protein expression with IPTG for induction. The expressed protein in its bacterial culture lysate form and/or purified form is then injected in mice for antibody production. The mice are bled, and the sera collected for agglutination, *in vitro* growth inhibition, and complement- dependent and - independent lysis tests.

### B. Specific Chimeric Nucleic Acid Sequences

Various chimeric nucleic acid sequences were generated. The nucleic acid sequences are described as encoding polypeptides from *Borrelia* proteins. The chimeric nucleic acid sequences are produced such that the nucleic acid sequence encoding one polypeptide is in the same reading frame as the nucleic acid sequence encoding the next polypeptide in the chimeric protein sequence encoded by the chimeric nucleic acid sequence. The proteins are listed sequentially (in order of presence of the encoding sequence) in the description of the chimeric nucleic acid sequence. For example, if a chimeric nucleic acid sequence consists of bp 1-650 from OspA-1 and bp 651-820 from OspA-2 were sequenced, the sequence of the chimer would include the first 650 base pairs from OspA-1 followed immediately by base pairs 651-820 of OspA-2.

OspA-K48/OspA-PGau A chimer of OspA from strain K48 (OspA-K48) and OspA from strain PGau (OspA-PGau) was generated using the method described above. This chimeric nucleic acid sequence included bp 1-654 from OspA-K48, followed by bp 655-820 from OspA-PGau. Primers used included: the amino-terminal sequence of OspA primer #607 (SEQ ID NO. 16); the fusion primer,
5'-AAAGTAGAAGTTTTTGAATCCCATTTTCCAGTTTTTTT-3' (minus strand primer #668-654) (SEQ ID NO. 84); the carboxy-terminal sequence of OspA primer #586 (SEQ ID NO. 19); and the sequence primers #369 (SEQ ID NO. 14) and #357 (SEQ ID NO. 15). The chimeric nucleic acid sequence is presented as SEQ ID NO. 85; the chimeric protein encoded by this chimeric nucleic acid sequence is presented as SEQ ID NO. 86.

OspA-B31/OspA-PGau A chimer of OspA from strain B31 (OspA-B31) and OspA from strain PGau (OspA-PGau) was generated using the method described above. This chimeric nucleic acid sequence included bp 1-651 from OspA-B31, followed by bp 652-820 from OspA-PGau. Primers used included: the fusion primer,
5'-AAAGTAGAAGTTTTTGAATTCCAAGCTGCAGTTTT-3' (minus strand primer #668-651) (SEQ ID NO. 87); and the sequence primer, #369 (SEQ ID NO. 14). The chimeric nucleic acid sequence is presented as SEQ ID NO. 88; the chimeric protein encoded by this chimeric nucleic acid sequence is presented as SEQ ID NO. 89.

OspA-B31/OspA-K48 A chimer of OspA from strain B31 (OspA-B31) and OspA from strain K48 (OspA-K48) was generated using the method described above. This chimeric nucleic acid sequence included bp 1-651 from OspA-B31, followed by bp 652-820 from OspA-K48. Primers used included: the fusion primer,
5'-AAAGTGGAAGTTTTTGAATTCCAAGCTGCAGTTTTTTT-3' (minus strand primer #671-651) (SEQ ID NO. 90); and the sequence primer, #369 (SEQ ID NO. 14). The chimeric nucleic acid sequence is presented as SEQ ID NO. 91; the chimeric protein encoded by this chimeric nucleic acid sequence is presented as SEQ ID NO. 92.

OspA-B31/OspA-25015 A chimer of OspA from strain B31 (OspA-B31) and OspA from strain 25015 (OspA-25015) was generated using the method described above. This chimeric nucleic acid sequence included bp 1-651 from OspA-B31, followed by bp 652-820 from OspA-25015. Primers used included: the fusion primer, 5'-TAAAGTTGAAGTGCCTGCATTCCAAGCTGCAGTTT-3' (SEQ ID NO. 93). The chimeric nucleic acid sequence is presented as SEQ ID NO. 94; the chimeric protein encoded by this chimeric nucleic acid sequence is presented as SEQ ID NO. 95.

OspA-K48/OspA-B31/OspA-K48 A chimer of OspA from strain B31 (OspA-B31) and OspA from strain K48 (OspA-K48) was generated using the method described above. This chimeric nucleic acid sequence included bp 1-570 from OspA-B31, followed by bp 570-651 from OspA-B31, followed by bp 650-820 from OspA-K48. Primers used included: the fusion primer, 5'-CCCCAGATTTTGAAATCTTGCTTAAAACAAC-3' (SEQ ID NO. 96); and the sequence primer, #357 (SEQ ID NO. 15). The chimeric nucleic acid sequence is presented as SEQ ID NO. 97; the chimeric protein encoded by this chimeric nucleic acid sequence is presented as SEQ ID NO. 98.

OspA-B31/OspA-K48/OspA-B31/OspA-K48 A chimer of OspA from strain B31 (OspA-B31) and OspA from strain K48 (OspA-K48) was generated using the method described above. This chimeric nucleic acid sequence included bp 1-420 from OspA-B31, followed by 420-570 from OspA-K48, followed by bp 570-650 from OspA-B31, followed by bp 651-820 from OspA-K48. Primers used included: the fusion primer, 5'-CAAGTCTGGTTCCAATTTGCTCTTGTTATTAT-3' (minus strand primer #436-420) (SEQ ID NO. 99); and the sequence primer, #357 (SEQ ID NO. 15). The chimeric nucleic acid sequence is presented as SEQ ID NO. 100; the chimeric protein encoded by this chimeric nucleic acid sequence is presented as SEQ ID NO. 101.

OspA-B31/OspB-B31 A chimer of OspA and OspB from strain B31 (OspA-B31, OspB-B31) was generated using the method described above. The chimeric nucleic acid sequence included bp 1-651 from OspA-B31, followed by bp 652-820 from OspB-B31. Primers used included: the fusion primer, 5'-GTTAAAGTGCTAGTACTGTCATTCCAAGCTGCAGTTTTTTT-3' (minus strand primer #740-651) (SEQ ID NO. 102); the carboxy-terminal sequence of OspB primer #1106 (SEQ ID NO. 25); and the sequence primer #357 (SEQ ID NO. 15). The chimeric nucleic acid sequence is presented as SEQ ID NO. 103; the chimeric protein encoded by this chimeric nucleic acid sequence is presented as SEQ ID NO. 104.

OspA-B31/OspB-B31/OspC-B31 A chimer of OspA, OspB and OspC from strain B31 (OspA-B31, OspB-B31, and OspC-B31) was generated using the method described above. The chimeric nucleic acid sequence included bp 1-650 from OspA-B31, followed by bp 652-820 from OspB-B31, followed by bp 74-630 of OspC-B31. Primers used included: the fusion primer, 5'-TGCAGATGTAATCCCATCCGCCATTTTTAAAGCGTTTTT-3' (SEQ ID NO. 105); and the carboxy-terminal sequence of OspC primer (SEQ ID NO. 28). The chimeric nucleic acid sequence is presented as SEQ ID NO. 106; the chimeric protein encoded by this chimeric nucleic acid sequence is presented as SEQ ID NO. 107.

OspC-B31/OspA-B31/OspB-B31 A chimer of OspA, OspB and OspC from strain B31 (OspA-B31, OspB-B31, and OspC-B31) was generated using the method described above. The chimeric nucleic acid sequence included bp 1-630 from OspC-B31, followed by bp 52-650 from OspA-B31, followed by bp 650-820 of OspB-B31. Primers used included: the amino-terminal sequence of OspC primer having SEQ ID NO. 27; the fusion primer, 5'-GCTGCTAACATTTTGCTTAGGTTTTTTTGGACTTTC-3' (minus strand primer #69-630) (SEQ ID NO. 108); and the sequence primers #520 (SEQ ID NO. 40) and #200 (SEQ ID NO. 18). The chimeric nucleic acid sequence is presented as SEQ ID NO. 109; the chimeric protein encoded by this chimeric nucleic acid sequence is presented as SEQ ID NO. 110.

### Additional Chimeric Nucleic Acid Sequences

Using the methods described above, other chimeric nucleic acid sequences were produced. These chimeric nucleic acid sequences, and the proteins encoded, are summarized in Table 3.

**Table III**

| Chimeric Nucleic acid Sequences and the Encoded Proteins | | |
|---|---|---|
| Chimers Generated (base pairs) | SEQ ID No. (nt) | SEQ ID NO. (protein) |
| OspA (52-882) / p93 (1168-2100) | 111 | 112 |
| OspB (45-891) / p41 (122-234) | 113 | 114 |
| OspB (45-891) / p41 (122-295) | 115 | 116 |
| OspB (45-891) / p41 (140-234) | 117 | 118 |
| OspB (45-891) / p41 (140-295) | 119 | 120 |
| OspB (45-891) / p41 (122-234) / OspC (58-633) | 121 | 122 |
| OspA-Tro/OspA-Bo | 137 | 138 |
| OspA-PGau/OspA-Bo | 139 | 140 |
| OspA-B31/OspA-PGau/OspA-B31/ OspA-K48 | 141 | 142 |
| OspA-PGau/OspA-B31/OspA-K48 | 143 | 144 |

### C. Purification of Proteins Generated by Chimeric Nucleic Acid Sequences

The chimeric nucleic acid sequences described above, as well as chimeric nucleic acid sequences produced by the methods described above, are used to produce chimeric proteins encoded by the nucleic acid sequences. Standard methods, such as those described above in Example 3, concerning the expression of proteins from Borrelia genes, can be used to express the proteins in a compatible host organism. The chimeric proteins can then be isolated and purified using standard techniques.

If the chimeric protein is soluble, it can be purified on a Sepharose column. Insoluble proteins can be solubilized in guanidine and purified on a Ni++ column; alternatively, they can be solubilized in 10 mM NaPO₄ with 0.1 - 1% TRIXON X 114, and subsequently purified over an S column (Pharmacia). Lipidated proteins were generally purified by the latter method. Solubility was determined by separating both soluble and insoluble fractions of cell lysate on a 12% PAGE gel, and checking for the localization of the protein by Coomasie staining, or by Western blotting with monoclonal antibodies directed to an antigenic polypeptide of the chimeric protein.

### Equivalents

Those skilled in the art will recognize, or be able to ascertain using no more than routine experimentation, many equivalents to the specific embodiments of the invention described herein. such equivalents are intended to be encompassed in the scope of the following claims.

## Claims

1. A recombinantly produced, chimeric protein comprising two polypeptides derived from outer surface protein A or outer surface protein B from different Lyme Disease causing strains of *Borrelia* wherein the first polypeptide comprises outer surface protein A or B from the N-terminus to and including a conserved tryptophan and the second polypeptide comprises outer surface protein A or B from the conserved tryptophan to the C-terminus of the protein wherein said first polypeptide and said second polypeptide are from different strains of Lyme Disease causing *Borrelia* and wherein the amino acid sequence of said chimeric protein is not the same as the amino acid sequence of either outer surface protein A or B from which said first polypeptide and said second polypeptide are obtained and wherein each polypeptide retains antigenicity in the chimeric protein.

2. The chimeric protein of Claim 1, wherein:
a) the first polypeptide comprises outer surface protein A or B from the N-terminus to and including a conserved tryptophan, wherein said first polypeptide includes hypervariable regions of outer surface protein A comprising residues 120 through 140, residues 150 through 180, and residues 200 through 217, and
b) the second polypeptide comprises outer surface protein A or outer surface protein B from the conserved tryptophan to the C-terminus of the protein; wherein the Lyme disease causing strain of *Borrelia* from which at least one of the group consisting of: any one of said hypervariable regions and said second polypeptide differs from the Lyme disease causing strain of *Borrelia* from which the remainder of said first polypeptide is obtained,
and wherein the hypervariable region comprising residues 200 through 217 and said second polypeptide are from different Lyme disease causing strains of *Borrelia,* and wherein the amino acid sequence of said chimeric protein is not the same as the amino acid sequence of any outer surface protein A or B from which said first polypeptide and said second polypeptide are obtained and wherein the chimeric protein retains antigenicity representative of the OspA or B protein of the parent *Borrelia* strains.

3. The chimeric protein of Claim 2, wherein the first and second hypervariable domains are derived from outer surface protein A from different strains of *Borrelia* burgdorferi.

4. The nucleic acid sequence encoding a recombinantly produced, chimeric protein comprising two polypeptides derived from outer surface protein A or outer surface protein B from different Lyme Disease causing strains of *Borrelia* wherein the first polypeptide comprises outer surface protein A or B from the N-terminus to and including a conserved tryptophan and the second polypeptide comprises outer surface protein A or B from the conserved tryptophan to the C-terminus of the protein wherein said first polypeptide and said second polypeptide are from different strains of Lyme Disease causing *Borrelia* and wherein the amino acid sequence of said chimeric protein is not the same as the amino acid sequence of either outer surface protein A or B from which said first polypeptide and said second polypeptide are obtained and wherein each polypeptide retains antigenicity in the chimeric protein.

5. The nucleic acid sequence of Claim 4, wherein:
a) the first polypeptide comprising outer surface protein A or B from the N-terminus to and including a conserved tryptophan, wherein said first polypeptide includes hypervariable regions of outer surface protein A comprising residues 120 through 140, residues 150 through 180, and residues 200 through 217, and
b) the second polypeptide comprising outer surface protein A or outer surface protein B from the conserved tryptophan to the C-terminus of the protein; wherein the Lyme disease causing strain of *Borrelia* from which at least one of the group consisting of: any one of said hypervariable regions and said second polypeptide differs from the Lyme disease causing strain of *Borrelia* from which the remainder of said first polypeptide is obtained,
and wherein the hypervariable region comprising residues 200 through 217 and said second polypeptide are from different Lyme disease causing strains of *Borrelia*, and wherein the amino acid sequence of said chimeric protein is not the same as the amino acid sequence of any outer surface protein A or B from which said first polypeptide and said second polypeptide are obtained and wherein the chimeric protein retains antigenicity representative of the OspA or B protein of the parent *Borrelia* strains.

6. The nucleic acid sequence of Claim 5, wherein the first and second hypervariable domains are derived from outer surface protein A from different strains of *Borrelia* burgdorferi.

7. A nucleic acid sequence having a sequence selected from the group consisting of: SEQ ID NO: 85, SEQ ID NO: 88, SEQ ID NO: 91, SEQ ID NO: 94, SEQ ID NO: 97, SEQ ID NO: 100, SEQ ID NO: 103, SEQ ID NO: 106, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 137, SEQ ID NO: 139, SEQ ID NO: 141, and SEQ NO: 143, or a protein having an amino acid sequence selected from the groups consisting of: SEQ ID NO: 86, SEQ ID NO: 89, SEQ ID NO: 92, SEQ ID NO: 95, SEQ ID NO: 98, SEQ ID NO: 101, SEQ ID NO: 104, SEQ ID NO: 107, SEQ ID NO: 110, SEQ ID NO: 112, SEQ ID NO: 114, SEQ ID NO: 116, SEQ ID NO: 118, SEQ ID NO: 120, SEQ ID NO: 122, SEQ ID NO: 138, SEQ ID NO: 140, SEQ ID NO: 142, and SEQ NO: 144.

8. A recombinantly produced, chimeric protein according to any one of Claims 1 to 3 and 7 for use in therapy or diagnosis, for example as a vaccine against *Borrelia burgdorferi* infection, in immunodiagnostic assays to detect the presence of antibodies to *Borrelia burgdorferi* or to measure T-cell reactivity, the immunodiagnostic assay for example being a dot blot, Western blot, ELISA or agglutination assay.

9. Use of the chimeric protein according to any one of Claims 1 to 3 and 7 or the nucleic acid sequence of any one of the Claim 4 to 7, for the manufacture of a compound for use in therapy or diagnosis, for example as a vaccine against *Borrelia burgdorferi* infection, in immunodiagnostic assays to detect the presence of antibodies to *Borrelia burgdorferi* or to measure T-cell reactivity, the immunodiagnostic assay for example being a dot blot, Western blot, ELISA or agglutination assay.

## Patentansprüche

1. Rekombinant hergestelltes, chimäres Protein, das zwei Polypeptide aufweist, die vom äußeren Oberflächenprotein A oder äußeren Oberflächenprotein B aus unterschiedlichen Lyme-Borreliose verursachenden Stämmen der Borrelia abgeleitet sind, wobei das erste Polypeptid des äußeren Oberflächenprotein A oder B vom N-Terminus bis einschließlich einem konservierten Tryptophan umfasst, und das zweite Polypeptid das äußere Oberflächenprotein A oder B vom konservierten Tryptophan zum C-terminalen Ende des Proteins umfasst, wobei besagtes erstes Polypeptid und besagtes zweites Polypeptid aus unterschiedlichen Stämmen der Lyme-Borreliose verursachenden Borrelia sind, und wobei die Aminosäuresequenz des besagten chimären Proteins nicht die gleiche ist wie die Aminosäuresequenz von entweder dem äußeren Oberflächenprotein A oder B, von denen das erste Polypeptid und das zweite Polypeptid erhalten werden, und worin jedes Polypeptid im chimären Protein Antigenizität bewahrt.

2. Chimäres Protein nach Anspruch 1, bei dem:
a) das erste Polypeptid das äußere Oberflächenprotein A oder B vom N-Terminus bis einschließlich einem konservierten Tryptophan umfasst, wobei das erste Polypeptid hypervariable Bereiche des äußeren Oberflächenproteins A, welche die Reste 120 bis einschließlich 140, die Reste 150 bis einschließlich 180 und die Reste 200 bis einschließlich 217 aufweisen, einschließt, und
b) das zweite Polypeptid das äußere Oberflächenprotein A oder das Oberflächenprotein B vom konservierten Tryptophan zum C-terminalen Ende des Proteins umfasst; , wobei der Lyme-Borreliose verursachende Stamm der Borrelia, von dem zumindest ein Bestandteil aus der Gruppe, bestehend aus: jeglichem der hypervariablen Bereiche und dem zweiten Polypeptid, erhalten ist, sich vom Lyme-Borreliose verursachenden Stamm der Borrelia, woraus der Rest des ersten Polypeptid erhalten ist, unterscheidet, und wobei der hypervariable Bereich, der die Reste 200 bis einschließlich 217 umfasst, und das zweite Polypeptid von verschiedenen Lyme-Borreliose verursachenden Stämmen der Borrelia sind, und worin die Aminosäuresequenz des chimären Proteins nicht die gleiche ist wie die Aminosäuresequenz eines beliebigen anderen, äußeren Oberflächenproteins A oder B, woraus das erstes Polypeptid und das zweite Polypeptid erhalten wurden, und worin das chimäre Protein die für das äußere Oberflächenprotein A oder B der Mutter-Borrelia-Stämme charakteristische Antigenizität bewahrt.

3. Chimäres Protein nach Anspruch 2, wobei der erste und der zweite hypervariable Bereich vom äußeren Oberflächenproteinen A aus unterschiedlichen Stämmen der Borrelia-burgdorferi abgeleitet sind.

4. Nucleinsäeresequenz, die ein rekombiniert hergestelltes chimäres Protein kodiert, das zwei Polypeptide aufweist, die vom äußeren Oberflächenprotein A oder äußeren Oberflächenprotein B aus unterschiedlichen Lyme-Borreliose verursachenden Stämmen der Borrelia abgeleitet sind, wobei das erste Polypeptid des äußeren Oberflächenprotein A oder B vom N-Terminus bis einschließlich einem konservierten Tryptophan umfasst, und das zweite Polypeptid das äußere Oberflächenprotein A oder B vom konservierten Tryptophan zum C-terminalen Ende des Proteins umfasst, wobei besagtes erstes Polypeptid und besagtes zweites Polypeptid aus unterschiedlichen Stämmen der Lyme-Borreliose verursachenden Borrelia sind, und wobei die Aminosäuresequenz des besagten chimären Proteins nicht die gleiche ist wie die Aminosäuresequenz von entweder dem äußeren Oberflächenprotein A oder B, von denen das erste Polypeptid und das zweite Polypeptid erhalten werden, und worin jedes Polypeptid im chimären Protein Antigenizität bewahrt.

5. Nucleinsäuresequent nach Anspruch 4, bei dem:
a) das erste Polypeptid das äußere Oberflächenprotein A oder B vom N-Terminus bis einschließlich einem konservierten Tryptophan umfasst, wobei das erste Polypeptid hypervariable Bereiche des äußeren Oberflächenproteins A, welche die Reste 120 bis einschließlich 140, die Reste 150 bis einschließlich 180 und die Reste 200 bis einschließlich 217 aufweisen, einschließt, und
b) das zweite Polypeptid das äußere Oberflächenprotein A oder das Oberflächenprotein B vom konservierten Tryptophan zum C-terminalen Ende des Proteins umfasst; wobei der Lyme-Borreliose verursachende Stamm der Borrelia, von dem zumindest ein Bestandteil aus der Gruppe, bestehend aus: jeglichem der hypervariablen Bereiche und dem zweiten Polypeptid, erhalten ist, sich vom Lyme-Borreliose verursachenden Stamm der Borrelia, woraus der Rest des ersten Polypeptid erhalten ist, unterscheidet, und wobei der hypervariable Bereich, der die Reste 200 bis einschließlich 217 umfasst, und das zweite Polypeptid von verschiedenen Lyme-Borreliose verursachenden Stämmen der Borrelia sind, und worin die Aminosäuresequenz des chimären Proteins nicht die gleiche ist wie die Aminosäuresequenz eines beliebigen anderen, äußeren Oberflächenproteins A oder B, woraus das erstes Polypeptid und das zweite Polypeptid erhalten wurden, und worin das chimäre Protein die für das äußere Oberflächenprotein A oder B der Mutter-Borrelia-Stämme charakteristische Antigenizität bewahrt.

6. Nucleinsäuresequenz nach Anspruch 5, wobei der erste und der zweite hypervariable Bereich vom äußeren Oberflächenprotein A aus unterschiedlichen Stämmen der Borrelia-burgdorferi abgeleitet sind.

7. Nucleinsäuresequenz mit einer Sequenz, ausgewählt aus der Gruppe bestehend aus: SEQ ID NO (Sequenzidentitätsnummer): 85, SEQ ID NO: 88, SEQ ID NO: 91, SEQ ID NO: 94, SEQ ID NO: 97, SEQ ID NO: 100, SEQ ID NO: 103, SEQ ID NO: 106, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 137, SEQ ID NO: 139, SEQ ID NO: 141 und SEQ ID NO: 143, oder ein Protein mit einer Aminosäuresequenz, ausgewählt aus der Gruppe, bestehend aus SEQ ID NO: 86, SEQ ID NO: 89, SEQ ID NO: 92, SEQ ID NO: 95, SEQ ID NO: 98, SEQ ID NO: 101, SEQ ID NO: 104, SEQ ID NO: 107, SEQ ID NO: 110, SEQ ID NO: 112, SEQ ID NO: 114, SEQ ID NO: 116, SEQ ID NO: 118, SEQ ID NO: 120, SEQ ID NO: 122, SEQ ID NO: 138, SEQ ID NO: 140, SEQ ID NO: 142 und SEQ ID NO: 144.

8. Rekombiniant hergestelltes, chimäres Protein nach einem der Ansprüche 1 bis 3 und 7 zur Verwendung in Therapie oder Diagnose, beispielsweise als Impfstoff gegen eine Borrelia burgdorferi Infektion, in immundiaganostischen Untersuchungen, um die Gegenwart von Antikörpern auf Borrelia burgdorferi zu testen oder zum Messen der T-Zellen Reaktivität, wobei die immundiagnostische Untersuchung beispielsweise ein Dotblot, ein Westemblot, ELISA oder ein Agglutinationsassay ist.

9. Verwendung eines chimären Proteins nach einem der Ansprüche 1 bis 3 und 7, oder der Nucleinsäuresequenz nach einem der Ansprüche 4 bis 7 zur Herstellung einer Verbindung zur Verwendung in Therapie und Diagnose, beispielsweise als Impfstoff gegen eine Borrelia burgdorferi Infektion, in immundiagnostischen Untersuchungen, um die Gegenwart von Antikörpern auf Borrelia burgdorferi zu testen oder zum Messen der T-Zellen Reaktivitäten, wobei die immundiagnostische Untersuchung beispielsweise ein Dotblot, ein Westemblot, ELISA oder ein Agglutinationsassay ist.

## Revendications

1. Protéine chimérique recombinante comprenant deux polypeptides dérivés de la protéine A de la surface externe ou de la protéine B de la surface externe de différentes souches de *Borrelia* responsables de la maladie de Lyme, dans laquelle le premier polypeptide comprend la protéine A ou B de la surface externe depuis l'extrémité N-terminale jusqu'à et comprenant un tryptophane conservé et le second polypeptide comprend la protéine A ou B de la surface externe depuis le tryptophane conservé jusqu'à l'extrémité C-terminale de la protéine, dans laquelle ledit premier polypeptide et ledit second polypeptide proviennent de différentes souches de *Borrelia* responsables de la maladie de Lyme et dans laquelle la séquence d'acides aminés de ladite protéine chimérique n'est pas la même que la séquence d'acides aminés de l'une ou l'autre de la protéine A ou B de la surface externe à partir de laquelle ledit premier polypeptide et ledit second polypeptide sont obtenus et dans laquelle chaque polypeptide conserve l'antigénicité de la protéine chimérique.

2. Protéine chimérique selon la revendication 1, dans laquelle :
a) le premier polypeptide comprend la protéine A ou B de la surface externe depuis l'extrémité N-terminale jusqu'à et comprenant un tryptophane conservé, dans laquelle ledit polypeptide comprend des régions hypervariables de la protéine A de la surface externe comprenant les résidus 120 à 140, les résidus 150 à 180 et les résidus 200 à 217 ; et
b) le second polypeptide comprend la protéine A de la surface externe ou la protéine B de la surface externe depuis le tryptophane conservé jusqu'à l'extrémité C-terminale de la protéine ;
dans laquelle la souche de *Borrelia* responsable de la maladie de Lyme à partir de laquelle au moins l'un du groupe constitué de : l'une quelconque desdites régions hypervariables et dudit second polypeptide, diffère de la souche de *Borrelia* responsable de la maladie de Lyme à partir de laquelle le reste dudit premier polypeptide est obtenu,
et dans laquelle la région hypervariable comprenant les résidus 200 à 217 et ledit second polypeptide proviennent de différentes souches de *Borrelia* responsables de la maladie de Lyme, et dans laquelle la séquence d'acides aminés de ladite protéine chimérique n'est pas la même que la séquence d'acides aminés de l'une ou l'autre de la protéine A ou B de la surface externe à partir de laquelle ledit premier polypeptide et ledit second polypeptide sont obtenus et dans laquelle la protéine chimérique conserve l'antigénicité représentative de la protéine OspA ou B des souches parentes de *Borrelia*.

3. Protéine chimérique selon la revendication 2, dans laquelle les premier et second domaines hypervariables sont dérivés de la protéine A de la surface externe provenant de différentes souches de *Borrelia burgdorferi*.

4. Séquence d'acide nucléique codant pour une protéine chimérique recombinante comprenant deux polypeptides dérivés de la protéine A de la surface externe ou de la protéine B de la surface externe provenant de différentes souches de *Borrelia* responsables de la maladie de Lyme, dans laquelle le premier polypeptide comprend la protéine A ou B de la surface externe depuis l'extrémité N-terminale jusqu'à et comprenant un tryptophane conservé et le second polypeptide comprend la protéine A ou B de la surface externe depuis le tryptophane conservé jusqu'à l'extrémité C-terminale de la protéine, dans laquelle ledit premier polypeptide et ledit second polypeptide proviennent de différentes souches de *Borrelia* responsables de la maladie de Lyme et dans laquelle la séquence d'acides aminés de ladite protéine chimérique n'est pas la même que la séquence d'acides aminés de l'une ou l'autre de la protéine A ou B de la surface externe à partir de laquelle ledit premier polypeptide et ledit second polypeptide sont obtenus et dans laquelle chaque polypeptide conserve l'antigénicité de la protéine chimérique.

5. Séquence d'acide nucléiques selon la revendication 4, dans laquelle :
a) le premier polypeptide comprenant la protéine A ou B de la surface externe depuis l'extrémité N-terminale jusqu'à et comprenant un tryptophane conservé, dans laquelle ledit premier polypeptide comprend des régions hypervariables de la protéine A de la surface externe comprenant les résidus 120 à 140, les résidus 150 à 180 et les résidus 200 à 217 et
b) le second polypeptide comprenant la protéine A de la surface externe ou la protéine B de la surface externe depuis le tryptophane conservé jusqu'à l'extrémité C-terminale de la protéine ;
dans laquelle la souche de *Borrelia* responsable de la maladie de Lyme à partir de laquelle au moins l'un du groupe constitué de : l'une quelconque desdites régions hypervariables et dudit second polypeptide, diffère de la souche de *Borrelia* responsable de la maladie de Lyme à partir de laquelle le reste dudit premier polypeptide est obtenu,
et dans laquelle la région hypervariable comprenant les résidus 200 à 217 et ledit second polypeptide proviennent de différentes souches de *Borrelia* responsables de la maladie de Lyme, et dans laquelle la séquence d'acides aminés de ladite protéine chimérique n'est pas la même que la séquence d'acides aminés de l'une ou l'autre de la protéine A ou B de la surface externe à partir de laquelle ledit premier polypeptide et ledit second polypeptide sont obtenus et dans laquelle la protéine chimérique conserve l'antigénicité représentative de la protéine OspA ou B des souches parentes de *Borrelia*.

6. Séquence d'acide nucléique selon la revendication 5, dans laquelle les premier et second domaines hypervariables sont dérivés de la protéine A de la surface externe provenant de différentes souches de *Borrelia burgdorferi*.

7. Séquence d'acides nucléiques ayant une séquence sélectionnée dans le groupe constitué de: SEQ ID n° 85, SEQ ID n° 88, SEQ ID n° 91, SEQ ID n° 94, SEQ ID n° 97, SEQ ID n° 100, SEQ ID n° 103, SEQ ID n° 106, SEQ ID n° 109, SEQ ID n° 111, SEQ ID n° 113, SEQ ID n° 115, SEQ ID n° 117, SEQ ID n° 119, SEQ ID n° 121, SEQ ID n° 137, SEQ ID n° 139, SEQ ID n° 141 et SEQ ID n° 143, ou protéine ayant une séquence d'acides aminés sélectionnée dans le groupe constitué de: SEQ ID n° 86, SEQ ID n° 89, SEQ ID n° 92, SEQ ID n° 95, SEQ ID n° 98, SEQ ID n° 101, SEQ ID n° 104, SEQ ID n° 107, SEQ ID n° 110, SEQ ID n° 112, SEQ ID n° 114, SEQ ID n° 116, SEQ ID n° 118, SEQ ID n° 120, SEQ ID n° 122, SEQ ID n° 138, SEQ ID n° 140, SEQ ID n° 142 et SEQ ID n° 144.

8. Protéine chimérique recombinante selon l'une quelconque des revendications 1 à 3 et 7 pour une utilisation en thérapie ou en diagnostic, par exemple en tant que vaccin contre une infection à *Borrelia burgdorferi*, dans des tests d'immunodiagnostic pour détecter la présence d'anticorps dirigés contre *Borrelia burgdorferi* ou pour mesurer la réactivité des cellules T, le test d'immunodiagnostic étant, par exemple, un test dot blot, Western blot, ELISA ou d'agglutination.

9. Utilisation de la protéine chimérique selon l'une quelconque des revendications 1 à 3 et 7 ou de la séquence d'acide nucléique selon l'une quelconque des revendications 4 à 7, pour la fabrication d'un composé pour une utilisation en thérapie ou en diagnostic, par exemple en tant que vaccin contre une infection à *Borrelia burgdorferi*, dans des tests d'immunodiagnostic pour détecter la présence d'anticorps dirigés contre *Borrelia burgdorferi* ou pour mesurer la réactivité des cellules T, le test d'immuno-diagnostic étant, par exemple, un test dot blot, Western blot, ELISA ou d'agglutination.


### SEQUENCE LISTING

(1) GENERAL INFORMATION:
   (i) APPLICANT:
      (A) NAME: Associated Universities, Inc.
      (B) STREET: Suite 730 1400 16th Street, NW
      (C) CITY: Washington
      (D) STATE/PROVINCE: District of Columbia
      (E) COUNTRY: United States of America
      (F) POSTAL CODE/ZIP: 20036
      (G) TELEPHONE: (516) 282-7338
      (I) TELEFAX:

   (i) INVENTOR:
      (A) NAME: Dunn, John J.
      (B) STREET: 5 Mott Drive
      (C) CITY: Bellport
      (D) STATE/PROVINCE: New York
      (E) COUNTRY: United States of America
      (F) POSTAL CODE/ZIP: 11713
      (G) TELEPHONE:
      (I) TELEFAX:

   (i) INVENTOR:
      (A) NAME: Luft, Benjamin J.
      (B) STREET: 223 Lincoln Avenue
      (C) CITY: Port Jefferson
      (D) STATE/PROVINCE: New York
      (E) COUNTRY: United States of America
      (F) POSTAL CODE/ZIP: 11777
      (G) TELEPHONE:
      (I) TELEFAX:
   (ii) TITLE OF INVENTION: Chimeric Proteins Comprising Borrelia Polypeptides; Uses Therefor
   (iii) NUMBER OF SEQUENCES: 144
   (iv) CORRESPONDENCE ADDRESS:
      (A) ADDRESSEE: Hamilton, Brook, Smith & Reynolds, P.C.
      (B) STREET: Two Militia Drive
      (C) CITY: Lexington
      (D) STATE: MA
      (E) COUNTRY: USA
      (F) ZIP: 02173
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT/US94/12352
      (B) FILING DATE: 27-OCT-1994
      (C) CLASSIFICATION:
   (vii) PRIOR APPLICATION DATA:
      (A) APPLICATION NUMBER: US 235,836
      (B) FILING DATE: 29-APRIL-94
   (vii) PRIOR APPLICATION DATA:
      (A) APPLICATION NUMBER: US 08/148,191
      (B) FILING DATE: 01-NOV-1993
   (viii) ATTORNEY/AGENT INFORMATION:
      (A) NAME: Granahan, Patricia
      (B) REGISTRATION NUMBER: 32,227
      (C) REFERENCE/DOCKET NUMBER: BNL93-28A PCT
   (ix) TELECOMMUNICATION INFORMATION:
      (A) TELEPHONE: (617) 861-6240
      (B) TELEFAX: (617) 861-9540
(2) INFORMATION FOR SEQ ID NO:1:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:1:
   CTTAATGACT CTGACACTAG TGC 23
(2) INFORMATION FOR SEQ ID NO:2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:2:
   GCTACTAAAA AAACCGGGAA ATGGAATTCA 30
(2) INFORMATION FOR SEQ ID NO:3:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:3:
   GCAGCTTGGG ATTCAAAAAC ATCCACTTTA ACA 33
(2) INFORMATION FOR SEQ ID NO:4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:4:
   GGAGAATATA TTATGAAA 18
(2) INFORMATION FOR SEQ ID NO:5:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:5:
   CTCCTTATTT TAAAGCG                                                17
(2) INFORMATION FOR SEQ ID NO:6:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Gly Thr Ser Thr Leu Thr
       210                 215                 220
   Ile Thr Val Asn Ser Lys Lys Thr Lys Asp Leu Val Phe Thr Lys Glu
   225                 230                 235                 240
   Asn Thr Ile Thr Val Gln Gln Tyr Asp Ser Asn Gly Thr Lys Leu Glu
                   245                 250                 255
   Gly Ser Ala Val Glu Ile Thr Lys Leu Asp Glu Ile Lys Asn Ala Leu
               260                 265                 270
   Lys
(2) INFORMATION FOR SEQ ID NO:8:
   (i) SEQUENCE CHARACTERISTICS:
      (A) LENGTH: 825 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..825
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 274 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Lys Thr Ser Thr Leu
       210                 215                 220
   Thr Ile Ser Val Asn Ser Gln Lys Thr Lys Asn Leu Val Phe Thr Lys
   225                 230                 235                 240
   Glu Asp Thr Ile Thr Val Gln Lys Tyr Asp Ser Ala Gly Thr Asn Leu
                   245                 250                 255
   Glu Gly Lys Ala Val Glu Ile Thr Thr Leu Lys Glu Leu Lys Asn Ala
               260                 265                 270
   Leu Lys
(2) INFORMATION FOR SEQ ID NO:10:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                     230                     235                     240
   Tyr Thr Ile Thr Val Lys Gln Tyr Asp Ser Ala Gly Thr Asn Leu Glu
                   245                     250                     255
   Gly Thr Ala Val Glu Ile Lys Thr Leu Asp Glu Leu Lys Asn Ala Leu
               260                     265                     270
   Lys
(2) INFORMATION FOR SEQ ID NO:12:
   (i) SEQUENCE CHARACTERISTICS:
      (A) LENGTH: 819 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..819
(2) INFORMATION FOR SEQ ID NO:13:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Met Ala Thr Val Asp Lys Leu Glu Leu Lys
        50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Val Ser Asp Asp Leu Ser Thr
                   85                  90                  95
   Thr Thr Leu Glu Val Leu Lys Glu Asp Gly Lys Thr Leu Val Ser Lys
               100                 105                 110
   Lys Arg Thr Ser Lys Asp Lys Ser Ser Thr Glu Glu Lys Phe Asn Glu
           115                 120                 125
   Lys Gly Glu Leu Val Glu Lys Ile Met Ala Arg Ala Asn Gly Thr Ile
       130                 135                 140
   Leu Glu Tyr Thr Gly Ile Lys Ser Asp Gly Ser Gly Lys Ala Lys Glu
   145                 150                 155                 160
   Thr Leu Lys Glu Tyr Val Leu Glu Gly Thr Leu Thr Ala Glu Lys Ala
                   165                 170                 175
   Thr Leu Val Val Lys Glu Gly Thr Val Thr Leu Ser Lys His Ile Ser
               180                 185                 190
   Lys Ser Gly Glu Val Thr Ala Glu Leu Asn Asp Thr Asp Ser Thr Gln
           195                 200                 205
   Ala Thr Lys Lys Thr Gly Lys Trp Asp Ala Gly Thr Ser Thr Leu Thr
       210                 215                 220
   Ile Thr Val Asn Asn Lys Lys Thr Lys Ala Leu Val Phe Thr Lys Gln
   225                 230                 235                 240
   Asp Thr Ile Thr Ser Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Arg
(2) INFORMATION FOR SEQ ID NO:14:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:14:
   GTCTGCAAAA ACCATGACAA G                                   21
(2) INFORMATION FOR SEQ ID NO:15:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:15:
   GTCATCAACA GAAGAAAAAT TC 22
(2) INFORMATION FOR SEQ ID NO:16:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:16:
   CCGGATCCAT ATGAAAAAAT ATTTATTGGG 30
(2) INFORMATION FOR SEQ ID NO:17:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:17:
   CCGGGATCCA TATGGCTAAG CAAAATGTTA GC 32
(2) INFORMATION FOR SEQ ID NO:18:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:18:
   GCGTTCAAGT ACTCCAGA 18
(2) INFORMATION FOR SEQ ID NO:19:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (xi) SEQUENCE DESCRIPTION: SEQ ID NO:19:
   GATATCTAGA TCTTATTTTA AAGCGTT                                       27
(2) INFORMATION FOR SEQ ID NO:20:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:20:
   GGATCCGGTG ACCTTTTAAA GCGTTTTTAA T                                  31
(2) INFORMATION FOR SEQ ID NO:21:
   (i) SEQUENCE CHARACTERISTICS:
      (A) LENGTH: 891 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..891
   (xi) SEQUENCE DESCRIPTION: SEQ ID NO:21:
(2) INFORMATION FOR SEQ ID NO:22:
   (i) SEQUENCE CHARACTERISTICS:
      (A) LENGTH: 296 amino acids
      (B) TYPE: amino acid
      (D) TOPOLOGY: linear
   (ii) MOLECULE TYPE: protein
   (xi) SEQUENCE DESCRIPTION: SEQ ID NO:22:
   Met Arg Leu Leu Ile Gly Phe Ala Leu Ala Leu Ala Leu Ile Gly Cys
    1               5                  10                  15
   Ala Gln Lys Gly Ala Glu Ser Ile Gly Ser Gln Lys Glu Asn Asp Leu
               20                  25                  30
   Asn Leu Glu Asp Ser Ser Lys Lys Ser His Gln Asn Ala Lys Gln Asp
           35                  40                  45
   Leu Pro Ala Val Thr Glu Asp Ser Val Ser Leu Phe Asn Gly Asn Lys
       50                  55                  60
   Ile Phe Val Ser Lys Glu Lys Asn Ser Ser Gly Lys Tyr Asp Leu Arg
   65                  70                  75                  80
   Ala Thr Ile Asp Gln Val Glu Leu Lys Gly Thr Ser Asp Lys Asn Asn
                   85                  90                  95
   Gly Ser Gly Thr Leu Glu Gly Ser Lys Pro Asp Lys Ser Lys Val Lys
               100                 105                 110
   Leu Thr Val Ser Ala Asp Leu Asn Thr Val Thr Leu Glu Ala Phe Asp
           115                 120                 125
   Ala Ser Asn Gln Lys Ile Ser Ser Lys Val Thr Lys Lys Gln Gly Ser
       130                 135                 140
   Ile Thr Glu Glu Thr Leu Lys Ala Asn Lys Leu Asp Ser Lys Lys Leu
   145                 150                 155                 160
   Thr Arg Ser Asn Gly Thr Thr Leu Glu Tyr Ser Gln Ile Thr Asp Ala
                   165                 170                 175
   Asp Asn Ala Thr Lys Ala Val Glu Thr Leu Lys Asn Ser Ile Lys Leu
               180                 185                 190
   Glu Gly Ser Leu Val Val Gly Lys Thr Thr Val Glu Ile Lys Glu Gly
           195                 200                 205
   Thr Val Thr Leu Lys Arg Glu Ile Glu Lys Asp Gly Lys Val Lys Val
       210                 215                 220
   Phe Leu Asn Asp Thr Ala Gly Ser Asn Lys Lys Thr Gly Lys Trp Glu
   225                 230                 235                 240
   Asp Ser Thr Ser Thr Leu Thr Ile Ser Ala Asp Ser Lys Lys Thr Lys
                   245                 250                 255
   Asp Leu Val Phe Leu Thr Asp Gly Thr Ile Thr Val Gln Gln Tyr Asn
               260                 265                 270
   Thr Ala Gly Thr Ser Leu Glu Gly Ser Ala Ser Glu Ile Lys Asn Leu
           275                 280                 285
   Ser Glu Leu Lys Asn Ala Leu Lys
       290                 295
(2) INFORMATION FOR SEQ ID NO:23:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:23:
   GGTACAATTA CAGTACAA 18
(2) INFORMATION FOR SEQ ID NO:24:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:24:
   CCGAGAATCT CATATGGCAC AAAAAGGTGC TGAGTCAATT GG 42
(2) INFORMATION FOR SEQ ID NO:25:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:25:
   CCGATATCGG ATCCTATTTT AAAGCGTTTT TAAGC 35
(2) INFORMATION FOR SEQ ID NO:26:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:26:
   GGATCCGGTG ACCTTTTAAA GCGTTTTTAA G 31
(2) INFORMATION FOR SEQ ID NO:27:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:27:
   GTGCGCGACC ATATGAAAAA GAATACATTA AGTGCG                                36
(2) INFORMATION FOR SEQ ID NO:28:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:28:
   GTCGGCGGAT CCTTAAGGTT TTTTTGGACT TTCTGC                                36
(2) INFORMATION FOR SEQ ID NO:29:
   (i) SEQUENCE CHARACTERISTICS:
      (A) LENGTH: 633 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..633
   (xi) SEQUENCE DESCRIPTION: SEQ ID NO:29:
(2) INFORMATION FOR SEQ ID NO:30:
   (i) SEQUENCE CHARACTERISTICS:
      (A) LENGTH: 210 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Lys Lys Asn Thr Leu Ser Ala Ile Leu Met Thr Leu Phe Leu Phe
     1               5                  10                  15
   Ile Ser Cys Asn Asn Ser Gly Lys Asp Gly Asn Thr Ser Ala Asn Ser
                   20                  25                  30
   Ala Asp Glu Ser Val Lys Gly Pro Asn Leu Thr Glu Ile Ser Lys Lys
               35                  40                  45
   Ile Thr Asp Ser Asn Ala Val Leu Leu Ala Val Lys Glu Val Glu Ala
       50                  55                  60
   Leu Leu Ser Ser Ile Asp Glu Ile Ala Ala Lys Ala Ile Gly Lys Lys
   65                  70                  75                  80
   Ile His Gln Asn Asn Gly Leu Asp Thr Glu Tyr Asn His Asn Gly Ser
                   85                  90                  95
   Leu Leu Ala Gly Arg Tyr Ala Ile Ser Thr Leu Ile Lys Gln Lys Leu
               100                 105                 110
   Asp Gly Leu Lys Asn Glu Gly Leu Lys Glu Lys Ile Asp Ala Ala Lys
           115                 120                 125
   Lys Cys Ser Glu Thr Phe Thr Asn Lys Leu Lys Glu Lys His Thr Asp
       130                 135                 140
   Leu Gly Lys Glu Gly Val Thr Asp Ala Asp Ala Lys Glu Ala Ile Leu
   145                 150                 155                 160
   Lys Thr Asn Gly Thr Lys Thr Lys Gly Ala Glu Glu Leu Gly Lys Leu
                   165                 170                 175
   Phe Glu Ser Val Glu Val Leu Ser Lys Ala Ala Lys Glu Met Leu Ala
               180                 185                 190
   Asn Ser Val Lys Glu Leu Thr Ser Pro Val Val Ala Glu Ser Pro Lys
           195                 200                 205
   Lys Pro
       210
(2) INFORMATION FOR SEQ ID NO:31:
   (i) SEQUENCE CHARACTERISTICS:
      (A) LENGTH: 630 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..630
   (xi) SEQUENCE DESCRIPTION: SEQ ID NO:31:
(2) INFORMATION FOR SEQ ID NO:32:
   (i) SEQUENCE CHARACTERISTICS:
      (A) LENGTH: 209 amino acids
      (B) TYPE: amino acid
      (D) TOPOLOGY: linear
   (ii) MOLECULE TYPE: protein
   (xi) SEQUENCE DESCRIPTION: SEQ ID NO:32:
   Met Lys Lys Asn Thr Leu Ser Ala Ile Leu Met Thr Leu Phe Leu Phe
    1               5                  10                  15
   Ile Ser Cys Asn Asn Ser Gly Gly Asp Thr Ala Ser Thr Asn Pro Asp
               20                  25                  30
   Glu Ser Ala Lys Gly Pro Asn Leu Thr Val Ile Ser Lys Lys Ile Thr
           35                  40                  45
   Asp Ser Asn Ala Phe Val Leu Ala Val Lys Glu Val Glu Ala Leu Ile
       50                  55                  60
   Ser Ser Ile Asp Glu Leu Ala Asn Lys Ala Ile Gly Lys Val Ile His
   65                  70                  75                  80
   Gln Asn Asn Gly Leu Asn Ala Asn Ala Gly Gln Asn Gly Ser Leu Leu
                   85                  90                  95
   Ala Gly Ala Tyr Ala Ile Ser Thr Leu Ile Thr Glu Lys Leu Ser Lys
               100                 105                 110
   Leu Lys Asn Ser Glu Glu Leu Asn Lys Lys Ile Glu Glu Ala Lys Asn
           115                 120                 125
   His Ser Glu Ala Phe Thr Asn Arg Leu Lys Gly Ser His Ala Gln Leu
       130                 135                 140
   Gly Val Ala Ala Ala Thr Asp Asp His Ala Lys Glu Ala Ile Leu Lys
   145                 150                 155                 160
   Ser Asn Pro Thr Lys Asp Lys Gly Ala Lys Ala Leu Lys Asp Leu Ser
                   165                 170                 175
   Glu Ser Val Glu Ser Leu Ala Lys Ala Ala Gln Glu Ala Leu Ala Asn
               180                 185                 190
   Ser Val Lys Glu Leu Thr Asn Pro Val Val Ala Glu Ser Pro Lys Lys
           195                 200                 205
   Pro
(2) INFORMATION FOR SEQ ID NO:33:
   (i) SEQUENCE CHARACTERISTICS:
      (A) LENGTH: 639 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..639
   (xi) SEQUENCE DESCRIPTION: SEQ ID NO:33:
(2) INFORMATION FOR SEQ ID NO:34:
   (i) SEQUENCE CHARACTERISTICS:
      (A) LENGTH: 212 amino acids
      (B) TYPE: amino acid
      (D) TOPOLOGY: linear
   (ii) MOLECULE TYPE: protein
   (xi) SEQUENCE DESCRIPTION: SEQ ID NO:34:
   Met Lys Lys Asn Thr Leu Ser Ala Ile Leu Met Thr Leu Phe Leu Phe
     1               5                  10                  15
   Ile Ser Cys Ser Asn Ser Gly Lys Gly Gly Asp Ser Ala Ser Thr Asn
                20                  25                  30
   Pro Ala Asp Glu Ser Ala Lys Gly Pro Asn Leu Thr Glu Ile Ser Lys
            35                  40                  45
   Lys Ile Thr Asp Ser Asn Ala Phe Val Leu Ala Val Lys Glu Val Glu
        50                  55                  60
   Thr Leu Val Leu Ser Ile Asp Glu Leu Ala Lys Lys Ala Ile Gly Gln
    65                  70                  75                      80
   Lys Ile Asp Asn Asn Asn Gly Leu Ala Ala Leu Asn Asn Gln Asn Gly
                    85                  90                  95
   Ser Leu Leu Ala Gly Ala Tyr Ala Ile Ser Thr Leu Ile Thr Glu Lys
               100                 105                 110
   Leu Ser Lys Leu Lys Asn Leu Glu Glu Leu Lys Thr Glu Ile Ala Lys
           115                 120                 125
   Ala Lys Lys Cys Ser Glu Glu Phe Thr Asn Lys Leu Lys Ser Gly His
       130                 135                 140
   Ala Asp Leu Gly Lys Gln Asp Ala Thr Asp Asp His Ala Lys Ala Ala
   145                 150                 155                 160
   Ile Leu Lys Thr His Ala Thr Thr Asp Lys Gly Ala Lys Glu Phe Lys
                   165                 170                 175
   Asp Leu Phe Glu Ser Val Glu Gly Leu Leu Lys Ala Ala Gln Val Ala
               180                 185                 190
   Leu Thr Asn Ser Val Lys Glu Leu Thr Ser Pro Val Val Ala Glu Ser
           195                 200                 205
   Pro Lys Lys Pro
       210
(2) INFORMATION FOR SEQ ID NO:35:
   (i) SEQUENCE CHARACTERISTICS:
      (A) LENGTH: 624 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..624
   (xi) SEQUENCE DESCRIPTION: SEQ ID NO:35:
(2) INFORMATION FOR SEQ ID NO:36:
   (i) SEQUENCE CHARACTERISTICS:
      (A) LENGTH: 207 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Met Lys Lys Asn Thr Leu Ser Ala Ile Leu Met Thr Leu Phe Leu Phe
    1               5                  10                  15
   Ile Ser Cys Asn Asn Ser Gly Gly Asp Ser Ala Ser Thr Asn Pro Asp
               20                  25                  30
   Glu Ser Ala Lys Gly Pro Asn Leu Thr Val Ile Ser Lys Lys Ile Thr
           35                  40                  45
   Asp Ser Asn Ala Phe Leu Leu Ala Val Lys Glu Val Glu Ala Leu Leu
       50                  55                  60
   Ser Ser Ile Asp Glu Leu Ser Lys Ala Ile Gly Lys Lys Ile Lys Asn
   65                  70                  75                  80
   Asp Gly Thr Leu Asp Asn Glu Ala Asn Arg Asn Glu Ser Leu Ile Ala
                   85                  90                  95
   Gly Ala Tyr Glu Ile Ser Lys Leu Ile Thr Gln Lys Leu Ser Val Leu
               100                 105                 110
   Asn Ser Glu Glu Leu Lys Lys Lys Ile Lys Glu Ala Lys Asp Cys Ser
           115                 120                 125
   Glu Lys Phe Thr Thr Lys Leu Lys Asp Ser His Ala Glu Leu Gly Ile
       130                 135                 140
   Gln Ser Val Gln Asp Asp Asn Ala Lys Lys Ala Ile Leu Lys Thr His
   145                 150                 155                 160
   Gly Thr Lys Asp Lys Gly Ala Lys Glu Leu Glu Glu Leu Phe Lys Ser
                   165                 170                 175
   Leu Glu Ser Leu Ser Lys Ala Ala Gln Ala Ala Leu Thr Asn Ser Val
               180                 185                 190
   Lys Glu Leu Thr Asn Pro Val Val Ala Glu Ser Pro Lys Lys Pro
           195                 200                 205
(2) INFORMATION FOR SEQ ID NO:37:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:37:
   GTGCGCGACC ATATGGCTAA TAATTCAGGG AAAGAT                    36
(2) INFORMATION FOR SEQ ID NO:38:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:38:
   GTGCGCGACC ATATGGGCTA GTAATTCAGG GAAAGGT 37
(2) INFORMATION FOR SEQ ID NO:39:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:39:
   GTGCGCGACC ATATGGCTAA TAATTCAGGT GGGGAT 36
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:40:
   CTTGGAAAAT TATTTGAA 18
(2) INFORMATION FOR SEQ ID NO:41:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:41:
   CACGGTCACC CCATGGGAAA TAATTCAGGG AAAGG 35
(2) INFORMATION FOR SEQ ID NO:42:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:42:
   TATAGATGAC AGCAACGC 18
(2) INFORMATION FOR SEQ ID NO:43:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:43:
   CCGGTGACCC CATGGTACCA GGTTTTTTTG GACTTTCTGC 40
(2) INFORMATION FOR SEQ ID NO:44:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:44:
   CCGGATCCAT ATGGTTAAAA AAATAATATT TATTTC 36
(2) INFORMATION FOR SEQ ID NO:45:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:45:
   GATATCTAGA TCTTTAATTG CTCTGCTCAC TCTCTTC 37
(2) INFORMATION FOR SEQ ID NO:46:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:46:
   CCGGGATCCA TATGGCTAGT GCAATTGGTC GTGG 34
(2) INFORMATION FOR SEQ ID NO:47:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:47:
   ATGATTATCA ATCATAAT 18
(2) INFORMATION FOR SEQ ID NO:48:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:48:
   TCTGAACAAT GACAAAAC 18
(2) INFORMATION FOR SEQ ID NO:49:
   (i) SEQUENCE CHARACTERISTICS:
      (A) LENGTH: 825 base pairs
      (B) TYPE: nucleic acid
      (C) STRANDEDNESS: double
      (D) TOPOLOGY: linear
   (ii) MOLECULE TYPE: DNA (genomic)
(2) INFORMATION FOR SEQ ID NO:50:
   (i) SEQUENCE CHARACTERISTICS:
      (A) LENGTH: 824 base pairs
      (B) TYPE: nucleic acid
      (C) STRANDEDNESS: double
      (D) TOPOLOGY: linear
   (xi) SEQUENCE DESCRIPTION: SEQ ID NO:50:
   ATGAAAAAAT ATTTATTGGG AATAGGTCTA ATATTAGCAT TAATAGCATG TAAGCAAAAT      60
   GTTAGCAGCC TTGATGAAAA AAATAGCGTT TCAGTAGATT TACCTGGTGG AATGCAAGTT     120
   CTTGTAAGTA AAGAAAAAGA CAAAGATGGT AAATACAGTC TAATGGCAAC AGTAGACAAG     180
   CTTGAGCTTA AAGGAACTTC TGATAAAAAC AACGGTTCTG GAACACTTGA AGGTGAAAAA     240
   ACTGACAAAA GTAAAGCAAA ATTAACAATT GCTGAGGATC TAAGTAAAAC CACATTTGAA     300
   ATCTTCAAAG AAGATGGCAA AACATTAGTA TCAAAAAAAG TAACCCTTAA AGACAAGTCA     360
   TCAACAGAAG AAAAATTCAA CGCAAAGGGT GAAGCATCTG AAAAAACAAT AGTAAGAGCA     420
   AATGGAACCA GACTTGAATA CACAGACATA AAAAGCGATA AAACCGGAAA AGCTAAAGAA     480
   GTTTTAAAAG ACTTTGCTCT TGAAGGAACT CTAGCTGCTG ACGGCAAAAC AACATTAAAA     540
   GTTACAGAAG GCACTGTTGT TTTAAGCAAA CACATTTCAA ACTCTGGAGA AATAACAGTT     600
   GAGCTTAATG ACTCTGACAC TACTCAGGCT ACTAAAAAAA CTGGAACATG GGATTCAAAG      660
   ACTTCCACTT TAACAATTAG TGTGAATAGC CGAAAAACCA AAAACCTTGT ATTCACAAAA      720
   GAAGACACAA TAACAGTACA AAAATACGAC TCAGCAGGCA CCAATCTAGA AGGCAAAGCA      780
   GTCGAAATTA CAACGCTTAA AGAACTTAAA GATGCTTTAA AATA                       824
(2) INFORMATION FOR SEQ ID NO:51:
   (i) SEQUENCE CHARACTERISTICS:
      (A) LENGTH: 1011 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..1011
   (xi) SEQUENCE DESCRIPTION: SEQ ID NO:51:
(2) INFORMATION FOR SEQ ID NO:52:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:52:
   Met Ile Ile Asn His Asn Thr Ser Ala Ile Asn Ala Ser Arg Asn Asn
    1               5                  10                  15
   Ala Ile Asn Ala Ala Asn Leu Ser Lys Thr Gln Glu Lys Leu Ser Ser
               20                  25                  30
   Gly Tyr Arg Ile Asn Arg Ala Ser Asp Asp Ala Ala Gly Met Gly Val
           35                  40                  45
   Ser Gly Lys Ile Asn Ala Gln Ile Thr Gly Leu Ser Gln Ala Ser Arg
       50                  55                  60
   Asn Thr Ser Lys Ala Ile Asn Phe Ile Gln Thr Thr Glu Gly Asn Leu
   65                  70                  75                  80
   Asn Glu Val Glu Lys Val Leu Val Arg Met Lys Glu Leu Ala Val Gln
                   85                  90                  95
   Ser Gly Asn Gly Thr Tyr Ser Asp Ala Asp Arg Gly Ser Ile Gln Ile
               100                 105                 110
   Glu Ile Glu Gln Leu Thr Asp Glu Ile Asn Arg Ile Ala Asp Gln Ala
           115                 120                 125
   Gln Tyr Asn Gln Met His Met Leu Ser Asn Lys Ser Ala Ser Gln Asn
       130                 135                 140
   Val Lys Thr Ala Glu Glu Leu Gly Met Gln Pro Ala Lys Ile Asn Thr
   145                 150                 155                 160
   Pro Ala Ser Leu Ser Gly Ser Gln Ala Ser Trp Thr Leu Arg Val His
                   165                 170                 175
   Val Gly Ala Asn Gln Asp Glu Ala Ile Ala Val Asn Ile Tyr Ser Ala
               180                 185                 190
   Asn Val Ala Asn Leu Phe Ala Gly Glu Gly Ala Gln Ala Ala Gln Ala
           195                 200                 205
   Ala Pro Val Gln Glu Gly Ala Gln Glu Glu Gly Ala Gln Gln Pro Thr
       210                 215                 220
   Pro Ala Thr Ala Pro Thr Gln Gly Gly Val Asn Ser Pro Val Asn Val
   225                 230                 235                 240
   Thr Thr Thr Val Asp Ala Asn Thr Ser Leu Ala Lys Ile Glu Asn Ala
                   245                 250                 255
   Ile Arg Met Ile Ser Asp Gln Arg Ala Asn Leu Gly Ala Phe Gln Asn
               260                 265                 270
   Arg Leu Glu Ser Ile Lys Asn Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Ser Ile Leu Thr Gln Ser Ala Met Ala Met
   305                 310                 315                 320
   Ile Ala Gln Ala Asn Gln Val Pro Gln Tyr Val Leu Ser Leu Leu Arg
                   325                 330                 335
(2) INFORMATION FOR SEQ ID NO:53:
   (i) SEQUENCE CHARACTERISTICS:
      (A) LENGTH: 1008 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..1008
   (xi) SEQUENCE DESCRIPTION: SEQ ID NO:53:
   ATG ATT ATC AAT CAT AAT ACA TCA GCT ATT AAT GCT TCA AGA AAT AAT        48
   Met Ile Ile Asn His Asn Thr Ser Ala Ile Asn Ala Ser Arg Asn Asn
     1               5                  10                  15
   GGT ATT AAT GCT GCT AAT CTT AGT AAA ACT CAA GAG AAG CTT TCT AGT        96
   Gly Ile Asn Ala Ala Asn Leu Ser Lys Thr Gln Glu Lys Leu Ser Ser
                20                  25                  30
   GGT TAC AGA ATT AAT AGA GCT TCT GAT GAT GCT GCT GGT ATG GGG GTT       144
   Gly Tyr Arg Ile Asn Arg Ala Ser Asp Asp Ala Ala Gly Met Gly Val
            35                  40                  45
   TCT GGG AAG ATT AAT GCT CAA ATA AGA GGT TTA TCA CAA GCT TCT AGA       192
   Ser Gly Lys Ile Asn Ala Gln Ile Arg Gly Leu Ser Gln Ala Ser Arg
        50                  55                  60
   AAC ACT TCA AAA GCT ATT AAT TTT ATT CAG ACA ACA GAA GGA AAT TTG       240
   Asn Thr Ser Lys Ala Ile Asn Phe Ile Gln Thr Thr Glu Gly Asn Leu
    65                  70                  75                  80
   AAT GAA GTA GAA AAA GTT TTA GTA AGA ATG AAA GAA TTA GCA GTT CAA       288
   Asn Glu Val Glu Lys Val Leu Val Arg Met Lys Glu Leu Ala Val Gln
                    85                  90                  95
   TCA GGT AAC GGT ACA TAT TCA GAC GCA GAC AGA GGT TCT ATA CAA ATT       336
   Ser Gly Asn Gly Thr Tyr Ser Asp Ala Asp Arg Gly Ser Ile Gln Ile
               100                 105                 110
   GAA ATA GAG CAA CTT ACA GAC GAA ATT AAT AGA ATT GCT GAT CAA GCT     384
   Glu Ile Glu Gln Leu Thr Asp Glu Ile Asn Arg Ile Ala Asp Gln Ala
           115                 120                 125
   CAA TAT AAC CAA ATG CAC ATG TTG TCA AAC AAA TCT GCT TCC CAA AAT     432
   Gln Tyr Asn Gln Met His Met Leu Ser Asn Lys Ser Ala Ser Gln Asn
       130                 135                 140
   GTA AGA ACA GCT GAA GAA CTT GGA ATG CAA CCT GCA AAA ATC AAC ACA     480
   Val Arg Thr Ala Glu Glu Leu Gly Met Gln Pro Ala Lys Ile Asn Thr
   145                 150                 155                 160
   CCA GCG TCA CTT TCA GGA TCT CAA GCT TCT TGG ACT TTA AGA GTT CAT     528
   Pro Ala Ser Leu Ser Gly Ser Gln Ala Ser Trp Thr Leu Arg Val His
                   165                 170                 175
   GTG GGA GCA AAT CAA GAT GAA GCG ATT GCT GTA AAT ATT TAT GCT GCT     576
   Val Gly Ala Asn Gln Asp Glu Ala Ile Ala Val Asn Ile Tyr Ala Ala
               180                 185                 190
   AAT GTT GCA AAT CTA TTC TCT GGT GAA GGA GCT CAG GCT GCT CAG ACT     624
   Asn Val Ala Asn Leu Phe Ser Gly Glu Gly Ala Gln Ala Ala Gln Thr
           195                 200                 205
   GCA CCT GTT CAA GAA GGT GCT CAA CAA GAA GGA GCT CAA CAA CCA GCA     672
   Ala Pro Val Gln Glu Gly Ala Gln Gln Glu Gly Ala Gln Gln Pro Ala
       210                 215                 220
   CCT GCT ACA GCG CCT TCT CAG GGT GGA GTT AAT TCT CCT GTT AAT GTT     720
   Pro Ala Thr Ala Pro Ser Gln Gly Gly Val Asn Ser Pro Val Asn Val
   225                 230                 235                 240
   ACA ACT ACA GTT GAC GCT AAT ACA TCT CTT GCT AAA ATA GAA AAT GCT     768
   Thr Thr Thr Val Asp Ala Asn Thr Ser Leu Ala Lys Ile Glu Asn Ala
                   245                 250                 255
   ATT AGA ATG ATA AGT GAT CAA AGA GCA AAT TTA GGT GCT TTC CAA AAT     816
   Ile Arg Met Ile Ser Asp Gln Arg Ala Asn Leu Gly Ala Phe Gln Asn
               260                 265                 270
   AGA CTT GAG TCT ATA AAG GAT AGT ACT GAG TAT GCT ATT GAA AAC CTA     864
   Arg Leu Glu Ser Ile Lys Asp Ser Thr Glu Tyr Ala Ile Glu Asn Leu
           275                 280                 285
   AAA GCA TCT TAT GCT CAA ATA AAA GAT GCT ACA ATG ACA GAT GAG GTT     912
   Lys Ala Ser Tyr Ala Gln Ile Lys Asp Ala Thr Met Thr Asp Glu Val
       290                 295                 300
   GTA GCA GCT ACA ACT AAT AGT ATT TTA ACA CAA TGT GCA ATG GCA ATG     960
   Val Ala Ala Thr Thr Asn Ser Ile Leu Thr Gln Cys Ala Met Ala Met
   305                 310                 315                 320
   ATT GCG CAA GCT AAT CAA GTT CCT CAA TAT GTT TTG TCA TTG CTT AGA    1008
   Ile Ala Gln Ala Asn Gln Val Pro Gln Tyr Val Leu Ser Leu Leu Arg
                   325                 330                 335
(2) INFORMATION FOR SEQ ID NO:54:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Met Ile Ile Asn His Asn Thr Ser Ala Ile Asn Ala Ser Arg Asn Asn
   1 5 10 15
   Gly Ile Asn Ala Ala Asn Leu Ser Lys Thr Gln Glu Lys Leu Ser Ser
   20 25 30
   Gly Tyr Arg Ile Asn Arg Ala Ser Asp Asp Ala Ala Gly Met Gly Val
   35 40 45
   Ser Gly Lys Ile Asn Ala Gln Ile Arg Gly Leu Ser Gln Ala Ser Arg
   50 55 60
   Asn Thr Ser Lys Ala Ile Asn Phe Ile Gln Thr Thr Glu Gly Asn Leu
   65 70 75 80
   Asn Glu Val Glu Lys Val Leu Val Arg Met Lys Glu Leu Ala Val Gln
   85 90 95
   Ser Gly Asn Gly Thr Tyr Ser Asp Ala Asp Arg Gly Ser Ile Gln Ile
   100 105 110
   Glu Ile Glu Gln Leu Thr Asp Glu Ile Asn Arg Ile Ala Asp Gln Ala
   115 120 125
   Gln Tyr Asn Gln Met His Met Leu Ser Asn Lys Ser Ala Ser Gln Asn
   130 135 140
   Val Arg Thr Ala Glu Glu Leu Gly Met Gln Pro Ala Lys Ile Asn Thr
   145 150 155 160
   Pro Ala Ser Leu Ser Gly Ser Gln Ala Ser Trp Thr Leu Arg Val His
   165 170 175
   Val Gly Ala Asn Gln Asp Glu Ala Ile Ala Val Asn Ile Tyr Ala Ala
   180 185 190
   Asn Val Ala Asn Leu Phe Ser Gly Glu Gly Ala Gln Ala Ala Gln Thr
   195 200 205
   Ala Pro Val Gln Glu Gly Ala Gln Gln Glu Gly Ala Gln Gln Pro Ala
   210 215 220
   Pro Ala Thr Ala Pro Ser Gln Gly Gly Val Asn Ser Pro Val Asn Val
   225 230 235 240
   Thr Thr Thr Val Asp Ala Asn Thr Ser Leu Ala Lys Ile Glu Asn Ala
   245 250 255
   Ile Arg Met Ile Ser Asp Gln Arg Ala Asn Leu Gly Ala Phe Gln Asn
   260 265 270
   Arg Leu Glu Ser Ile Lys Asp Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Ser Ile Leu Thr Gln Cys Ala Met Ala Met
   305                 310                 315                 320
   Ile Ala Gln Ala Asn Gln Val Pro Gln Tyr Val Leu Ser Leu Leu Arg
                   325                 330                 335
(2) INFORMATION FOR SEQ ID NO:55:
   (i) SEQUENCE CHARACTERISTICS:
      (A) LENGTH: 821 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:55:
   ATGAAAAAAT ATTTATTGGG AATAGGTCTA ATATTAGCCT TAATAGCATG CAAGCAAAAT    60
   GTTAGCAGCC TTGATGAAAA AAACAGCGCT TCAGTAGATT TGCCTGGTGA GATGAAAGTT   120
   CTTGTAAGTA AAGAAAAAGA CAAAGACGGT AAGTACAGTC TAAAGGCAAC AGTAGACAAG   180
   ATTGAGCTAA AAGGAACTTC TGATAAAGAC AATGGTTCTG GGGTGCTTGA AGGTACAAAA   240
   GATGACAAAA GTAAAGCAAA ATTAACAATT GCTGACGATC TAGGTAAAAC CACATTCGAA   300
   CTTTTCAAAG AAGATGGCAA AACATTAGTG TCAAGAAAAG TAAGTTCTAA AGACAAAACA   360
   TCAACAGATG AAATGTTCAA TGAAAAAGGT GAATTGTCTG CAAAAACCAT GACAAGAGAA   420
   AATGGAACCA AACTTGAATA TACAGAAATG AAAAGCGATG GAACCGGAAA AGCTAAAGAA   480
   GTTTTAAAAA ACTTTACTCT TGAAGGAAAA GTAGCTAATG ATAAAGTAAC ATTGGAAGTA   540
   AAAGAAGGAA CCGTTACTTT AAGTAAGGAA ATTGCAAAAT CTGGAGAAGT AACAGTTGCT   600
   CTTAATGACA CTAACACTAC TCAGGCTACT AAAAAAACTG GCGCATGGGA TTCAAAAACT   660
   TCTACTTTAA CAATTAGTGT TAACAGCAAA AAAACTACAC AACTTGTGTT TACTAAACAA   720
   GACACAATAA CTGTACAAAA ATACGACTCC GCAGGTACCA ATTTAGAAGG CACAGCAGTC   780
   GAAATTAAAA CACTTGATGA ACTTAAAAAC GCTTTAAAAT A                       821
(2) INFORMATION FOR SEQ ID NO:56:
   (i) SEQUENCE CHARACTERISTICS:
      (A) LENGTH: 821 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:56:
   ATGAAAAAAT ATTTATTGGG AATAGGTCTA ATATTAGCCT TAATAGCATG CAAGCAAAAT      60
   GTTAGCAGCC TTGATGAAAA AAACAGCGCT TCAGTAGATT TGCCTGGTGA GATTAAAGTT     120
   CTTGTAAGTA AAGAAAAAGA CAAAGACGGT AAGTACAGTC TAAAGGCAAC AGTAGACAAG     180
   ATTGAGCTAA AAGGAACTTC TGATAAAGAC AATGGTTCTG GAGTGCTTGA AGGTACAAAA     240
   GATGACAAAA GTAAAGCAAA ATTAACAATT GCTGACGATC TAAGTAAAAC CACATTCGAA     300
   CTTTTCAAAG AAGATGGCAA AACATTAGTG TCAAGAAAAG TAAGTTCTAA AGACAAAACA     360
   TCAACAGATG AAATGTTCAA TGAAAAAGGT GAATTGTCTG CAAAAACCAT GACAAGAGAA     420
   AATGGAACCA AACTTGAATA TACAGAAATG AAAAGCGATG GAACCGGAAA AGCTAAAGAA     480
   GTTTTAAAAA ACTTTACTCT TGAAGGAAAA GTAGCTAATG ATAAAGTAAC ATTGGAAGTA     540
   AAAGAAGGAA CCGTTACTTT AAGTAAGGAA ATTGCAAAAT CTGGAGAAGT AACAGTTGCT     600
   CTTAATGACA CTAACACTAC TCAGGCTACT AAAAAAACTG GCGCATGGGA TTCAAAAACT     660
   TCTACTTTAA CAATTAGTGT TAACAGTAAA AAAACTACAC AACTTGTGTT TACTAAACAA     720
   GACACAATAA CTGTACAAAA ATACGACTCC GCAGGTACCA ATTTAGAAGG CACAGCAGTC     780
   GAAATTAAAA CACTTGATGA ACTTAAAAAC GCTTTAAAAT A                         821
(2) INFORMATION FOR SEQ ID NO:57:
   (i) SEQUENCE CHARACTERISTICS:
      (A) LENGTH: 821 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:57:
   ATGAAAAAAT ATTTATTGGG AATAGGTCTA ATATTAGCCT TAATAGCATG CAAGCAAAAT      60
   GTTAGCAGCC TTGATGAAAA AAACAGCGCT TCAGTAGATT TGCCTGGTGA GATGAAAGTT     120
   CTTGTAAGTA AAGAAAAAGA CAAAGACGGT AAGTACAGTC TAAAGGCAAC AGTAGACAAG     180
   ATTGAGCTAA AAGGAACTTC TGATAAAGAC AATGGTTCTG GGGTGCTTGA AGGTACAAAA     240
   GATGACAAAA GTAAAGCAAA ATTAACAATT GCTGACGATC TAGGTAAAAC CACATTCGAA     300
   CTTTTCAAAG AAGATGGCAA AACATTAGTG TCAAGAAAAG TAAGTTCTAA AGACAAAACA     360
   TCAACAGATG AAATGTTCAA TGAAAAAGGT GAATTGTCTG CAAAAACCAT GACAAGAGAA 420
   AATGGAACCA AACTTGAATA TACAGAAATG AAAAGCGATG GAACCGGAAA AGCTAAAGAA 480
   GTTTTAAAAA ACTTTACTCT TGAAGGAAAA GTAGCTAATG ATAAAGTAAC ATTGGAAGTA 540
   AAAGAAGGAA CCGTTACTTT AAGTAAGGAA ATTGCAAAAT CTGGAGAAGT AACAGTTGCT 600
   CTTAATGACA CTAACACTAC TCAGGCTACT AAAAAAACTG GCGCATGGGA TTCAAAAACT 660
   TCTACTTTAA CAATTAGTGT TAACAGCAAA AAAACTACAC AACTTGTGTT TACTAAACAA 720
   GACACAATAA CTGTACAAAA ATACGACTCC GCAGGTACCA ATTTAGAAGG CACAGCAGTC 780
   GAAATTAAAA CACTTGATGA ACTTAAAAAC GCTTTAAAAT A 821
(2) INFORMATION FOR SEQ ID NO:58:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:58:
   ATGAAAAAAT ATTTATTGGG AATAGGTCTA ATATTAGCCT TAATAGCATG CAAGCAAAAT 60
   GTTAGCAGCC TTGATGAAAA AAACAGCGCT TCAGTAGATT TGCCTGGTGA GATGAAAGTT 120
   CTTGTAAGTA AAGAAAAAGA CAAAGACGGT AAGTACAGTC TAAAGGCAAC AGTAGACAAG 180
   ATTGAGCTAA AAGGAACTTC TGATAAAGAC AATGGTTCTG GAGTGCTTGA AGGTACAAAA 240
   GATGACAAAA GTAAAGCAAA ATTAACAATT GCTGACGATC TAAGTAAAAC CACATTCGAA 300
   CTTTTCAAAG AAGATGGCAA AACATTAGTG TCAAGAAAAG TAAGTTCTAA AGACAAAACA 360
   TCAACAGATG AAATGTTCAA TGAAAAAGGT GAATTGTCTG CAAAAACCAT GACAAGAGAA 420
   AATGGAACCA AACTTGAATA TACAGAAATG AAAAGCGATG GAACCGGAAA AGCTAAAGAA 480
   GTTTTAAAAA ACTTTACTCT TGAAGGAAAA GTAGCTAATG ATAAAGTAAC ATTGGAAGTA 540
   AAAGAAGGAA CCGTTACTTT AAGTAAGGAA ATTGCAAAAT CTGGAGAAGT AACAGTTGCT 600
   CTTAATGACA CTAACACTAC TCAGGCTACT AAAAAAACTG GCGCATGGGA TTCAAAAACT 660
   TCTACTTTAA CAATTAGTGT TAACAGCAAA AAAACTACAC AACTTGTGTT TACTAAACAA 720
   GACACAATAA CTGTACAAAA ATACGACTCC GCAGGTACCA ATTTAGAAGG CACAGCAGTC 780
   GAAATTAAAA CACTTGATGA ACTTAAAAAC GCTTTGAAAT AA 822
(2) INFORMATION FOR SEQ ID NO:59:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:59:
   TTGGATCCGG TCACCCCATG GCTCAATATA ACCAATG 37
(2) INFORMATION FOR SEQ ID NO:60:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:60:
   TTGGATCCGG TCACCCCATG GCTTCTCAAA ATGTAAG 37
(2) INFORMATION FOR SEQ ID NO:61:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:61:
   TTGGATCCGG TGACCAACTC CGCCTTGAGA AGG 33
(2) INFORMATION FOR SEQ ID NO:62:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:62:
   TTGGATCCGG TGACCTATTT GAGCATAAGA TGC 33
(2) INFORMATION FOR SEQ ID NO:63:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:63:
   GGTGAATTTA GTTGGTAAGG 20
(2) INFORMATION FOR SEQ ID NO:64:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:64:
   CACCAGTTTC TTTAAGCTGC TCCTGC 26
(2) INFORMATION FOR SEQ ID NO:65:
   (i) SEQUENCE CHARACTERISTICS:
      (A) LENGTH: 2102 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..2100
   (xi) SEQUENCE DESCRIPTION: SEQ ID NO:65:
   ATG AAA AAA ATG TTA CTA ATC TTT AGT TTT TTT CTT ATT TTC TTG AAT        48
   Met Lys Lys Met Leu Leu Ile Phe Ser Phe Phe Leu Ile Phe Leu Asn
     1               5                  10                  15
   GGA TTT CCT GTT AGT GCA AGA GAA GTT GAT AGG GAA AAA TTA AAG GAC        96
   Gly Phe Pro Val Ser Ala Arg Glu Val Asp Arg Glu Lys Leu Lys Asp
                20                  25                  30
   TTT GTT AAT ATG GAT CTT GAG TTT GTA AAT TAT AAA GGC CCT TAT GAT       144
   Phe Val Asn Met Asp Leu Glu Phe Val Asn Tyr Lys Gly Pro Tyr Asp
            35                  40                  45
   TCT ACA AAT ACA TAT GAA CAA ATA GTG GGT ATT GGG GAG TTT TTA GCA       192
   Ser Thr Asn Thr Tyr Glu Gln Ile Val Gly Ile Gly Glu Phe Leu Ala
        50                  55                  60
   AGA CCG TTG ACC AAT TCC AAT AGC AAC TCA AGT TAT TAT GGT AAA TAT       240
   Arg Pro Leu Thr Asn Ser Asn Ser Asn Ser Ser Tyr Tyr Gly Lys Tyr
    65                  70                  75                  80
   TTT ATT AAT AGA TTT ATT GAT GAT CAA GAT AAA AAA GCA AGC GTT GAT     288
   Phe Ile Asn Arg Phe Ile Asp Asp Gln Asp Lys Lys Ala Ser Val Asp
                    85                  90                  95
   GTT TTT TCT ATT GGT AGT AAG TCA GAG CTT GAC AGT ATA TTG AAT TTA     336
   Val Phe Ser Ile Gly Ser Lys Ser Glu Leu Asp Ser Ile Leu Asn Leu
               100                 105                 110
   AGA AGA ATT CTT ACA GGG TAT TTA ATA AAG TCT TTC GAT TAT GAC AGG     384
   Arg Arg Ile Leu Thr Gly Tyr Leu Ile Lys Ser Phe Asp Tyr Asp Arg
           115                 120                 125
   TCT AGT GCA GAA TTA ATT GCT AAG GTT ATT ACA ATA TAT AAT GCT GTT     432
   Ser Ser Ala Glu Leu Ile Ala Lys Val Ile Thr Ile Tyr Asn Ala Val
       130                 135                 140
   TAT AGA GGA GAT TTG GAT TAT TAT AAA GGG TTT TAT ATT GAG GCT GCT     480
   Tyr Arg Gly Asp Leu Asp Tyr Tyr Lys Gly Phe Tyr Ile Glu Ala Ala
   145                 150                 155                 160
   TTA AAG TCT TTA AGT AAA GAA AAT GCA GGT CTT TCT AGG GTT TAT AGT     528
   Leu Lys Ser Leu Ser Lys Glu Asn Ala Gly Leu Ser Arg Val Tyr Ser
                   165                 170                 175
   CAG TGG GCT GGA AAG ACA CAA ATA TTT ATT CCT CTT AAA AAG GAT ATT     576
   Gln Trp Ala Gly Lys Thr Gln Ile Phe Ile Pro Leu Lys Lys Asp Ile
               180                 185                 190
   TTG TCT GGA AAT ATT GAG TCT GAC ATT GAT ATT GAC AGT TTA GTT ACA     624
   Leu Ser Gly Asn Ile Glu Ser Asp Ile Asp Ile Asp Ser Leu Val Thr
           195                 200                 205
   GAT AAG GTG GTG GCA GCT CTT TTA AGT GAA AAT GAA GCA GGT GTT AAC     672
   Asp Lys Val Val Ala Ala Leu Leu Ser Glu Asn Glu Ala Gly Val Asn
       210                 215                 220
   TTT GCA AGA GAT ATT ACA GAT ATT CAA GGC GAA ACT CAT AAG GCA GAT     720
   Phe Ala Arg Asp Ile Thr Asp Ile Gln Gly Glu Thr His Lys Ala Asp
   225                 230                 235                 240
   CAA GAT AAA ATT GAT ATT GAA TTA GAC AAT ATT CAT GAA AGT GAT TCC     768
   Gln Asp Lys Ile Asp Ile Glu Leu Asp Asn Ile His Glu Ser Asp Ser
                   245                 250                 255
   AAT ATA ACA GAA ACT ATT GAA AAT TTA AGG GAT CAG CTT GAA AAA GCT     816
   Asn Ile Thr Glu Thr Ile Glu Asn Leu Arg Asp Gln Leu Glu Lys Ala
               260                 265                 270
   ACA GAT GAA GAG CAT AAA AAA GAG ATT GAA AGT CAG GTT GAT GCT AAA     864
   Thr Asp Glu Glu His Lys Lys Glu Ile Glu Ser Gln Val Asp Ala Lys
           275                 280                 285
   AAG AAA CAA AAG GAA GAG CTA GAT AAA AAG GCA ATA AAT CTT GAT AAA     912
   Lys Lys Gln Lys Glu Glu Leu Asp Lys Lys Ala Ile Asn Leu Asp Lys
       290                 295                 300
   GCT CAG CAA AAA TTA GAT TCT GCT GAA GAT AAT TTA GAT GTT CAA AGA     960
   Ala Gln Gln Lys Leu Asp Ser Ala Glu Asp Asn Leu Asp Val Gln Arg
   305                 310                 315                 320
   AAT ACT GTT AGA GAG AAA ATT CAA GAG GAT ATT AAC GAA ATT AAC AAG    1008
   Asn Thr Val Arg Glu Lys Ile Gln Glu Asp Ile Asn Glu Ile Asn Lys
                   325                 330                 335
   GAA AAG AAT TTA CCA AAG CCT GGT GAT GTA AGT TCT CCT AAA GTT GAT    1056
   Glu Lys Asn Leu Pro Lys Pro Gly Asp Val Ser Ser Pro Lys Val Asp
               340                 345                 350
   AAG CAA CTA CAA ATA AAA GAG AGC CTG GAA GAT TTG CAG GAG CAG CTT    1104
   Lys Gln Leu Gln Ile Lys Glu Ser Leu Glu Asp Leu Gln Glu Gln Leu
           355                 360                 365
   AAA GAA ACT GGT GAT GAA AAT CAG AAA AGA GAA ATT GAA AAG CAA ATT    1152
   Lys Glu Thr Gly Asp Glu Asn Gln Lys Arg Glu Ile Glu Lys Gln Ile
       370                 375                 380
   GAA ATC AAA AAA AGT GAT GAA AAG CTT TTA AAA AGT AAA GAT GAT AAA    1200
   Glu Ile Lys Lys Ser Asp Glu Lys Leu Leu Lys Ser Lys Asp Asp Lys
   385                 390                 395                 400
   GCA AGT AAA GAT GGT AAA GCC TTG GAT CTT GAT CGA GAA TTA AAT TCT    1248
   Ala Ser Lys Asp Gly Lys Ala Leu Asp Leu Asp Arg Glu Leu Asn Ser
                   405                 410                 415
   AAA GCT TCT AGC AAA GAA AAA AGT AAA GCC AAG GAA GAA GAA ATA ACC    1296
   Lys Ala Ser Ser Lys Glu Lys Ser Lys Ala Lys Glu Glu Glu Ile Thr
               420                 425                 430
   AAG GGT AAG TCA CAG AAA AGC TTA GGC GAT TTG AAT AAT GAT GAA AAT    1344
   Lys Gly Lys Ser Gln Lys Ser Leu Gly Asp Leu Asn Asn Asp Glu Asn
           435                 440                 445
   CTT ATG ATG CCA GAA GAT CAA AAA TTA CCT GAG GTT AAA AAA TTA GAT    1392
   Leu Met Met Pro Glu Asp Gln Lys Leu Pro Glu Val Lys Lys Leu Asp
       450                 455                 460
   AGC AAA AAA GAA TTT AAA CCT GTT TCT GAG GTT GAG AAA TTA GAT AAG    1440
   Ser Lys Lys Glu Phe Lys Pro Val Ser Glu Val Glu Lys Leu Asp Lys
   465                 470                 475                 480
   ATT TTC AAG TCT AAT AAC AAT GTT GGA GAA TTA TCA CCG TTA GAT AAA    1488
   Ile Phe Lys Ser Asn Asn Asn Val Gly Glu Leu Ser Pro Leu Asp Lys
                   485                 490                 495
   TCT TCT TAT AAA GAC ATT GAT TCA AAA GAG GAG ACA GTT AAT AAA GAT    1536
   Ser Ser Tyr Lys Asp Ile Asp Ser Lys Glu Glu Thr Val Asn Lys Asp
               500                 505                 510
   GTT AAT TTG CAA AAG ACT AAG CCT CAG GTT AAA GAC CAA GTT ACT TCT    1584
   Val Asn Leu Gln Lys Thr Lys Pro Gln Val Lys Asp Gln Val Thr Ser
           515                 520                 525
   TTG AAT GAA GAT TTG ACT ACT ATG TCT ATA GAT TCC AGT AGT CCT GTA    1632
   Leu Asn Glu Asp Leu Thr Thr Met Ser Ile Asp Ser Ser Ser Pro Val
       530                 535                 540
   TTT TTA GAG GTT ATT GAT CCA ATT ACA AAT TTA GGA ACT CTT CAA CTT    1680
   Phe Leu Glu Val Ile Asp Pro Ile Thr Asn Leu Gly Thr Leu Gln Leu
   545                 550                 555                 560
   ATT GAT TTA AAT ACT GGT GTT AGG CTT AAA GAA AGC ACT CAG CAA GGC     1728
   Ile Asp Leu Asn Thr Gly Val Arg Leu Lys Glu Ser Thr Gln Gln Gly
                   565                 570                 575
   ATT CAG CGG TAT GGA ATT TAT GAA CGT GAA AAA GAT TTG GTT GTT ATT     1776
   Ile Gln Arg Tyr Gly Ile Tyr Glu Arg Glu Lys Asp Leu Val Val Ile
               580                 585                 590
   AAA ATG GAT TCA GGA AAA GCT AAG CTT CAG ATA CTT GAT AAA CTT GAA     1824
   Lys Met Asp Ser Gly Lys Ala Lys Leu Gln Ile Leu Asp Lys Leu Glu
           595                 600                 605
   AAT TTA AAA GTG GTA TCA GAG TCT AAT TTT GAG ATT AAT AAA AAT TCA     1872
   Asn Leu Lys Val Val Ser Glu Ser Asn Phe Glu Ile Asn Lys Asn Ser
       610                 615                 620
   TCT CTT TAT GTT GAT TCT AAA ATG ATT TTA GTA GCT GTT AGG GAT AAA     1920
   Ser Leu Tyr Val Asp Ser Lys Met Ile Leu Val Ala Val Arg Asp Lys
   625                 630                 635                 640
   GAT AGT AGT AAT GAT TGG AGA TTG GCC AAA TTT TCT CCT AAA AAT TTA     1968
   Asp Ser Ser Asn Asp Trp Arg Leu Ala Lys Phe Ser Pro Lys Asn Leu
                   645                 650                 655
   GAT GAG TTT ATT CTT TCA GAG AAT AAA ATT ATG CCT TTT ACT AGC TTT     2016
   Asp Glu Phe Ile Leu Ser Glu Asn Lys Ile Met Pro Phe Thr Ser Phe
               660                 665                 670
   TCT GTG AGA AAA AAT TTT ATT TAT TTG CAA GAT GAG TTT AAA AGT CTA     2064
   Ser Val Arg Lys Asn Phe Ile Tyr Leu Gln Asp Glu Phe Lys Ser Leu
           675                 680                 685
   GTT ATT TTA GAT GTA AAT ACT TTA AAA AAA GTT AAG TA                  2102
   Val Ile Leu Asp Val Asn Thr Leu Lys Lys Val Lys
       690                 695                 700
(2) INFORMATION FOR SEQ ID NO:66:
   (i) SEQUENCE CHARACTERISTICS:
      (A) LENGTH: 700 amino acids
      (B) TYPE: amino acid
      (D) TOPOLOGY: linear
   (ii) MOLECULE TYPE: protein
   (xi) SEQUENCE DESCRIPTION: SEQ ID NO:66:
   Met Lys Lys Met Leu Leu Ile Phe Ser Phe Phe Leu Ile Phe Leu Asn
   1               5                   10                  15
   Gly Phe Pro Val Ser Ala Arg Glu Val Asp Arg Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Thr Asn Ser Asn Ser Asn Ser Ser Tyr Tyr Gly Lys Tyr
   65 70 75 80
   Phe Ile Asn Arg Phe Ile Asp Asp Gln Asp Lys Lys Ala Ser Val Asp
   85 90 95
   Val Phe Ser Ile Gly Ser Lys Ser Glu Leu Asp Ser Ile Leu Asn Leu
   100 105 110
   Arg Arg Ile Leu Thr Gly Tyr Leu Ile Lys Ser Phe Asp Tyr Asp Arg
   115 120 125
   Ser Ser Ala Glu Leu Ile Ala Lys Val Ile Thr Ile Tyr Asn Ala Val
   130 135 140
   Tyr Arg Gly Asp Leu Asp Tyr Tyr Lys Gly Phe Tyr Ile Glu Ala Ala
   145 150 155 160
   Leu Lys Ser Leu Ser Lys Glu Asn Ala Gly Leu Ser Arg Val Tyr Ser
   165 170 175
   Gln Trp Ala Gly Lys Thr Gln Ile Phe Ile Pro Leu Lys Lys Asp Ile
   180 185 190
   Leu Ser Gly Asn Ile Glu Ser Asp Ile Asp Ile Asp Ser Leu Val Thr
   195 200 205
   Asp Lys Val Val Ala Ala Leu Leu Ser Glu Asn Glu Ala Gly Val Asn
   210 215 220
   Phe Ala Arg Asp Ile Thr Asp Ile Gln Gly Glu Thr His Lys Ala Asp
   225 230 235 240
   Gln Asp Lys Ile Asp Ile Glu Leu Asp Asn Ile His Glu Ser Asp Ser
   245 250 255
   Asn Ile Thr Glu Thr Ile Glu Asn Leu Arg Asp Gln Leu Glu Lys Ala
   260 265 270
   Thr Asp Glu Glu His Lys Lys Glu Ile Glu Ser Gln Val Asp Ala Lys
   275 280 285
   Lys Lys Gln Lys Glu Glu Leu Asp Lys Lys Ala Ile Asn Leu Asp Lys
   290 295 300
   Ala Gln Gln Lys Leu Asp Ser Ala Glu Asp Asn Leu Asp Val Gln Arg
   305 310 315 320
   Asn Thr Val Arg Glu Lys Ile Gln Glu Asp Ile Asn Glu Ile Asn Lys
   325 330 335
   Glu Lys Asn Leu Pro Lys Pro Gly Asp Val Ser Ser Pro Lys Val Asp
   340 345 350
   Lys Gln Leu Gln Ile Lys Glu Ser Leu Glu Asp Leu Gln Glu Gln Leu
   355 360 365
   Lys Glu Thr Gly Asp Glu Asn Gln Lys Arg Glu Ile Glu Lys Gln Ile
   370 375 380
   Glu Ile Lys Lys Ser Asp Glu Lys Leu Leu Lys Ser Lys Asp Asp Lys
   385                 390                 395                 400
   Ala Ser Lys Asp Gly Lys Ala Leu Asp Leu Asp Arg Glu Leu Asn Ser
                   405                 410                 415
   Lys Ala Ser Ser Lys Glu Lys Ser Lys Ala Lys Glu Glu Glu Ile Thr
               420                 425                 430
   Lys Gly Lys Ser Gln Lys Ser Leu Gly Asp Leu Asn Asn Asp Glu Asn
           435                 440                 445
   Leu Met Met Pro Glu Asp Gln Lys Leu Pro Glu Val Lys Lys Leu Asp
       450                 455                 460
   Ser Lys Lys Glu Phe Lys Pro Val Ser Glu Val Glu Lys Leu Asp Lys
   465                 470                 475                 480
   Ile Phe Lys Ser Asn Asn Asn Val Gly Glu Leu Ser Pro Leu Asp Lys
                   485                 490                 495
   Ser Ser Tyr Lys Asp Ile Asp Ser Lys Glu Glu Thr Val Asn Lys Asp
               500                 505                 510
   Val Asn Leu Gln Lys Thr Lys Pro Gln Val Lys Asp Gln Val Thr Ser
           515                 520                 525
   Leu Asn Glu Asp Leu Thr Thr Met Ser Ile Asp Ser Ser Ser Pro Val
       530                 535                 540
   Phe Leu Glu Val Ile Asp Pro Ile Thr Asn Leu Gly Thr Leu Gln Leu
   545                 550                 555                 560
   Ile Asp Leu Asn Thr Gly Val Arg Leu Lys Glu Ser Thr Gln Gln Gly
                   565                 570                 575
   Ile Gln Arg Tyr Gly Ile Tyr Glu Arg Glu Lys Asp Leu Val Val Ile
               580                 585                 590
   Lys Met Asp Ser Gly Lys Ala Lys Leu Gln Ile Leu Asp Lys Leu Glu
           595                 600                 605
   Asn Leu Lys Val Val Ser Glu Ser Asn Phe Glu Ile Asn Lys Asn Ser
       610                 615                 620
   Ser Leu Tyr Val Asp Ser Lys Met Ile Leu Val Ala Val Arg Asp Lys
   625                 630                 635                 640
   Asp Ser Ser Asn Asp Trp Arg Leu Ala Lys Phe Ser Pro Lys Asn Leu
                   645                 650                 655
   Asp Glu Phe Ile Leu Ser Glu Asn Lys Ile Met Pro Phe Thr Ser Phe
               660                 665                 670
   Ser Val Arg Lys Asn Phe Ile Tyr Leu Gln Asp Glu Phe Lys Ser Leu
           675                 680                 685
   Val Ile Leu Asp Val Asn Thr Leu Lys Lys Val Lys
       690                 695                 700
(2) INFORMATION FOR SEQ ID NO:67:
   (i) SEQUENCE CHARACTERISTICS:
      (A) LENGTH: 2081 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..2079
   (xi) SEQUENCE DESCRIPTION: SEQ ID NO:67:
   ATG AAA AAA TTG TTA CTA ATC TTT AGT TTT TTT CTT ATT TCT TTG AAT       48
   Met Lys Lys Leu Leu Leu Ile Phe Ser Phe Phe Leu Ile Ser Leu Asn
     1               5                  10                  15
   GGA TTT CCT CTT AAT TCA AGG GAA GTT GAT AAG GAA AAA TTA AAG GAT       96
   Gly Phe Pro Leu Asn Ser Arg Glu Val Asp Lys Glu Lys Leu Lys Asp
                20                  25                  30
   TTT GTT AAT ATG GAT CTT GAG TTT GTA AAC TAT AAA GGT CCT TAT GAT      144
   Phe Val Asn Met Asp Leu Glu Phe Val Asn Tyr Lys Gly Pro Tyr Asp
            35                  40                  45
   TCT ACA AAT ACA TAT GAA CAA ATA GTA GGT ATT GGT GAG TTT TTA GCA      192
   Ser Thr Asn Thr Tyr Glu Gln Ile Val Gly Ile Gly Glu Phe Leu Ala
        50                  55                  60
   AGA CCA TTG ATT AAT TCC AAT AGC AAC TCA ATT TAT TAT GGT AAA TAT      240
   Arg Pro Leu Ile Asn Ser Asn Ser Asn Ser Ile Tyr Tyr Gly Lys Tyr
    65                  70                  75                  80
   TTT ATT AAT AGA TTT ATT GAT GAT CAA GAT AAA AAA GCA AGC GTT GAT      288
   Phe Ile Asn Arg Phe Ile Asp Asp Gln Asp Lys Lys Ala Ser Val Asp
                    85                  90                  95
   GTT TTT TCT ATT GGT AGT AGG TCA CAG CTT GAC AGT ATA TTG AAT CTA      336
   Val Phe Ser Ile Gly Ser Arg Ser Gln Leu Asp Ser Ile Leu Asn Leu
               100                 105                 110
   AGA AGA ATT CTT ACA GGG TAT TTG ATA AAG TCT TTT GAT TAT GAA AGA      384
   Arg Arg Ile Leu Thr Gly Tyr Leu Ile Lys Ser Phe Asp Tyr Glu Arg
           115                 120                 125
   TCT AGT GCT GAA TTA ATT GCT AAG GTT ATT ACA ATA CAT AAT GCT GTT      432
   Ser Ser Ala Glu Leu Ile Ala Lys Val Ile Thr Ile His Asn Ala Val
       130                 135                 140
   TAT AGA GGG GAT TTA AAT TAT TAT AAA GAG GTT TAT ATT GAG GCT GCT      480
   Tyr Arg Gly Asp Leu Asn Tyr Tyr Lys Glu Val Tyr Ile Glu Ala Ala
   145                 150                 155                 160
   TTA AAG TCT TTA ACT AAA GAA AAT GCA GGT CTT TCT AGA GTG TAC AGT      528
   Leu Lys Ser Leu Thr Lys Glu Asn Ala Gly Leu Ser Arg Val Tyr Ser
                   165                 170                 175
   CAA TGG GCT GGA AAG ACA CAA ATA TTT ATT CCT CTT AAA AAG AAT ATT      576
   Gln Trp Ala Gly Lys Thr Gln Ile Phe Ile Pro Leu Lys Lys Asn Ile
               180                 185                 190
   TTA TCT GGA AAA GTT GAG TCT GAC ATT GAT ATT GAC AGT TTG GTT ACA      624
   Leu Ser Gly Lys Val Glu Ser Asp Ile Asp Ile Asp Ser Leu Val Thr
           195                 200                 205
   GAT AAG GTT GTG GCA GCT CTT TTA AGC GAG AAT GAA GCA GGT GTT AAC      672
   Asp Lys Val Val Ala Ala Leu Leu Ser Glu Asn Glu Ala Gly Val Asn
       210                 215                 220
   TTT GCA AGA GAT ATT ACA GAT ATT CAA GGC GAA ACT CAT AAA GCA GAT      720
   Phe Ala Arg Asp Ile Thr Asp Ile Gln Gly Glu Thr His Lys Ala Asp
   225                 230                 235                 240
   CAA GAT AAA ATT GAT ATT GAA TTA GAT AAT GTT CAT AAA AGT GAT TCC      768
   Gln Asp Lys Ile Asp Ile Glu Leu Asp Asn Val His Lys Ser Asp Ser
                   245                 250                 255
   AAT ATA ACA GAG ACT ATT GAG AAT TTA AGA GAT CAG CTT GAA AAG GCT      816
   Asn Ile Thr Glu Thr Ile Glu Asn Leu Arg Asp Gln Leu Glu Lys Ala
               260                 265                 270
   ACA GAT GAA GAG CAT AGA AAA GAG ATT GAA AGT CAG GTT GAT GCT AAA      864
   Thr Asp Glu Glu His Arg Lys Glu Ile Glu Ser Gln Val Asp Ala Lys
           275                 280                 285
   AAG AAA CAA AAA GAA GAA CTA GAT AAA AAG GCA ATC GAT CTT GAT AAA      912
   Lys Lys Gln Lys Glu Glu Leu Asp Lys Lys Ala Ile Asp Leu Asp Lys
       290                 295                 300
   GCC CAA CAA AAA TTA GAT TCT TCT GAA GAT AAT TTA GAT ATT CAA AGG      960
   Ala Gln Gln Lys Leu Asp Ser Ser Glu Asp Asn Leu Asp Ile Gln Arg
   305                 310                 315                 320
   GAT ACT GTT AGA GAG AAG ATT CAA GAG GAT ATT GAC GAG ATT AAT AAA     1008
   Asp Thr Val Arg Glu Lys Ile Gln Glu Asp Ile Asp Glu Ile Asn Lys
                   325                 330                 335
   GAA AAG AAT TTG CCA AAA CCT GGT GAT GTA AGT TCT CCT AAA GTT GAT     1056
   Glu Lys Asn Leu Pro Lys Pro Gly Asp Val Ser Ser Pro Lys Val Asp
               340                 345                 350
   AAG CAG CTA CAA ATA AAA GAG AGT CTA GAA GAC TTG CAG GAA CAG CTT     1104
   Lys Gln Leu Gln Ile Lys Glu Ser Leu Glu Asp Leu Gln Glu Gln Leu
           355                 360                 365
   AAA GAA ACT AGC GAT GAA AAT CAA AAA AGA GAA ATT GAA AAG CAA ATT     1152
   Lys Glu Thr Ser Asp Glu Asn Gln Lys Arg Glu Ile Glu Lys Gln Ile
       370                 375                 380
   GAA ATC AAA AAA AGT GAT GAA GAA CTT TTA AAA AGT AAA GAT CCT AAA     1200
   Glu Ile Lys Lys Ser Asp Glu Glu Leu Leu Lys Ser Lys Asp Pro Lys
   385                 390                 395                 400
   GCA TTA GAT CTT AAT GGA GAT TTA AAT TCT AAA GTT TCT AGT AAA GAA        1248
   Ala Leu Asp Leu Asn Gly Asp Leu Asn Ser Lys Val Ser Ser Lys Glu
                   405                 410                 415
   AAA ATT AAA GGC AAA GAA GGA GAA ATA GTC AAA GAG GAA TCA AAG GCA        1296
   Lys Ile Lys Gly Lys Glu Gly Glu Ile Val Lys Glu Glu Ser Lys Ala
               420                 425                 430
   AGT TTA GCT GAT TTG AAT AAT GAC GAA AAT CTT ATG AGG CCG GAA GAT        1344
   Ser Leu Ala Asp Leu Asn Asn Asp Glu Asn Leu Met Arg Pro Glu Asp
           435                 440                 445
   CAA AAA TTA TCT GAG GAT AAA AAA TTA GAT AGT AAA AAA AAT TTA AAA        1392
   Gln Lys Leu Ser Glu Asp Lys Lys Leu Asp Ser Lys Lys Asn Leu Lys
       450                 455                 460
   CCT GTT TCT GAG ATT GAG AGA GTA AAT GAA ATT TCG AAG TCT AAC AAC        1440
   Pro Val Ser Glu Ile Glu Arg Val Asn Glu Ile Ser Lys Ser Asn Asn
   465                 470                 475                 480
   AAT GAG ATT AGT GAA TCA TCA CCA TTA TAT AAG CCT TCT TAT AGC GAT        1488
   Asn Glu Ile Ser Glu Ser Ser Pro Leu Tyr Lys Pro Ser Tyr Ser Asp
                   485                 490                 495
   ATG GAT TCA AAA GAG GGT ATA GAT AAT AAA GAT GTT AAC TTG CAA GAA        1536
   Met Asp Ser Lys Glu Gly Ile Asp Asn Lys Asp Val Asn Leu Gln Glu
               500                 505                 510
   ACC AAG TCT CAA ACT AAA AGT CAA CCT ACT TCT TTA AAT CAA GAT TTG        1584
   Thr Lys Ser Gln Thr Lys Ser Gln Pro Thr Ser Leu Asn Gln Asp Leu
           515                 520                 525
   ACT ACT ATG TCT ATA GAT TCT AGT AAT CCT GTA TTT TTA GAG GTT ATT        1632
   Thr Thr Met Ser Ile Asp Ser Ser Asn Pro Val Phe Leu Glu Val Ile
       530                 535                 540
   GAT CCT ATT ACA AAT TTA GGA ACG CTT CAA CTT ATT GAT TTG AAT ACC        1680
   Asp Pro Ile Thr Asn Leu Gly Thr Leu Gln Leu Ile Asp Leu Asn Thr
   545                 550                 555                 560
   GGT GTT AGA CTT AAA GAA AGT ACT CAG CAA GGC ATT CAG CGG TAT GGA        1728
   Gly Val Arg Leu Lys Glu Ser Thr Gln Gln Gly Ile Gln Arg Tyr Gly
                   565                 570                 575
   ATT TAT GAA CGT GAA AAA GAT TTA GTT GTT ATT AAA ATG GAT TCA GGA        1776
   Ile Tyr Glu Arg Glu Lys Asp Leu Val Val Ile Lys Met Asp Ser Gly
               580                 585                 590
   AAA GCC AAG CTT CAA ATA CTT AAT AAA CTT GAG AAT TTA AAA GTG ATA        1824
   Lys Ala Lys Leu Gln Ile Leu Asn Lys Leu Glu Asn Leu Lys Val Ile
           595                 600                 605
   TCG GAG TCT AAT TTT GAG ATT AAT AAA AAT TCA TCT CTT TAT GTT GAC        1872
   Ser Glu Ser Asn Phe Glu Ile Asn Lys Asn Ser Ser Leu Tyr Val Asp
       610                 615                 620
   TCT AAA ATG ATT TTA GTA GTT GTG AGA GAT AGT GGT AAT GTT TGG AGA        1920
   Ser Lys Met Ile Leu Val Val Val Arg Asp Ser Gly Asn Val Trp Arg
   625                 630                 635                 640
   TTG GCT AAA TTT TCT CCT AAA AAT TTA AAT GAG TTT ATT CTT TCA GAG      1968
   Leu Ala Lys Phe Ser Pro Lys Asn Leu Asn Glu Phe Ile Leu Ser Glu
                   645                 650                 655
   AAT AAA ATT TTG CCT TTT ACT AGC TTT TCT GTG AGA AAG AAT TTT ATT      2016
   Asn Lys Ile Leu Pro Phe Thr Ser Phe Ser Val Arg Lys Asn Phe Ile
               660                 665                 670
   TAT TTG CAG GAT GAG TTT AAA AGT CTT ATT ACT TTA GAT GTA AAT ACT      2064
   Tyr Leu Gln Asp Glu Phe Lys Ser Leu Ile Thr Leu Asp Val Asn Thr
           675                 680                 685
   TTA AAA AAA GTT AAG TA                                               2081
   Leu Lys Lys Val Lys
       690
(2) INFORMATION FOR SEQ ID NO:68:
   (i) SEQUENCE CHARACTERISTICS:
      (A) LENGTH: 693 amino acids
      (B) TYPE: amino acid
      (D) TOPOLOGY: linear
   (ii) MOLECULE TYPE: protein
   (xi) SEQUENCE DESCRIPTION: SEQ ID NO:68:
   Met Lys Lys Leu Leu Leu Ile Phe Ser Phe Phe Leu Ile Ser Leu Asn
    1               5                  10                  15
   Gly Phe Pro Leu Asn Ser Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Ser Asn Ser Asn Ser Ile Tyr Tyr Gly Lys Tyr
   65                  70                  75                  80
   Phe Ile Asn Arg Phe Ile Asp Asp Gln Asp Lys Lys Ala Ser Val Asp
                   85                  90                  95
   Val Phe Ser Ile Gly Ser Arg Ser Gln Leu Asp Ser Ile Leu Asn Leu
               100                 105                 110
   Arg Arg Ile Leu Thr Gly Tyr Leu Ile Lys Ser Phe Asp Tyr Glu Arg
           115                 120                 125
   Ser Ser Ala Glu Leu Ile Ala Lys Val Ile Thr Ile His Asn Ala Val
       130                 135                 140
   Tyr Arg Gly Asp Leu Asn Tyr Tyr Lys Glu Val Tyr Ile Glu Ala Ala
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
   180 185 190
   Leu Ser Gly Lys Val Glu Ser Asp Ile Asp Ile Asp Ser Leu Val Thr
   195 200 205
   Asp Lys Val Val Ala Ala Leu Leu Ser Glu Asn Glu Ala Gly Val Asn
   210 215 220
   Phe Ala Arg Asp Ile Thr Asp Ile Gln Gly Glu Thr His Lys Ala Asp
   225 230 235 240
   Gln Asp Lys Ile Asp Ile Glu Leu Asp Asn Val His Lys Ser Asp Ser
   245 250 255
   Asn Ile Thr Glu Thr Ile Glu Asn Leu Arg Asp Gln Leu Glu Lys Ala
   260 265 270
   Thr Asp Glu Glu His Arg Lys Glu Ile Glu Ser Gln Val Asp Ala Lys
   275 280 285
   Lys Lys Gln Lys Glu Glu Leu Asp Lys Lys Ala Ile Asp Leu Asp Lys
   290 295 300
   Ala Gln Gln Lys Leu Asp Ser Ser Glu Asp Asn Leu Asp Ile Gln Arg
   305 310 315 320
   Asp Thr Val Arg Glu Lys Ile Gln Glu Asp Ile Asp Glu Ile Asn Lys
   325 330 335
   Glu Lys Asn Leu Pro Lys Pro Gly Asp Val Ser Ser Pro Lys Val Asp
   340 345 350
   Lys Gln Leu Gln Ile Lys Glu Ser Leu Glu Asp Leu Gln Glu Gln Leu
   355 360 365
   Lys Glu Thr Ser Asp Glu Asn Gln Lys Arg Glu Ile Glu Lys Gln Ile
   370 375 380
   Glu Ile Lys Lys Ser Asp Glu Glu Leu Leu Lys Ser Lys Asp Pro Lys
   385 390 395 400
   Ala Leu Asp Leu Asn Gly Asp Leu Asn Ser Lys Val Ser Ser Lys Glu
   405 410 415
   Lys Ile Lys Gly Lys Glu Gly Glu Ile Val Lys Glu Glu Ser Lys Ala
   420 425 430
   Ser Leu Ala Asp Leu Asn Asn Asp Glu Asn Leu Met Arg Pro Glu Asp
   435 440 445
   Gln Lys Leu Ser Glu Asp Lys Lys Leu Asp Ser Lys Lys Asn Leu Lys
   450 455 460
   Pro Val Ser Glu Ile Glu Arg Val Asn Glu Ile Ser Lys Ser Asn Asn
   465 470 475 480
   Asn Glu Ile Ser Glu Ser Ser Pro Leu Tyr Lys Pro Ser Tyr Ser Asp
   485 490 495
   Met Asp Ser Lys Glu Gly Ile Asp Asn Lys Asp Val Asn Leu Gln Glu
               500                 505                 510
   Thr Lys Ser Gln Thr Lys Ser Gln Pro Thr Ser Leu Asn Gln Asp Leu
           515                 520                 525
   Thr Thr Met Ser Ile Asp Ser Ser Asn Pro Val Phe Leu Glu Val Ile
       530                 535                 540
   Asp Pro Ile Thr Asn Leu Gly Thr Leu Gln Leu Ile Asp Leu Asn Thr
   545                 550                 555                 560
   Gly Val Arg Leu Lys Glu Ser Thr Gln Gln Gly Ile Gln Arg Tyr Gly
                   565                 570                 575
   Ile Tyr Glu Arg Glu Lys Asp Leu Val Val Ile Lys Met Asp Ser Gly
               580                 585                 590
   Lys Ala Lys Leu Gln Ile Leu Asn Lys Leu Glu Asn Leu Lys Val Ile
           595                 600                 605
   Ser Glu Ser Asn Phe Glu Ile Asn Lys Asn Ser Ser Leu Tyr Val Asp
       610                 615                 620
   Ser Lys Met Ile Leu Val Val Val Arg Asp Ser Gly Asn Val Trp Arg
   625                 630                 635                 640
   Leu Ala Lys Phe Ser Pro Lys Asn Leu Asn Glu Phe Ile Leu Ser Glu
                   645                 650                 655
   Asn Lys Ile Leu Pro Phe Thr Ser Phe Ser Val Arg Lys Asn Phe Ile
               660                 665                 670
   Tyr Leu Gln Asp Glu Phe Lys Ser Leu Ile Thr Leu Asp Val Asn Thr
           675                 680                 685
   Leu Lys Lys Val Lys
       690
(2) INFORMATION FOR SEQ ID NO:69:
   (i) SEQUENCE CHARACTERISTICS:
      (A) LENGTH: 1991 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..1989
   (xi) SEQUENCE DESCRIPTION: SEQ ID NO:69:
   ATG AAA AAA ATG TTA CTA ATC TTT AGT TTT TTT CTT GTT TTT TTA AAT      48
   Met Lys Lys Met Leu Leu Ile Phe Ser Phe Phe Leu Val Phe Leu Asn
    1               5                  10                  15
   GGA TTT CCT CTT AAT GCA AGG GAA GTT GAT AAG GAA AAA TTA AAG GAC          96
   Gly Phe Pro Leu Asn Ala Arg Glu Val Asp Lys Glu Lys Leu Lys Asp
                20                  25                  30
   TTT GTT AAT ATG GAT CTT GAA TTT GTT AAT TAC AAG GGT CCT TAT GAT         144
   Phe Val Asn Met Asp Leu Glu Phe Val Asn Tyr Lys Gly Pro Tyr Asp
            35                  40                  45
   TCT ACA GAT ACA TAT GAA CAA ATA GTA GGT ATT GGG GAG TTT TTA GCA         192
   Ser Thr Asp Thr Tyr Glu Gln Ile Val Gly Ile Gly Glu Phe Leu Ala
        50                  55                  60
   AGG CCG TTG AAC AAT TCC AAT AGT AAT TCA AGT TAT TAT GGT AAA TAT         240
   Arg Pro Leu Asn Asn Ser Asn Ser Asn Ser Ser Tyr Tyr Gly Lys Tyr
    65                  70                  75                  80
   TTT GTT AAT AGA TTT ATT GAC GAT CAA GAT AAA AAA GCA AGT GTT GAT         288
   Phe Val Asn Arg Phe Ile Asp Asp Gln Asp Lys Lys Ala Ser Val Asp
                    85                  90                  95
   ATT TTT TCT ATT GGT AGT AAG TCA GAG CTT GAT AGT ATA TTA AAT CTA         336
   Ile Phe Ser Ile Gly Ser Lys Ser Glu Leu Asp Ser Ile Leu Asn Leu
               100                 105                 110
   AGA AGA ATT CTT ACA GGG TAT TTA ATG AAG TCT TTT GAT TAT GAG AGG         384
   Arg Arg Ile Leu Thr Gly Tyr Leu Met Lys Ser Phe Asp Tyr Glu Arg
           115                 120                 125
   TCT AGT GCG GAA TTA ATT GCT AAA GCT ATT ACA ATA TAT AAT GCT GTT         432
   Ser Ser Ala Glu Leu Ile Ala Lys Ala Ile Thr Ile Tyr Asn Ala Val
       130                 135                 140
   TAT AGA GGA GAT TTA GAT TAT TAC AAA GAG TTT TAT ATT GAG GCT TCT         480
   Tyr Arg Gly Asp Leu Asp Tyr Tyr Lys Glu Phe Tyr Ile Glu Ala Ser
   145                 150                 155                 160
   TTG AAG TCT TTG ACT AAA GAA AAT GCA GGT CTT TCT AGG GTG TAC AGT         528
   Leu Lys Ser Leu Thr Lys Glu Asn Ala Gly Leu Ser Arg Val Tyr Ser
                   165                 170                 175
   CAA TGG GCT GGG AAG ACA CAA ATA TTT ATT CCT CTT AAA AAG AAT ATT         576
   Gln Trp Ala Gly Lys Thr Gln Ile Phe Ile Pro Leu Lys Lys Asn Ile
               180                 185                 190
   TTA TCT GGA AAT GTT GAG TCT GAC ATT GAT ATT GAT AGT TTG GTT ACA         624
   Leu Ser Gly Asn Val Glu Ser Asp Ile Asp Ile Asp Ser Leu Val Thr
           195                 200                 205
   GAT AAG GTG GTG GCA GCT CTT TTA AGT GAG AAT GAA TCA GGT GTT AAC         672
   Asp Lys Val Val Ala Ala Leu Leu Ser Glu Asn Glu Ser Gly Val Asn
       210                 215                 220
   TTT GCA AGA GAT ATT ACA GAC ATT CAA GGC GAA ACT CAT AAA GCA GAT         720
   Phe Ala Arg Asp Ile Thr Asp Ile Gln Gly Glu Thr His Lys Ala Asp
   225                 230                 235                 240
   CAA GAT AAA ATT GAT ATT GAA TTA GAT AAT TTT CAT GAA AGT GAT TCC         768
   Gln Asp Lys Ile Asp Ile Glu Leu Asp Asn Phe His Glu Ser Asp Ser
                   245                 250                 255
   AAT ATA ACA GAA ACT ATT GAG AAT TTA AGG GAT CAG CTT GAA AAA GCT        816
   Asn Ile Thr Glu Thr Ile Glu Asn Leu Arg Asp Gln Leu Glu Lys Ala
               260                 265                 270
   ACA GAT GAA GAG CAT AAA AAA GAG ATT GAA AGT CAG GTT GAT GCT AAA        864
   Thr Asp Glu Glu His Lys Lys Glu Ile Glu Ser Gln Val Asp Ala Lys
           275                 280                 285
   AAG AAA CAA AAG GAA GAA TTA GAT AAA AAG GCA ATT GAT CTT GAT AAA        912
   Lys Lys Gln Lys Glu Glu Leu Asp Lys Lys Ala Ile Asp Leu Asp Lys
       290                 295                 300
   GCT CAA CAA AAA TTA GAT TTT GCT GAA GAT AAT CTA GAT ATT CAA AGG        960
   Ala Gln Gln Lys Leu Asp Phe Ala Glu Asp Asn Leu Asp Ile Gln Arg
   305                 310                 315                 320
   GAT ACT GTT AGA GAG AAG CTT CAA GAA AAT ATT AAC GAG ACT AAT AAG       1008
   Asp Thr Val Arg Glu Lys Leu Gln Glu Asn Ile Asn Glu Thr Asn Lys
                   325                 330                 335
   GAA AAG AAT TTA CCA AAG CCT GGT GAT GTA AGT TCT CCT AAG GTT GAT       1056
   Glu Lys Asn Leu Pro Lys Pro Gly Asp Val Ser Ser Pro Lys Val Asp
               340                 345                 350
   AAG CAG TTG CAG ATA AAA GAG AGT CTA GAA GAT TTG CAA GAG CAG CTT       1104
   Lys Gln Leu Gln Ile Lys Glu Ser Leu Glu Asp Leu Gln Glu Gln Leu
           355                 360                 365
   AAA GAA GCT AGT GAT GAA AAT CAA AAA AGA GAA ATA GAA AAG CAA ATT       1152
   Lys Glu Ala Ser Asp Glu Asn Gln Lys Arg Glu Ile Glu Lys Gln Ile
       370                 375                 380
   GAA ATC AAA AAA AAT GAT GAA GAA CTT TTT AAA AAT AAA GAT CAT AAA       1200
   Glu Ile Lys Lys Asn Asp Glu Glu Leu Phe Lys Asn Lys Asp His Lys
   385                 390                 395                 400
   GCA TTA GAT CTT AAG CAA GAA TTA AAT TCT AAA GCT TCT AGT AAA GAA       1248
   Ala Leu Asp Leu Lys Gln Glu Leu Asn Ser Lys Ala Ser Ser Lys Glu
                   405                 410                 415
   AAA ATT GAA GGC GAA GAA GAG GAT AAA GAA TTA GAT AGT AAA AAA AAT       1296
   Lys Ile Glu Gly Glu Glu Glu Asp Lys Glu Leu Asp Ser Lys Lys Asn
               420                 425                 430
   TTA GAG CCT GTT TCT GAG GCT GAT AAA GTA GAT AAA ATT TCC AAG TCT       1344
   Leu Glu Pro Val Ser Glu Ala Asp Lys Val Asp Lys Ile Ser Lys Ser
           435                 440                 445
   AAC AAC AAT GAG GTT AGT AAA TTA TCC CCG TTA GAT GAG CCT TCT TAT       1392
   Asn Asn Asn Glu Val Ser Lys Leu Ser Pro Leu Asp Glu Pro Ser Tyr
       450                 455                 460
   AGC GAC ATT GAT TCG AAA GAG GGT GTA GAT AAC AAA GAT GTT GAT TTG       1440
   Ser Asp Ile Asp Ser Lys Glu Gly Val Asp Asn Lys Asp Val Asp Leu
   465                 470                 475                 480
   CAA AAA ACT AAA CCC CAA GTT GAA AGT CAA CCT ACT TCG TTA AAT GAA       1488
   Gln Lys Thr Lys Pro Gln Val Glu Ser Gln Pro Thr Ser Leu Asn Glu
                   485                 490                 495
   GAT TTG ATT GAT GTG TCT ATA GAT TCC AGT AAT CCT GTC TTT TTA GAG        1536
   Asp Leu Ile Asp Val Ser Ile Asp Ser Ser Asn Pro Val Phe Leu Glu
               500                 505                 510
   GTT ATC GAT CCG ATT ACA AAT TTA GGA ACG CTT CAA CTT ATT GAT TTG        1584
   Val Ile Asp Pro Ile Thr Asn Leu Gly Thr Leu Gln Leu Ile Asp Leu
           515                 520                 525
   AAT ACC GGT GTT AGA CTT AAA GAA AGT GCT CAA CAA GGT ATT CAG CGA        1632
   Asn Thr Gly Val Arg Leu Lys Glu Ser Ala Gln Gln Gly Ile Gln Arg
       530                 535                 540
   TAT GGA ATT TAT GAA CGT GAA AAA GAT TTG GTT GTT ATT AAA ATA GAT        1680
   Tyr Gly Ile Tyr Glu Arg Glu Lys Asp Leu Val Val Ile Lys Ile Asp
   545                 550                 555                 560
   TCA GGA AAA GCT AAG CTT CAG ATA CTT GAT AAA CTC GAG AAT TTA AAA        1728
   Ser Gly Lys Ala Lys Leu Gln Ile Leu Asp Lys Leu Glu Asn Leu Lys
                   565                 570                 575
   GTG ATA TCA GAG TCT AAT TTT GAG ATT AAT AAA AAT TCA TCT CTT TAT        1776
   Val Ile Ser Glu Ser Asn Phe Glu Ile Asn Lys Asn Ser Ser Leu Tyr
               580                 585                 590
   GTT GAC TCT AGA ATG ATT TTA GTA GTT GTT AAG GAC GAT AGT AAT GCT        1824
   Val Asp Ser Arg Met Ile Leu Val Val Val Lys Asp Asp Ser Asn Ala
           595                 600                 605
   TGG AGA TTG GCT AAA TTT TCT CCT AAA AAT TTA GAT GAA TTT ATT CTG        1872
   Trp Arg Leu Ala Lys Phe Ser Pro Lys Asn Leu Asp Glu Phe Ile Leu
       610                 615                 620
   TCA GAA AAT AAA ATT TTG CCT TTT ACT AGC TTT GCT GTG AGA AAG AAT        1920
   Ser Glu Asn Lys Ile Leu Pro Phe Thr Ser Phe Ala Val Arg Lys Asn
   625                 630                 635                 640
   TTT ATT TAT TTG CAA GAT GAA CTT AAA AGC TTA GTT ACT TTA GAT GTA        1968
   Phe Ile Tyr Leu Gln Asp Glu Leu Lys Ser Leu Val Thr Leu Asp Val
                   645                 650                 655
   AAT ACT TTA AAA AAA GTT AAG TA                                         1991
   Asn Thr Leu Lys Lys Val Lys
               660
(2) INFORMATION FOR SEQ ID NO:70:
   (i) SEQUENCE CHARACTERISTICS:
      (A) LENGTH: 663 amino acids
      (B) TYPE: amino acid
      (D) TOPOLOGY: linear
   (ii) MOLECULE TYPE: protein
   (xi) SEQUENCE DESCRIPTION: SEQ ID NO:70:
   Met Lys Lys Met Leu Leu Ile Phe Ser Phe Phe Leu Val Phe Leu Asn
   1               5                   10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asp Thr Tyr Glu Gln Ile Val Gly Ile Gly Glu Phe Leu Ala
       50                  55                  60
   Arg Pro Leu Asn Asn Ser Asn Ser Asn Ser Ser Tyr Tyr Gly Lys Tyr
   65                  70                  75                  80
   Phe Val Asn Arg Phe Ile Asp Asp Gln Asp Lys Lys Ala Ser Val Asp
                   85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Phe His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ala Glu Asp Asn Leu Asp Ile Gln Arg
   305                 310                 315                 320
   Asp Thr Val Arg Glu Lys Leu Gln Glu Asn Ile Asn Glu Thr Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Ala Ser Asp Glu Asn Gln Lys Arg Glu Ile Glu Lys Gln Ile
       370                 375                 380
   Glu Ile Lys Lys Asn Asp Glu Glu Leu Phe Lys Asn Lys Asp His Lys
   385                 390                 395                 400
   Ala Leu Asp Leu Lys Gln Glu Leu Asn Ser Lys Ala Ser Ser Lys Glu
                   405                 410                 415
   Lys Ile Glu Gly Glu Glu Glu Asp Lys Glu Leu Asp Ser Lys Lys Asn
               420                 425                 430
   Leu Glu Pro Val Ser Glu Ala Asp Lys Val Asp Lys Ile Ser Lys Ser
           435                 440                 445
   Asn Asn Asn Glu Val Ser Lys Leu Ser Pro Leu Asp Glu Pro Ser Tyr
       450                 455                 460
   Ser Asp Ile Asp Ser Lys Glu Gly Val Asp Asn Lys Asp Val Asp Leu
   465                 470                 475                 480
   Gln Lys Thr Lys Pro Gln Val Glu Ser Gln Pro Thr Ser Leu Asn Glu
                   485                 490                 495
   Asp Leu Ile Asp Val Ser Ile Asp Ser Ser Asn Pro Val Phe Leu Glu
               500                 505                 510
   Val Ile Asp Pro Ile Thr Asn Leu Gly Thr Leu Gln Leu Ile Asp Leu
           515                 520                 525
   Asn Thr Gly Val Arg Leu Lys Glu Ser Ala Gln Gln Gly Ile Gln Arg
       530                 535                 540
   Tyr Gly Ile Tyr Glu Arg Glu Lys Asp Leu Val Val Ile Lys Ile Asp
   545                 550                 555                 560
   Ser Gly Lys Ala Lys Leu Gln Ile Leu Asp Lys Leu Glu Asn Leu Lys
                   565                 570                 575
   Val Ile Ser Glu Ser Asn Phe Glu Ile Asn Lys Asn Ser Ser Leu Tyr
               580                 585                 590
   Val Asp Ser Arg Met Ile Leu Val Val Val Lys Asp Asp Ser Asn Ala
           595                 600                 605
   Trp Arg Leu Ala Lys Phe Ser Pro Lys Asn Leu Asp Glu Phe Ile Leu
       610                 615                 620
   Ser Glu Asn Lys Ile Leu Pro Phe Thr Ser Phe Ala Val Arg Lys Asn
   625                 630                 635                 640
   Phe Ile Tyr Leu Gln Asp Glu Leu Lys Ser Leu Val Thr Leu Asp Val
                   645                 650                 655
   Asn Thr Leu Lys Lys Val Lys
               660
(2) INFORMATION FOR SEQ ID NO:71:
   (i) SEQUENCE CHARACTERISTICS:
      (A) LENGTH: 2081 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..2079
   (xi) SEQUENCE DESCRIPTION: SEQ ID NO:71:
   ATG AAA AAA ATG TTA CTA ATC TTT AGT TTT TTT CTT ATT TCT TTG AAT       48
   Met Lys Lys Met Leu Leu Ile Phe Ser Phe Phe Leu Ile Ser Leu Asn
     1               5                  10                  15
   GGA TTT CCC CTT AAT GCA AGG GAA GTT GAT AAG GAA AAA TTA AAG GAC       96
   Gly Phe Pro Leu Asn Ala Arg Glu Val Asp Lys Glu Lys Leu Lys Asp
                20                  25                  30
   TTT GTT AAT ATG GAT CTT GAG TTT GTA AAC TAT AAA GGT CCT TAT GAT      144
   Phe Val Asn Met Asp Leu Glu Phe Val Asn Tyr Lys Gly Pro Tyr Asp
            35                  40                  45
   TCT ACA AAT ACA TAT GAA CAA ATA GTA GGT ATT GGT GAG TTT TTA GCA      192
   Ser Thr Asn Thr Tyr Glu Gln Ile Val Gly Ile Gly Glu Phe Leu Ala
        50                  55                  60
   AGA CCA TTG ATT AAT TTC AAT AGC AAC TCA AGT TAT TAT GGT AAA TAT      240
   Arg Pro Leu Ile Asn Phe Asn Ser Asn Ser Ser Tyr Tyr Gly Lys Tyr
    65                  70                  75                  80
   TTT ATT AAT AGA TTT ATT GAC GAT CAA GAT AAA AAA GCA AGC GTT GAT      288
   Phe Ile Asn Arg Phe Ile Asp Asp Gln Asp Lys Lys Ala Ser Val Asp
                    85                  90                  95
   GTT TTT TCT ATT AGT AGT AAG TCA CAG CTT GAC AGT ATA TTG AAT TTA      336
   Val Phe Ser Ile Ser Ser Lys Ser Gln Leu Asp Ser Ile Leu Asn Leu
               100                 105                 110
   AGA AGA ATT CTT ACA GGG TAT TTG ATA AAG TCT TTT GAT TAT GAA AGA      384
   Arg Arg Ile Leu Thr Gly Tyr Leu Ile Lys Ser Phe Asp Tyr Glu Arg
           115                 120                 125
   TCT AGT GCT GAA TTA ATT GCC AAG GTT ATT ACA ATA CAT AAT GCT GTT      432
   Ser Ser Ala Glu Leu Ile Ala Lys Val Ile Thr Ile His Asn Ala Val
       130                 135                 140
   TAT AGA GGT GAT TTA AAT TAT TAT AAA GAG TTT TAT ATT GAG TCT GCT      480
   Tyr Arg Gly Asp Leu Asn Tyr Tyr Lys Glu Phe Tyr Ile Glu Ser Ala
   145                 150                 155                 160
   TTA AAG TCT TTA ACT AAA GAA AAT GCA GGT CTT TCT AGA GTG TAC AGT      528
   Leu Lys Ser Leu Thr Lys Glu Asn Ala Gly Leu Ser Arg Val Tyr Ser
                   165                 170                 175
   CAA TGG GCT GGA AAG ACA CAA ATA TTT ATT CCT CTT AAA AAG AAT ATT      576
   Gln Trp Ala Gly Lys Thr Gln Ile Phe Ile Pro Leu Lys Lys Asn Ile
               180                 185                 190
   TTA TCT GGA AAA ATT GAG TCT GAC ATT GAT ATT GAT AGT TTG GTT ACA      624
   Leu Ser Gly Lys Ile Glu Ser Asp Ile Asp Ile Asp Ser Leu Val Thr
           195                 200                 205
   GAT AAG GTT GTG GCA GCT CTT TTA AGC GAA AAT GAA GCA GGT GTT AAC      672
   Asp Lys Val Val Ala Ala Leu Leu Ser Glu Asn Glu Ala Gly Val Asn
       210                 215                 220
   TTT GCA AGG GAT ATT ACA GAT ATT CAA GGA GAA ACT CAT AAA GCA GAT      720
   Phe Ala Arg Asp Ile Thr Asp Ile Gln Gly Glu Thr His Lys Ala Asp
   225                 230                 235                 240
   CAA GAT AAA ATT GAT ATT GAA TTA GAT AAT GTT CAT GAA AGT GAT TCC      768
   Gln Asp Lys Ile Asp Ile Glu Leu Asp Asn Val His Glu Ser Asp Ser
                   245                 250                 255
   AAT ATA ACA GAA ACT ATT GAG AAT TTA AGA GAT CAG CTT GAA AAG GCT      816
   Asn Ile Thr Glu Thr Ile Glu Asn Leu Arg Asp Gln Leu Glu Lys Ala
               260                 265                 270
   ACA GAT GAA GAG CAT AGA AAA GAG ATT GAA AGT CAA GTT GAT GCT AAA      864
   Thr Asp Glu Glu His Arg Lys Glu Ile Glu Ser Gln Val Asp Ala Lys
           275                 280                 285
   AAG AAA CAA AAA GAA GAA CTA GAT AAA AAG GCA ATC GAT CTT GAT AAA      912
   Lys Lys Gln Lys Glu Glu Leu Asp Lys Lys Ala Ile Asp Leu Asp Lys
       290                 295                 300
   GCC CAA CAA AAA TTA GAT TTT TCT GAA GAT AAT TTA GAT ATT CAA AGG      960
   Ala Gln Gln Lys Leu Asp Phe Ser Glu Asp Asn Leu Asp Ile Gln Arg
   305                 310                 315                 320
   GAT ACT GTT AGA GAG AAG ATT CAA GAG GAT ATT AAC GAG ATT AAT AAG     1008
   Asp Thr Val Arg Glu Lys Ile Gln Glu Asp Ile Asn Glu Ile Asn Lys
                   325                 330                 335
   GAA AAG AAT TTA CCA AAA CCT GGT GAT GTA AGT TCT CCT AAA GTT GAT     1056
   Glu Lys Asn Leu Pro Lys Pro Gly Asp Val Ser Ser Pro Lys Val Asp
               340                 345                 350
   AAG CAG CTA CAA ATA AAA GAG AGT CTA GAA GAC TTG CAG GAG CAG CTT     1104
   Lys Gln Leu Gln Ile Lys Glu Ser Leu Glu Asp Leu Gln Glu Gln Leu
           355                 360                 365
   AAA GAA ACT AGC GAT GAA AAT CAA AAA AGA GAA ATT GAA AAG CAA ATT     1152
   Lys Glu Thr Ser Asp Glu Asn Gln Lys Arg Glu Ile Glu Lys Gln Ile
       370                 375                 380
   GAA ATC AAA AAA AGT GAT GAA GAA CTT TTA AAA AGC AAA GAT CCT AAA     1200
   Glu Ile Lys Lys Ser Asp Glu Glu Leu Leu Lys Ser Lys Asp Pro Lys
   385                 390                 395                 400
   GCA TTA GAT CTT AAT CGA GAT TTA AAT TCT AAA GCT TCT AGT AAA GAA      1248
   Ala Leu Asp Leu Asn Arg Asp Leu Asn Ser Lys Ala Ser Ser Lys Glu
                   405                 410                 415
   AAA ATT AAA GGC AAA GAA AAA GAA ATA GTC AAA GAG AAA TCA AAG GTA      1296
   Lys Ile Lys Gly Lys Glu Lys Glu Ile Val Lys Glu Lys Ser Lys Val
               420                 425                 430
   AGT TTA GGT GAT TTG GAT AAT GAC GAA ACC CTT ATG ACG CCG GAA GAT      1344
   Ser Leu Gly Asp Leu Asp Asn Asp Glu Thr Leu Met Thr Pro Glu Asp
           435                 440                 445
   CAA AAA TTA TCT GAG GAT AAA AAA TTA GAT AGT AAA AAA AAT TTA AAA      1392
   Gln Lys Leu Ser Glu Asp Lys Lys Leu Asp Ser Lys Lys Asn Leu Lys
       450                 455                 460
   CCT GTT TCT GAG ATT GAG AGA GTA AAT GAA ATT TCA AAG TCT AAC AAC      1440
   Pro Val Ser Glu Ile Glu Arg Val Asn Glu Ile Ser Lys Ser Asn Asn
   465                 470                 475                 480
   AAT GAG GTT AGC AAA TCA TCA CCA TTA GAT AAG CCT TCT TAT AGT GAT      1488
   Asn Glu Val Ser Lys Ser Ser Pro Leu Asp Lys Pro Ser Tyr Ser Asp
                   485                 490                 495
   ATC GAT TCA AAA GAG GTT GTA GAT AAT AAA GAT GTT AAT TTG CAA GAA      1536
   Ile Asp Ser Lys Glu Val Val Asp Asn Lys Asp Val Asn Leu Gln Glu
               500                 505                 510
   ACC AAG CCT CAA GCT AAA AGT CAA TCT ACT TCT TTA AAT CAA GAT TTG      1584
   Thr Lys Pro Gln Ala Lys Ser Gln Ser Thr Ser Leu Asn Gln Asp Leu
           515                 520                 525
   ATT ACT ATG TCT ATA GAT TCT AGT AAT CCT GTA TTT TTA GAG GTT ATT      1632
   Ile Thr Met Ser Ile Asp Ser Ser Asn Pro Val Phe Leu Glu Val Ile
       530                 535                 540
   GAT CCT ATT ACA AAT TTA GGA ATG CTT CAA CTT ATT GAT TTA AAT ACT      1680
   Asp Pro Ile Thr Asn Leu Gly Met Leu Gln Leu Ile Asp Leu Asn Thr
   545                 550                 555                 560
   GGT GTT AGA CTT AAA GAA AGC ACT CAG CAA GGC ATT CAG CGT TAT GGA      1728
   Gly Val Arg Leu Lys Glu Ser Thr Gln Gln Gly Ile Gln Arg Tyr Gly
                   565                 570                 575
   ATT TAT GAA CGT GAA AAA GAT TTA GTT GTT ATT AAA ATG GAT TCA GGA      1776
   Ile Tyr Glu Arg Glu Lys Asp Leu Val Val Ile Lys Met Asp Ser Gly
               580                 585                 590
   AAA GCT AAG CTT CAA ATA CTT AAT AAA CTT GAG AAT TTA AAA GTG ATA      1824
   Lys Ala Lys Leu Gln Ile Leu Asn Lys Leu Glu Asn Leu Lys Val Ile
           595                 600                 605
   TCA GAG TCT AAT TTT GAG ATT AAT AAA AAT TCA TCT CTT TAT GTT GAC      1872
   Ser Glu Ser Asn Phe Glu Ile Asn Lys Asn Ser Ser Leu Tyr Val Asp
       610                 615                 620
   TCT AAA ATG ATT TTA GTA GCT GTG AAA GAT AGT GGT AAT GTT TGG AGA      1920
   Ser Lys Met Ile Leu Val Ala Val Lys Asp Ser Gly Asn Val Trp Arg
   625                 630                 635                 640
   TTG GCT AAA TTT TCT CCT AAA AAT TTA GAT GAG TTT ATT CTT TCA GAG      1968
   Leu Ala Lys Phe Ser Pro Lys Asn Leu Asp Glu Phe Ile Leu Ser Glu
                   645                 650                 655
   AAT AAA ATT TTG CCT TTT ACT AGC TTT TCT GTG AGA AAG AAT TTT ATT      2016
   Asn Lys Ile Leu Pro Phe Thr Ser Phe Ser Val Arg Lys Asn Phe Ile
               660                 665                 670
   TAT TTG CAA GAT GAG TTT AAA AGT CTT ATT ACT TTA GAT GTA AAT ACT      2064
   Tyr Leu Gln Asp Glu Phe Lys Ser Leu Ile Thr Leu Asp Val Asn Thr
           675                 680                 685
   TTA AAA AAA GTT AAG TA                                               2081
   Leu Lys Lys Val Lys
       690
(2) INFORMATION FOR SEQ ID NO:72:
   (i) SEQUENCE CHARACTERISTICS:
      (A) LENGTH: 693 amino acids
      (B) TYPE: amino acid
      (D) TOPOLOGY: linear
   (ii) MOLECULE TYPE: protein
   (xi) SEQUENCE DESCRIPTION: SEQ ID NO:72:
   Met Lys Lys Met Leu Leu Ile Phe Ser Phe Phe Leu Ile Ser Leu Asn
    1               5                  10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Phe Asn Ser Asn Ser Ser Tyr Tyr Gly Lys Tyr
   65                  70                  75                  80
   Phe Ile Asn Arg Phe Ile Asp Asp Gln Asp Lys Lys Ala Ser Val Asp
                   85                  90                  95
   Val Phe Ser Ile Ser Ser Lys Ser Gln Leu Asp Ser Ile Leu Asn Leu
               100                 105                 110
   Arg Arg Ile Leu Thr Gly Tyr Leu Ile Lys Ser Phe Asp Tyr Glu Arg
           115                 120                 125
   Ser Ser Ala Glu Leu Ile Ala Lys Val Ile Thr Ile His Asn Ala Val
       130                 135                 140
   Tyr Arg Gly Asp Leu Asn Tyr Tyr Lys Glu Phe Tyr Ile Glu Ser Ala
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Lys Ile Glu Ser Asp Ile Asp Ile Asp Ser Leu Val Thr
           195                 200                 205
   Asp Lys Val Val Ala Ala Leu Leu Ser Glu Asn Glu Ala Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Val His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Arg Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ser Glu Asp Asn Leu Asp Ile Gln Arg
   305                 310                 315                 320
   Asp Thr Val Arg Glu Lys Ile Gln Glu Asp Ile Asn Glu Ile Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Thr Ser Asp Glu Asn Gln Lys Arg Glu Ile Glu Lys Gln Ile
       370                 375                 380
   Glu Ile Lys Lys Ser Asp Glu Glu Leu Leu Lys Ser Lys Asp Pro Lys
   385                 390                 395                 400
   Ala Leu Asp Leu Asn Arg Asp Leu Asn Ser Lys Ala Ser Ser Lys Glu
                   405                 410                 415
   Lys Ile Lys Gly Lys Glu Lys Glu Ile Val Lys Glu Lys Ser Lys Val
               420                 425                 430
   Ser Leu Gly Asp Leu Asp Asn Asp Glu Thr Leu Met Thr Pro Glu Asp
           435                 440                 445
   Gln Lys Leu Ser Glu Asp Lys Lys Leu Asp Ser Lys Lys Asn Leu Lys
       450                 455                 460
   Pro Val Ser Glu Ile Glu Arg Val Asn Glu Ile Ser Lys Ser Asn Asn
   465                 470                 475                 480
   Asn Glu Val Ser Lys Ser Ser Pro Leu Asp Lys Pro Ser Tyr Ser Asp
                   485                 490                 495
   Ile Asp Ser Lys Glu Val Val Asp Asn Lys Asp Val Asn Leu Gln Glu
               500                 505                 510
   Thr Lys Pro Gln Ala Lys Ser Gln Ser Thr Ser Leu Asn Gln Asp Leu
           515                 520                 525
   Ile Thr Met Ser Ile Asp Ser Ser Asn Pro Val Phe Leu Glu Val Ile
       530                 535                 540
   Asp Pro Ile Thr Asn Leu Gly Met Leu Gln Leu Ile Asp Leu Asn Thr
   545                 550                 555                 560
   Gly Val Arg Leu Lys Glu Ser Thr Gln Gln Gly Ile Gln Arg Tyr Gly
                   565                 570                 575
   Ile Tyr Glu Arg Glu Lys Asp Leu Val Val Ile Lys Met Asp Ser Gly
               580                 585                 590
   Lys Ala Lys Leu Gln Ile Leu Asn Lys Leu Glu Asn Leu Lys Val Ile
           595                 600                 605
   Ser Glu Ser Asn Phe Glu Ile Asn Lys Asn Ser Ser Leu Tyr Val Asp
       610                 615                 620
   Ser Lys Met Ile Leu Val Ala Val Lys Asp Ser Gly Asn Val Trp Arg
   625                 630                 635                 640
   Leu Ala Lys Phe Ser Pro Lys Asn Leu Asp Glu Phe Ile Leu Ser Glu
                   645                 650                 655
   Asn Lys Ile Leu Pro Phe Thr Ser Phe Ser Val Arg Lys Asn Phe Ile
               660                 665                 670
   Tyr Leu Gln Asp Glu Phe Lys Ser Leu Ile Thr Leu Asp Val Asn Thr
           675                 680                 685
   Leu Lys Lys Val Lys
       690
(2) INFORMATION FOR SEQ ID NO:73:
   (i) SEQUENCE CHARACTERISTICS:
      (A) LENGTH: 2107 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..2100
   (xi) SEQUENCE DESCRIPTION: SEQ ID NO:73:
   ATG AAA AAA ATG TTA CTA ATC TTT AGT TTT TTT CTT GTT TTT TTA AAT      48
   Met Lys Lys Met Leu Leu Ile Phe Ser Phe Phe Leu Val Phe Leu Asn
    1               5                  10                  15
   GGA TTT CCT CTT AAT GCA AGG GAA GTT GAT AAG GAA AAA TTA AAG GAC      96
   Gly Phe Pro Leu Asn Ala Arg Glu Val Asp Lys Glu Lys Leu Lys Asp
               20                  25                  30
   TTT GTT AAT ATG GAT CTT GAA TTT GTT AAT TAC AAG GGT CCT TAT GAT     144
   Phe Val Asn Met Asp Leu Glu Phe Val Asn Tyr Lys Gly Pro Tyr Asp
           35                  40                  45
   TCT ACA AAT ACA TAT GAA CAA ATA GTA GGT ATT GGG GAG TTT TTA GCA     192
   Ser Thr Asn Thr Tyr Glu Gln Ile Val Gly Ile Gly Glu Phe Leu Ala
       50                  55                  60
   AGG CCG TTG ATC AAT TCC AAT AGT AAT TCA AGT TAT TAT GGT AAA TAT     240
   Arg Pro Leu Ile Asn Ser Asn Ser Asn Ser Ser Tyr Tyr Gly Lys Tyr
   65                  70                  75                  80
   TTT GTT AAT AGA TTT ATT GAC GAT CAA GAT AAA AAA GCA AGT GTT GAT     288
   Phe Val Asn Arg Phe Ile Asp Asp Gln Asp Lys Lys Ala Ser Val Asp
                   85                  90                  95
   ATT TTT TCT ATT GGT AGT AAG TCA GAG CTT GAT AGT ATA TTA AAT CTA     336
   Ile Phe Ser Ile Gly Ser Lys Ser Glu Leu Asp Ser Ile Leu Asn Leu
               100                 105                 110
   AGA AGA ATT CTT ACA GGG TAT TTA ATG AAG TCT TTT GAT TAT GAG AGG     384
   Arg Arg Ile Leu Thr Gly Tyr Leu Met Lys Ser Phe Asp Tyr Glu Arg
           115                 120                 125
   TCT AGT GCG GAA TTA ATT GCT AAA GCT ATT ACA ATA TAT AAT GCT GTT     432
   Ser Ser Ala Glu Leu Ile Ala Lys Ala Ile Thr Ile Tyr Asn Ala Val
       130                 135                 140
   TAT AGA GGA GAT TTA GAT TAT TAC AAA GAG TTT TAT ATT GAG GCT TCT     480
   Tyr Arg Gly Asp Leu Asp Tyr Tyr Lys Glu Phe Tyr Ile Glu Ala Ser
   145                 150                 155                 160
   TTG AAG TCT TTG ACT AAA GAA AAT GCA GGT CTT TCT AGG GTG TAC AGT     528
   Leu Lys Ser Leu Thr Lys Glu Asn Ala Gly Leu Ser Arg Val Tyr Ser
                   165                 170                 175
   CAA TGG GCT GGG AAG ACA CAA ATA TTT ATT CCT CTT AAA AAG AAT ATT     576
   Gln Trp Ala Gly Lys Thr Gln Ile Phe Ile Pro Leu Lys Lys Asn Ile
               180                 185                 190
   TTA TCT GGA AAT GTT GAG TCT GAC ATT GAT ATT GAT AGT TTG GTT ACA     624
   Leu Ser Gly Asn Val Glu Ser Asp Ile Asp Ile Asp Ser Leu Val Thr
           195                 200                 205
   GAT AAG GTG GTG GCA GCT CTT TTA AGT GAG AAT GAA TCA GGT GTT AAC     672
   Asp Lys Val Val Ala Ala Leu Leu Ser Glu Asn Glu Ser Gly Val Asn
       210                 215                 220
   TTT GCA AGA GAT ATT ACA GAC ATT CAA GGC GAA ACT CAT AAA GCA GAT      720
   Phe Ala Arg Asp Ile Thr Asp Ile Gln Gly Glu Thr His Lys Ala Asp
   225                 230                 235                 240
   CAA GAT AAA ATT GAT ATT GAA TTA GAT AAT ATT CAT GAA AGT GAT TCC      768
   Gln Asp Lys Ile Asp Ile Glu Leu Asp Asn Ile His Glu Ser Asp Ser
                   245                 250                 255
   AAT ATA ACA GAA ACT ATT GAG AAT TTA AGG GAT CAG CTT GAA AAA GCT      816
   Asn Ile Thr Glu Thr Ile Glu Asn Leu Arg Asp Gln Leu Glu Lys Ala
               260                 265                 270
   ACA GAT GAA GAG CAT AAA AAA GAG ATT GAA AGT CAG GTT GAT GCT AAA      864
   Thr Asp Glu Glu His Lys Lys Glu Ile Glu Ser Gln Val Asp Ala Lys
           275                 280                 285
   AAG AAA CAA AAG GAA GAA TTA GAT AAA AAG GCA ATT GAT CTT GAT AAA      912
   Lys Lys Gln Lys Glu Glu Leu Asp Lys Lys Ala Ile Asp Leu Asp Lys
       290                 295                 300
   GCT CAA CAA AAA TTA GAT TTT GCT GAA GAT AAT CTA GAT ATT CAA AGG      960
   Ala Gln Gln Lys Leu Asp Phe Ala Glu Asp Asn Leu Asp Ile Gln Arg
   305                 310                 315                 320
   GAT ACT GTT AGA GAG AAG CTT CAA GAG AAT ATT AAC GAG ACT AAT AAG     1008
   Asp Thr Val Arg Glu Lys Leu Gln Glu Asn Ile Asn Glu Thr Asn Lys
                   325                 330                 335
   GAA AAG AAT TTA CCA AAG CCT GGT GAT GTA AGT TCT CCT AAA GTT GAT     1056
   Glu Lys Asn Leu Pro Lys Pro Gly Asp Val Ser Ser Pro Lys Val Asp
               340                 345                 350
   AAG CAA CTA CAA ATA AAA GAG AGC CTG GAA GAT TTG CAG GAG CAG CTT     1104
   Lys Gln Leu Gln Ile Lys Glu Ser Leu Glu Asp Leu Gln Glu Gln Leu
           355                 360                 365
   AAA GAA ACT GGT GAT GAA AAT CAG AAA AGA GAA ATT GAA AAG CAA ATT     1152
   Lys Glu Thr Gly Asp Glu Asn Gln Lys Arg Glu Ile Glu Lys Gln Ile
       370                 375                 380
   GAA ATC AAA AAA AGT GAT GAA AAG CTT TTA AAA AGT AAA GAT GAT AAA     1200
   Glu Ile Lys Lys Ser Asp Glu Lys Leu Leu Lys Ser Lys Asp Asp Lys
   385                 390                 395                 400
   GCA AGT AAA GAT GGT AAA GCC TTG GAT CTT GAT CGA GAA TTA AAT TCT     1248
   Ala Ser Lys Asp Gly Lys Ala Leu Asp Leu Asp Arg Glu Leu Asn Ser
                   405                 410                 415
   AAA GCT TCT AGC AAA GAA AAA AGT AAA GCC AAG GAA GAA GAA ATA ACC     1296
   Lys Ala Ser Ser Lys Glu Lys Ser Lys Ala Lys Glu Glu Glu Ile Thr
               420                 425                 430
   AAG GGT AAG TCA CAG AAA AGC TTA GGC GAT TTG AAT AAT GAT GAA AAT     1344
   Lys Gly Lys Ser Gln Lys Ser Leu Gly Asp Leu Asn Asn Asp Glu Asn
           435                 440                 445
   CTT ATG ATG CCA GAA GAT CAA AAA TTA CCT GAG GTT AAA AAA TTA GAT     1392
   Leu Met Met Pro Glu Asp Gln Lys Leu Pro Glu Val Lys Lys Leu Asp
       450                 455                 460
   AGC AAA AAA GAA TTT AAA CCT GTT TCT GAG GTT GAG AAA TTA GAT AAG      1440
   Ser Lys Lys Glu Phe Lys Pro Val Ser Glu Val Glu Lys Leu Asp Lys
   465                 470                 475                 480
   ATT TTC AAG TCT AAT AAC AAT GTT GGA GAA TTA TCA CCG TTA GAT AAA      1488
   Ile Phe Lys Ser Asn Asn Asn Val Gly Glu Leu Ser Pro Leu Asp Lys
                   485                 490                 495
   TCT TCT TAT AAA GAC ATT GAT TCA AAA GAG GAG ACA GTT AAT AAA GAT      1536
   Ser Ser Tyr Lys Asp Ile Asp Ser Lys Glu Glu Thr Val Asn Lys Asp
               500                 505                 510
   GTT AAT TTG CAA AAG ACT AAG CCT CAG GTT AAA GAC CAA GTT ACT TCT      1584
   Val Asn Leu Gln Lys Thr Lys Pro Gln Val Lys Asp Gln Val Thr Ser
           515                 520                 525
   TTG AAT GAA GAT TTG ACT ACT ATG TCT ATA GAT TCC AGT AGT CCT GTA      1632
   Leu Asn Glu Asp Leu Thr Thr Met Ser Ile Asp Ser Ser Ser Pro Val
       530                 535                 540
   TTT TTA GAG GTT ATT GAT CCA ATT ACA AAT TTA GGA ACT CTT CAA CTT      1680
   Phe Leu Glu Val Ile Asp Pro Ile Thr Asn Leu Gly Thr Leu Gln Leu
   545                 550                 555                 560
   ATT GAT TTA AAT ACT GGT GTT AGG CTT AAA GAA AGC ACT CAG CAA GGC      1728
   Ile Asp Leu Asn Thr Gly Val Arg Leu Lys Glu Ser Thr Gln Gln Gly
                   565                 570                 575
   ATT CAG CGG TAT GGA ATT TAT GAA CGT GAA AAA GAT TTG GTT GTT ATT      1776
   Ile Gln Arg Tyr Gly Ile Tyr Glu Arg Glu Lys Asp Leu Val Val Ile
               580                 585                 590
   AAA ATG GAT TCA GGA AAA GCT AAG CTT CAG ATA CTT GAT AAA CTT GAA      1824
   Lys Met Asp Ser Gly Lys Ala Lys Leu Gln Ile Leu Asp Lys Leu Glu
           595                 600                 605
   AAT TTA AAA GTG GTA TCA GAG TCT AAT TTT GAG ATT AAT AAA AAT TCA      1872
   Asn Leu Lys Val Val Ser Glu Ser Asn Phe Glu Ile Asn Lys Asn Ser
       610                 615                 620
   TCT CTT TAT GTT GAT TCT AAA ATG ATT TTA GTA GCT GTT AGG GAT AAA      1920
   Ser Leu Tyr Val Asp Ser Lys Met Ile Leu Val Ala Val Arg Asp Lys
   625                 630                 635                 640
   GAT AGT AGT AAT GAT TGG AGA TTG GCC AAA TTT TCT CCT AAA AAT TTA      1968
   Asp Ser Ser Asn Asp Trp Arg Leu Ala Lys Phe Ser Pro Lys Asn Leu
                   645                 650                 655
   GAT GAG TTT ATT CTT TCA GAG AAT AAA ATT ATG CCT TTT ACT AGC TTT      2016
   Asp Glu Phe Ile Leu Ser Glu Asn Lys Ile Met Pro Phe Thr Ser Phe
               660                 665                 670
   TCT GTG AGA AAA AAT TTT ATT TAT TTG CAA GAT GAG TTT AAA AGT CTA      2064
   Ser Val Arg Lys Asn Phe Ile Tyr Leu Gln Asp Glu Phe Lys Ser Leu
           675                 680                 685
   GTT ATT TTA GAT GTA AAT ACT TTA AAA AAA GTT AAG TAAAGCC              2107
   Val Ile Leu Asp Val Asn Thr Leu Lys Lys Val Lys
       690                 695                 700
(2) INFORMATION FOR SEQ ID NO:74:
   (i) SEQUENCE CHARACTERISTICS:
      (A) LENGTH: 700 amino acids
      (B) TYPE: amino acid
      (D) TOPOLOGY: linear
   (ii) MOLECULE TYPE: protein
   (xi) SEQUENCE DESCRIPTION: SEQ ID NO:74:
   Met Lys Lys Met Leu Leu Ile Phe Ser Phe Phe Leu Val Phe Leu Asn
    1               5                  10                  15
   Gly Phe Pro Leu Asn Ala Arg Glu Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Ile Asn Ser Asn Ser Asn Ser Ser Tyr Tyr Gly Lys Tyr
   65                  70                  75                  80
   Phe Val Asn Arg Phe Ile Asp Asp Gln Asp Lys Lys Ala Ser Val Asp
                   85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Ile Glu Leu Asp Asn Ile His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Gln Lys Glu Glu Leu Asp Lys Lys Ala Ile Asp Leu Asp Lys
       290                 295                 300
   Ala Gln Gln Lys Leu Asp Phe Ala Glu Asp Asn Leu Asp Ile Gln Arg
   305                 310                 315                 320
   Asp Thr Val Arg Glu Lys Leu Gln Glu Asn Ile Asn Glu Thr Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Thr Gly Asp Glu Asn Gln Lys Arg Glu Ile Glu Lys Gln Ile
       370                 375                 380
   Glu Ile Lys Lys Ser Asp Glu Lys Leu Leu Lys Ser Lys Asp Asp Lys
   385                 390                 395                 400
   Ala Ser Lys Asp Gly Lys Ala Leu Asp Leu Asp Arg Glu Leu Asn Ser
                   405                 410                 415
   Lys Ala Ser Ser Lys Glu Lys Ser Lys Ala Lys Glu Glu Glu Ile Thr
               420                 425                 430
   Lys Gly Lys Ser Gln Lys Ser Leu Gly Asp Leu Asn Asn Asp Glu Asn
           435                 440                 445
   Leu Met Met Pro Glu Asp Gln Lys Leu Pro Glu Val Lys Lys Leu Asp
       450                 455                 460
   Ser Lys Lys Glu Phe Lys Pro Val Ser Glu Val Glu Lys Leu Asp Lys
   465                 470                 475                 480
   Ile Phe Lys Ser Asn Asn Asn Val Gly Glu Leu Ser Pro Leu Asp Lys
                   485                 490                 495
   Ser Ser Tyr Lys Asp Ile Asp Ser Lys Glu Glu Thr Val Asn Lys Asp
               500                 505                 510
   Val Asn Leu Gln Lys Thr Lys Pro Gln Val Lys Asp Gln Val Thr Ser
           515                 520                 525
   Leu Asn Glu Asp Leu Thr Thr Met Ser Ile Asp Ser Ser Ser Pro Val
       530                 535                 540
   Phe Leu Glu Val Ile Asp Pro Ile Thr Asn Leu Gly Thr Leu Gln Leu
   545                 550                 555                 560
   Ile Asp Leu Asn Thr Gly Val Arg Leu Lys Glu Ser Thr Gln Gln Gly
                   565                 570                 575
   Ile Gln Arg Tyr Gly Ile Tyr Glu Arg Glu Lys Asp Leu Val Val Ile
               580                 585                 590
   Lys Met Asp Ser Gly Lys Ala Lys Leu Gln Ile Leu Asp Lys Leu Glu
           595                 600                 605
   Asn Leu Lys Val Val Ser Glu Ser Asn Phe Glu Ile Asn Lys Asn Ser
       610                 615                 620
   Ser Leu Tyr Val Asp Ser Lys Met Ile Leu Val Ala Val Arg Asp Lys
   625                 630                 635                 640
   Asp Ser Ser Asn Asp Trp Arg Leu Ala Lys Phe Ser Pro Lys Asn Leu
                   645                 650                 655
   Asp Glu Phe Ile Leu Ser Glu Asn Lys Ile Met Pro Phe Thr Ser Phe
               660                 665                 670
   Ser Val Arg Lys Asn Phe Ile Tyr Leu Gln Asp Glu Phe Lys Ser Leu
           675                 680                 685
   Val Ile Leu Asp Val Asn Thr Leu Lys Lys Val Lys
       690                 695                 700
(2) INFORMATION FOR SEQ ID NO:75:
   (i) SEQUENCE CHARACTERISTICS:
      (A) LENGTH: 2126 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..2124
   (xi) SEQUENCE DESCRIPTION: SEQ ID NO:75:
   ATG AAA AAA ATG TTA CTA ATC TTT AGT TTT TTT CTT ATT TTT TTG AAT       48
   Met Lys Lys Met Leu Leu Ile Phe Ser Phe Phe Leu Ile Phe Leu Asn
     1               5                  10                  15
   GGA TTT CCT CTT AAT GCA AGG AAA GTT GAT AAG GAA AAA TTA AAG GAT       96
   Gly Phe Pro Leu Asn Ala Arg Lys Val Asp Lys Glu Lys Leu Lys Asp
               20                  25                  30
   TTT GTT AAT ATG GAT CTT GAG TTT GTA AAT TAT AAA GGT CCT TAT GAT      144
   Phe Val Asn Met Asp Leu Glu Phe Val Asn Tyr Lys Gly Pro Tyr Asp
           35                  40                  45
   TCT ACA AAT ACG TAT GAA CAA ATA GTG GGT ATT GGG GAG TTT TTA GCA      192
   Ser Thr Asn Thr Tyr Glu Gln Ile Val Gly Ile Gly Glu Phe Leu Ala
       50                  55                  60
   AGA CCG CTG ACC AAT TCC AAT AGC AAC TCA AGT TAT TAT GGC AAA TAT      240
   Arg Pro Leu Thr Asn Ser Asn Ser Asn Ser Ser Tyr Tyr Gly Lys Tyr
   65                  70                  75                  80
   TTT ATT AAT AGA TTT ATT GAT GAT CAA GAT AAA AAA GCA AGT GTT GAT      288
   Phe Ile Asn Arg Phe Ile Asp Asp Gln Asp Lys Lys Ala Ser Val Asp
                   85                  90                  95
   GTT TTT TCT ATA AGC AGC AAA TCA GAG CTT GAC AGT ATA TTG AAT TTA      336
   Val Phe Ser Ile Ser Ser Lys Ser Glu Leu Asp Ser Ile Leu Asn Leu
               100                 105                 110
   AGA AGA ATT CTT ACA GGG TAT ATA ATA AAG TCT TTC GAT TAT GAC AGG      384
   Arg Arg Ile Leu Thr Gly Tyr Ile Ile Lys Ser Phe Asp Tyr Asp Arg
           115                 120                 125
   TCT AGT GCA GAA TTA ATT GCT AAG GTT ATT ACA ATA TAT AAT GCT GTT      432
   Ser Ser Ala Glu Leu Ile Ala Lys Val Ile Thr Ile Tyr Asn Ala Val
       130                 135                 140
   TAT AGA GGA GAT TTG GAT TAT TAT AAA GGG TTT TAT ATT GAG CCT GCT      480
   Tyr Arg Gly Asp Leu Asp Tyr Tyr Lys Gly Phe Tyr Ile Glu Pro Ala
   145                 150                 155                 160
   TTG AAG TCT TTA ACT AAA GAA AAC GCA GGT CTT TCT AGG GTT TAC AGT      528
   Leu Lys Ser Leu Thr Lys Glu Asn Ala Gly Leu Ser Arg Val Tyr Ser
                   165                 170                 175
   CAG TGG GCT GGA AAG ACT CAA ATA TTT ATT CCT CTT AAA AAG GAT ATT      576
   Gln Trp Ala Gly Lys Thr Gln Ile Phe Ile Pro Leu Lys Lys Asp Ile
               180                 185                 190
   TTG TCT GGA AAT ATT GAA TCT GAC ATT GAT ATT GAC AGT TTG GTT ACA      624
   Leu Ser Gly Asn Ile Glu Ser Asp Ile Asp Ile Asp Ser Leu Val Thr
           195                 200                 205
   GAT AAG GTG ATA GCA GCT CTT TTA AGC GAA AAT GAA GCA GGC GTT AAC      672
   Asp Lys Val Ile Ala Ala Leu Leu Ser Glu Asn Glu Ala Gly Val Asn
       210                 215                 220
   TTT GCA AGA GAT ATT ACA GAT ATT CAA GGC GAA ACT CAT AAG GCA GAT      720
   Phe Ala Arg Asp Ile Thr Asp Ile Gln Gly Glu Thr His Lys Ala Asp
   225                 230                 235                 240
   CAA GAT AAG ATT GAT ACT GAA TTA GAC AAT ATC CAT GAA AGC GAT TCT      768
   Gln Asp Lys Ile Asp Thr Glu Leu Asp Asn Ile His Glu Ser Asp Ser
                   245                 250                 255
   AAT ATA ACA GAA ACT ATT GAA AAT TTA AGG GAT CAG CTT GAA AAA GCT      816
   Asn Ile Thr Glu Thr Ile Glu Asn Leu Arg Asp Gln Leu Glu Lys Ala
               260                 265                 270
   ACA GAT GAA GAG CAT AAA AAA GAG ATT GAA AGT CAG GTT GAT GCT AAA      864
   Thr Asp Glu Glu His Lys Lys Glu Ile Glu Ser Gln Val Asp Ala Lys
           275                 280                 285
   AAG AAA GAA AAG GAA GAG CTA GAT AAA AAG GCA ATC AAT CTT GAT AAA      912
   Lys Lys Glu Lys Glu Glu Leu Asp Lys Lys Ala Ile Asn Leu Asp Lys
       290                 295                 300
   GCT CAG CAA AAA TTA GAC TCT GCT GAA GAT AAT TTA GAT GTT CAA AGA      960
   Ala Gln Gln Lys Leu Asp Ser Ala Glu Asp Asn Leu Asp Val Gln Arg
   305                 310                 315                 320
   GAT ACT GTT AGA GAG AAA ATT CAA GAG GAT ATT AAT GAG ATT AAT AAG     1008
   Asp Thr Val Arg Glu Lys Ile Gln Glu Asp Ile Asn Glu Ile Asn Lys
                   325                 330                 335
   GAA AAG AAT TTG CCA AAA CCT GGT GAT GTA AGT TCT CCT AAA GTT GAT      1056
   Glu Lys Asn Leu Pro Lys Pro Gly Asp Val Ser Ser Pro Lys Val Asp
               340                 345                 350
   AAG CAA CTG CAA ATA AAA GAG AGT CTA GAA GAT TTG CAG GAG CAG CTT      1104
   Lys Gln Leu Gln Ile Lys Glu Ser Leu Glu Asp Leu Gln Glu Gln Leu
           355                 360                 365
   AAA GAA GCT GGT GAT GAA AAT CAG AAA AGA GAA ATT GAG AAG CAA ATT      1152
   Lys Glu Ala Gly Asp Glu Asn Gln Lys Arg Glu Ile Glu Lys Gln Ile
       370                 375                 380
   GAA ATC AAA AAA AGG GAC GAA GAA CTT TTA AAA AGT AAA GAT GGC AAA      1200
   Glu Ile Lys Lys Arg Asp Glu Glu Leu Leu Lys Ser Lys Asp Gly Lys
   385                 390                 395                 400
   GTA AGT AAA GAT TAT GAA GCA TTA GAT CTT GAT CGA GAA TTA TCC AAA      1248
   Val Ser Lys Asp Tyr Glu Ala Leu Asp Leu Asp Arg Glu Leu Ser Lys
                   405                 410                 415
   GCT TCT AGT AAA GAA AAA AGT AAG GTC AAG GAA GAA GAA ATA ACT AAA      1296
   Ala Ser Ser Lys Glu Lys Ser Lys Val Lys Glu Glu Glu Ile Thr Lys
               420                 425                 430
   GGT AAA TCA CGG GCA AGC TTA GGC GAT TTG AAT AAT GAT AAA AAC CTT      1344
   Gly Lys Ser Arg Ala Ser Leu Gly Asp Leu Asn Asn Asp Lys Asn Leu
           435                 440                 445
   ATG TTG CCA GAA GAT CAA AAA TTA CCT GAA GAT AAA AAA TTG GAT AGT      1392
   Met Leu Pro Glu Asp Gln Lys Leu Pro Glu Asp Lys Lys Leu Asp Ser
       450                 455                 460
   AAA TTA GAT GGT AAA AAA GAA TTT AAA CCA GTT TCT GAG GTT GAA AAA      1440
   Lys Leu Asp Gly Lys Lys Glu Phe Lys Pro Val Ser Glu Val Glu Lys
   465                 470                 475                 480
   TTA GAT AAG ATT TCC AAG TCT AAT AAC AAT GAG GTT GGC AAG TTA TCA      1488
   Leu Asp Lys Ile Ser Lys Ser Asn Asn Asn Glu Val Gly Lys Leu Ser
                   485                 490                 495
   CCA TTA GAT AAG CCT TCT TAT GAT GAT ATT GAT TCA AAA GAG GAG GTA      1536
   Pro Leu Asp Lys Pro Ser Tyr Asp Asp Ile Asp Ser Lys Glu Glu Val
               500                 505                 510
   GAT AAT AAA GCT ATT AAT TTG CAA AAG ATC GAC CCT AAA GTT AAA GAC      1584
   Asp Asn Lys Ala Ile Asn Leu Gln Lys Ile Asp Pro Lys Val Lys Asp
           515                 520                 525
   CAA ACT ACT TCT TTG AAT GAA GAT TTG GAT AAA GAT TTG ACT ACT ATG      1632
   Gln Thr Thr Ser Leu Asn Glu Asp Leu Asp Lys Asp Leu Thr Thr Met
       530                 535                 540
   TCT ATA GAT TCC AGC AGT CCT GTA TTT CTA GAG GTT ATT GAT CCT ATT      1680
   Ser Ile Asp Ser Ser Ser Pro Val Phe Leu Glu Val Ile Asp Pro Ile
   545                 550                 555                 560
   ACA AAT TTA GGA ACC CTG CAG CTT ATT GAT TTA AAT ACT GGG GTT AGG      1728
   Thr Asn Leu Gly Thr Leu Gln Leu Ile Asp Leu Asn Thr Gly Val Arg
                   565                 570                 575
   CTT AAG GAA AGC ACT CAG CAA GGC ATT CAG CGG TAT GGA ATT TAT GAA        1776
   Leu Lys Glu Ser Thr Gln Gln Gly Ile Gln Arg Tyr Gly Ile Tyr Glu
               580                 585                 590
   CGT GAA AAA GAT TTG GTT GTT ATT AAA ATG GAT TCA GGA AAG GCT AAG        1824
   Arg Glu Lys Asp Leu Val Val Ile Lys Met Asp Ser Gly Lys Ala Lys
           595                 600                 605
   CTT CAA ATA CTT AAT AAG CTT GAA AAT TTG AAA GTG GTA TCA GAG TCT        1872
   Leu Gln Ile Leu Asn Lys Leu Glu Asn Leu Lys Val Val Ser Glu Ser
       610                 615                 620
   AAT TTT GAG ATC AAT AAA AAT TCA TCT CTT TAT GTT GAC TCT AAA ATG        1920
   Asn Phe Glu Ile Asn Lys Asn Ser Ser Leu Tyr Val Asp Ser Lys Met
   625                 630                 635                 640
   ATT TTG GCA GCT GTT AGA GAT AAG GAT GAT AGC AAT GCT TGG AGA TTG        1968
   Ile Leu Ala Ala Val Arg Asp Lys Asp Asp Ser Asn Ala Trp Arg Leu
                   645                 650                 655
   GCT AAA TTT TCT CCT AAA AAT TTG GAT GAG TTT ATT CTT TCA GAG AAT        2016
   Ala Lys Phe Ser Pro Lys Asn Leu Asp Glu Phe Ile Leu Ser Glu Asn
               660                 665                 670
   AAA ATT TTG CCT TTT ACT AGC TTT TCT GTG AGA AAA AAT TTT ATT TAT        2064
   Lys Ile Leu Pro Phe Thr Ser Phe Ser Val Arg Lys Asn Phe Ile Tyr
           675                 680                 685
   TTG CAA GAT GAG CTT AAA AAT CTA GTT ATT TTA GAT GTA AAT ACT TTA        2112
   Leu Gln Asp Glu Leu Lys Asn Leu Val Ile Leu Asp Val Asn Thr Leu
       690                 695                 700
   AAA AAA GTT AAG TA                                                     2126
   Lys Lys Val Lys
   705
(2) INFORMATION FOR SEQ ID NO:76:
   (i) SEQUENCE CHARACTERISTICS:
      (A) LENGTH: 708 amino acids
      (B) TYPE: amino acid
      (D) TOPOLOGY: linear
   (ii) MOLECULE TYPE: protein
   (xi) SEQUENCE DESCRIPTION: SEQ ID NO:76:
   Met Lys Lys Met Leu Leu Ile Phe Ser Phe Phe Leu Ile Phe Leu Asn
   1               5                   10                  15
   Gly Phe Pro Leu Asn Ala Arg Lys Val Asp Lys Glu Lys Leu Lys Asp
               20                  25                  30
   Phe Val Asn Met Asp Leu Glu Phe Val Asn Tyr Lys Gly Pro Tyr Asp
           35                  40                  45
   Ser Thr Asn Thr Tyr Glu Gln Ile Val Gly Ile Gly Glu Phe Leu Ala
       50                  55                  60
   Arg Pro Leu Thr Asn Ser Asn Ser Asn Ser Ser Tyr Tyr Gly Lys Tyr
    65                  70                  75                  80
   Phe Ile Asn Arg Phe Ile Asp Asp Gln Asp Lys Lys Ala Ser Val Asp
                    85                  90                  95
   Val Phe Ser Ile Ser Ser Lys Ser Glu Leu Asp Ser Ile Leu Asn Leu
               100                 105                 110
   Arg Arg Ile Leu Thr Gly Tyr Ile Ile Lys Ser Phe Asp Tyr Asp Arg
           115                 120                 125
   Ser Ser Ala Glu Leu Ile Ala Lys Val Ile Thr Ile Tyr Asn Ala Val
       130                 135                 140
   Tyr Arg Gly Asp Leu Asp Tyr Tyr Lys Gly Phe Tyr Ile Glu Pro Ala
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asp Ile
               180                 185                 190
   Leu Ser Gly Asn Ile Glu Ser Asp Ile Asp Ile Asp Ser Leu Val Thr
           195                 200                 205
   Asp Lys Val Ile Ala Ala Leu Leu Ser Glu Asn Glu Ala Gly Val Asn
       210                 215                 220
   Phe Ala Arg Asp Ile Thr Asp Ile Gln Gly Glu Thr His Lys Ala Asp
   225                 230                 235                 240
   Gln Asp Lys Ile Asp Thr Glu Leu Asp Asn Ile His Glu Ser Asp Ser
                   245                 250                 255
   Asn Ile Thr Glu Thr Ile Glu Asn Leu Arg Asp Gln Leu Glu Lys Ala
               260                 265                 270
   Thr Asp Glu Glu His Lys Lys Glu Ile Glu Ser Gln Val Asp Ala Lys
           275                 280                 285
   Lys Lys Glu Lys Glu Glu Leu Asp Lys Lys Ala Ile Asn Leu Asp Lys
       290                 295                 300
   Ala Gln Gln Lys Leu Asp Ser Ala Glu Asp Asn Leu Asp Val Gln Arg
   305                 310                 315                 320
   Asp Thr Val Arg Glu Lys Ile Gln Glu Asp Ile Asn Glu Ile Asn Lys
                   325                 330                 335
   Glu Lys Asn Leu Pro Lys Pro Gly Asp Val Ser Ser Pro Lys Val Asp
               340                 345                 350
   Lys Gln Leu Gln Ile Lys Glu Ser Leu Glu Asp Leu Gln Glu Gln Leu
           355                 360                 365
   Lys Glu Ala Gly Asp Glu Asn Gln Lys Arg Glu Ile Glu Lys Gln Ile
       370                 375                 380
   Glu Ile Lys Lys Arg Asp Glu Glu Leu Leu Lys Ser Lys Asp Gly Lys
   385                 390                 395                 400
   Val Ser Lys Asp Tyr Glu Ala Leu Asp Leu Asp Arg Glu Leu Ser Lys
                   405                 410                 415
   Ala Ser Ser Lys Glu Lys Ser Lys Val Lys Glu Glu Glu Ile Thr Lys
               420                 425                 430
   Gly Lys Ser Arg Ala Ser Leu Gly Asp Leu Asn Asn Asp Lys Asn Leu
           435                 440                 445
   Met Leu Pro Glu Asp Gln Lys Leu Pro Glu Asp Lys Lys Leu Asp Ser
       450                 455                 460
   Lys Leu Asp Gly Lys Lys Glu Phe Lys Pro Val Ser Glu Val Glu Lys
   465                 470                 475                 480
   Leu Asp Lys Ile Ser Lys Ser Asn Asn Asn Glu Val Gly Lys Leu Ser
                   485                 490                 495
   Pro Leu Asp Lys Pro Ser Tyr Asp Asp Ile Asp Ser Lys Glu Glu Val
               500                 505                 510
   Asp Asn Lys Ala Ile Asn Leu Gln Lys Ile Asp Pro Lys Val Lys Asp
           515                 520                 525
   Gln Thr Thr Ser Leu Asn Glu Asp Leu Asp Lys Asp Leu Thr Thr Met
       530                 535                 540
   Ser Ile Asp Ser Ser Ser Pro Val Phe Leu Glu Val Ile Asp Pro Ile
   545                 550                 555                 560
   Thr Asn Leu Gly Thr Leu Gln Leu Ile Asp Leu Asn Thr Gly Val Arg
                   565                 570                 575
   Leu Lys Glu Ser Thr Gln Gln Gly Ile Gln Arg Tyr Gly Ile Tyr Glu
               580                 585                 590
   Arg Glu Lys Asp Leu Val Val Ile Lys Met Asp Ser Gly Lys Ala Lys
           595                 600                 605
   Leu Gln Ile Leu Asn Lys Leu Glu Asn Leu Lys Val Val Ser Glu Ser
       610                 615                 620
   Asn Phe Glu Ile Asn Lys Asn Ser Ser Leu Tyr Val Asp Ser Lys Met
   625                 630                 635                 640
   Ile Leu Ala Ala Val Arg Asp Lys Asp Asp Ser Asn Ala Trp Arg Leu
                   645                 650                 655
   Ala Lys Phe Ser Pro Lys Asn Leu Asp Glu Phe Ile Leu Ser Glu Asn
               660                 665                 670
   Lys Ile Leu Pro Phe Thr Ser Phe Ser Val Arg Lys Asn Phe Ile Tyr
           675                 680                 685
   Leu Gln Asp Glu Leu Lys Asn Leu Val Ile Leu Asp Val Asn Thr Leu
       690                 695                 700
   Lys Lys Val Lys
   705
(2) INFORMATION FOR SEQ ID NO:77:
   (i) SEQUENCE CHARACTERISTICS:
      (A) LENGTH: 1991 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..1989
   (xi) SEQUENCE DESCRIPTION: SEQ ID NO:77:
   ATG AAA AAA ATG TTA CTA ATC TTT AGT TTT TTT CTT GTT TTT TTA AAT       48
   Met Lys Lys Met Leu Leu Ile Phe Ser Phe Phe Leu Val Phe Leu Asn
     1               5                  10                  15
   GGA TTT CCT CTT AAT GCA AGG GAA GTT GAT AAG GAA AAA TTA AAG GAC       96
   Gly Phe Pro Leu Asn Ala Arg Glu Val Asp Lys Glu Lys Leu Lys Asp
                20                  25                  30
   TTT GTT AAT ATG GAT CTT GAA TTT GTT AAT TAC AAG GGT CCT TAT GAT      144
   Phe Val Asn Met Asp Leu Glu Phe Val Asn Tyr Lys Gly Pro Tyr Asp
            35                  40                  45
   TCT ACA AAT ACA TAT GAA CAA ATA GTA GGT ATT GGG GAG TTT TTA GCA      192
   Ser Thr Asn Thr Tyr Glu Gln Ile Val Gly Ile Gly Glu Phe Leu Ala
        50                  55                  60
   AGG CCG TTG ATC AAT TCC AAT AGT AAT TCA AGT TAT TAT GGT AAA TAT      240
   Arg Pro Leu Ile Asn Ser Asn Ser Asn Ser Ser Tyr Tyr Gly Lys Tyr
    65                  70                  75                  80
   TTT GTT AAT AGA TTT ATT GAC GAT CAA GAT AAA AAA GCA AGT GTT GAT      288
   Phe Val Asn Arg Phe Ile Asp Asp Gln Asp Lys Lys Ala Ser Val Asp
                    85                  90                  95
   ATT TTT TCT ATT GGT AGT AAG TCA GAG CTT GAT AGT ATA TTA AAT CTA      336
   Ile Phe Ser Ile Gly Ser Lys Ser Glu Leu Asp Ser Ile Leu Asn Leu
               100                 105                 110
   AGA AGA ATT CTT ACA GGG TAT TTA ATG AAG TCT TTT GAT TAT GAG AGG      384
   Arg Arg Ile Leu Thr Gly Tyr Leu Met Lys Ser Phe Asp Tyr Glu Arg
           115                 120                 125
   TCT AGT GCG GAA TTA ATT GCT AAA GCT ATT ACA ATA TAT AAT GCT GTT      432
   Ser Ser Ala Glu Leu Ile Ala Lys Ala Ile Thr Ile Tyr Asn Ala Val
       130                 135                 140
   TAT AGA GGA GAT TTA GAT TAT TAC AAA GAG TTT TAT ATT GAG GCT TCT      480
   Tyr Arg Gly Asp Leu Asp Tyr Tyr Lys Glu Phe Tyr Ile Glu Ala Ser
   145                 150                 155                 160
   TTG AAG TCT TTG ACT AAA GAA AAT GCA GGT CTT TCT AGG GTG TAC AGT        528
   Leu Lys Ser Leu Thr Lys Glu Asn Ala Gly Leu Ser Arg Val Tyr Ser
               165                 170                 175
   CAA TGG GCT GGG AAG ACA CAA ATA TTT ATT CCT CTT AAA AAG AAT ATT        576
   Gln Trp Ala Gly Lys Thr Gln Ile Phe Ile Pro Leu Lys Lys Asn Ile
           180                 185                 190
   TTA TCT GGA AAT GTT GAG TCT GAC ATT GAT ATT GAT AGT TTG GTT ACA        624
   Leu Ser Gly Asn Val Glu Ser Asp Ile Asp Ile Asp Ser Leu Val Thr
       195                 200                 205
   GAT AAG GTG GTG GCA GCT CTT TTA AGT GAG AAT GAA TCA GGT GTT AAC        672
   Asp Lys Val Val Ala Ala Leu Leu Ser Glu Asn Glu Ser Gly Val Asn
       210                 215                 220
   TTT GCA AGA GAT ATT ACA GAC ATT CAA GGC GAA ACT CAT AAA GCA GAT        720
   Phe Ala Arg Asp Ile Thr Asp Ile Gln Gly Glu Thr His Lys Ala Asp
   225                 230                 235                 240
   CAA GAT AAA ATT GAT ATT GAA TTA GAT AAT TTT CAT GAA AGT GAT TCC        768
   Gln Asp Lys Ile Asp Ile Glu Leu Asp Asn Phe His Glu Ser Asp Ser
               245                 250                 255
   AAT ATA ACA GAA ACT ATT GAG AAT TTA AGG GAT CAG CTT GAA AAA GCT        816
   Asn Ile Thr Glu Thr Ile Glu Asn Leu Arg Asp Gln Leu Glu Lys Ala
           260                 265                 270
   ACA GAT GAA GAG CAT AAA AAA GAG ATT GAA AGT CAG GTT GAT GCT AAA        864
   Thr Asp Glu Glu His Lys Lys Glu Ile Glu Ser Gln Val Asp Ala Lys
       275                 280                 285
   AAG AAA CAA AAG GAA GAA TTA GAT AAA AAG GCA ATT GAT CTT GAT AAA        912
   Lys Lys Gln Lys Glu Glu Leu Asp Lys Lys Ala Ile Asp Leu Asp Lys
       290                 295                 300
   GCT CAA CAA AAA TTA GAT TTT GCT GAA GAT AAT CTA GAT ATT CAA AGG        960
   Ala Gln Gln Lys Leu Asp Phe Ala Glu Asp Asn Leu Asp Ile Gln Arg
   305                 310                 315                 320
   GAT ACT GTT AGA GAG AAG CTT CAA GAA AAT ATT AAC GAG ACT AAT AAG       1008
   Asp Thr Val Arg Glu Lys Leu Gln Glu Asn Ile Asn Glu Thr Asn Lys
               325                 330                 335
   GAA AAG AAT TTA CCA AAG CCT GGT GAT GTA AGT TCT CCT AAG GTT GAT       1056
   Glu Lys Asn Leu Pro Lys Pro Gly Asp Val Ser Ser Pro Lys Val Asp
           340                 345                 350
   AAG CAG TTG CAG ATA AAA GAG AGT CTA GAA GAT TTG CAA GAG CAG CTT       1104
   Lys Gln Leu Gln Ile Lys Glu Ser Leu Glu Asp Leu Gln Glu Gln Leu
       355                 360                 365
   AAA GAA GCT AGT GAT GAA AAT CAA AAA AGA GAA ATA GAA AAG CAA ATT       1152
   Lys Glu Ala Ser Asp Glu Asn Gln Lys Arg Glu Ile Glu Lys Gln Ile
       370                 375                 380
   GAA ATC AAA AAA AAT GAT GAA GAA CTT TTT AAA AAT AAA GAT CAT AAA       1200
   Glu Ile Lys Lys Asn Asp Glu Glu Leu Phe Lys Asn Lys Asp His Lys
   385                 390                 395                 400
   GCA TTA GAT CTT AAG CAA GAA TTA AAT TCT AAA GCT TCT AGT AAA GAA     1248
   Ala Leu Asp Leu Lys Gln Glu Leu Asn Ser Lys Ala Ser Ser Lys Glu
               405                 410                 415
   AAA ATT GAA GGC GAA GAA GAG GAT AAA GAA TTA GAT AGT AAA AAA AAT     1296
   Lys Ile Glu Gly Glu Glu Glu Asp Lys Glu Leu Asp Ser Lys Lys Asn
           420                 425                 430
   TTA GAG CCT GTT TCT GAG GCT GAT AAA GTA GAT AAA ATT TCC AAG TCT     1344
   Leu Glu Pro Val Ser Glu Ala Asp Lys Val Asp Lys Ile Ser Lys Ser
       435                 440                 445
   AAC AAC AAT GAG GTT AGT AAA TTA TCC CCG TTA GAT GAG CCT TCT TAT     1392
   Asn Asn Asn Glu Val Ser Lys Leu Ser Pro Leu Asp Glu Pro Ser Tyr
       450                 455                 460
   AGC GAC ATT GAT TCG AAA GAG GGT GTA GAT AAC AAA GAT GTT GAT TTG     1440
   Ser Asp Ile Asp Ser Lys Glu Gly Val Asp Asn Lys Asp Val Asp Leu
   465                 470                 475                 480
   CAA AAA ACT AAA CCC CAA GTT GAA AGT CAA CCT ACT TCG TTA AAT GAA     1488
   Gln Lys Thr Lys Pro Gln Val Glu Ser Gln Pro Thr Ser Leu Asn Glu
               485                 490                 495
   GAC TTG ATT GAT GTG TCT ATA GAT TCC AGT AAT CCT GTC TTT TTA GAG     1536
   Asp Leu Ile Asp Val Ser Ile Asp Ser Ser Asn Pro Val Phe Leu Glu
           500                 505                 510
   GTT ATC GAT CCG ATT ACA AAT TTA GGA ACG CTT CAA CTT ATT GAT TTG     1584
   Val Ile Asp Pro Ile Thr Asn Leu Gly Thr Leu Gln Leu Ile Asp Leu
       515                 520                 525
   AAT ACC GGT GTT AGA CTT AAA GAA AGT GCT CAA CAA GGT ATT CAG CGA     1632
   Asn Thr Gly Val Arg Leu Lys Glu Ser Ala Gln Gln Gly Ile Gln Arg
       530                 535                 540
   TAT GGA ATT TAT GAA CGT GAA AAA GAT TTG GTT GTT ATT AAA ATA GAT     1680
   Tyr Gly Ile Tyr Glu Arg Glu Lys Asp Leu Val Val Ile Lys Ile Asp
   545                 550                 555                 560
   TCA GGA AAA GCT AAG CTT CAG ATA CTT GAT AAA CTC GAG AAT TTA AAA     1728
   Ser Gly Lys Ala Lys Leu Gln Ile Leu Asp Lys Leu Glu Asn Leu Lys
               565                 570                 575
   GTG ATA TCA GAG TCT AAT TTT GAG ATT AAT AAA AAT TCA TCT CTT TAT     1776
   Val Ile Ser Glu Ser Asn Phe Glu Ile Asn Lys Asn Ser Ser Leu Tyr
           580                 585                 590
   GTT GAC TCT AGA ATG ATT TTA GTA GTT GTT AAG GAC GAT AGT AAT GCT     1824
   Val Asp Ser Arg Met Ile Leu Val Val Val Lys Asp Asp Ser Asn Ala
       595                 600                 605
   TGG AGA TTG GCT AAA TTT TCT CCT AAA AAT TTA GAT GAA TTT ATT CTG     1872
   Trp Arg Leu Ala Lys Phe Ser Pro Lys Asn Leu Asp Glu Phe Ile Leu
       610                 615                 620
   TCA GAA AAT AAA ATT TTG CCT TTT ACT AGC TTT GCT GTG AGA AAG AAT     1920
   Ser Glu Asn Lys Ile Leu Pro Phe Thr Ser Phe Ala Val Arg Lys Asn
   625                 630                 635                 640
   TTT ATT TAT TTG CAA GAT GAA CTT AAA AGC TTA GTT ACT TTA GAT GTA        1968
   Phe Ile Tyr Leu Gln Asp Glu Leu Lys Ser Leu Val Thr Leu Asp Val
                   645                 650                 655
   AAT ACT TTA AAA AAA GTT AAG TA                                         1991
   Asn Thr Leu Lys Lys Val Lys
               660
(2) INFORMATION FOR SEQ ID NO:78:
   (i) SEQUENCE CHARACTERISTICS:
      (A) LENGTH: 663 amino acids
      (B) TYPE: amino acid
      (D) TOPOLOGY: linear
   (ii) MOLECULE TYPE: protein
   (xi) SEQUENCE DESCRIPTION: SEQ ID NO:78:
   Met Lys Lys Met Leu Leu Ile Phe Ser Phe Phe Leu Val Phe Leu Asn
     1               5                  10                  15
   Gly Phe Pro Leu Asn Ala Arg Glu Val Asp Lys Glu Lys Leu Lys Asp
                20                  25                  30
   Phe Val Asn Met Asp Leu Glu Phe Val Asn Tyr Lys Gly Pro Tyr Asp
            35                  40                  45
   Ser Thr Asn Thr Tyr Glu Gln Ile Val Gly Ile Gly Glu Phe Leu Ala
        50                  55                  60
   Arg Pro Leu Ile Asn Ser Asn Ser Asn Ser Ser Tyr Tyr Gly Lys Tyr
    65                  70                  75                  80
   Phe Val Asn Arg Phe Ile Asp Asp Gln Asp Lys Lys Ala Ser Val Asp
                    85                  90                  95
   Ile Phe Ser Ile Gly Ser Lys Ser Glu Leu Asp Ser Ile Leu Asn Leu
               100                 105                 110
   Arg Arg Ile Leu Thr Gly Tyr Leu Met Lys Ser Phe Asp Tyr Glu Arg
           115                 120                 125
   Ser Ser Ala Glu Leu Ile Ala Lys Ala Ile Thr Ile Tyr Asn Ala Val
       130                 135                 140
   Tyr Arg Gly Asp Leu Asp Tyr Tyr Lys Glu Phe Tyr Ile Glu Ala Ser
   145                 150                 155                 160
   Leu Lys Ser Leu Thr Lys Glu Asn Ala Gly Leu Ser Arg Val Tyr Ser
                   165                 170                 175
   Gln Trp Ala Gly Lys Thr Gln Ile Phe Ile Pro Leu Lys Lys Asn Ile
               180                 185                 190
   Leu Ser Gly Asn Val Glu Ser Asp Ile Asp Ile Asp Ser Leu Val Thr
           195                 200                 205
   Asp Lys Val Val Ala Ala Leu Leu Ser Glu Asn Glu Ser Gly Val Asn
       210                 215                 220
   Phe Ala Arg Asp Ile Thr Asp Ile Gln Gly Glu Thr His Lys Ala Asp
   225 230 235 240
   Gln Asp Lys Ile Asp Ile Glu Leu Asp Asn Phe His Glu Ser Asp Ser
   245 250 255
   Asn Ile Thr Glu Thr Ile Glu Asn Leu Arg Asp Gln Leu Glu Lys Ala
   260 265 270
   Thr Asp Glu Glu His Lys Lys Glu Ile Glu Ser Gln Val Asp Ala Lys
   275 280 285
   Lys Lys Gln Lys Glu Glu Leu Asp Lys Lys Ala Ile Asp Leu Asp Lys
   290 295 300
   Ala Gln Gln Lys Leu Asp Phe Ala Glu Asp Asn Leu Asp Ile Gln Arg
   305 310 315 320
   Asp Thr Val Arg Glu Lys Leu Gln Glu Asn Ile Asn Glu Thr Asn Lys
   325 330 335
   Glu Lys Asn Leu Pro Lys Pro Gly Asp Val Ser Ser Pro Lys Val Asp
   340 345 350
   Lys Gln Leu Gln Ile Lys Glu Ser Leu Glu Asp Leu Gln Glu Gln Leu
   355 360 365
   Lys Glu Ala Ser Asp Glu Asn Gln Lys Arg Glu Ile Glu Lys Gln Ile
   370 375 380
   Glu Ile Lys Lys Asn Asp Glu Glu Leu Phe Lys Asn Lys Asp His Lys
   385 390 395 400
   Ala Leu Asp Leu Lys Gln Glu Leu Asn Ser Lys Ala Ser Ser Lys Glu
   405 410 415
   Lys Ile Glu Gly Glu Glu Glu Asp Lys Glu Leu Asp Ser Lys Lys Asn
   420 425 430
   Leu Glu Pro Val Ser Glu Ala Asp Lys Val Asp Lys Ile Ser Lys Ser
   435 440 445
   Asn Asn Asn Glu Val Ser Lys Leu Ser Pro Leu Asp Glu Pro Ser Tyr
   450 455 460
   Ser Asp Ile Asp Ser Lys Glu Gly Val Asp Asn Lys Asp Val Asp Leu
   465 470 475 480
   Gln Lys Thr Lys Pro Gln Val Glu Ser Gln Pro Thr Ser Leu Asn Glu
   485 490 495
   Asp Leu Ile Asp Val Ser Ile Asp Ser Ser Asn Pro Val Phe Leu Glu
   500 505 510
   Val Ile Asp Pro Ile Thr Asn Leu Gly Thr Leu Gln Leu Ile Asp Leu
   515 520 525
   Asn Thr Gly Val Arg Leu Lys Glu Ser Ala Gln Gln Gly Ile Gln Arg
   530 535 540
   Tyr Gly Ile Tyr Glu Arg Glu Lys Asp Leu Val Val Ile Lys Ile Asp
   545 550 555 560
   Ser Gly Lys Ala Lys Leu Gln Ile Leu Asp Lys Leu Glu Asn Leu Lys
                   565                 570                 575
   Val Ile Ser Glu Ser Asn Phe Glu Ile Asn Lys Asn Ser Ser Leu Tyr
               580                 585                 590
   Val Asp Ser Arg Met Ile Leu Val Val Val Lys Asp Asp Ser Asn Ala
           595                 600                 605
   Trp Arg Leu Ala Lys Phe Ser Pro Lys Asn Leu Asp Glu Phe Ile Leu
       610                 615                 620
   Ser Glu Asn Lys Ile Leu Pro Phe Thr Ser Phe Ala Val Arg Lys Asn
   625                 630                 635                 640
   Phe Ile Tyr Leu Gln Asp Glu Leu Lys Ser Leu Val Thr Leu Asp Val
                   645                 650                 655
   Asn Thr Leu Lys Lys Val Lys
               660
(2) INFORMATION FOR SEQ ID NO:79:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:79:
   CCGGTCACCC CATGGCTGCT TTAAAGTCTT TA                                32
(2) INFORMATION FOR SEQ ID NO:80:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:80:
   CCGGTCACCC CATGAATCTT GATAAAGCTC AG                                32
(2) INFORMATION FOR SEQ ID NO:81:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:81:
   CCGGTCACCC CATGGATGAA AAGCTTTTAA AAAGT                             35
(2) INFORMATION FOR SEQ ID NO:82:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:82:
   CCGGTCACCC CCATGGTTGA GAAATTAGAT AAG 33
(2) INFORMATION FOR SEQ ID NO:83:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:83:
   TTGGATCCGG TGACCCTTAA CTTTTTTTAA AG 32
(2) INFORMATION FOR SEQ ID NO:84:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:84:
   AAAGTAGAAG TTTTTGAATC CCATTTTCCA GTTTTTTT 38
(2) INFORMATION FOR SEQ ID NO:85:
   (i) SEQUENCE CHARACTERISTICS:
      (A) LENGTH: 825 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..825
   (xi) SEQUENCE DESCRIPTION: SEQ ID NO:85:
(2) INFORMATION FOR SEQ ID NO:86:
   (i) SEQUENCE CHARACTERISTICS:
      (A) LENGTH: 274 amino acids
      (B) TYPE: amino acid
      (D) TOPOLOGY: linear
   (ii) MOLECULE TYPE: protein
   (xi) SEQUENCE DESCRIPTION: SEQ ID NO:86:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Lys Thr Ser Thr Leu
       210                 215                 220
   Thr Ile Ser Val Asn Ser Lys Lys Thr Thr Gln Leu Val Phe Thr Lys
   225                 230                 235                 240
   Gln Tyr Thr Ile Thr Val Lys Gln Tyr Asp Ser Ala Gly Thr Asn Leu
                   245                 250                 255
   Glu Gly Thr Ala Val Glu Ile Lys Thr Leu Asp Glu Leu Lys Asn Ala
               260                 265                 270
   Leu Lys
(2) INFORMATION FOR SEQ ID NO:87:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:87:
   AAAGTAGAAG TTTTTGAATT CCAAGCTGCA GTTTT                              35
(2) INFORMATION FOR SEQ ID NO:88:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:88:
(2) INFORMATION FOR SEQ ID NO:89:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:89:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                 230                 235                 240
   Tyr Thr Ile Thr Val Lys Gln Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Lys
(2) INFORMATION FOR SEQ ID NO:90:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:90:
   AAAGTGGAAG TTTTTGAATT CCAAGCTGCA GTTTTTTT                    38
(2) INFORMATION FOR SEQ ID NO:91:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:91:
(2) INFORMATION FOR SEQ ID NO:92:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:92:
   Met Lys Lys Tyr Leu Leu Gly Ile Gly Leu Ile Leu Ala Leu Ile Ala
   1 5 10 15
   Cys Lys Gln Asn Val Ser Ser Leu Asp Glu Lys Asn Ser Val Ser Val
   20 25 30
   Asp Leu Pro Gly Glu Met Lys Val Leu Val Ser Lys Glu Lys Asn Lys
   35 40 45
   Asp Gly Lys Tyr Asp Leu Ile Ala Thr Val Asp Lys Leu Glu Leu Lys
   50 55 60
   Gly Thr Ser Asp Lys Asn Asn Gly Ser Gly Val Leu Glu Gly Val Lys
   65 70 75 80
   Ala Asp Lys Ser Lys Val Lys Leu Thr Ile Ser Asp Asp Leu Gly Gln
   85 90 95
   Thr Thr Leu Glu Val Phe Lys Glu Asp Gly Lys Thr Leu Val Ser Lys
   100 105 110
   Lys Val Thr Ser Lys Asp Lys Ser Ser Thr Glu Glu Lys Phe Asn Glu
   115 120 125
   Lys Gly Glu Val Ser Glu Lys Ile Ile Thr Arg Ala Asp Gly Thr Arg
   130 135 140
   Leu Glu Tyr Thr Gly Ile Lys Ser Asp Gly Ser Gly Lys Ala Lys Glu
   145 150 155 160
   Val Leu Lys Gly Tyr Val Leu Glu Gly Thr Leu Thr Ala Glu Lys Thr
   165 170 175
   Thr Leu Val Val Lys Glu Gly Thr Val Thr Leu Ser Lys Asn Ile Ser
   180 185 190
   Lys Ser Gly Glu Val Ser Val Glu Leu Asn Asp Thr Asp Ser Ser Ala
   195 200 205
   Ala Thr Lys Lys Thr Ala Ala Trp Asn Ser Lys Thr Ser Thr Leu Thr
   210 215 220
   Ile Ser Val Asn Ser Gln Lys Thr Lys Asn Leu Val Phe Thr Lys Glu
   225 230 235 240
   Asp Thr Ile Thr Val Gln Lys Tyr Asp Ser Ala Gly Thr Asn Leu Glu
   245 250 255
   Gly Lys Ala Val Glu Ile Thr Thr Leu Lys Glu Leu Lys Asn Ala Leu
   260 265 270
   Lys
(2) INFORMATION FOR SEQ ID NO:93:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:93:
   TAAAGTTGAA GTGCCTGCAT TCCAAGCTGC AGTTT                                35
(2) INFORMATION FOR SEQ ID NO:94:
   (i) SEQUENCE CHARACTERISTICS:
      (A) LENGTH: 819 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..819
   (xi) SEQUENCE DESCRIPTION: SEQ ID NO:94:
   ATG AAA AAA TAT TTA TTG GGA ATA GGT CTA ATA TTA GCC TTA ATA GCA       48
   Met Lys Lys Tyr Leu Leu Gly Ile Gly Leu Ile Leu Ala Leu Ile Ala
     1               5                  10                  15
   TGT AAG CAA AAT GTT AGC AGC CTT GAC GAG AAA AAC AGC GTT TCA GTA       96
   Cys Lys Gln Asn Val Ser Ser Leu Asp Glu Lys Asn Ser Val Ser Val
                20                  25                  30
   GAT TTG CCT GGT GAA ATG AAA GTT CTT GTA AGC AAA GAA AAA AAC AAA      144
   Asp Leu Pro Gly Glu Met Lys Val Leu Val Ser Lys Glu Lys Asn Lys
            35                  40                  45
   GAC GGC AAG TAC GAT CTA ATT GCA ACA GTA GAC AAG CTT GAG CTT AAA      192
   Asp Gly Lys Tyr Asp Leu Ile Ala Thr Val Asp Lys Leu Glu Leu Lys
        50                  55                  60
   GGA ACT TCT GAT AAA AAC AAT GGA TCT GGA GTA CTT GAA GGC GTA AAA      240
   Gly Thr Ser Asp Lys Asn Asn Gly Ser Gly Val Leu Glu Gly Val Lys
    65                  70                  75                  80
   GCT GAC AAA AGT AAA GTA AAA TTA ACA ATT TCT GAC GAT CTA GGT CAA      288
   Ala Asp Lys Ser Lys Val Lys Leu Thr Ile Ser Asp Asp Leu Gly Gln
                    85                  90                  95
   ACC ACA CTT GAA GTT TTC AAA GAA GAT GGC AAA ACA CTA GTA TCA AAA      336
   Thr Thr Leu Glu Val Phe Lys Glu Asp Gly Lys Thr Leu Val Ser Lys
               100                 105                 110
   AAA GTA ACT TCC AAA GAC AAG TCA TCA ACA GAA GAA AAA TTC AAT GAA      384
   Lys Val Thr Ser Lys Asp Lys Ser Ser Thr Glu Glu Lys Phe Asn Glu
           115                 120                 125
   AAA GGT GAA GTA TCT GAA AAA ATA ATA ACA AGA GCA GAC GGA ACC AGA      432
   Lys Gly Glu Val Ser Glu Lys Ile Ile Thr Arg Ala Asp Gly Thr Arg
       130                 135                 140
   CTT GAA TAC ACA GGA ATT AAA AGC GAT GGA TCT GGA AAA GCT AAA GAG      480
   Leu Glu Tyr Thr Gly Ile Lys Ser Asp Gly Ser Gly Lys Ala Lys Glu
   145                 150                 155                 160
   GTT TTA AAA GGC TAT GTT CTT GAA GGA ACT CTA ACT GCT GAA AAA ACA      528
   Val Leu Lys Gly Tyr Val Leu Glu Gly Thr Leu Thr Ala Glu Lys Thr
                   165                 170                 175
   ACA TTG GTG GTT AAA GAA GGA ACT GTT ACT TTA AGC AAA AAT ATT TCA      576
   Thr Leu Val Val Lys Glu Gly Thr Val Thr Leu Ser Lys Asn Ile Ser
               180                 185                 190
   AAA TCT GGG GAA GTT TCA GTT GAA CTT AAT GAC ACT GAC AGT AGT GCT      624
   Lys Ser Gly Glu Val Ser Val Glu Leu Asn Asp Thr Asp Ser Ser Ala
           195                 200                 205
   GCT ACT AAA AAA ACT GCA GCT TGG AAT GCA GGC ACT TCA ACT TTA ACA      672
   Ala Thr Lys Lys Thr Ala Ala Trp Asn Ala Gly Thr Ser Thr Leu Thr
       210                 215                 220
   ATT ACT GTA AAC AAC AAA AAA ACT AAA GCC CTT GTA TTT ACA AAA CAA      720
   Ile Thr Val Asn Asn Lys Lys Thr Lys Ala Leu Val Phe Thr Lys Gln
   225                 230                 235                 240
   GAC ACA ATT ACA TCA CAA AAA TAC GAC TCA GCA GGA ACC AAC TTG GAA      768
   Asp Thr Ile Thr Ser Gln Lys Tyr Asp Ser Ala Gly Thr Asn Leu Glu
                   245                 250                 255
   GGC ACA GCA GTC GAA ATT AAA ACA CTT GAT GAA CTT AAA AAC GCT TTA      816
   Gly Thr Ala Val Glu Ile Lys Thr Leu Asp Glu Leu Lys Asn Ala Leu
               260                 265                 270
   AGA                                                                  819
   Arg
(2) INFORMATION FOR SEQ ID NO:95:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:95:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
            35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ala Gly Thr Ser Thr Leu Thr
       210                 215                 220
   Ile Thr Val Asn Asn Lys Lys Thr Lys Ala Leu Val Phe Thr Lys Gln
   225                 230                 235                 240
   Asp Thr Ile Thr Ser Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Arg
(2) INFORMATION FOR SEQ ID NO:96:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:96:
   CCCCAGATTT TGAAATCTTG CTTAAAACAA C                                31
(2) INFORMATION FOR SEQ ID NO:97:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:97:
(2) INFORMATION FOR SEQ ID NO:98:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:98:
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
(2) INFORMATION FOR SEQ ID NO:99:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:99:
   CAAGTCTGGT TCCAATTTGC TCTTGTTATT AT                                  32
(2) INFORMATION FOR SEQ ID NO:100:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..822
(2) INFORMATION FOR SEQ ID NO:101:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:101:
   Met Lys Lys Tyr Leu Leu Gly Ile Gly Leu Ile Leu Ala Leu Ile Ala
     1               5                  10                  15
   Cys Lys Gln Asn Val Ser Ser Leu Asp Glu Lys Asn Ser Val Ser Val
                20                  25                  30
   Asp Leu Pro Gly Gly Met Thr Val Leu Val Ser Lys Glu Lys Asp Lys
            35                  40                  45
   Asp Gly Lys Tyr Ser Leu Glu Ala Thr Val Asp Lys Leu Glu Leu Lys
        50                  55                  60
   Gly Thr Ser Asp Lys Asn Asn Gly Ser Gly Thr Leu Glu Gly Glu Lys
    65                  70                  75                  80
   Thr Asp Lys Ser Lys Val Lys Leu Thr Ile Ala Asp Asp Leu Ser Gln
                    85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
(2) INFORMATION FOR SEQ ID NO:102:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:102:
   GTTAAAGTGC TAGTACTGTC ATTCCAAGCT GCAGTTTTTT T                    41
(2) INFORMATION FOR SEQ ID NO:103:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..822
   (xi) SEQUENCE DESCRIPTION: SEQ ID NO:103:
(2) INFORMATION FOR SEQ ID NO:104:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:104:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Asp Ser Thr Ser Thr Leu Thr
       210                 215                 220
   Ile Ser Ala Asp Ser Lys Lys Thr Lys Asp Leu Val Phe Leu Thr Asp
   225                 230                 235                 240
   Gly Thr Ile Thr Val Gln Gln Tyr Asn Thr Ala Gly Thr Ser Leu Glu
                   245                 250                 255
   Gly Ser Ala Ser Glu Ile Lys Asn Leu Ser Glu Leu Lys Asn Ala Leu
               260                 265                 270
   Lys
(2) INFORMATION FOR SEQ ID NO:105:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:105:
   TGCAGATGTA ATCCCATCCG CCATTTTTAA AGCGTTTTT                              39
(2) INFORMATION FOR SEQ ID NO:106:
   (i) SEQUENCE CHARACTERISTICS:
      (A) LENGTH: 1401 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1401
   (xi) SEQUENCE DESCRIPTION: SEQ ID NO:106:
(2) INFORMATION FOR SEQ ID NO:107:
   (i) SEQUENCE CHARACTERISTICS:
      (A) LENGTH: 466 amino acids
      (B) TYPE: amino acid
      (D) TOPOLOGY: linear
   (ii) MOLECULE TYPE: protein
   (xi) SEQUENCE DESCRIPTION: SEQ ID NO:107:
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65                  70                  75                  80
   Ala Asp Lys Ser Lys Val Lys Leu Thr Ile Ser Asp Asp Leu Gly Gln
                   85                  90                  95
   Thr Thr Leu Glu Val Phe Lys Glu Asp Gly Lys Thr Leu Val Ser Lys
               100                 105                 110
   Lys Val Thr Ser Lys Asp Lys Ser Ser Thr Glu Glu Lys Phe Asn Glu
           115                 120                 125
   Lys Gly Glu Val Ser Glu Lys Ile Ile Thr Arg Ala Asp Gly Thr Arg
       130                 135                 140
   Leu Glu Tyr Thr Gly Ile Lys Ser Asp Gly Ser Gly Lys Ala Lys Glu
   145                 150                 155                 160
   Val Leu Lys Gly Tyr Val Leu Glu Gly Thr Leu Thr Ala Glu Lys Thr
                   165                 170                 175
   Thr Leu Val Val Lys Glu Gly Thr Val Thr Leu Ser Lys Asn Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Asp Ser Thr Ser Thr Leu Thr
       210                 215                 220
   Ile Ser Ala Asp Ser Lys Lys Thr Lys Asp Leu Val Phe Leu Thr Asp
   225                 230                 235                 240
   Gly Thr Ile Thr Val Gln Gln Tyr Asn Thr Ala Gly Thr Ser Leu Glu
                   245                 250                 255
   Gly Ser Ala Ser Glu Ile Lys Asn Leu Ser Glu Leu Lys Asn Ala Leu
               260                 265                 270
   Lys Met Ala Asn Asn Ser Gly Lys Asp Gly Asn Thr Ser Ala Asn Ser
           275                 280                 285
   Ala Asp Glu Ser Val Lys Gly Pro Asn Leu Thr Glu Ile Ser Lys Lys
       290                 295                 300
   Ile Thr Asp Ser Asn Ala Val Leu Leu Ala Val Lys Glu Val Glu Ala
   305                 310                 315                 320
   Leu Leu Ser Ser Ile Asp Glu Ile Ala Ala Lys Ala Ile Gly Lys Lys
                   325                 330                 335
   Ile His Gln Asn Asn Gly Leu Asp Thr Glu Tyr Asn His Asn Gly Ser
               340                 345                 350
   Leu Leu Ala Gly Arg Tyr Ala Ile Ser Thr Leu Ile Lys Gln Lys Leu
           355                 360                 365
   Asp Gly Leu Lys Asn Glu Gly Leu Lys Glu Lys Ile Asp Ala Ala Lys
       370                 375                 380
   Lys Cys Ser Glu Thr Phe Thr Asn Lys Leu Lys Ala Lys His Thr Asp
   385                 390                 395                 400
   Leu Gly Lys Glu Gly Val Thr Asp Ala Asp Ala Lys Glu Ala Ile Leu
                   405                 410                 415
   Lys Thr Asn Gly Thr Lys Thr Lys Gly Ala Glu Glu Leu Gly Lys Leu
               420                 425                 430
   Phe Glu Ser Val Glu Val Leu Ser Lys Ala Ala Lys Glu Met Leu Ala
           435                 440                 445
   Asn Ser Val Lys Glu Leu Thr Ser Pro Val Val Ala Glu Ser Pro Lys
       450                 455                 460
   Lys Pro
   465
(2) INFORMATION FOR SEQ ID NO:108:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:108:
   GCTGCTAACA TTTTGCTTAG GTTTTTTTGG ACTTTC                                36
(2) INFORMATION FOR SEQ ID NO:109:
   (i) SEQUENCE CHARACTERISTICS:
      (A) LENGTH: 1401 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1401
   (xi) SEQUENCE DESCRIPTION: SEQ ID NO:109:
(2) INFORMATION FOR SEQ ID NO:110:
   (i) SEQUENCE CHARACTERISTICS:
      (A) LENGTH: 466 amino acids
      (B) TYPE: amino acid
      (D) TOPOLOGY: linear
   (ii) MOLECULE TYPE: protein
   (xi) SEQUENCE DESCRIPTION: SEQ ID NO:110:
   Met Lys Lys Asn Thr Leu Ser Ala Ile Leu Met Thr Leu Phe Leu Phe
   1               5                   10                  15
   Ile Ser Cys Asn Asn Ser Gly Lys Asp Gly Asn Thr Ser Ala Asn Ser
               20                  25                  30
   Ala Asp Glu Ser Val Lys Gly Pro Asn Leu Thr Glu Ile Ser Lys Lys
           35                  40                  45
   Ile Thr Asp Ser Asn Ala Val Leu Leu Ala Val Lys Glu Val Glu Ala
   50 55 60
   Leu Leu Ser Ser Ile Asp Glu Ile Ala Ala Lys Ala Ile Gly Lys Lys
   65 70 75 80
   Ile His Gln Asn Asn Gly Leu Asp Thr Glu Tyr Asn His Asn Gly Ser
   85 90 95
   Leu Leu Ala Gly Arg Tyr Ala Ile Ser Thr Leu Ile Lys Gln Lys Leu
   100 105 110
   Asp Gly Leu Lys Asn Glu Gly Leu Lys Glu Lys Ile Asp Ala Ala Lys
   115 120 125
   Lys Cys Ser Glu Thr Phe Thr Asn Lys Leu Lys Ala Lys His Thr Asp
   130 135 140
   Leu Gly Lys Glu Gly Val Thr Asp Ala Asp Ala Lys Glu Ala Ile Leu
   145 150 155 160
   Lys Thr Asn Gly Thr Lys Thr Lys Gly Ala Glu Glu Leu Gly Lys Leu
   165 170 175
   Phe Glu Ser Val Glu Val Leu Ser Lys Ala Ala Lys Glu Met Leu Ala
   180 185 190
   Asn Ser Val Lys Glu Leu Thr Ser Pro Val Val Ala Glu Ser Pro Lys
   195 200 205
   Lys Pro Lys Gln Asn Val Ser Ser Leu Asp Glu Lys Asn Ser Val Ser
   210 215 220
   Val Asp Leu Pro Gly Glu Met Lys Val Leu Val Ser Lys Glu Lys Asn
   225 230 235 240
   Lys Asp Gly Lys Tyr Asp Leu Ile Ala Thr Val Asp Lys Leu Glu Leu
   245 250 255
   Lys Gly Thr Ser Asp Lys Asn Asn Gly Ser Gly Val Leu Glu Gly Val
   260 265 270
   Lys Ala Asp Lys Ser Lys Val Lys Leu Thr Ile Ser Asp Asp Leu Gly
   275 280 285
   Gln Thr Thr Leu Glu Val Phe Lys Glu Asp Gly Lys Thr Leu Val Ser
   290 295 300
   Lys Lys Val Thr Ser Lys Asp Lys Ser Ser Thr Glu Glu Lys Phe Asn
   305 310 315 320
   Glu Lys Gly Glu Val Ser Glu Lys Ile Ile Thr Arg Ala Asp Gly Thr
   325 330 335
   Arg Leu Glu Tyr Thr Gly Ile Lys Ser Asp Gly Ser Gly Lys Ala Lys
   340 345 350
   Glu Val Leu Lys Gly Tyr Val Leu Glu Gly Thr Leu Thr Ala Glu Lys
   355 360 365
   Thr Thr Leu Val Val Lys Glu Gly Thr Val Thr Leu Ser Lys Asn Ile
   370 375 380
   Ser Lys Ser Gly Glu Val Ser Val Glu Leu Asn Asp Thr Asp Ser Ser
   385                 390                 395                 400
   Ala Ala Thr Lys Lys Thr Ala Ala Trp Asn Asp Ser Thr Ser Thr Leu
                   405                 410                 415
   Thr Ile Ser Ala Asp Ser Lys Lys Thr Lys Asp Leu Val Phe Leu Thr
               420                 425                 430
   Asp Gly Thr Ile Thr Val Gln Gln Tyr Asn Thr Ala Gly Thr Ser Leu
           435                 440                 445
   Glu Gly Ser Ala Ser Glu Ile Lys Asn Leu Ser Glu Leu Lys Asn Ala
       450                 455                 460
   Leu Lys
   465
(2) INFORMATION FOR SEQ ID NO:111:
   (i) SEQUENCE CHARACTERISTICS:
      (A) LENGTH: 1720 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1719
   (xi) SEQUENCE DESCRIPTION: SEQ ID NO:111:
   AAG CAA AAT GTT AGC AGC CTT GAC GAG AAA AAC AGC GTT TCA GTA GAT      48
   Lys Gln Asn Val Ser Ser Leu Asp Glu Lys Asn Ser Val Ser Val Asp
     1               5                  10                  15
   TTG CCT GGT GAA ATG AAA GTT CTT GTA AGC AAA GAA AAA AAC AAA GAC      96
   Leu Pro Gly Glu Met Lys Val Leu Val Ser Lys Glu Lys Asn Lys Asp
                20                  25                  30
   GGC AAG TAC GAT CTA ATT GCA ACA GTA GAC AAG CTT GAG CTT AAA GGA     144
   Gly Lys Tyr Asp Leu Ile Ala Thr Val Asp Lys Leu Glu Leu Lys Gly
            35                  40                  45
   ACT TCT GAT AAA AAC AAT GGA TCT GGA GTA CTT GAA GGC GTA AAA GCT     192
   Thr Ser Asp Lys Asn Asn Gly Ser Gly Val Leu Glu Gly Val Lys Ala
        50                  55                  60
   GAC AAA AGT AAA GTA AAA TTA ACA ATT TCT GAC GAT CTA GGT CAA ACC     240
   Asp Lys Ser Lys Val Lys Leu Thr Ile Ser Asp Asp Leu Gly Gln Thr
    65                  70                  75                  80
   ACA CTT GAA GTT TTC AAA GAA GAT GGC AAA ACA CTA GTA TCA AAA AAA     288
   Thr Leu Glu Val Phe Lys Glu Asp Gly Lys Thr Leu Val Ser Lys Lys
                    85                  90                  95
   GTA ACT TCC AAA GAC AAG TCA TCA ACA GAA GAA AAA TTC AAT GAA AAA      336
   Val Thr Ser Lys Asp Lys Ser Ser Thr Glu Glu Lys Phe Asn Glu Lys
               100                 105                 110
   GGT GAA GTA TCT GAA AAA ATA ATA ACA AGA GCA GAC GGA ACC AGA CTT      384
   Gly Glu Val Ser Glu Lys Ile Ile Thr Arg Ala Asp Gly Thr Arg Leu
           115                 120                 125
   GAA TAC ACA GGA ATT AAA AGC GAT GGA TCT GGA AAA GCT AAA GAG GTT      432
   Glu Tyr Thr Gly Ile Lys Ser Asp Gly Ser Gly Lys Ala Lys Glu Val
       130                 135                 140
   TTA AAA GGC TAT GTT CTT GAA GGA ACT CTA ACT GCT GAA AAA ACA ACA      480
   Leu Lys Gly Tyr Val Leu Glu Gly Thr Leu Thr Ala Glu Lys Thr Thr
   145                 150                 155                 160
   TTG GTG GTT AAA GAA GGA ACT GTT ACT TTA AGC AAA AAT ATT TCA AAA      528
   Leu Val Val Lys Glu Gly Thr Val Thr Leu Ser Lys Asn Ile Ser Lys
                   165                 170                 175
   TCT GGG GAA GTT TCA GTT GAA CTT AAT GAC ACT GAC AGT AGT GCT GCT      576
   Ser Gly Glu Val Ser Val Glu Leu Asn Asp Thr Asp Ser Ser Ala Ala
               180                 185                 190
   ACT AAA AAA ACT GCA GCT TGG AAT TCA GGC ACT TCA ACT TTA ACA ATT      624
   Thr Lys Lys Thr Ala Ala Trp Asn Ser Gly Thr Ser Thr Leu Thr Ile
           195                 200                 205
   ACT GTA AAC AGT AAA AAA ACT AAA GAC CTT GTG TTT ACA AAA GAA AAC      672
   Thr Val Asn Ser Lys Lys Thr Lys Asp Leu Val Phe Thr Lys Glu Asn
       210                 215                 220
   ACA ATT ACA GTA CAA CAA TAC GAC TCA AAT GGC ACC AAA TTA GAG GGG      720
   Thr Ile Thr Val Gln Gln Tyr Asp Ser Asn Gly Thr Lys Leu Glu Gly
   225                 230                 235                 240
   TCA GCA GTT GAA ATT ACA AAA CTT GAT GAA ATT AAA AAC GCT TTA AAA      768
   Ser Ala Val Glu Ile Thr Lys Leu Asp Glu Ile Lys Asn Ala Leu Lys
                   245                 250                 255
   GGT CAC CCC ATG GAT GAA AAG CTT TTA AAA AGT AAA GAT GAT AAA GCA      816
   Gly His Pro Met Asp Glu Lys Leu Leu Lys Ser Lys Asp Asp Lys Ala
               260                 265                 270
   AGT AAA GAT GGT AAA GCC TTG GAT CTT GAT CGA GAA TTA AAT TCT AAA      864
   Ser Lys Asp Gly Lys Ala Leu Asp Leu Asp Arg Glu Leu Asn Ser Lys
           275                 280                 285
   GCT TCT AGC AAA GAA AAA AGT AAA GCC AAG GAA GAA GAA ATA ACC AAG      912
   Ala Ser Ser Lys Glu Lys Ser Lys Ala Lys Glu Glu Glu Ile Thr Lys
       290                 295                 300
   GGT AAG TCA CAG AAA AGC TTA GGC GAT TTG AAT AAT GAT GAA AAT CTT      960
   Gly Lys Ser Gln Lys Ser Leu Gly Asp Leu Asn Asn Asp Glu Asn Leu
   305                 310                 315                 320
   ATG ATG CCA GAA GAT CAA AAA TTA CCT GAG GTT AAA AAA TTA GAT AGC     1008
   Met Met Pro Glu Asp Gln Lys Leu Pro Glu Val Lys Lys Leu Asp Ser
                   325                 330                 335
   AAA AAA GAA TTT AAA CCT GTT TCT GAG GTT GAG AAA TTA GAT AAG ATT      1056
   Lys Lys Glu Phe Lys Pro Val Ser Glu Val Glu Lys Leu Asp Lys Ile
               340                 345                 350
   TTC AAG TCT AAT AAC AAT GTT GGA GAA TTA TCA CCG TTA GAT AAA TCT      1104
   Phe Lys Ser Asn Asn Asn Val Gly Glu Leu Ser Pro Leu Asp Lys Ser
           355                 360                 365
   TCT TAT AAA GAC ATT GAT TCA AAA GAG GAG ACA GTT AAT AAA GAT GTT      1152
   Ser Tyr Lys Asp Ile Asp Ser Lys Glu Glu Thr Val Asn Lys Asp Val
       370                 375                 380
   AAT TTG CAA AAG ACT AAG CCT CAG GTT AAA GAC CAA GTT ACT TCT TTG      1200
   Asn Leu Gln Lys Thr Lys Pro Gln Val Lys Asp Gln Val Thr Ser Leu
   385                 390                 395                 400
   AAT GAA GAT TTG ACT ACT ATG TCT ATA GAT TCC AGT AGT CCT GTA TTT      1248
   Asn Glu Asp Leu Thr Thr Met Ser Ile Asp Ser Ser Ser Pro Val Phe
                   405                 410                 415
   TTA GAG GTT ATT GAT CCA ATT ACA AAT TTA GGA ACT CTT CAA CTT ATT      1296
   Leu Glu Val Ile Asp Pro Ile Thr Asn Leu Gly Thr Leu Gln Leu Ile
               420                 425                 430
   GAT TTA AAT ACT GGT GTT AGG CTT AAA GAA AGC ACT CAG CAA GGC ATT      1344
   Asp Leu Asn Thr Gly Val Arg Leu Lys Glu Ser Thr Gln Gln Gly Ile
           435                 440                 445
   CAG CGG TAT GGA ATT TAT GAA CGT GAA AAA GAT TTG GTT GTT ATT AAA      1392
   Gln Arg Tyr Gly Ile Tyr Glu Arg Glu Lys Asp Leu Val Val Ile Lys
       450                 455                 460
   ATG GAT TCA GGA AAA GCT AAG CTT CAG ATA CTT GAT AAA CTT GAA AAT      1440
   Met Asp Ser Gly Lys Ala Lys Leu Gln Ile Leu Asp Lys Leu Glu Asn
   465                 470                 475                 480
   TTA AAA GTG GTA TCA GAG TCT AAT TTT GAG ATT AAT AAA AAT TCA TCT      1488
   Leu Lys Val Val Ser Glu Ser Asn Phe Glu Ile Asn Lys Asn Ser Ser
                   485                 490                 495
   CTT TAT GTT GAT TCT AAA ATG ATT TTA GTA GCT GTT AGG GAT AAA GAT      1536
   Leu Tyr Val Asp Ser Lys Met Ile Leu Val Ala Val Arg Asp Lys Asp
               500                 505                 510
   AGT AGT AAT GAT TGG AGA TTG GCC AAA TTT TCT CCT AAA AAT TTA GAT      1584
   Ser Ser Asn Asp Trp Arg Leu Ala Lys Phe Ser Pro Lys Asn Leu Asp
           515                 520                 525
   GAG TTT ATT CTT TCA GAG AAT AAA ATT ATG CCT TTT ACT AGC TTT TCT      1632
   Glu Phe Ile Leu Ser Glu Asn Lys Ile Met Pro Phe Thr Ser Phe Ser
       530                 535                 540
   GTG AGA AAA AAT TTT ATT TAT TTG CAA GAT GAG TTT AAA AGT CTA GTT      1680
   Val Arg Lys Asn Phe Ile Tyr Leu Gln Asp Glu Phe Lys Ser Leu Val
   545                 550                 555                 560
   ATT TTA GAT GTA AAT ACT TTA AAA AAA GTT AAG GGT CAC C                1720
   Ile Leu Asp Val Asn Thr Leu Lys Lys Val Lys Gly His
                   565                 570
(2) INFORMATION FOR SEQ ID NO:112:
   (i) SEQUENCE CHARACTERISTICS:
      (A) LENGTH: 573 amino acids
      (B) TYPE: amino acid
      (D) TOPOLOGY: linear
   (ii) MOLECULE TYPE: protein
   (xi) SEQUENCE DESCRIPTION: SEQ ID NO:112:
   Lys Gln Asn Val Ser Ser Leu Asp Glu Lys Asn Ser Val Ser Val Asp
    1               5                  10                  15
   Leu Pro Gly Glu Met Lys Val Leu Val Ser Lys Glu Lys Asn Lys Asp
               20                  25                  30
   Gly Lys Tyr Asp Leu Ile Ala Thr Val Asp Lys Leu Glu Leu Lys Gly
           35                  40                  45
   Thr Ser Asp Lys Asn Asn Gly Ser Gly Val Leu Glu Gly Val Lys Ala
       50                  55                  60
   Asp Lys Ser Lys Val Lys Leu Thr Ile Ser Asp Asp Leu Gly Gln Thr
   65                  70                  75                  80
   Thr Leu Glu Val Phe Lys Glu Asp Gly Lys Thr Leu Val Ser Lys Lys
                   85                  90                  95
   Val Thr Ser Lys Asp Lys Ser Ser Thr Glu Glu Lys Phe Asn Glu Lys
               100                 105                 110
   Gly Glu Val Ser Glu Lys Ile Ile Thr Arg Ala Asp Gly Thr Arg Leu
           115                 120                 125
   Glu Tyr Thr Gly Ile Lys Ser Asp Gly Ser Gly Lys Ala Lys Glu Val
       130                 135                 140
   Leu Lys Gly Tyr Val Leu Glu Gly Thr Leu Thr Ala Glu Lys Thr Thr
   145                 150                 155                 160
   Leu Val Val Lys Glu Gly Thr Val Thr Leu Ser Lys Asn Ile Ser Lys
                   165                 170                 175
   Ser Gly Glu Val Ser Val Glu Leu Asn Asp Thr Asp Ser Ser Ala Ala
               180                 185                 190
   Thr Lys Lys Thr Ala Ala Trp Asn Ser Gly Thr Ser Thr Leu Thr Ile
           195                 200                 205
   Thr Val Asn Ser Lys Lys Thr Lys Asp Leu Val Phe Thr Lys Glu Asn
       210                 215                 220
   Thr Ile Thr Val Gln Gln Tyr Asp Ser Asn Gly Thr Lys Leu Glu Gly
   225                 230                 235                 240
   Ser Ala Val Glu Ile Thr Lys Leu Asp Glu Ile Lys Asn Ala Leu Lys
                   245                 250                 255
   Gly His Pro Met Asp Glu Lys Leu Leu Lys Ser Lys Asp Asp Lys Ala
               260                 265                 270
   Ser Lys Asp Gly Lys Ala Leu Asp Leu Asp Arg Glu Leu Asn Ser Lys
           275                 280                 285
   Ala Ser Ser Lys Glu Lys Ser Lys Ala Lys Glu Glu Glu Ile Thr Lys
       290                 295                 300
   Gly Lys Ser Gln Lys Ser Leu Gly Asp Leu Asn Asn Asp Glu Asn Leu
   305                 310                 315                 320
   Met Met Pro Glu Asp Gln Lys Leu Pro Glu Val Lys Lys Leu Asp Ser
                   325                 330                 335
   Lys Lys Glu Phe Lys Pro Val Ser Glu Val Glu Lys Leu Asp Lys Ile
               340                 345                 350
   Phe Lys Ser Asn Asn Asn Val Gly Glu Leu Ser Pro Leu Asp Lys Ser
           355                 360                 365
   Ser Tyr Lys Asp Ile Asp Ser Lys Glu Glu Thr Val Asn Lys Asp Val
       370                 375                 380
   Asn Leu Gln Lys Thr Lys Pro Gln Val Lys Asp Gln Val Thr Ser Leu
   385                 390                 395                 400
   Asn Glu Asp Leu Thr Thr Met Ser Ile Asp Ser Ser Ser Pro Val Phe
                   405                 410                 415
   Leu Glu Val Ile Asp Pro Ile Thr Asn Leu Gly Thr Leu Gln Leu Ile
               420                 425                 430
   Asp Leu Asn Thr Gly Val Arg Leu Lys Glu Ser Thr Gln Gln Gly Ile
           435                 440                 445
   Gln Arg Tyr Gly Ile Tyr Glu Arg Glu Lys Asp Leu Val Val Ile Lys
       450                 455                 460
   Met Asp Ser Gly Lys Ala Lys Leu Gln Ile Leu Asp Lys Leu Glu Asn
   465                 470                 475                 480
   Leu Lys Val Val Ser Glu Ser Asn Phe Glu Ile Asn Lys Asn Ser Ser
                   485                 490                 495
   Leu Tyr Val Asp Ser Lys Met Ile Leu Val Ala Val Arg Asp Lys Asp
               500                 505                 510
   Ser Ser Asn Asp Trp Arg Leu Ala Lys Phe Ser Pro Lys Asn Leu Asp
           515                 520                 525
   Glu Phe Ile Leu Ser Glu Asn Lys Ile Met Pro Phe Thr Ser Phe Ser
       530                 535                 540
   Val Arg Lys Asn Phe Ile Tyr Leu Gln Asp Glu Phe Lys Ser Leu Val
   545                 550                 555                 560
   Ile Leu Asp Val Asn Thr Leu Lys Lys Val Lys Gly His
                   565                 570
(2) INFORMATION FOR SEQ ID NO:113:
   (i) SEQUENCE CHARACTERISTICS:
      (A) LENGTH: 1180 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1179
   (xi) SEQUENCE DESCRIPTION: SEQ ID NO:113:
   GCA CAA AAA GGT GCT GAG TCA ATT GGT TCT CAA AAA GAA AAT GAT CTA      48
   Ala Gln Lys Gly Ala Glu Ser Ile Gly Ser Gln Lys Glu Asn Asp Leu
     1               5                  10                  15
   AAC CTT GAA GAC TCT AGT AAA AAA TCA CAT CAA AAC GCT AAA CAA GAC      96
   Asn Leu Glu Asp Ser Ser Lys Lys Ser His Gln Asn Ala Lys Gln Asp
                20                  25                  30
   CTT CCT GCG GTG ACA GAA GAC TCA GTG TCT TTG TTT AAT GGT AAT AAA     144
   Leu Pro Ala Val Thr Glu Asp Ser Val Ser Leu Phe Asn Gly Asn Lys
            35                  40                  45
   ATT TTT GTA AGC AAA GAA AAA AAT AGC TCC GGC AAA TAT GAT TTA AGA     192
   Ile Phe Val Ser Lys Glu Lys Asn Ser Ser Gly Lys Tyr Asp Leu Arg
        50                  55                  60
   GCA ACA ATT GAT CAG GTT GAA CTT AAA GGA ACT TCC GAT AAA AAC AAT     240
   Ala Thr Ile Asp Gln Val Glu Leu Lys Gly Thr Ser Asp Lys Asn Asn
    65                  70                  75                  80
   GGT TCT GGA ACC CTT GAA GGT TCA AAG CCT GAC AAG AGT AAA GTA AAA     288
   Gly Ser Gly Thr Leu Glu Gly Ser Lys Pro Asp Lys Ser Lys Val Lys
                    85                  90                  95
   TTA ACA GTT TCT GCT GAT TTA AAC ACA GTA ACC TTA GAA GCA TTT GAT     336
   Leu Thr Val Ser Ala Asp Leu Asn Thr Val Thr Leu Glu Ala Phe Asp
               100                 105                 110
   GCC AGC AAC CAA AAA ATT TCA AGT AAA GTT ACT AAA AAA CAG GGG TCA     384
   Ala Ser Asn Gln Lys Ile Ser Ser Lys Val Thr Lys Lys Gln Gly Ser
           115                 120                 125
   ATA ACA GAG GAA ACT CTC AAA GCT AAT AAA TTA GAC TCA AAG AAA TTA     432
   Ile Thr Glu Glu Thr Leu Lys Ala Asn Lys Leu Asp Ser Lys Lys Leu
       130                 135                 140
   ACA AGA TCA AAC GGA ACT ACA CTT GAA TAC TCA CAA ATA ACA GAT GCT     480
   Thr Arg Ser Asn Gly Thr Thr Leu Glu Tyr Ser Gln Ile Thr Asp Ala
   145                 150                 155                 160
   GAC AAT GCT ACA AAA GCA GTA GAA ACT CTA AAA AAT AGC ATT AAG CTT     528
   Asp Asn Ala Thr Lys Ala Val Glu Thr Leu Lys Asn Ser Ile Lys Leu
                   165                 170                 175
   GAA GGA AGT CTT GTA GTC GGA AAA ACA ACA GTG GAA ATT AAA GAA GGT        576
   Glu Gly Ser Leu Val Val Gly Lys Thr Thr Val Glu Ile Lys Glu Gly
               180                 185                 190
   ACT GTT ACT CTA AAA AGA GAA ATT GAA AAA GAT GGA AAA GTA AAA GTC        624
   Thr Val Thr Leu Lys Arg Glu Ile Glu Lys Asp Gly Lys Val Lys Val
           195                 200                 205
   TTT TTG AAT GAC ACT GCA GGT TCT AAC AAA AAA ACA GGT AAA TGG GAA        672
   Phe Leu Asn Asp Thr Ala Gly Ser Asn Lys Lys Thr Gly Lys Trp Glu
       210                 215                 220
   GAC AGT ACT AGC ACT TTA ACA ATT AGT GCT GAC AGC AAA AAA ACT AAA        720
   Asp Ser Thr Ser Thr Leu Thr Ile Ser Ala Asp Ser Lys Lys Thr Lys
   225                 230                 235                 240
   GAT TTG GTG TTC TTA ACA GAT GGT ACA ATT ACA GTA CAA CAA TAC AAC        768
   Asp Leu Val Phe Leu Thr Asp Gly Thr Ile Thr Val Gln Gln Tyr Asn
                   245                 250                 255
   ACA GCT GGA ACC AGC CTA GAA GGA TCA GCA AGT GAA ATT AAA AAT CTT        816
   Thr Ala Gly Thr Ser Leu Glu Gly Ser Ala Ser Glu Ile Lys Asn Leu
               260                 265                 270
   TCA GAG CTT AAA AAC GCT TTA AAA GGT CAC CCC ATG GCT CAA TAT AAC        864
   Ser Glu Leu Lys Asn Ala Leu Lys Gly His Pro Met Ala Gln Tyr Asn
           275                 280                 285
   CAA ATG CAC ATG TTA TCA AAC AAA TCT GCT TCT CAA AAT GTA AGA ACA        912
   Gln Met His Met Leu Ser Asn Lys Ser Ala Ser Gln Asn Val Arg Thr
       290                 295                 300
   GCT GAA GAG CTT GGA ATG CAG CCT GCA AAA ATT AAC ACA CCA GCA TCA        960
   Ala Glu Glu Leu Gly Met Gln Pro Ala Lys Ile Asn Thr Pro Ala Ser
   305                 310                 315                 320
   CTT TCA GGG CTT CAA GCG TCT TGG ACT TTA AGA GTT CAT GTT GGA GCA       1008
   Leu Ser Gly Leu Gln Ala Ser Trp Thr Leu Arg Val His Val Gly Ala
                   325                 330                 335
   ACC CAA GAT GAA GCT ATT GCT GTA AAT ATT TAT GCA GCT AAT GTT GCA       1056
   Thr Gln Asp Glu Ala Ile Ala Val Asn Ile Tyr Ala Ala Asn Val Ala
               340                 345                 350
   AAT CTT TTC TCT GGT GAG GGA GCT CAA ACT GCT CAG GCT GCA CCG GTT       1104
   Asn Leu Phe Ser Gly Glu Gly Ala Gln Thr Ala Gln Ala Ala Pro Val
           355                 360                 365
   CAA GAG GGT GTT CAA CAG GAA GGA GCT CAA CAG CCA GCA CCT GCT ACA       1152
   Gln Glu Gly Val Gln Gln Glu Gly Ala Gln Gln Pro Ala Pro Ala Thr
       370                 375                 380
   GCA CCT TCT CAA GGC GGA GTT GGT CAC C                                 1180
   Ala Pro Ser Gln Gly Gly Val Gly His
   385                 390
(2) INFORMATION FOR SEQ ID NO:114:
   (i) SEQUENCE CHARACTERISTICS:
      (A) LENGTH: 393 amino acids
      (B) TYPE: amino acid
      (D) TOPOLOGY: linear
   (ii) MOLECULE TYPE: protein
   (xi) SEQUENCE DESCRIPTION: SEQ ID NO:114:
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Gln Tyr Asn
           275                 280                 285
   Gln Met His Met Leu Ser Asn Lys Ser Ala Ser Gln Asn Val Arg Thr
       290                 295                 300
   Ala Glu Glu Leu Gly Met Gln Pro Ala Lys Ile Asn Thr Pro Ala Ser
   305                 310                 315                 320
   Leu Ser Gly Leu Gln Ala Ser Trp Thr Leu Arg Val His Val Gly Ala
                   325                 330                 335
   Thr Gln Asp Glu Ala Ile Ala Val Asn Ile Tyr Ala Ala Asn Val Ala
               340                 345                 350
   Asn Leu Phe Ser Gly Glu Gly Ala Gln Thr Ala Gln Ala Ala Pro Val
           355                 360                 365
   Gln Glu Gly Val Gln Gln Glu Gly Ala Gln Gln Pro Ala Pro Ala Thr
       370                 375                 380
   Ala Pro Ser Gln Gly Gly Val Gly His
   385                 390
(2) INFORMATION FOR SEQ ID NO:115:
   (i) SEQUENCE CHARACTERISTICS:
      (A) LENGTH: 1363 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1362
   (xi) SEQUENCE DESCRIPTION: SEQ ID NO:115:
   GCA CAA AAA GGT GCT GAG TCA ATT GGT TCT CAA AAA GAA AAT GAT CTA       48
   Ala Gln Lys Gly Ala Glu Ser Ile Gly Ser Gln Lys Glu Asn Asp Leu
     1               5                  10                  15
   AAC CTT GAA GAC TCT AGT AAA AAA TCA CAT CAA AAC GCT AAA CAA GAC       96
   Asn Leu Glu Asp Ser Ser Lys Lys Ser His Gln Asn Ala Lys Gln Asp
                20                  25                  30
   CTT CCT GCG GTG ACA GAA GAC TCA GTG TCT TTG TTT AAT GGT AAT AAA      144
   Leu Pro Ala Val Thr Glu Asp Ser Val Ser Leu Phe Asn Gly Asn Lys
            35                  40                  45
   ATT TTT GTA AGC AAA GAA AAA AAT AGC TCC GGC AAA TAT GAT TTA AGA      192
   Ile Phe Val Ser Lys Glu Lys Asn Ser Ser Gly Lys Tyr Asp Leu Arg
        50                  55                  60
   GCA ACA ATT GAT CAG GTT GAA CTT AAA GGA ACT TCC GAT AAA AAC AAT      240
   Ala Thr Ile Asp Gln Val Glu Leu Lys Gly Thr Ser Asp Lys Asn Asn
    65                  70                  75                  80
   GGT TCT GGA ACC CTT GAA GGT TCA AAG CCT GAC AAG AGT AAA GTA AAA      288
   Gly Ser Gly Thr Leu Glu Gly Ser Lys Pro Asp Lys Ser Lys Val Lys
                    85                  90                  95
   TTA ACA GTT TCT GCT GAT TTA AAC ACA GTA ACC TTA GAA GCA TTT GAT      336
   Leu Thr Val Ser Ala Asp Leu Asn Thr Val Thr Leu Glu Ala Phe Asp
               100                 105                 110
   GCC AGC AAC CAA AAA ATT TCA AGT AAA GTT ACT AAA AAA CAG GGG TCA      384
   Ala Ser Asn Gln Lys Ile Ser Ser Lys Val Thr Lys Lys Gln Gly Ser
           115                 120                 125
   ATA ACA GAG GAA ACT CTC AAA GCT AAT AAA TTA GAC TCA AAG AAA TTA      432
   Ile Thr Glu Glu Thr Leu Lys Ala Asn Lys Leu Asp Ser Lys Lys Leu
       130                 135                 140
   ACA AGA TCA AAC GGA ACT ACA CTT GAA TAC TCA CAA ATA ACA GAT GCT      480
   Thr Arg Ser Asn Gly Thr Thr Leu Glu Tyr Ser Gln Ile Thr Asp Ala
   145                 150                 155                 160
   GAC AAT GCT ACA AAA GCA GTA GAA ACT CTA AAA AAT AGC ATT AAG CTT      528
   Asp Asn Ala Thr Lys Ala Val Glu Thr Leu Lys Asn Ser Ile Lys Leu
                   165                 170                 175
   GAA GGA AGT CTT GTA GTC GGA AAA ACA ACA GTG GAA ATT AAA GAA GGT      576
   Glu Gly Ser Leu Val Val Gly Lys Thr Thr Val Glu Ile Lys Glu Gly
               180                 185                 190
   ACT GTT ACT CTA AAA AGA GAA ATT GAA AAA GAT GGA AAA GTA AAA GTC      624
   Thr Val Thr Leu Lys Arg Glu Ile Glu Lys Asp Gly Lys Val Lys Val
           195                 200                 205
   TTT TTG AAT GAC ACT GCA GGT TCT AAC AAA AAA ACA GGT AAA TGG GAA      672
   Phe Leu Asn Asp Thr Ala Gly Ser Asn Lys Lys Thr Gly Lys Trp Glu
       210                 215                 220
   GAC AGT ACT AGC ACT TTA ACA ATT AGT GCT GAC AGC AAA AAA ACT AAA      720
   Asp Ser Thr Ser Thr Leu Thr Ile Ser Ala Asp Ser Lys Lys Thr Lys
   225                 230                 235                 240
   GAT TTG GTG TTC TTA ACA GAT GGT ACA ATT ACA GTA CAA CAA TAC AAC      768
   Asp Leu Val Phe Leu Thr Asp Gly Thr Ile Thr Val Gln Gln Tyr Asn
                   245                 250                 255
   ACA GCT GGA ACC AGC CTA GAA GGA TCA GCA AGT GAA ATT AAA AAT CTT      816
   Thr Ala Gly Thr Ser Leu Glu Gly Ser Ala Ser Glu Ile Lys Asn Leu
               260                 265                 270
   TCA GAG CTT AAA AAC GCT TTA AAA GGT CAC CCC ATG GCT CAA TAT AAC      864
   Ser Glu Leu Lys Asn Ala Leu Lys Gly His Pro Met Ala Gln Tyr Asn
           275                 280                 285
   CAA ATG CAC ATG TTA TCA AAC AAA TCT GCT TCT CAA AAT GTA AGA ACA      912
   Gln Met His Met Leu Ser Asn Lys Ser Ala Ser Gln Asn Val Arg Thr
       290                 295                 300
   GCT GAA GAG CTT GGA ATG CAG CCT GCA AAA ATT AAC ACA CCA GCA TCA      960
   Ala Glu Glu Leu Gly Met Gln Pro Ala Lys Ile Asn Thr Pro Ala Ser
   305                 310                 315                 320
   CTT TCA GGG CTT CAA GCG TCT TGG ACT TTA AGA GTT CAT GTT GGA GCA     1008
   Leu Ser Gly Leu Gln Ala Ser Trp Thr Leu Arg Val His Val Gly Ala
                   325                 330                 335
   ACC CAA GAT GAA GCT ATT GCT GTA AAT ATT TAT GCA GCT AAT GTT GCA     1056
   Thr Gln Asp Glu Ala Ile Ala Val Asn Ile Tyr Ala Ala Asn Val Ala
               340                 345                 350
   AAT CTT TTC TCT GGT GAG GGA GCT CAA ACT GCT CAG GCT GCA CCG GTT      1104
   Asn Leu Phe Ser Gly Glu Gly Ala Gln Thr Ala Gln Ala Ala Pro Val
           355                 360                 365
   CAA GAG GGT GTT CAA CAG GAA GGA GCT CAA CAG CCA GCA CCT GCT ACA      1152
   Gln Glu Gly Val Gln Gln Glu Gly Ala Gln Gln Pro Ala Pro Ala Thr
       370                 375                 380
   GCA CCT TCT CAA GGC GGA GTT AAT TCT CCT GTT AAT GTT ACA ACT ACA      1200
   Ala Pro Ser Gln Gly Gly Val Asn Ser Pro Val Asn Val Thr Thr Thr
   385                 390                 395                 400
   GTT GAT GCT AAT ACA TCA CTT GCT AAA ATT GAA AAT GCT ATT AGA ATG      1248
   Val Asp Ala Asn Thr Ser Leu Ala Lys Ile Glu Asn Ala Ile Arg Met
                   405                 410                 415
   ATA AGT GAT CAA AGG GCA AAT TTA GGT GCT TTC CAA AAT AGA CTT GAA      1296
   Ile Ser Asp Gln Arg Ala Asn Leu Gly Ala Phe Gln Asn Arg Leu Glu
               420                 425                 430
   TCT ATA AAG AAT AGT ACT GAG TAT GCA ATT GAA AAT CTA AAA GCA TCT      1344
   Ser Ile Lys Asn Ser Thr Glu Tyr Ala Ile Glu Asn Leu Lys Ala Ser
           435                 440                 445
   TAT GCT CAA ATA GGT CAC C                                            1363
   Tyr Ala Gln Ile Gly His
       450
(2) INFORMATION FOR SEQ ID NO:116:
   (i) SEQUENCE CHARACTERISTICS:
      (A) LENGTH: 454 amino acids
      (B) TYPE: amino acid
      (D) TOPOLOGY: linear
   (ii) MOLECULE TYPE: protein
   (xi) SEQUENCE DESCRIPTION: SEQ ID NO:116:
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Gln Tyr Asn
           275                 280                 285
   Gln Met His Met Leu Ser Asn Lys Ser Ala Ser Gln Asn Val Arg Thr
       290                 295                 300
   Ala Glu Glu Leu Gly Met Gln Pro Ala Lys Ile Asn Thr Pro Ala Ser
   305                 310                 315                 320
   Leu Ser Gly Leu Gln Ala Ser Trp Thr Leu Arg Val His Val Gly Ala
                   325                 330                 335
   Thr Gln Asp Glu Ala Ile Ala Val Asn Ile Tyr Ala Ala Asn Val Ala
               340                 345                 350
   Asn Leu Phe Ser Gly Glu Gly Ala Gln Thr Ala Gln Ala Ala Pro Val
           355                 360                 365
   Gln Glu Gly Val Gln Gln Glu Gly Ala Gln Gln Pro Ala Pro Ala Thr
       370                 375                 380
   Ala Pro Ser Gln Gly Gly Val Asn Ser Pro Val Asn Val Thr Thr Thr
   385                 390                 395                 400
   Val Asp Ala Asn Thr Ser Leu Ala Lys Ile Glu Asn Ala Ile Arg Met
                   405                 410                 415
   Ile Ser Asp Gln Arg Ala Asn Leu Gly Ala Phe Gln Asn Arg Leu Glu
               420                 425                 430
   Ser Ile Lys Asn Ser Thr Glu Tyr Ala Ile Glu Asn Leu Lys Ala Ser
           435                 440                 445
   Tyr Ala Gln Ile Gly His
       450
(2) INFORMATION FOR SEQ ID NO:117:
   (i) SEQUENCE CHARACTERISTICS:
      (A) LENGTH: 1141 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1140
   (xi) SEQUENCE DESCRIPTION: SEQ ID NO:117:
   GCA CAA AAA GGT GCT GAG TCA ATT GGT TCT CAA AAA GAA AAT GAT CTA       48
   Ala Gln Lys Gly Ala Glu Ser Ile Gly Ser Gln Lys Glu Asn Asp Leu
     1               5                  10                  15
   AAC CTT GAA GAC TCT AGT AAA AAA TCA CAT CAA AAC GCT AAA CAA GAC       96
   Asn Leu Glu Asp Ser Ser Lys Lys Ser His Gln Asn Ala Lys Gln Asp
                20                  25                  30
   CTT CCT GCG GTG ACA GAA GAC TCA GTG TCT TTG TTT AAT GGT AAT AAA      144
   Leu Pro Ala Val Thr Glu Asp Ser Val Ser Leu Phe Asn Gly Asn Lys
            35                  40                  45
   ATT TTT GTA AGC AAA GAA AAA AAT AGC TCC GGC AAA TAT GAT TTA AGA      192
   Ile Phe Val Ser Lys Glu Lys Asn Ser Ser Gly Lys Tyr Asp Leu Arg
        50                  55                  60
   GCA ACA ATT GAT CAG GTT GAA CTT AAA GGA ACT TCC GAT AAA AAC AAT      240
   Ala Thr Ile Asp Gln Val Glu Leu Lys Gly Thr Ser Asp Lys Asn Asn
    65                  70                  75                  80
   GGT TCT GGA ACC CTT GAA GGT TCA AAG CCT GAC AAG AGT AAA GTA AAA      288
   Gly Ser Gly Thr Leu Glu Gly Ser Lys Pro Asp Lys Ser Lys Val Lys
                    85                  90                  95
   TTA ACA GTT TCT GCT GAT TTA AAC ACA GTA ACC TTA GAA GCA TTT GAT      336
   Leu Thr Val Ser Ala Asp Leu Asn Thr Val Thr Leu Glu Ala Phe Asp
               100                 105                 110
   GCC AGC AAC CAA AAA ATT TCA AGT AAA GTT ACT AAA AAA CAG GGG TCA      384
   Ala Ser Asn Gln Lys Ile Ser Ser Lys Val Thr Lys Lys Gln Gly Ser
           115                 120                 125
   ATA ACA GAG GAA ACT CTC AAA GCT AAT AAA TTA GAC TCA AAG AAA TTA      432
   Ile Thr Glu Glu Thr Leu Lys Ala Asn Lys Leu Asp Ser Lys Lys Leu
       130                 135                 140
   ACA AGA TCA AAC GGA ACT ACA CTT GAA TAC TCA CAA ATA ACA GAT GCT      480
   Thr Arg Ser Asn Gly Thr Thr Leu Glu Tyr Ser Gln Ile Thr Asp Ala
   145                 150                 155                 160
   GAC AAT GCT ACA AAA GCA GTA GAA ACT CTA AAA AAT AGC ATT AAG CTT      528
   Asp Asn Ala Thr Lys Ala Val Glu Thr Leu Lys Asn Ser Ile Lys Leu
                   165                 170                 175
   GAA GGA AGT CTT GTA GTC GGA AAA ACA ACA GTG GAA ATT AAA GAA GGT        576
   Glu Gly Ser Leu Val Val Gly Lys Thr Thr Val Glu Ile Lys Glu Gly
               180                 185                 190
   ACT GTT ACT CTA AAA AGA GAA ATT GAA AAA GAT GGA AAA GTA AAA GTC        624
   Thr Val Thr Leu Lys Arg Glu Ile Glu Lys Asp Gly Lys Val Lys Val
           195                 200                 205
   TTT TTG AAT GAC ACT GCA GGT TCT AAC AAA AAA ACA GGT AAA TGG GAA        672
   Phe Leu Asn Asp Thr Ala Gly Ser Asn Lys Lys Thr Gly Lys Trp Glu
       210                 215                 220
   GAC AGT ACT AGC ACT TTA ACA ATT AGT GCT GAC AGC AAA AAA ACT AAA        720
   Asp Ser Thr Ser Thr Leu Thr Ile Ser Ala Asp Ser Lys Lys Thr Lys
   225                 230                 235                 240
   GAT TTG GTG TTC TTA ACA GAT GGT ACA ATT ACA GTA CAA CAA TAC AAC        768
   Asp Leu Val Phe Leu Thr Asp Gly Thr Ile Thr Val Gln Gln Tyr Asn
                   245                 250                 255
   ACA GCT GGA ACC AGC CTA GAA GGA TCA GCA AGT GAA ATT AAA AAT CTT        816
   Thr Ala Gly Thr Ser Leu Glu Gly Ser Ala Ser Glu Ile Lys Asn Leu
               260                 265                 270
   TCA GAG CTT AAA AAC GCT TTA AAA GGT CAC CCC ATG GCT TCT CAA AAT        864
   Ser Glu Leu Lys Asn Ala Leu Lys Gly His Pro Met Ala Ser Gln Asn
           275                 280                 285
   GTA AGA ACA GCT GAA GAG CTT GGA ATG CAG CCT GCA AAA ATT AAC ACA        912
   Val Arg Thr Ala Glu Glu Leu Gly Met Gln Pro Ala Lys Ile Asn Thr
       290                 295                 300
   CCA GCA TCA CTT TCA GGG CTT CAA GCG TCT TGG ACT TTA AGA GTT CAT        960
   Pro Ala Ser Leu Ser Gly Leu Gln Ala Ser Trp Thr Leu Arg Val His
   305                 310                 315                 320
   GTT GGA GCA ACC CAA GAT GAA GCT ATT GCT GTA AAT ATT TAT GCA GCT       1008
   Val Gly Ala Thr Gln Asp Glu Ala Ile Ala Val Asn Ile Tyr Ala Ala
                   325                 330                 335
   AAT GTT GCA AAT CTT TTC TCT GGT GAG GGA GCT CAA ACT GCT CAG GCT       1056
   Asn Val Ala Asn Leu Phe Ser Gly Glu Gly Ala Gln Thr Ala Gln Ala
               340                 345                 350
   GCA CCG GTT CAA GAG GGT GTT CAA CAG GAA GGA GCT CAA CAG CCA GCA       1104
   Ala Pro Val Gln Glu Gly Val Gln Gln Glu Gly Ala Gln Gln Pro Ala
           355                 360                 365
   CCT GCT ACA GCA CCT TCT CAA GGC GGA GTT GGT CAC C                     1141
   Pro Ala Thr Ala Pro Ser Gln Gly Gly Val Gly His
       370                 375                 380
(2) INFORMATION FOR SEQ ID NO:118:
   (i) SEQUENCE CHARACTERISTICS:
      (A) LENGTH: 380 amino acids
      (B) TYPE: amino acid
      (D) TOPOLOGY: linear
   (ii) MOLECULE TYPE: protein
   (xi) SEQUENCE DESCRIPTION: SEQ ID NO:118:
   Ala Gln Lys Gly Ala Glu Ser Ile Gly Ser Gln Lys Glu Asn Asp Leu
    1               5                  10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Ser Gln Asn
           275                 280                 285
   Val Arg Thr Ala Glu Glu Leu Gly Met Gln Pro Ala Lys Ile Asn Thr
       290                 295                 300
   Pro Ala Ser Leu Ser Gly Leu Gln Ala Ser Trp Thr Leu Arg Val His
   305                 310                 315                 320
   Val Gly Ala Thr Gln Asp Glu Ala Ile Ala Val Asn Ile Tyr Ala Ala
                   325                 330                 335
   Asn Val Ala Asn Leu Phe Ser Gly Glu Gly Ala Gln Thr Ala Gln Ala
               340                 345                 350
   Ala Pro Val Gln Glu Gly Val Gln Gln Glu Gly Ala Gln Gln Pro Ala
           355                 360                 365
   Pro Ala Thr Ala Pro Ser Gln Gly Gly Val Gly His
       370                 375                 380
(2) INFORMATION FOR SEQ ID NO:119:
   (i) SEQUENCE CHARACTERISTICS:
      (A) LENGTH: 1324 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1323
   (xi) SEQUENCE DESCRIPTION: SEQ ID NO:119:
   GCA CAA AAA GGT GCT GAG TCA ATT GGT TCT CAA AAA GAA AAT GAT CTA      48
   Ala Gln Lys Gly Ala Glu Ser Ile Gly Ser Gln Lys Glu Asn Asp Leu
     1               5                  10                  15
   AAC CTT GAA GAC TCT AGT AAA AAA TCA CAT CAA AAC GCT AAA CAA GAC      96
   Asn Leu Glu Asp Ser Ser Lys Lys Ser His Gln Asn Ala Lys Gln Asp
                20                  25                  30
   CTT CCT GCG GTG ACA GAA GAC TCA GTG TCT TTG TTT AAT GGT AAT AAA     144
   Leu Pro Ala Val Thr Glu Asp Ser Val Ser Leu Phe Asn Gly Asn Lys
            35                  40                  45
   ATT TTT GTA AGC AAA GAA AAA AAT AGC TCC GGC AAA TAT GAT TTA AGA     192
   Ile Phe Val Ser Lys Glu Lys Asn Ser Ser Gly Lys Tyr Asp Leu Arg
        50                  55                  60
   GCA ACA ATT GAT CAG GTT GAA CTT AAA GGA ACT TCC GAT AAA AAC AAT     240
   Ala Thr Ile Asp Gln Val Glu Leu Lys Gly Thr Ser Asp Lys Asn Asn
    65                  70                  75                  80
   GGT TCT GGA ACC CTT GAA GGT TCA AAG CCT GAC AAG AGT AAA GTA AAA     288
   Gly Ser Gly Thr Leu Glu Gly Ser Lys Pro Asp Lys Ser Lys Val Lys
                    85                  90                  95
   TTA ACA GTT TCT GCT GAT TTA AAC ACA GTA ACC TTA GAA GCA TTT GAT     336
   Leu Thr Val Ser Ala Asp Leu Asn Thr Val Thr Leu Glu Ala Phe Asp
               100                 105                 110
   GCC AGC AAC CAA AAA ATT TCA AGT AAA GTT ACT AAA AAA CAG GGG TCA     384
   Ala Ser Asn Gln Lys Ile Ser Ser Lys Val Thr Lys Lys Gln Gly Ser
           115                 120                 125
   ATA ACA GAG GAA ACT CTC AAA GCT AAT AAA TTA GAC TCA AAG AAA TTA        432
   Ile Thr Glu Glu Thr Leu Lys Ala Asn Lys Leu Asp Ser Lys Lys Leu
       130                 135                 140
   ACA AGA TCA AAC GGA ACT ACA CTT GAA TAC TCA CAA ATA ACA GAT GCT        480
   Thr Arg Ser Asn Gly Thr Thr Leu Glu Tyr Ser Gln Ile Thr Asp Ala
   145                 150                 155                 160
   GAC AAT GCT ACA AAA GCA GTA GAA ACT CTA AAA AAT AGC ATT AAG CTT        528
   Asp Asn Ala Thr Lys Ala Val Glu Thr Leu Lys Asn Ser Ile Lys Leu
                   165                 170                 175
   GAA GGA AGT CTT GTA GTC GGA AAA ACA ACA GTG GAA ATT AAA GAA GGT        576
   Glu Gly Ser Leu Val Val Gly Lys Thr Thr Val Glu Ile Lys Glu Gly
               180                 185                 190
   ACT GTT ACT CTA AAA AGA GAA ATT GAA AAA GAT GGA AAA GTA AAA GTC        624
   Thr Val Thr Leu Lys Arg Glu Ile Glu Lys Asp Gly Lys Val Lys Val
           195                 200                 205
   TTT TTG AAT GAC ACT GCA GGT TCT AAC AAA AAA ACA GGT AAA TGG GAA        672
   Phe Leu Asn Asp Thr Ala Gly Ser Asn Lys Lys Thr Gly Lys Trp Glu
       210                 215                 220
   GAC AGT ACT AGC ACT TTA ACA ATT AGT GCT GAC AGC AAA AAA ACT AAA        720
   Asp Ser Thr Ser Thr Leu Thr Ile Ser Ala Asp Ser Lys Lys Thr Lys
   225                 230                 235                 240
   GAT TTG GTG TTC TTA ACA GAT GGT ACA ATT ACA GTA CAA CAA TAC AAC        768
   Asp Leu Val Phe Leu Thr Asp Gly Thr Ile Thr Val Gln Gln Tyr Asn
                   245                 250                 255
   ACA GCT GGA ACC AGC CTA GAA GGA TCA GCA AGT GAA ATT AAA AAT CTT        816
   Thr Ala Gly Thr Ser Leu Glu Gly Ser Ala Ser Glu Ile Lys Asn Leu
               260                 265                 270
   TCA GAG CTT AAA AAC GCT TTA AAA GGT CAC CCC ATG GCT TCT CAA AAT        864
   Ser Glu Leu Lys Asn Ala Leu Lys Gly His Pro Met Ala Ser Gln Asn
           275                 280                 285
   GTA AGA ACA GCT GAA GAG CTT GGA ATG CAG CCT GCA AAA ATT AAC ACA        912
   Val Arg Thr Ala Glu Glu Leu Gly Met Gln Pro Ala Lys Ile Asn Thr
       290                 295                 300
   CCA GCA TCA CTT TCA GGG CTT CAA GCG TCT TGG ACT TTA AGA GTT CAT        960
   Pro Ala Ser Leu Ser Gly Leu Gln Ala Ser Trp Thr Leu Arg Val His
   305                 310                 315                 320
   GTT GGA GCA ACC CAA GAT GAA GCT ATT GCT GTA AAT ATT TAT GCA GCT       1008
   Val Gly Ala Thr Gln Asp Glu Ala Ile Ala Val Asn Ile Tyr Ala Ala
                   325                 330                 335
   AAT GTT GCA AAT CTT TTC TCT GGT GAG GGA GCT CAA ACT GCT CAG GCT       1056
   Asn Val Ala Asn Leu Phe Ser Gly Glu Gly Ala Gln Thr Ala Gln Ala
               340                 345                 350
   GCA CCG GTT CAA GAG GGT GTT CAA CAG GAA GGA GCT CAA CAG CCA GCA       1104
   Ala Pro Val Gln Glu Gly Val Gln Gln Glu Gly Ala Gln Gln Pro Ala
           355                 360                 365
   CCT GCT ACA GCA CCT TCT CAA GGC GGA GTT AAT TCT CCT GTT AAT GTT        1152
   Pro Ala Thr Ala Pro Ser Gln Gly Gly Val Asn Ser Pro Val Asn Val
       370                 375                 380
   ACA ACT ACA GTT GAT GCT AAT ACA TCA CTT GCT AAA ATT GAA AAT GCT        1200
   Thr Thr Thr Val Asp Ala Asn Thr Ser Leu Ala Lys Ile Glu Asn Ala
   385                 390                 395                 400
   ATT AGA ATG ATA AGT GAT CAA AGG GCA AAT TTA GGT GCT TTC CAA AAT        1248
   Ile Arg Met Ile Ser Asp Gln Arg Ala Asn Leu Gly Ala Phe Gln Asn
                   405                 410                 415
   AGA CTT GAA TCT ATA AAG AAT AGT ACT GAG TAT GCA ATT GAA AAT CTA        1296
   Arg Leu Glu Ser Ile Lys Asn Ser Thr Glu Tyr Ala Ile Glu Asn Leu
               420                 425                 430
   AAA GCA TCT TAT GCT CAA ATA GGT CAC C                                  1324
   Lys Ala Ser Tyr Ala Gln Ile Gly His
           435                 440
(2) INFORMATION FOR SEQ ID NO:120:
   (i) SEQUENCE CHARACTERISTICS:
      (A) LENGTH: 441 amino acids
      (B) TYPE: amino acid
      (D) TOPOLOGY: linear
   (ii) MOLECULE TYPE: protein
   (xi) SEQUENCE DESCRIPTION: SEQ ID NO:120:
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
               100                 105                 110
   Ala Ser Asn Gln Lys Ile Ser Ser Lys Val Thr Lys Lys Gln Gly Ser
           115                 120                 125
   Ile Thr Glu Glu Thr Leu Lys Ala Asn Lys Leu Asp Ser Lys Lys Leu
       130                 135                 140
   Thr Arg Ser Asn Gly Thr Thr Leu Glu Tyr Ser Gln Ile Thr Asp Ala
   145                 150                 155                 160
   Asp Asn Ala Thr Lys Ala Val Glu Thr Leu Lys Asn Ser Ile Lys Leu
                   165                 170                 175
   Glu Gly Ser Leu Val Val Gly Lys Thr Thr Val Glu Ile Lys Glu Gly
               180                 185                 190
   Thr Val Thr Leu Lys Arg Glu Ile Glu Lys Asp Gly Lys Val Lys Val
           195                 200                 205
   Phe Leu Asn Asp Thr Ala Gly Ser Asn Lys Lys Thr Gly Lys Trp Glu
       210                 215                 220
   Asp Ser Thr Ser Thr Leu Thr Ile Ser Ala Asp Ser Lys Lys Thr Lys
   225                 230                 235                 240
   Asp Leu Val Phe Leu Thr Asp Gly Thr Ile Thr Val Gln Gln Tyr Asn
                   245                 250                 255
   Thr Ala Gly Thr Ser Leu Glu Gly Ser Ala Ser Glu Ile Lys Asn Leu
               260                 265                 270
   Ser Glu Leu Lys Asn Ala Leu Lys Gly His Pro Met Ala Ser Gln Asn
           275                 280                 285
   Val Arg Thr Ala Glu Glu Leu Gly Met Gln Pro Ala Lys Ile Asn Thr
       290                 295                 300
   Pro Ala Ser Leu Ser Gly Leu Gln Ala Ser Trp Thr Leu Arg Val His
   305                 310                 315                 320
   Val Gly Ala Thr Gln Asp Glu Ala Ile Ala Val Asn Ile Tyr Ala Ala
                   325                 330                 335
   Asn Val Ala Asn Leu Phe Ser Gly Glu Gly Ala Gln Thr Ala Gln Ala
               340                 345                 350
   Ala Pro Val Gln Glu Gly Val Gln Gln Glu Gly Ala Gln Gln Pro Ala
           355                 360                 365
   Pro Ala Thr Ala Pro Ser Gln Gly Gly Val Asn Ser Pro Val Asn Val
       370                 375                 380
   Thr Thr Thr Val Asp Ala Asn Thr Ser Leu Ala Lys Ile Glu Asn Ala
   385                 390                 395                 400
   Ile Arg Met Ile Ser Asp Gln Arg Ala Asn Leu Gly Ala Phe Gln Asn
                   405                 410                 415
   Arg Leu Glu Ser Ile Lys Asn Ser Thr Glu Tyr Ala Ile Glu Asn Leu
               420                 425                 430
   Lys Ala Ser Tyr Ala Gln Ile Gly His
           435                 440
(2) INFORMATION FOR SEQ ID NO:121:
   (i) SEQUENCE CHARACTERISTICS:
      (A) LENGTH: 1765 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1764
   (xi) SEQUENCE DESCRIPTION: SEQ ID NO:121:
   GCA CAA AAA GGT GCT GAG TCA ATT GGT TCT CAA AAA GAA AAT GAT CTA      48
   Ala Gln Lys Gly Ala Glu Ser Ile Gly Ser Gln Lys Glu Asn Asp Leu
     1               5                  10                  15
   AAC CTT GAA GAC TCT AGT AAA AAA TCA CAT CAA AAC GCT AAA CAA GAC      96
   Asn Leu Glu Asp Ser Ser Lys Lys Ser His Gln Asn Ala Lys Gln Asp
                20                  25                  30
   CTT CCT GCG GTG ACA GAA GAC TCA GTG TCT TTG TTT AAT GGT AAT AAA     144
   Leu Pro Ala Val Thr Glu Asp Ser Val Ser Leu Phe Asn Gly Asn Lys
            35                  40                  45
   ATT TTT GTA AGC AAA GAA AAA AAT AGC TCC GGC AAA TAT GAT TTA AGA     192
   Ile Phe Val Ser Lys Glu Lys Asn Ser Ser Gly Lys Tyr Asp Leu Arg
        50                  55                  60
   GCA ACA ATT GAT CAG GTT GAA CTT AAA GGA ACT TCC GAT AAA AAC AAT     240
   Ala Thr Ile Asp Gln Val Glu Leu Lys Gly Thr Ser Asp Lys Asn Asn
    65                  70                  75                  80
   GGT TCT GGA ACC CTT GAA GGT TCA AAG CCT GAC AAG AGT AAA GTA AAA     288
   Gly Ser Gly Thr Leu Glu Gly Ser Lys Pro Asp Lys Ser Lys Val Lys
                    85                  90                  95
   TTA ACA GTT TCT GCT GAT TTA AAC ACA GTA ACC TTA GAA GCA TTT GAT     336
   Leu Thr Val Ser Ala Asp Leu Asn Thr Val Thr Leu Glu Ala Phe Asp
               100                 105                 110
   GCC AGC AAC CAA AAA ATT TCA AGT AAA GTT ACT AAA AAA CAG GGG TCA     384
   Ala Ser Asn Gln Lys Ile Ser Ser Lys Val Thr Lys Lys Gln Gly Ser
           115                 120                 125
   ATA ACA GAG GAA ACT CTC AAA GCT AAT AAA TTA GAC TCA AAG AAA TTA     432
   Ile Thr Glu Glu Thr Leu Lys Ala Asn Lys Leu Asp Ser Lys Lys Leu
       130                 135                 140
   ACA AGA TCA AAC GGA ACT ACA CTT GAA TAC TCA CAA ATA ACA GAT GCT     480
   Thr Arg Ser Asn Gly Thr Thr Leu Glu Tyr Ser Gln Ile Thr Asp Ala
   145                 150                 155                 160
   GAC AAT GCT ACA AAA GCA GTA GAA ACT CTA AAA AAT AGC ATT AAG CTT     528
   Asp Asn Ala Thr Lys Ala Val Glu Thr Leu Lys Asn Ser Ile Lys Leu
                   165                 170                 175
   GAA GGA AGT CTT GTA GTC GGA AAA ACA ACA GTG GAA ATT AAA GAA GGT     576
   Glu Gly Ser Leu Val Val Gly Lys Thr Thr Val Glu Ile Lys Glu Gly
               180                 185                 190
   ACT GTT ACT CTA AAA AGA GAA ATT GAA AAA GAT GGA AAA GTA AAA GTC     624
   Thr Val Thr Leu Lys Arg Glu Ile Glu Lys Asp Gly Lys Val Lys Val
           195                 200                 205
   TTT TTG AAT GAC ACT GCA GGT TCT AAC AAA AAA ACA GGT AAA TGG GAA     672
   Phe Leu Asn Asp Thr Ala Gly Ser Asn Lys Lys Thr Gly Lys Trp Glu
       210                 215                 220
   GAC AGT ACT AGC ACT TTA ACA ATT AGT GCT GAC AGC AAA AAA ACT AAA      720
   Asp Ser Thr Ser Thr Leu Thr Ile Ser Ala Asp Ser Lys Lys Thr Lys
   225                 230                 235                 240
   GAT TTG GTG TTC TTA ACA GAT GGT ACA ATT ACA GTA CAA CAA TAC AAC      768
   Asp Leu Val Phe Leu Thr Asp Gly Thr Ile Thr Val Gln Gln Tyr Asn
                   245                 250                 255
   ACA GCT GGA ACC AGC CTA GAA GGA TCA GCA AGT GAA ATT AAA AAT CTT      816
   Thr Ala Gly Thr Ser Leu Glu Gly Ser Ala Ser Glu Ile Lys Asn Leu
               260                 265                 270
   TCA GAG CTT AAA AAC GCT TTA AAA GGT CAC CCC ATG GGA AAT AAT TCA      864
   Ser Glu Leu Lys Asn Ala Leu Lys Gly His Pro Met Gly Asn Asn Ser
           275                 280                 285
   GGG AAA GAT GGG AAT ACA TCT GCA AAT TCT GCT GAT GAG TCT GTT AAA      912
   Gly Lys Asp Gly Asn Thr Ser Ala Asn Ser Ala Asp Glu Ser Val Lys
       290                 295                 300
   GGG CCT AAT CTT ACA GAA ATA AGT AAA AAA ATT ACG GAT TCT AAT GCG      960
   Gly Pro Asn Leu Thr Glu Ile Ser Lys Lys Ile Thr Asp Ser Asn Ala
   305                 310                 315                 320
   GTT TTA CTT GCT GTG AAA GAG GTT GAA GCG TTG CTG TCA TCT ATA GAT     1008
   Val Leu Leu Ala Val Lys Glu Val Glu Ala Leu Leu Ser Ser Ile Asp
                   325                 330                 335
   GAA ATT GCT GCT AAA GCT ATT GGT AAA AAA ATA CAC CAA AAT AAT GGT     1056
   Glu Ile Ala Ala Lys Ala Ile Gly Lys Lys Ile His Gln Asn Asn Gly
               340                 345                 350
   TTG GAT ACC GAA TAT AAT CAC AAT GGA TCA TTG TTA GCG GGA CGT TAT     1104
   Leu Asp Thr Glu Tyr Asn His Asn Gly Ser Leu Leu Ala Gly Arg Tyr
           355                 360                 365
   GCA ATA TCA ACC CTA ATA AAA CAA AAA TTA GAT GGA TTG AAA AAT GAA     1152
   Ala Ile Ser Thr Leu Ile Lys Gln Lys Leu Asp Gly Leu Lys Asn Glu
       370                 375                 380
   GGA TTA AAG GAA AAA ATT GAT GCG GCT AAG AAA TGT TCT GAA ACA TTT     1200
   Gly Leu Lys Glu Lys Ile Asp Ala Ala Lys Lys Cys Ser Glu Thr Phe
   385                 390                 395                 400
   ACT AAT AAA TTA AAA GAA AAA CAC ACA GAT CTT GGT AAA GAA GGT GTT     1248
   Thr Asn Lys Leu Lys Glu Lys His Thr Asp Leu Gly Lys Glu Gly Val
                   405                 410                 415
   ACT GAT GCT GAT GCA AAA GAA GCC ATT TTA AAA ACA AAT GGT ACT AAA     1296
   Thr Asp Ala Asp Ala Lys Glu Ala Ile Leu Lys Thr Asn Gly Thr Lys
               420                 425                 430
   ACT AAA GGT GCT GAA GAA CTT GGA AAA TTA TTT GAA TCA GTA GAG GTC     1344
   Thr Lys Gly Ala Glu Glu Leu Gly Lys Leu Phe Glu Ser Val Glu Val
           435                 440                 445
   TTG TCA AAA GCA GCT AAA GAG ATG CTT GCT AAT TCA GTT AAA GAG CTT     1392
   Leu Ser Lys Ala Ala Lys Glu Met Leu Ala Asn Ser Val Lys Glu Leu
       450                 455                 460
   ACA AGC CCT GTT GTG GCA GAA AGT CCA AAA AAA CCT GGT ACC ATG GCT      1440
   Thr Ser Pro Val Val Ala Glu Ser Pro Lys Lys Pro Gly Thr Met Ala
   465                 470                 475                 480
   CAA TAT AAC CAA ATG CAC ATG TTA TCA AAC AAA TCT GCT TCT CAA AAT      1488
   Gln Tyr Asn Gln Met His Met Leu Ser Asn Lys Ser Ala Ser Gln Asn
                   485                 490                 495
   GTA AGA ACA GCT GAA GAG CTT GGA ATG CAG CCT GCA AAA ATT AAC ACA      1536
   Val Arg Thr Ala Glu Glu Leu Gly Met Gln Pro Ala Lys Ile Asn Thr
               500                 505                 510
   CCA GCA TCA CTT TCA GGG CTT CAA GCG TCT TGG ACT TTA AGA GTT CAT      1584
   Pro Ala Ser Leu Ser Gly Leu Gln Ala Ser Trp Thr Leu Arg Val His
           515                 520                 525
   GTT GGA GCA ACC CAA GAT GAA GCT ATT GCT GTA AAT ATT TAT GCA GCT      1632
   Val Gly Ala Thr Gln Asp Glu Ala Ile Ala Val Asn Ile Tyr Ala Ala
       530                 535                 540
   AAT GTT GCA AAT CTT TTC TCT GGT GAG GGA GCT CAA ACT GCT CAG GCT      1680
   Asn Val Ala Asn Leu Phe Ser Gly Glu Gly Ala Gln Thr Ala Gln Ala
   545                 550                 555                 560
   GCA CCG GTT CAA GAG GGT GTT CAA CAG GAA GGA GCT CAA CAG CCA GCA      1728
   Ala Pro Val Gln Glu Gly Val Gln Gln Glu Gly Ala Gln Gln Pro Ala
                   565                 570                 575
   CCT GCT ACA GCA CCT TCT CAA GGC GGA GTT GGT CAC C                    1765
   Pro Ala Thr Ala Pro Ser Gln Gly Gly Val Gly His
               580                 585
(2) INFORMATION FOR SEQ ID NO:122:
   (i) SEQUENCE CHARACTERISTICS:
      (A) LENGTH: 588 amino acids
      (B) TYPE: amino acid
      (D) TOPOLOGY: linear
   (ii) MOLECULE TYPE: protein
   (xi) SEQUENCE DESCRIPTION: SEQ ID NO:122:
   Ala Gln Lys Gly Ala Glu Ser Ile Gly Ser Gln Lys Glu Asn Asp Leu
   1               5                   10                  15
   Asn Leu Glu Asp Ser Ser Lys Lys Ser His Gln Asn Ala Lys Gln Asp
               20                  25                  30
   Leu Pro Ala Val Thr Glu Asp Ser Val Ser Leu Phe Asn Gly Asn Lys
           35                  40                  45
   Ile Phe Val Ser Lys Glu Lys Asn Ser Ser Gly Lys Tyr Asp Leu Arg
       50                  55                  60
   Ala Thr Ile Asp Gln Val Glu Leu Lys Gly Thr Ser Asp Lys Asn Asn
   65                  70                  75                  80
   Gly Ser Gly Thr Leu Glu Gly Ser Lys Pro Asp Lys Ser Lys Val Lys
                   85                  90                  95
   Leu Thr Val Ser Ala Asp Leu Asn Thr Val Thr Leu Glu Ala Phe Asp
   100 105 110
   Ala Ser Asn Gln Lys Ile Ser Ser Lys Val Thr Lys Lys Gln Gly Ser
   115 120 125
   Ile Thr Glu Glu Thr Leu Lys Ala Asn Lys Leu Asp Ser Lys Lys Leu
   130 135 140
   Thr Arg Ser Asn Gly Thr Thr Leu Glu Tyr Ser Gln Ile Thr Asp Ala
   145 150 155 160
   Asp Asn Ala Thr Lys Ala Val Glu Thr Leu Lys Asn Ser Ile Lys Leu
   165 170 175
   Glu Gly Ser Leu Val Val Gly Lys Thr Thr Val Glu Ile Lys Glu Gly
   180 185 190
   Thr Val Thr Leu Lys Arg Glu Ile Glu Lys Asp Gly Lys Val Lys Val
   195 200 205
   Phe Leu Asn Asp Thr Ala Gly Ser Asn Lys Lys Thr Gly Lys Trp Glu
   210 215 220
   Asp Ser Thr Ser Thr Leu Thr Ile Ser Ala Asp Ser Lys Lys Thr Lys
   225 230 235 240
   Asp Leu Val Phe Leu Thr Asp Gly Thr Ile Thr Val Gln Gln Tyr Asn
   245 250 255
   Thr Ala Gly Thr Ser Leu Glu Gly Ser Ala Ser Glu Ile Lys Asn Leu
   260 265 270
   Ser Glu Leu Lys Asn Ala Leu Lys Gly His Pro Met Gly Asn Asn Ser
   275 280 285
   Gly Lys Asp Gly Asn Thr Ser Ala Asn Ser Ala Asp Glu Ser Val Lys
   290 295 300
   Gly Pro Asn Leu Thr Glu Ile Ser Lys Lys Ile Thr Asp Ser Asn Ala
   305 310 315 320
   Val Leu Leu Ala Val Lys Glu Val Glu Ala Leu Leu Ser Ser Ile Asp
   325 330 335
   Glu Ile Ala Ala Lys Ala Ile Gly Lys Lys Ile His Gln Asn Asn Gly
   340 345 350
   Leu Asp Thr Glu Tyr Asn His Asn Gly Ser Leu Leu Ala Gly Arg Tyr
   355 360 365
   Ala Ile Ser Thr Leu Ile Lys Gln Lys Leu Asp Gly Leu Lys Asn Glu
   370 375 380
   Gly Leu Lys Glu Lys Ile Asp Ala Ala Lys Lys Cys Ser Glu Thr Phe
   385 390 395 400
   Thr Asn Lys Leu Lys Glu Lys His Thr Asp Leu Gly Lys Glu Gly Val
   405 410 415
   Thr Asp Ala Asp Ala Lys Glu Ala Ile Leu Lys Thr Asn Gly Thr Lys
   420 425 430
   Thr Lys Gly Ala Glu Glu Leu Gly Lys Leu Phe Glu Ser Val Glu Val
           435                 440                 445
   Leu Ser Lys Ala Ala Lys Glu Met Leu Ala Asn Ser Val Lys Glu Leu
       450                 455                 460
   Thr Ser Pro Val Val Ala Glu Ser Pro Lys Lys Pro Gly Thr Met Ala
   465                 470                 475                 480
   Gln Tyr Asn Gln Met His Met Leu Ser Asn Lys Ser Ala Ser Gln Asn
                   485                 490                 495
   Val Arg Thr Ala Glu Glu Leu Gly Met Gln Pro Ala Lys Ile Asn Thr
               500                 505                 510
   Pro Ala Ser Leu Ser Gly Leu Gln Ala Ser Trp Thr Leu Arg Val His
           515                 520                 525
   Val Gly Ala Thr Gln Asp Glu Ala Ile Ala Val Asn Ile Tyr Ala Ala
       530                 535                 540
   Asn Val Ala Asn Leu Phe Ser Gly Glu Gly Ala Gln Thr Ala Gln Ala
   545                 550                 555                 560
   Ala Pro Val Gln Glu Gly Val Gln Gln Glu Gly Ala Gln Gln Pro Ala
                   565                 570                 575
   Pro Ala Thr Ala Pro Ser Gln Gly Gly Val Gly His
               580                 585
(2) INFORMATION FOR SEQ ID NO:123:
   (i) SEQUENCE CHARACTERISTICS:
      (A) LENGTH: 704 base pairs
      (B) TYPE: nucleic acid
      (C) STRANDEDNESS: double
      (D) TOPOLOGY: linear
   (xi) SEQUENCE DESCRIPTION: SEQ ID NO:123:
   CTACTGTTAA GTTTATTTTT ATTGCTCTCA ATATCTTGTT CTTTAGATAA TGAAGGTGTA      60
   AACTCAAAAG ATTACGAGTC AAAAAAACAG AGTATACTAG GTGAATTAAA TCAGCTATTG     120
   GGGCAAACTA CAAATTCACT AAAAGAAGCA AAAAATACAA CAGATAATTT AAATGCATCA     180
   AATGAGGCAA ATAAAGTTGT AGAAGCAGTT ATAAGTGTGG TTAATTTAAT TTCATCTGCT     240
   GCAGATCAGG TAAAAGGTCA ACAACAAATA TGCACGATTT AGCTCAAATG GCAGAAATAG     300
   ATTTAGAAAA AATAAAGGAA TCTAGTGATA AAGTAATAGT TGCGGCTAAT GTTGCGAAAG     360
   AAGCATATAA CCTTACTAAA GCAGTAGAAC AAAATATGCA AAAACTGTAC AAAGAGCAAG     420
   AAGAGCAACT AAAACACTAT CTGATTCTGA TGAAACAGAA CGAGTTTCTG ATGAAATAAA     480
   ACAAGCTAAA GAGGCTGTAG AAATAGCTTG GAAAGCCACA GTAAAAGTAA AAGATGAGTT     540
   AATTGATGTA GAAAATGCAG TCAAAGAGGC ATTGGATAAA ATAAAGACAG AAACCGCGAA     600
   CAATACAAAA CTTACAGATA TAGAAGAAGT AGCAGAGTTA GTATTACAGA TAGCCAAAAA 660
   TGTAGCGGAA ATAGCGCAAG AAGTTGTGGC CTTGTTAAAT ACTT 704
   (i) SEQUENCE CHARACTERISTICS:
      (A) LENGTH: 704 base pairs
      (B) TYPE: nucleic acid
      (C) STRANDEDNESS: double
      (D) TOPOLOGY: linear
   (xi) SEQUENCE DESCRIPTION: SEQ ID NO:124:
(2) INFORMATION FOR SEQ ID NO:125:
   (i) SEQUENCE CHARACTERISTICS:
      (A) LENGTH: 704 base pairs
      (B) TYPE: nucleic acid
      (C) STRANDEDNESS: double
      (D) TOPOLOGY: linear
   (xi) SEQUENCE DESCRIPTION: SEQ ID NO:125:
   CTACTGCTAA GTTTATTTTT ATTGCTCTCA ATATCTGGTT CTTTAGATAA TGAAGGTGTA 60
   AACTCAAAAG ATTACGAGTC AAAAAAACAG AGTATACTAG GTGAATTAAA TCAGCTATTG 120
   GGGCAAACTA CAAATTCACT AAAAGAAGCA AAAAATACAA CAGATAATTT AAATGCATCA 180
   AATGAGGCAA ATAAAGTTGT AGAAGCAGTT ATAAGTGTGG TTAATTTAAT TTCATCTGCT 240
   GCAGATCAGG TGAAAGGTCA ACAACAAATA TGCACGATTT AGCTCAAATG GCAGAAATAG 300
   ATTTAGAAAA AATAAAGGAA TCTAGTGATA AAGTAATAGT TGCGGCTAAT GTTGCGAAAG 360
   AAGCATATAA CCTTACTAAA GCAGTAGAAC AAAATATGCA AAAACTGTAC AAAGAGCAAG 420
   AAGAGCAACT AAAACACTAT CTGATTCTGA TGAAGCAGAA CGAGTTTCTG ATGAAATAAA 480
   ACAAGCTAAA GAGGCTGTAG AAATAGCTTG GAAAGCCACA GTAAAAGTAA AAGATGAGTT 540
   AATTGATGTA GAAAATGCAG TCAAAGAGGC ATTGGATAAA ATAAAGACAG AAACCGCGAA 600
   CAATACAAAA CTTACAGATA TAGAAGAAGT AGCAGAGTTA GTATTACAAA TAGCCAAAAA 660
   TGTAGCGGAA ATAGCGCAAG AAGTTGTGGC CTTGTTAAAT ACTT 704
(2) INFORMATION FOR SEQ ID NO:126:
   (i) SEQUENCE CHARACTERISTICS:
      (A) LENGTH: 704 base pairs
      (B) TYPE: nucleic acid
      (C) STRANDEDNESS: double
      (D) TOPOLOGY: linear
   (xi) SEQUENCE DESCRIPTION: SEQ ID NO:126:
   CTACTGTTAA GTTTATTTTT ATTGCTCTCA ATATCTTGTT CTTTAGATAA TGAAGGTGTA 60
   AGCTCAAAAG ATTACGAGTC AAAAAAACAG AGTATACTAG GTGAATTAAA TCAGCTATTG 120
   GGGCAAACTA CAAATTCACT AAAAGAAGCA AAAAATACAA CAGATAATTT AAATGCATCA 180
   AATGAGGCAA ATAAAGTTGT AGAAGCAGTT ATAAGTGTGG TTAATTTAAT TTCATCTGCT 240
   GCAGATCAGG TGAAAGGTCA ACAACAAATA TGCACGATTT AGCTCAAATG GCAGAAATAG 300
   ATTTAGAAAA AATAAAGGAA TCTAGTGATA AAGTAATAGT TGCGGCTAAT GTTGCGAAAG 360
   AAGCATATAA CCTTACTAAA GCAGTAGAAC AAAATATGCA AAAACTGTAC AAAGAGCAAG 420
   AAGAGCAACT AAAACACTAT CTGATTCTGA TGAAGCAGAA CGAGTTTCTG ATGAAATAAA 480
   ACAAGCTAAA GAGGCTGTAG AAATAGCTTG GAAAGCCACA GTAAAAGTAA AAGATGAGTT 540
   AATTGATGTA GAAAATGCAG TCAAAGAGGC ATTGGATAAA ATAAAGACAG GAACCGCGAA 600
   CAATACAAAA CTTACAGATA TAGAAGAAGT AGCAGAGTTA GTATTACAAA TAGCCAAAAA 660
   TGTAGCGGAA ATAGCGCAAG AAGTTGTGGC CTTGTTAAAT ACTT 704
(2) INFORMATION FOR SEQ ID NO:127:
   (i) SEQUENCE CHARACTERISTICS:
      (A) LENGTH: 1011 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1011
(2) INFORMATION FOR SEQ ID NO:128:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:128:
   Met Ile Ile Asn His Asn Thr Ser Ala Ile Asn Ala Ser Arg Asn Asn
   1               5                   10                  15
   Gly Ile Asn Ala Ala Asn Leu Ser Lys Thr Gln Glu Lys Leu Ser Ser
               20                  25                  30
   Gly Tyr Arg Ile Asn Arg Ala Ser Asp Asp Ala Ala Gly Met Gly Val
           35                  40                  45
   Ser Gly Lys Ile Asn Ala Gln Ile Arg Gly Leu Ser Gln Ala Ser Arg
       50                  55                  60
   Asn Thr Ser Lys Ala Ile Asn Phe Ile Gln Thr Thr Glu Gly Asn Leu
   65                  70                  75                  80
   Asn Glu Val Glu Lys Val Leu Val Arg Met Lys Glu Leu Ala Val Gln
                   85                  90                  95
   Ser Gly Asn Gly Thr Tyr Ser Asp Ala Asp Arg Gly Ser Ile Gln Ile
               100                 105                 110
   Glu Ile Glu Gln Leu Thr Asp Glu Ile Asn Arg Ile Ala Asp Gln Ala
           115                 120                 125
   Gln Tyr Asn Gln Met His Met Leu Ser Asn Lys Ser Ala Ser Gln Asn
       130                 135                 140
   Val Arg Thr Ala Glu Glu Leu Gly Met Gln Pro Ala Lys Ile Asn Thr
   145                 150                 155                 160
   Pro Ala Ser Leu Ser Gly Leu Gln Ala Ser Trp Thr Leu Arg Val His
                   165                 170                 175
   Val Gly Ala Thr Gln Asp Glu Ala Ile Ala Val Asn Ile Tyr Ala Ala
               180                 185                 190
   Asn Val Ala Asn Leu Phe Ser Gly Glu Gly Ala Gln Thr Ala Gln Ala
           195                 200                 205
   Ala Pro Val Gln Glu Gly Val Gln Gln Glu Gly Ala Gln Gln Pro Ala
       210                 215                 220
   Pro Ala Thr Ala Pro Ser Gln Gly Gly Val Asn Ser Pro Val Asn Val
   225                 230                 235                 240
   Thr Thr Thr Val Asp Ala Asn Thr Ser Leu Ala Lys Ile Glu Asn Ala
                   245                 250                 255
   Ile Arg Met Ile Ser Asp Gln Arg Ala Asn Leu Gly Ala Phe Gln Asn
               260                 265                 270
   Arg Leu Glu Ser Ile Lys Asn Ser Thr Glu Tyr Ala Ile Glu Asn Leu
           275                 280                 285
   Lys Ala Ser Tyr Ala Gln Ile Lys Asp Ala Thr Met Thr Asp Glu Val
       290                 295                 300
   Val Ala Ala Thr Thr Asn Met Ile Leu Thr Gln Ser Ala Met Ala Met
   305                 310                 315                 320
   Ile Ala Gln Ala Asn Gln Val Pro Gln Tyr Val Leu Ser Leu Leu Arg
                   325                 330                 335
(2) INFORMATION FOR SEQ ID NO:129:
   (i) SEQUENCE CHARACTERISTICS:
      (A) LENGTH: 1008 base pairs
      (B) TYPE: nucleic acid
      (C) STRANDEDNESS: double
      (D) TOPOLOGY: linear
   (xi) SEQUENCE DESCRIPTION: SEQ ID NO:129:
   ATGATTATCA ATCATAATAC ATCAGCTATT AATGCTTCAA GAAATAATGG CATTAACGCT     60
   GCTAATCTTA GTAAAACTCA AGAAAAGCTT TCTAGTGGGT ACAGAATTAA TCGAGCTTCT    120
   GATGATGCTG CTGGCATGGG AGTTTCTGGT AAGATTAATG CTCAAATAAG AGGTTTGTCA 180
   CAAGCTTCTA GAAATACTTC AAAGGCTATT AATTTTATTC AGACAACAGA AGGGAATTTA 240
   AATGAAGTAG AAAAAGTCTT AGTAAGAATG AAGGAATTGG CAGTTCAATC AGGTAACGGC 300
   ACATATTCAG ATGCAGACAG AGGTTCTATA CAAATTGAAA TAGAGCAACT TACAGACGAA 360
   ATTAATAGAA TTGCTGATCA AGCTCAATAT AACCAAATGC ACATGTTATC AAACAAATCT 420
   GCTTCTCAAA ATGTAAGAAC AGCTGAAGAG CTTGGAATGC AGCCTGCAAA AATTAACACA 480
   CCAGCATCAC TTTCAGGGTC TCAAGCGTCT TGGACTTTAA GAGTTCATGT TGGAGCAAAC 540
   CAAGATGAAG CTATTGCTGT AAATATTTAT GCAGCTAATG TTGCAAATCT TTTCTCTGGT 600
   GAGGGAGCTC AAACTGCTCA GGCTGCACCG GTTCAAGAGG GTGTTCAACA GGAAGGAGCT 660
   CAACAGCCAG CACCTGCTAC AGCACCTTCT CAAGGCGGAG TTAATTCTCC TGTTAATGTT 720
   ACAACTACAG TTGATGCTAA TACATCACTT GCTAAAATTG AAAATGCTAT TAGAATGATA 780
   AGTGATCAAA GGGCAAATTT AGGTGCTTTC CAAAATAGAC TTGAATCTAT AAAGAATAGT 840
   ACTGAGTATG CAATTGAAAA TCTAAAAGCA TCTTATGCTC AAATAAAAGA TGCTACAATG 900
   ACAGATGAGG TTGTAGCAGC AACAACTAAT AGTATTTTAA CACAATCTGC AATGGCAATG 960
   ATTGCGCAGG CTAATCAAGT TCCCCAATAT GTTTTGTCAT TGCTTAGA 1008
(2) INFORMATION FOR SEQ ID NO:130:
   (i) SEQUENCE CHARACTERISTICS:
      (A) LENGTH: 1008 base pairs
      (B) TYPE: nucleic acid
      (C) STRANDEDNESS: double
      (D) TOPOLOGY: linear
   (xi) SEQUENCE DESCRIPTION: SEQ ID NO:130:
   ATGATTATCA ATCATAATAC ATCAGCTATT AATGCTTCAA GAAATAATGC CATTAATGCT 60
   GCTAATCTTA GTAAAACCCA AGAGAAGCTT TCTAGTGGTT ACAGAATTAA TCGAGCTTCT 120
   GATGATGCTG CTGGTATGGG GGTTTCTGGC AAGATTAATG CTCAAATAAG AGGCTTATCA 180
   CAAGCTTCTA GAAACACTTC AAAAGCTATC AATTTTATTC AGACAACAGA AGGAAATTTA 240
   AATGAAGTAG AAAAAGTTTT AGTAAGAATG AAAGAATTAG CAGTTCAATC AGGTAACGGA 300
   ACGTATTCAG ACTCAGACAG AGGTTCTATA CAGATTGAAA TAGAGCAACT TACAGACGAA 360
   ATTAATAGAA TTGCTGATCA GGCTCAATAT AACCAAATGC ACATGTTGTC AAACAAATCT 420
   GCTTCCCAAA ATGTAAAAAC AGCTGAAGAG CTTGGAATGC AGCCTGCAAA AATTAACACA 480
   CCAGCATCAC TTTCAGGATC TCAAGCTTCT TGGACTTTAA GAGTTCATGT GGGAGCAAAT 540
   CAAGATGAAG CAATTGCTGT AAATATTTAT TCAGCTAATG TTGCAAATCT TTTTGCTGGT 600
   GAGGGAGCTC AAGCTGCTCA GGCTGCACCT GTTCAAGAGG GTGCTCAAGA AGAAGGAGCT 660
   CAGCAACCAA CACCTGCTAC AGCACCTACT CAAGGTGGAG TTAATTCTCC TGTTAATGTT 720
   ACAACCACAG TTGATGCTAA TACATCACTT GCTAAAATAG AAAATGCTAT TAGAATGATA 780
   AGTGATCAAA GAGCAAATTT AGGTGCTTTC CAAAATAGAC TTGAATCTAT AAAGAATAGC 840
   ACTGAGTATG CTATTGAAAA TCTAAAAGCA TCTTATGCTC AAATAAAAGA TGCTACAATG 900
   ACAGATGAGG TTGTAGCAGC TACAACTAAT AGTATTTTAA CTCAATCTGC AATGGCAATG 960
   ATTGCACAGG CTAATCAAGT TCCTCAATAT GTTTTGTCAT TGCTTAGA 1008
(2) INFORMATION FOR SEQ ID NO:131:
   (i) SEQUENCE CHARACTERISTICS:
      (A) LENGTH: 1008 base pairs
      (B) TYPE: nucleic acid
      (C) STRANDEDNESS: double
      (D) TOPOLOGY: linear
   (xi) SEQUENCE DESCRIPTION: SEQ ID NO:131:
   ATGATTATCA ATCATAATAC ATCAGCTATT AATGCTTCAA GAAATAATGC CATTAATGCT 60
   GCTAATCTTA GTAAAACCCA AGAGAAGCCT TCTAGTGGTT ACAGAATTAA TCGAGCTTCT 120
   GATGATGCTG CTGGTATGGG GGTTTCTGGC AAGATTAATG CTCAAATAAG AGGCTTATCA 180
   CAAGCTTCTA GAAACACTTC AAAAGCTATC AATTTTATTC AGACAACAGA AGGAAATTTA 240
   AATGAAGTAG AAAAAGTTTT AGTAAGAATG AAAGAATTAG CAGTTCAATC AGGTAACGGA 300
   ACGTATTCAG ACTCAGACAG AGGTTCTATA CAGATTGAAA TAGAGCAACT TACAGACGAA 360
   ATTAATAGAA TTGCTGATCA GGCTCAATAT AACCAAATGC ACATGTTGTC AAACAAATCT 420
   GCTTCCCAAA ATGTAAAAAC AGCTGAAGAG CTTGGAATGC AGCCTGCAAA AATTAACACA 480
   CCAGCATCAC TTTCAGGATC TCAAGCTTCT TGGACTTTAA GAGTTCATGT GGGAGCAAAT 540
   CAAGATGAAG CAATTGCTGT AAATATTTAT TCAGCTAATG TTGCAAATCT TTTTGCTGGT 600
   GAGGGAGCTC AAGCTGCTCA GGCTGCACCT GTTCAAGAGG GTGCTCAAGA AGAAGGAGCT 660
   CAGCAACCAA CACCTGCTAC AGCACCTACT CAAGGTGGAG TTAATTCTCC TGTTAATGTT 720
   ACAACCACAG TTGATGCTAA TACATCACTT GCTAAAATAG AAAATGCTAT TAGAATGATA 780
   AGTGATCAAA GAGCAAATTT AGGTGCTTTC CAAAATAGAC TTGAATCTAT AAAGAATAGC 840
   ACTGAGTATG CTATTGAAAA TCTAAAAGCA TCTTATGCTC AAATAAAAGA TGCTACAATG 900
   ACAGATGAGG TTGTAGCAGC TACAACTAAA AGTATTTTAA CTCAATCTGC AATGGCAATG 960
   ATTGCACAGG CTAATCAAGT TCCTCAATAT GTTTTGTCAT TGCTTAGA 1008
(2) INFORMATION FOR SEQ ID NO:132:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (xi) SEQUENCE DESCRIPTION: SEQ ID NO:132:
   ATGAAAAAAT ATTTATTGGG AATAGGTCTA ATATTAGCCT TAATAGCATG TAAGCAAAAT    60
   GTTAGCAGCC TTGACGAGAA AAACAGCGTT TCAGTAGATT TGCCTGGTGA AATGAAAGTT   120
   CTTGTAAGCA AAGAAAAAAA CAAAGACGGC AAGTACGATC TAATTGCAAC AGTAGACAAG   180
   CTTGAGCTTA AAGGAACTTC TGATAAAAAC AATGGATCTG GAGTACTTGA AGGCGTAAAA   240
   GCTGACAAAA GTAAAGTAAA ATTAACAATT TCTGACGATC TAGGTCAAAC CACACTTGAA   300
   GTTTTCAAAG AAGATGGCAA AACACTAGTA TCAAAAAAAG TAACTTCCAA AGACAAGTCA   360
   TCAACAGAAG AAAAATTCAA TGAAAAAGGT GAAGTATCTG AAAAAATAAT AACAAGAGCA   420
   GACGGAACCA GACTTGAATA CACAGGAATT AAAAGCGATG GATCTGGAAA AGCTAAAGAG   480
   GTTTTAAAAG GCTATGTTCT TGAAGGAACT CTAACTGCTG AAAAAACAAC ATTGGTGGTT   540
   AAAGAAGGAA CTGTTACTTT AAGCAAAAAT ATTTCAAAAT CTGGGGAAGT TTCAGTTGAA   600
   CTTAATGACA CTGACAGTAG TGCTGCTACT AAAAAAACTG CAGCTTGGAA TTCAGGCACT   660
   TCAACTTTAA CAATTACTGT AAACAGTAAA AAAACTAAAG ACCTTGTGTT TACAAAAGAA   720
   AACACAATTA CAGTACAACA ATACGACTCA AATGGCACCA AATTAGAGGG GTCAGCAGTT   780
   GAAATTACAA AACTTGATGA AATTAAAAAC GCTTTAAAAT AA                      822
(2) INFORMATION FOR SEQ ID NO:133:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (xi) SEQUENCE DESCRIPTION: SEQ ID NO:133:
   ATGAAAAAAT ATTTATTGGG AATAGGTCTA ATATTAGCCT TAATAGCATG TAAGCAAAAT    60
   GTTAGCAGCC TTGACGAGAA AAACAGCGTT TCAGTAGATT TGCCTGGTGA AATGAACGTT   120
   CTTGTAAGCA AAGAAAAAAA CAAAGACGGC AAGTACGATC TAATTGCAAC AGTAGACAAG   180
   CTTGAGCTTA AAGGAACTTC TGATAAAAAC AATGGATCTG GAGTACTTGA AGGCGTAAAA   240
   GCTGACAAAA GTAAAGTAAA ATTAACAATT TCTGACGATC TAGGTCAAAC CACACTTGAA   300
   GTTTTCAAAG AAGATGGCAA AACACTAGTA TCAAAAAAAG TAACTTCCAA AGACAAGTCA 360
   TCAACAGAAG AAAAATTCAA TGAAAAAGGT GAAGTATCTG AAAAAATAAT AACAAGAGCA 420
   GACGGAACCA GACTTGAATA CACAGAAATT AAAAGCGATG GATCTGGAAA AGCTAAAGAG 480
   GTTTTAAAAG GCTATGTTCT TGAAGGAACT CTAACTGCTG AAAAAACAAC ATTGGTGGTT 540
   AAAGAAGGAA CTGTTACTTT AAGCAAAAAT ATTTCAAAAT CTGGGGAAGT TTCAGTTGAA 600
   CTTAATGACA CTGACAGTAG TGCTGCTACT AAAAAAACTG CAGCTTGGAA TTCAGGCACT 660
   TCAACTTTAA CAATTACTGT AAACAGTAAA AAAACTAAAG ACCTTGTGTT TACAAAAGAA 720
   AACACAATTA CAGTACAACA ATACGACTCA AATGGCACCA AATTAGAGGG GTCAGCAGTT 780
   GAAATTACAA AACTTGATGA AATTAAAAAC GCTTTAAAAT AA 822
(2) INFORMATION FOR SEQ ID NO:134:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (xi) SEQUENCE DESCRIPTION: SEQ ID NO:134:
   ATGAAAAAAT ATTTATTGGG AATAGGTCTA ATATTAGCCT TAATAGCATG TAAGCAAAAT 60
   GTTAGCAGCC TTGACGAGAA AAACAGCGTT TCAGTAGATT TGCCTGGTGA AATGAACGTT 120
   CTTGTAAGCA AAGAAAAAAA CAAAGACGGC AAGTACGATC TAATTGCAAC AGTAGACAAG 180
   CTTGAGCTTA AAGGAACTTC TGATAAAAAC AATGGATCTG GAGTACTTGA AGGCGTAAAA 240
   GCTGACAAAA GTAAAGTAAA ATTAACAATT TCTGACGATC TAGGTCAAAC CACACTTGAA 300
   GTTTTCAAAG AAGATGGCAA AACACTAGTA TCAAAAAAAG TAACTTCCAA AGACAAGTCA 360
   TCAACAGAAG AAAAATTCAA TGAAAAAGGT GAAGTATCTG AAAAAATAAT AACAAGAGCA 420
   GACGGAACCA GACTTGAATA CACAGAAATT AAAAGCGATG GATCTGGAAA AGCTAAAGAG 480
   GTTTTAAAAA GCTATGTTCT TGAAGGAACT TTAACTGCTG AAAAAACAAC ATTGGTGGTT 540
   AAAGAAGGAA CTGTTACTTT AAGCAAAAAT ATTTCAAAAT CTGGGGAAGT TTCAGTTGAA 600
   CTTAATGACA CTGACAGTAG TGCTGCTACT AAAAAAACTG CAGCTTGGAA TTCAGGCACT 660
   TCAACTTTAA CAATTACTGT AAACAGTAAA AAAACTAAAG ACCTTGTGTT TACAAAAGAA 720
   AACACAATTA CAGTACAACA ATACGACTCA AATGGCACCA AATTAGAGGG GTCAGCAGTT 780
   GAAATTACAA AACTTGATGA AATTAAAAAC GCTTTAAAAT AA 822
(2) INFORMATION FOR SEQ ID NO:135:
   (i) SEQUENCE CHARACTERISTICS:
      (A) LENGTH: 821 base pairs
      (B) TYPE: nucleic acid
      (C) STRANDEDNESS: double
      (D) TOPOLOGY: linear
   (xi) SEQUENCE DESCRIPTION: SEQ ID NO:135:
   ATGAAAAAAT ATTTATTGGG AATAGGTCTA ATATTAGCCT TAATAGCATG TAAGCAAAAT    60
   GTTAGCAGCC TTGATGAGAA AAACAGCGTT TCAGTAGATT TACCTGGTGA AATGAAAGTT   120
   CTTGTAAGCA AAGAAAAAGA CAAAGATGGT AAATACAGTC TAATGGCAAC AGTAGACAAG   180
   CTAGAGCTTA AAGGAACTTC TGATAAAAGC AACGGTTCTG GAACACTTGA AGGTGAAAAA   240
   TCTGACAAAA GTAAAGCAAA ATTAACAATT TCTGAAGATC TAAGTAAAAC CACATTTGAA   300
   ATTTTCAAAG AAGATGGCAA AACATTAGTA TCAAAAAAAG TAAATTCTAA AGATAAGTCA   360
   TCAATAGAAG AAAAATTCAA CGCAAAAGGT GAATTATCTG AAAAAACAAT ACTAAGAGCA   420
   AACGGAACCA GGCTTGAATA CACAGAAATA AAAAGCGATG GAACCGGAAA AGCTAAAGAA   480
   GCTTTAAAAG ACTTTGCTCT TGAAGGAACT CTAGCTGCCG ACAAAACAAC ATTGAAAGTT   540
   ACAGAAGGCA CTGTTGTTTT AAGCAAACAC ATTCCAAACT CTGGAGAAAT AACAGTTGAG   600
   CTTAATGACT CTAACTCTAC TCAGGCTACT AAAAAAACTG GAAAATGGGA TTCAAATACT   660
   TCCACTTTAA CAATTAGTGT GAATAGCAAA AAAACTAAAA ACATTGTATT TACAAAAGAA   720
   GACACAATAA CAGTACAAAA ATACGACTCA GCAGGCACCA ATCTAGAAGG CAACGCAGTC   780
   GAAATTAAAA CACTTGATGA ACTTAAAAAC GCTTTAAAAT A                       821
(2) INFORMATION FOR SEQ ID NO:136:
   (i) SEQUENCE CHARACTERISTICS:
      (A) LENGTH: 821 base pairs
      (B) TYPE: nucleic acid
      (C) STRANDEDNESS: double
      (D) TOPOLOGY: linear
   (xi) SEQUENCE DESCRIPTION: SEQ ID NO:136:
   ATGAAAAAAT ATTTATTGGG AATAGGTCTA ATATTAGCCT TAATAGCATG TAAGCAAAAT    60
   GTTAGCAGCC TTGATGAAAA AAATAGCGTT TCAGTAGATT TACCTGGTGG AATGAAAGTT   120
   CTTGTAAGTA AAGAAAAAGA CAAAGATGGT AAATACAGTC TAATGGCAAC AGTAGAAAAG   180
   CTTGAGCTTA AAGGAACTTC TGATAAAAAC AACGGTTCTG GAACACTTGA AGGTGAAAAA   240
   ACTGACAAAA GTAAAGTAAA ATTAACAATT GCTGAGGATC TAAGTAAAAC CACATTTGAA   300
   ATCTTCAAAG AAGATGGCAA AACATTAGTA TCGAAAAAAG TAACCCTTAA AGACAAGTCA     360
   TCAACAGAAG AAAAATTCAA CGAAAAGGGT GAAATATCTG AAAAAACAAT AGTAAGAGCA     420
   AATGGAACCA GACTTGAATA CACAGACATA AAAAGCGATA AAACCGGAAA AGCTAAAGAA     480
   GTTTTAAAAG ACTTTACTCT TGAAGGAACT CTAGCTGCTG ACGGCAAAAC AACATTGAAA     540
   GTTACAGAGG GCACTGTTAC TTTAAGCAAG AACATTTCAA AATCCGGAGA AATAACAGTT     600
   GCACTTGATG ACACTGACTC TAGCGGCAAT AAAAAATCCG GAACATGGGA TTCAGGTACT     660
   TCTACTTTAA CAATTAGTAA AAACAGACAA AAAACTAAAC AACTTGTATT CACAAAAGAA     720
   GACACAATAA CAGTACAAAA CTACGACTCA GCAGGCACCA ATCTAGAAGG CAAAGCAGTC     780
   GAAATTACAA CACTTAAAGA ACTTAAAAAC GCTTTAAAAT A                         821
(2) INFORMATION FOR SEQ ID NO:137:
   (i) SEQUENCE CHARACTERISTICS:
      (A) LENGTH: 825 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..825
   (xi) SEQUENCE DESCRIPTION: SEQ ID NO:137:
(2) INFORMATION FOR SEQ ID NO:138:
   (i) SEQUENCE CHARACTERISTICS:
      (A) LENGTH: 274 amino acids
      (B) TYPE: amino acid
      (D) TOPOLOGY: linear
   (ii) MOLECULE TYPE: protein
   (xi) SEQUENCE DESCRIPTION: SEQ ID NO:138:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Val Ser Val
               20                  25                  30
   Asp Leu Pro Gly Gly Met Thr Val Leu Val Ser Lys Glu Lys Asp Lys
           35                  40                  45
   Asp Gly Lys Tyr Ser Leu Glu Ala Thr Val Asp Lys Leu Glu Leu Lys
       50                  55                  60
   Gly Thr Ser Asp Lys Asn Asn Gly Ser Gly Thr Leu Glu Gly Glu Lys
   65                  70                  75                  80
   Thr Asp Lys Ser Lys Val Lys Leu Thr Ile Ala Asp Asp Leu Ser Gln
                   85                  90                  95
   Thr Lys Phe Glu Ile Phe Lys Glu Asp Ala Lys Thr Leu Val Ser Lys
               100                 105                 110
   Lys Val Thr Leu Lys Asp Lys Ser Ser Thr Glu Glu Lys Phe Asn Glu
           115                 120                 125
   Lys Gly Glu Thr Ser Glu Lys Thr Ile Val Arg Ala Asn Gly Thr Arg
       130                 135                 140
   Leu Glu Tyr Thr Asp Ile Lys Ser Asp Gly Ser Gly Lys Ala Lys Glu
   145                 150                 155                 160
   Val Leu Lys Asp Phe Thr Leu Glu Gly Thr Leu Ala Ala Asp Gly Lys
                   165                 170                 175
   Thr Thr Leu Lys Val Thr Glu Gly Thr Val Val Leu Ser Lys Asn Ile
               180                 185                 190
   Leu Lys Ser Gly Glu Ile Thr Val Ala Leu Asp Asp Ser Asp Thr Thr
           195                 200                 205
   Gln Ala Thr Lys Lys Thr Gly Lys Trp Asp Ser Asn Thr Ser Thr Leu
       210                 215                 220
   Thr Ile Ser Val Asn Ser Lys Lys Thr Lys Asn Ile Val Phe Thr Lys
   225                 230                 235                 240
   Glu Asp Thr Ile Thr Val Gln Lys Tyr Asp Ser Ala Gly Thr Asn Leu
                   245                 250                 255
   Glu Gly Asn Ala Val Glu Ile Lys Thr Leu Asp Glu Leu Lys Asn Ala
               260                 265                 270
   Leu Lys
(2) INFORMATION FOR SEQ ID NO:139:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:139:
(2) INFORMATION FOR SEQ ID NO:140:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:140:
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ala
               180                 185                 190
   Lys Ser Gly Glu Val Thr Val Ala Leu Asn Asp Thr Asn Thr Thr Gln
           195                 200                 205
   Ala Thr Lys Lys Thr Gly Ala Trp Asp Ser Lys Thr Ser Thr Leu Thr
       210                 215                 220
   Ile Ser Val Asn Ser Lys Lys Thr Thr Gln Leu Val Phe Thr Lys Gln
   225                 230                 235                 240
   Asp Thr Ile Thr Val Gln Lys Tyr Asp Ser Ala Gly Thr Asn Leu Glu
                   245                 250                 255
   Gly Thr Ala Val Glu Ile Lys Thr Leu Asp Glu Leu Lys Asn Ala Leu
               260                 265                 270
   Lys
(2) INFORMATION FOR SEQ ID NO:141:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:141:
(2) INFORMATION FOR SEQ ID NO:142:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:142:
   Met Lys Lys Tyr Leu Leu Gly Ile Gly Leu Ile Leu Ala Leu Ile Ala
    1               5                  10                  15
   Cys Lys Gln Asn Val Ser Ser Leu Asp Glu Lys Asn Ser Ala Ser Val
               20                  25                  30
   Asp Leu Pro Gly Glu Met Lys Val Leu Val Ser Lys Glu Lys Asp Lys
           35                  40                  45
   Asp Gly Lys Tyr Ser Leu Lys Ala Thr Val Asp Lys Ile Glu Leu Lys
       50                  55                  60
   Gly Thr Ser Asp Lys Asp Asn Gly Ser Gly Val Leu Glu Gly Thr Lys
   65                  70                  75                  80
   Asp Asp Lys Ser Lys Ala Lys Leu Thr Ile Ala Asp Asp Leu Ser Lys
                   85                  90                  95
   Thr Thr Phe Glu Leu Leu Lys Glu Asp Gly Lys Thr Leu Val Ser Arg
               100                 105                 110
   Lys Val Ser Ser Arg Asp Lys Thr Ser Thr Asp Glu Met Phe Asn Glu
           115                 120                 125
   Lys Gly Glu Leu Ser Ala Lys Thr Met Thr Arg Glu Asn Gly Thr Lys
       130                 135                 140
   Leu Glu Tyr Thr Glu Met Lys Ser Asp Gly Thr Gly Lys Ala Lys Glu
   145                 150                 155                 160
   Val Leu Lys Lys Phe Thr Leu Glu Gly Lys Val Ala Asn Asp Lys Val
                   165                 170                 175
   Thr Leu Glu Val Lys Glu Gly Thr Val Thr Leu Ser Lys Glu Ile Ser
               180                 185                 190
   Lys Ser Gly Glu Val Ser Val Glu Leu Asn Asp Thr Asp Ser Ser Ala
           195                 200                 205
   Ala Thr Lys Lys Thr Ala Ala Trp Asn Ser Lys Thr Ser Thr Leu Thr
       210                 215                 220
   Ile Ser Val Asn Ser Gln Lys Thr Lys Asn Leu Val Phe Thr Lys Glu
   225                 230                 235                 240
   Asp Thr Ile Thr Val Gln Lys Tyr Asp Ser Ala Gly Thr Asn Leu Glu
                   245                 250                 255
   Gly Lys Ala Val Glu Ile Thr Thr Leu Lys Glu Leu Lys Asn Ala Leu
               260                 265                 270
   Lys
(2) INFORMATION FOR SEQ ID NO:143:
   (i) SEQUENCE CHARACTERISTICS:
      (A) LENGTH: 822 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..822
   (xi) SEQUENCE DESCRIPTION: SEQ ID NO:143:
(2) INFORMATION FOR SEQ ID NO:144:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:144:
   Met Lys Lys Tyr Leu Leu Gly Ile Gly Leu Ile Leu Ala Leu Ile Ala
   1               5                   10                  15
   Cys Lys Gln Asn Val Ser Ser Leu Asp Glu Lys Asn Ser Val Ser Val
               20                  25                  30
   Asp Leu Pro Gly Glu Met Lys Val Leu Val Ser Lys Glu Lys Asn Lys
           35                  40                  45
   Asp Gly Lys Tyr Asp Leu Ile Ala Thr Val Asp Lys Leu Glu Leu Lys
       50                  55                  60
   Gly Thr Ser Asp Lys Asn Asn Gly Ser Gly Val Leu Glu Gly Val Lys
   65 70 75 80
   Ala Asp Lys Ser Lys Val Lys Leu Thr Ile Ser Asp Asp Leu Gly Gln
   85 90 95
   Thr Thr Leu Glu Val Phe Lys Glu Asp Gly Lys Thr Leu Val Ser Lys
   100 105 110
   Lys Val Thr Ser Lys Asp Lys Ser Ser Thr Glu Glu Lys Phe Asn Glu
   115 120 125
   Lys Gly Glu Val Ser Glu Lys Ile Ile Thr Arg Ala Asn Gly Thr Lys
   130 135 140
   Leu Glu Tyr Thr Glu Met Lys Ser Asp Gly Thr Gly Lys Ala Lys Glu
   145 150 155 160
   Val Leu Lys Lys Phe Thr Leu Glu Gly Lys Val Ala Asn Asp Lys Val
   165 170 175
   Thr Leu Glu Val Lys Glu Gly Thr Val Thr Leu Ser Lys Glu Ile Ser
   180 185 190
   Lys Ser Gly Glu Val Ser Val Glu Leu Asn Asp Thr Asp Ser Ser Ala
   195 200 205
   Ala Thr Lys Lys Thr Ala Ala Trp Asn Ser Lys Thr Ser Thr Leu Thr
   210 215 220
   Ile Ser Val Asn Ser Gln Lys Thr Lys Asn Leu Val Phe Thr Lys Glu
   225 230 235 240
   Asp Thr Ile Thr Val Gln Lys Tyr Asp Ser Ala Gly Thr Asn Leu Glu
   245 250 255
   Gly Lys Ala Val Glu Ile Thr Thr Leu Lys Glu Leu Lys Asn Ala Leu
   260 265 270
   Lys